# EUROPÄISCHE PATENTSCHRIFT

(11) **EP 1 951 842 B1**
(45) Veröffentlichungstag und Bekanntmachung des Hinweises auf die Patenterteilung: **27.07.2016**
(21) Anmeldenummer: 06829934.6
(22) Anmeldetag: 06.11.2006
(51) Int. Cl.: C09K 11/06, H01L 31/0256, H01L 31/04, H01L 51/30, H01L 51/42, C07D 491/06, C07D 491/16, C07D 311/92, C07D 405/10, C07D 471/06, C07D 417/14, C07D 417/10, C07D 409/14, C07D 413/10, C07D 405/12

(54) **VERWENDUNG VON RYLENDERIVATEN ALS PHOTOSENSIBILISATOREN IN SOLARZELLEN**
USE OF RYLENE DERIVATIVES AS PHOTOSENSITIZERS IN SOLAR CELLS
UTILISATION DE DERIVES DE RYLENE EN TANT QUE PHOTOSENSIBILISATEURS DANS DES CELLULES SOLAIRES

(30) Priorität: 10.11.2005 DE 102005053995
(43) Veröffentlichungstag der Anmeldung: 06.08.2008
(73) Patentinhaber: BASF SE, 67056 Ludwigshafen am Rhein (DE); Max-Planck-Gesellschaft zur Förderung der Wissenschaften e.V., 80539 München (DE)
(72) Erfinder: PSCHIRER, Neil Gregory, 55116 Mainz (DE); EICKEMEYER, Felix, 68159 Mannheim (DE); SCHÖNEBOOM, Jan, 68199 Mannheim (DE); QU, Jianqiang, 67061 Ludwigshafen (DE); KÖNEMANN, Martin, 68163 Mannheim (DE); MÜLLEN, Klaus, 50939 Köln (DE); LI, Chen, 55128 Mainz (DE); HERRMANN, Andreas, 65199 Wiesbaden (DE); ERK, Peter, 67227 Frankenthal (DE); NORDMANN, Gero, 69120 Heidelberg (DE); KUHN, Alfred, 67125 Dannstadt-schauernheim (DE); HAGFELDT, Anders, S-74030 Björklingen (SE); EDVINSSON, Tomas, S-75337 Uppsala (SE)
(74) Vertreter: BASF IP Association
(86) Internationale Anmeldenummer: PCT/EP2006/068102
(87) Internationale Veröffentlichungsnummer: WO 2007/054470

(56) Entgegenhaltungen:
- WO-A-02/066438
- WO-A-2006/117383
- DE-A1- 19 757 312
- DE-A1- 19 809 840
- DE-A1- 19 848 555
- JP-A- 2000 243 463
- JP-A- 2001 093 589
- US-A- 4 599 408
- FERRERE S ET AL: "NEW PERYLENES FOR DYE SENSITIZATION OF TIO2" NEW JOURNAL OF CHEMISTRY, CNRS-GAUTHIER-VILLARS, MONTROUGE, FR, Bd. 26, Nr. 9, 2002, Seiten 1155-1160, XP008024830 ISSN: 1144-0546 in der Anmeldung erwähnt

## Beschreibung

Die vorliegende Erfindung betrifft die Verwendung von Rylenderivaten der allgemeinen Formel I 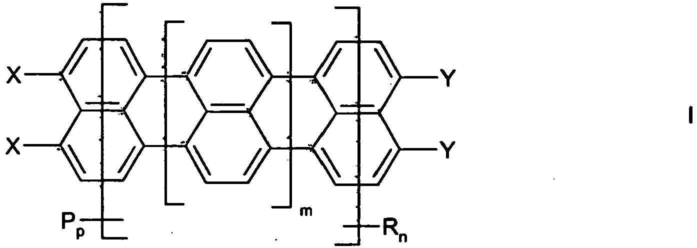 in der die Variablen folgende Bedeutung haben:
- X: miteinander unter Ausbildung eines Sechsrings verbunden zu einem Rest der Formel (x1), (x2) oder (x3) 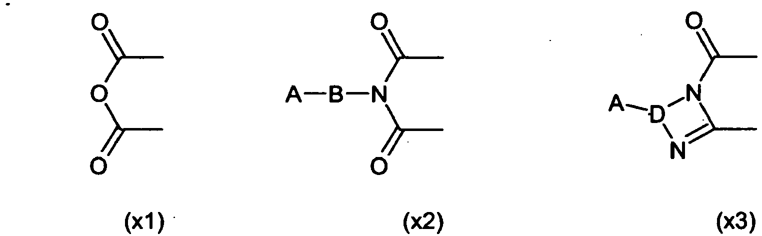
beide ein Rest -COOM;
- Y: einer der beiden Reste ein Rest der Formel (y1)

-L-NR¹R² (y1)

oder ein Rest der Formel (y2)

-L-Z-R³ (y2)

und der andere Rest jeweils Wasserstoff; miteinander unter Ausbildung eines Sechsrings verbunden zu einem Rest der Formel (y3) oder (y4) 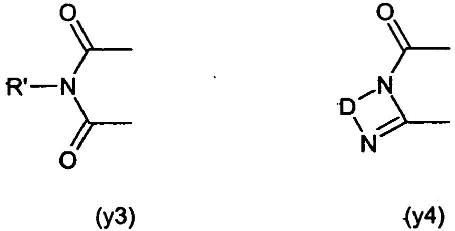
oder beide Wasserstoff;
- R: gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, anneliert sein können, wobei das gesamte Ring-system ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ ,-POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR⁴-, -N=CR4-, -CR⁴=CR⁴-, -CO-, -SO- und/oder -SO₂- unterbrochen sein kann, anneliert sein können, wobei das gesamte Ringsystem einoder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen,-Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem einoder mehrfach substituiert sein kann durch: C₁-C₁₈-Alkyl, -C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, C₁-C₆-Alkylthio, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷ substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR³-, -CO-, -SO- oder -SO₂- bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
- P: ein Aminorest -NR¹R²;
- B: C₁-C₆-Alkylen, Phenylen oder Kombinationen dieser Brückenglieder, wobei die Phenylenreste ein- oder mehrfach durch C₁-C₁₂-Alkyl, Nitro, Cyano und/oder Halogen substituiert sein können;
- A: -COOM, -SO₃M oder -PO₃M;
- D: Phenylen, Naphthylen oder Pyridylen, das jeweils ein- oder mehrfach durch C₁-C₁₂-Alkyl, C₁-C₁₂-Alkoxy, Hydroxy, Nitro und/oder Halogen substituiert sein kann;
- M: Wasserstoff, einweriges oder zweiwertiges Metallkation, insbesondere Erdalkali- oder Alkalimetallkation, Ammoniumsalze von cyclischen Aminen Guanidiniumsalze oder [NR⁵₄]⁺;;
- L: eine chemische Bindung oder ein direkt oder über Ethenylen oder Ethinylen an das Rylengerüst gebundener Arylen- oder Hetarylenrest der Formeln

-Ar- -Ar-E-Ar-

in denen die (Het)Arylenreste Ar gleich oder verschieden sein können, Heteroatome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl, C₁-C₁₂-Alkoxy, C₁-C₁₂-Alkylthio und/oder -NR⁵R⁶ substituiert sein kann;
- E: eine chemische Bindung oder eine Gruppierung -O-, -S-, -NR⁴-, -C≡C-, -CR⁴=CR⁴- oder C₁-C₆-Alkylen;
- R¹, R²: unabhängig voneinander einer der als Substituenten für die Reste R genannten Alkylreste (i), Cycloalkylreste (ii) oder (Het)Arylreste (iii); miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anne-liert sein können, deren Kohlenstoffkette ebenfalls durch diese Gruppierungen und/oder -N= unterbrochen sein kann, wobei das gesamte Ringsystem einoder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
- Z: -O- oder -S-;
- R³: einer der als Substituenten für die Reste R genannten Alkylreste (i) oder (Het)Arylreste (iii);
- R': Wasserstoff; C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach durch die als Substituenten für die Reste R genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR⁴-; -N=CR⁴-, -CR⁴=CR⁴-, -CO-, -SO- und/oder -SO₂- unterbrochen sein kann, anneliert sein können, wobei das gesamte Ringsystem ein- oder mehrfach durch die als Substituenten für die Reste R genannten Reste (i), (ii), (iii), (iv) und/oder (v) substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch die als Substituenten für die Reste R genannten Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
- R⁴: Wasserstoff oder C₁-C₁₈-Alkyl, wobei die Reste R⁴ gleich oder verschieden sein können, wenn sie mehrfach auftreten;
R⁵, R⁶ unabhängig voneinander: Wasserstoff; C₁-C₁₈-Alkyl, dessen Kohlenstoffkette durch eine oder mehrere Gruppierungen -O-, -S-, -CO-, -SO- und/oder -SO₂- unterbrochen sein kann und das ein-oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁵ gleich oder verschieden sein können, wenn sie mehrfach auftreten;
R⁷ C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₂-Alkoxy, C₁-C₆-Alkylthlo, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁷ gleich oder verschieden sein können, wenn sie mehrfach auftreten;
R⁸ C₁-C₁₈-Alkyl;
R⁹, R¹⁰ C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴², Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, -S-,-NR⁴-, -N=CR⁴- und/oder -CR⁴=CR⁴- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; Aryl oder Hetaryl, an das weitere gesättigte oder ungesättigte 5- bis 7-gliedrige Ringe, deren Kohlenstoffgerüst durch eine oder mehrere Gruppierungen O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem gesättigten oder ungesättigen, 5-bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
m 0, 1 oder 2;
n 0, 2 oder 4 für m = 0 oder 1; 0, 4 oder 6 für m = 2;
p 0, 2 oder 4 für m = 0, wobei n + p = 2 oder 4 ist oder auch 0 sein kann, wenn die beiden Reste X miteinander unter Ausbildung eines Sechsrings verbunden sind zu einem Rest der Formel (x1) oder (x2) oder beide einen Rest -COOM bedeuten und einer der beiden Reste Y einen Rest der Formel (y1) oder (y2) und der andere Rest Wasserstoff bedeutet, wobei mindestens einer der beiden Reste R¹ oder R² im Rest (y1) einen der als Substituenten für die Reste R genannten (Het)-Arylreste (iii) bedeutet, wenn L für eine chemische Bindung steht; 0, 2 oder 4 für m = 1, wobei n + p = 0, 2 oder 4 ist; 0, 4 oder 6 für m = 2, wobei n + p = 0, 4 oder 6 ist,
oder von Mischungen der Rylenderivate als Photosensibilisatoren in Solarzellen.

Außerdem betrifft die Erfindung neue farbstoffsensibilisierte Solarzellen, die die Rylenderivate I enthalten, sowie neue Rylenderivate la und Ib.

Die Direktumwandlung von Solarenergie in elektrische Energie in Solarzellen beruht auf dem inneren Photoeffekt eines Halbleitermaterials, d.h. der Erzeugung von Elektron-Loch-Paaren durch Absorption von Photonen und der Trennung der negativen und positiven Ladungsträger an einem p-n-Übergang oder einem Schottky-Kontakt. Die so erzeugte Photospannung kann in einem äußeren Stromkreis einen Photostrom bewirken, durch den die Solarzelle ihre Leistung abgibt.

Von dem Halbleiter können dabei nur solche Photonen absorbiert werden, die eine Energie aufweisen, die größer als seine Bandlücke ist. Die Größe der Halbleiterbandlücke bestimmt also den Anteil des Sonnenlichts, der in elektrische Energie umgewandelt werden kann.

Dünne Schichten oder Filme von Metalloxiden stellen bekanntermaßen kostengünstige feste Halbleitermaterialien (n-Halbleiter) dar, jedoch liegt ihre Absorption aufgrund großer Bandlücken üblicherweise nicht im sichtbaren Bereich des elektromagnetischen Spektrums. Für die Anwendung in Solarzellen müssen die Metalloxide daher mit einem Photosensibilisator kombiniert werden, der im Wellenlängenbereich des Sonnenlichts, also bei 300 bis 2000 nm, absorbiert und im elektronischangeregten Zustand Elektronen in das Leitungsband des Halbleiters injiziert. Mithilfe eines zusätzlich in der Zelle eingesetzten Redoxsystems, das an der Gegenelektrode reduziert wird, werden Elektronen zum Sensibilisator zurückgeführt und dieser so regeneriert.

Von besonderem Interesse für die Anwendung in Solarzellen sind die Halbleiter Zinkoxid, Zinndioxid und insbesondere Titandioxid, die in Form nanokristalliner poröser Schichten zum Einsatz kommen. Diese Schichten weisen eine große Oberfläche auf, die mit dem Sensibilisator beschichtet wird, so daß eine hohe Absorption des Sonnenlichts erreicht wird.

Farbstoffsensibilisierte Solarzellen, die auf Titandioxid als Halbleitermaterial basieren, sind z.B. in US-A-4 927 721, Nature 353, S. 737-740 (1991) und US-A-5 350 644 sowie Nature 395, S. 583-585 (1998) und EP-A-1 176 646 beschrieben. Diese Solarzellen enthalten monomolekulare Filme aus Übergangsmetallkomplexen, insbesondere Rutheniumkomplexen, die über Säuregruppen an die Titandioxidschicht gebunden sind, als Sensibilisatoren und in gelöster Form vorliegende Iod/Iodid-Redoxsysteme bzw. amorphe organische p-Leiter auf Basis von Spirobifluorenen.

Als Sensibilisatoren wurden nicht zuletzt aus Kostengründen auch wiederholt metallfreie organische Farbstoffe vorgeschlagen.

So beschreibt US-A-6 359 211 für diesen Zweck Cyanin-, Oxazin-, Thiazin- und Acridinfarbstoffe, die über einen Alkylenrest gebundene Carboxylgruppen zur Fixierung an den Titandioxidhalbleiter aufweisen.

In den JP-A-10-189065, 2000-243463, 2001-093589, 2000-100464 und 10-334954 werden verschiedene im Perylengerüst unsubstituierte Perylen-3,4:9,10-tetracarbonsäurederivate für die Anwendung in Halbleitersolarzellen beschrieben. Im einzelnen handelt es sich um: Perylentetracarbonsäuredümide, die an den Imidstickstoffatomen Carboxyalkyl-, Carboxyaryl-, Carboxyarylalkyl- oder Carboxyalkylarylreste tragen und/oder mit p-Diaminobenzolderivaten imidiert sind, bei denen das Stickstoffatom der Aminogruppe in p-Stellung durch zwei weitere Phenylreste substituiert oder Bestandteil eines heteroaromatischen tricyclischen Systems ist; Perylen-3,4:9,10-tetracarbonsäuremonoanhydridmonoimide, die am Imidstickstoffatom die vorstehend genannten Reste oder nicht weiter funktionalisierte Alkyl- oder Arylreste tragen, oder Semikondensate von Perylen-3,4:9,10-tetracarbonsäuredianhydrid mit 1,2-Diaminobenzolen oder 1,8-Diaminonaphthalinen, die durch weitere Reaktion mit primärem Amin in die entsprechenden Diimide bzw. doppelten Kondensate überführt werden; Kondensate von Perylen-3,4:9,10-tetracarbonsäuredianhydrid mit 1,2-Diaminobenzolen, die durch Carboxyl- oder Aminoreste funktionalisiert sind; sowie Perylen-3,4:9,10-tetracarbonsäurediimide, die mit aliphatischen oder aromatischen Diaminen imidiert sind.

In New J. Chem. 26, S. 1155-1160 (2002) wird die Sensibilisierung von Titandioxid mit Perylenderivaten untersucht, die im Perylengerüst (bay-Positionen) nicht substituiert sind. Im einzelnen genannt werden 9-Dialkylaminoperylen-3,4-dicarbonsäureanhydride, Perylen-3,4-dicarbonsäureimide, die in 9-Stellung durch Dialkylamino oder Carboxymethylamino substituiert sind und am Imidstickstoffatom einen Carboxymethyl- bzw. einen 2,5-Di(tert.-butyl)phenylrest tragen, und N-Dodecylaminoperylen-3,4:9,10-tetracarbonsäuremonoanhydridmonoimid genannt. Die Flüssigelektrolytsolarzellen auf Basis dieser Perylenderivate zeigten jedoch wesentlich geringere Wirkungsgrade als eine zum Vergleich mit einem Rutheniumkomplex sensibilisierte Solarzelle.

Die erfindungsgemäßen Perylenderivate I unterscheiden sich von den in der Literatur beschriebenen Perylenderivaten durch die Art der Endgruppen und/oder die Substitution im Perylengerüst.

Schließlich wird in Adv. Mater. 17, S. 813-815 (2005) für Solarzellen mit Spirobifluorenen als amorphem organischem p-Leiter ein Indolinfarbstoff vorgeschlagen.

Der Erfindung lag die Aufgabe zugrunde, organische Farbmittel bereitzustellen, die sich durch vorteilhafte Anwendungseigenschaften, insbesondere starke Lichtabsorption und hohe Stabilität, auszeichnen und Solarzellen mit guten Wirkungsgraden ergeben. Insbesondere sollte Absorptionsspektrum möglichst breit sein, vor allem sollte es den NIR-Bereich umfassen.

Demgemäß wurde die Verwendung von Rylenderivaten der eingangs definierten Formel I 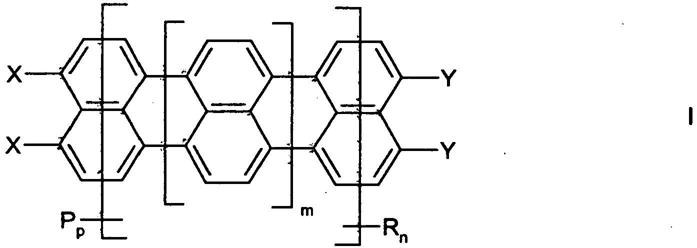 und ihrer Mischungen als Photosensibilisatoren in Solarzellen gefunden.

Außerdem wurden farbstoffsensibilisierte Solarzellen gefunden, die die Rylenderivaten I als Photosensibilisatoren enthalten.

Weiterhin wurden Rylenderivate der allgemeinen Formel Ia 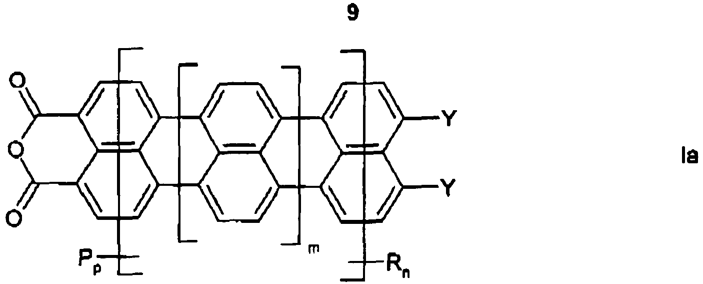 in der der Dicarbonsäureanhydridrest auch stellvertretend für zwei Carboxylreste -COOM stehen kann und die Variablen folgende Bedeutung haben:
- Y: einer der beiden Reste ein Rest der Formel (y1)

-L-NR¹R² (y1)

oder ein Rest der Formel (y2)

-L-Z-R³ (y2)

und der andere Rest jeweils Wasserstoff;
- R: gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann; (i) C1-C30-Alkyl, dessen Kohlenstoffkette durch eine oder mehrere Gruppierungen -O-, -S-, -NR4-, -N=CR4-, -C≡C-, -CR4=CR4-, -CO-, -SO- und/oderSO2- unterbrochen sein kann und das ein- oder mehrfach substituiert sein kann durch:
C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; (ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷; (iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem einoder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, C₁-C₆-Alkylthio, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷ substituiert sein können; (iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR³-, -CO-, -SO- oder -SO₂- bedeutet; (v) C₁-C₁₂-Alkoxy, C₁-C₆-Alkylthio, -C≡CR⁴, CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
- P: ein Aminorest -NR¹R²;
- M: Wasserstoff, einweriges oder zweiwertiges Metallkation, insbesondere Erdalkali- oder Alkalimetallkation, Ammoniumsalze von cyclischen Aminen Guanidiniumsalze oder [NR⁵₄]⁺;
- L: eine chemische Bindung oder ein direkt oder über Ethenylen oder Ethinylen an das Rylengerüst gebundener Arylen- oder Hetarylenrest der Formeln

-Ar- -Ar-E-Ar-

in denen die (Het)Arylenreste Ar gleich oder verschieden sein können, Hetero-atome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl, C₁-C₁₂-Alkoxy, C₁-C₁₂-Alkylthio und/oder -NR⁵R⁶ substituiert sein kann;
- E: eine chemische Bindung oder eine Gruppierung -O-, -S-, -NR⁴-, -C≡C-, -CR⁴=CR⁴- oder C₁-C₆-Alkylen;
- R¹, R²: unabhängig voneinander einer der als Substituenten für die Reste R genannten Alkylreste (i), Cycloalkylreste (ii) oder (Het)Arylreste (iii); miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
- Z: -O- oder -S-;
- R³: einer der als Substituenten für die Reste R genannten Alkylreste (i) oder (Het)Arylreste (iii);
- R⁴: Wasserstoff oder C₁-C₁₈-Alkyl, wobei die Reste R⁴ gleich oder verschieden sein können, wenn sie mehrfach auftreten;
- R⁵, R⁶: unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁵ gleich oder verschieden sein können, wenn sie mehrfach auftreten;
- R⁷: C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁷ gleich oder verschieden sein können, wenn sie mehrfach auftreten;
- R⁸: C₁-C₁₈-Alkyl;
- R⁹, R¹⁰: C₁-C₃₀-Alkyl, dessen Kohlenstoffkette durch eine oder mehrere Gruppierungen -O-, -S-, -NR⁴-, -N=CR⁴=, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, -S-,-NR⁴-, -N=CR⁴- und/oder -CR⁴=CR⁴- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem gesättigten oder ungesättigten, 5-bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
- m: 0, 1 oder 2;
- n: 0, 2 oder 4 für m = 0 oder 1;
0, 4 oder 6 für m = 2;
- p: 0, 2 oder 4 für m = 0, wobei n + p = 2 oder 4 ist; 0, 2 oder 4 für m = 1, wobei n + p = 0, 2 oder 4 ist, wobei für den Fall, daß n + p = 0 ist und einer der beiden Reste Y einen Rest der Formel (y1) und der andere Rest Wasserstoff bedeutet, mindestens einer der beiden Reste R¹ oder R² einen der als Substituenten für die Reste R genannten (Het)Arylreste (iii) bedeutet, wenn L für eine chemische Bindung steht; 0, 4 oder 6 für m = 2, wobei n + p = 0, 4 oder 6 ist.

Außerdem wurden Rylenderivate der allgemeinen Formel Ib 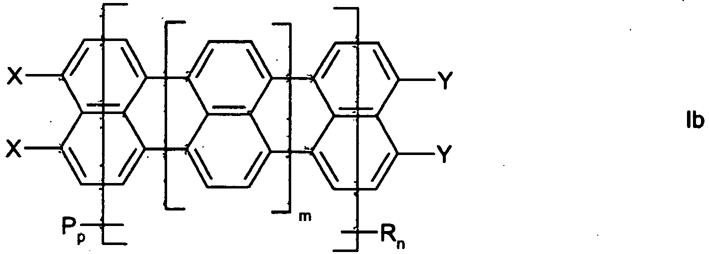 in der die Variablen folgende Bedeutung haben:
- X: miteinander unter Ausbildung eines Sechsrings verbunden zu einem Rest der Formel (x2) oder (x3)

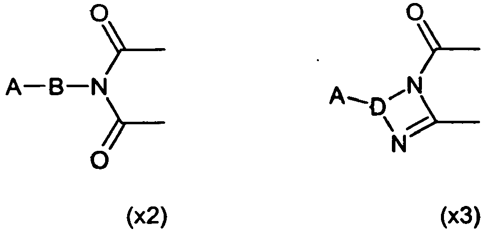

- Y: einer der beiden Reste ein Rest der Formel (y1)

-L-NR¹R² (y1)

oder ein Rest der Formel (y2) -L-Z-R³ (y2) und der andere Rest jeweils Wasserstoff; miteinander unter Ausbildung eines Sechsrings verbunden zu einem Rest der Formel (y3) oder (y4) 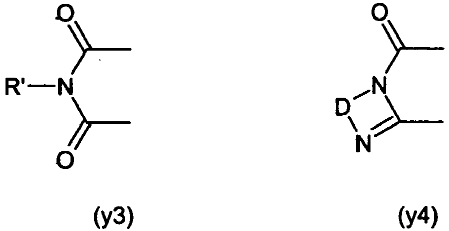 oder beide Wasserstoff;
- R: gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, anneliert sein können, wobei das gesamte Ring-system ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ ,-POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierunggen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem einoder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl, (Het)Aryloxy und/oder (Het)Arylthio, wobei die (Het)Aryl-reste jeweils ein- oder mehrfach durch C₁-C₁₈-Alkyl, C₁-C₁₂-Alkoxy, C₁-C₆-Alkylthio, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷ substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR⁴-, -CO-, -SO- oder -SO₂- bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
- P: ein Aminorest -N¹R²;
- B: C₁-C₆-Alkylen, Phenylen oder Kombinationen dieser Brückenglieder, wobei die Phenylenreste ein- oder mehrfach durch C₁-C₁₂-Alkyl, Nitro, Cyano und/oder Halogen substituiert sein können;
- A: -COOM, -SO₃M oder -PO₃M;
- D: Phenylen, Naphthylen oder Pyridylen, das jeweils ein- oder mehrfach durch C₁-C₁₂-Alkyl, C₁-C₁₂-Alkoxy, Hydroxy, Nitro und/oder Halogen substituiert sein kann;
- M: Wasserstoff, einweriges oder zweiwertiges Metallkation, insbesondere Erdalkali- oder Alkalimetallkation, Ammoniumsalze von cyclischen Aminen Guanidiniumsalze oder [NR⁵₄]⁺;
- L: eine chemische Bindung oder ein direkt oder über Ethenylen oder Ethinylen an das Rylengerüst gebundener Arylen- oder Hetarylenrest der Formeln

-Ar- -Ar-E-Ar-

in denen die (Het)Arylenreste Ar gleich oder verschieden sein können, Heteroatome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl, C₁-C₁₂-Alkoxy, C₁-C₁₂-Alkylthio und/oder -NR⁵R⁶ substituiert sein kann;
- E: eine chemische Bindung oder eine Gruppierung -O-, -S-, -NR⁴-, -C≡C-, -CR⁴=CR⁴- oder C₁-C₆-Alkylen;
- R¹, R²: unabhängig voneinander einer der als Substituenten für die Reste R genannten Alkylreste (i), Cycloalkylreste (ii) oder (Het)Arylreste (iii); miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem einoder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
- Z: -O- oder -S-;
- R³: einer der als Substituenten für die Reste R genannten Alkylreste (i) oder (Het)Arylreste (iii);
- R': Wasserstoff;
C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach durch die als Substituenten für die Reste R genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach durch die als Substituenten für die Reste R genannten Reste (i), (ii), (iii), (iv) und/oder (v) substituiert sein kann;
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch die als Substituenten für die Reste R genannten Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
- R⁴: Wasserstoff oder C₁-C₁₈-Alkyl, wobei die Reste R⁴ gleich oder verschieden sein können, wenn sie mehrfach auftreten;
- R⁵, R⁶: unabhängig voneinander:
Wasserstoff; C₁-C₁₈-Alkyl, dessen Kohlenstoffkette durch eine oder mehrere Gruppierungen -O-, -S-, -CO-, -SO- und/oder -SO₂- unterbrochen sein kann und das ein-oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁵ gleich oder verschieden sein können, wenn sie mehrfach auftreten;
- R⁷: C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann,
wobei die Reste R⁷ gleich oder verschieden sein können, wenn sie mehrfach auftreten;
- R⁸: C₁-C₁₈-Alkyl;
- R⁷, R⁸: C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, S-,-NR⁴-, -N=CR⁴- und/oder -CR⁴=CR⁴- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können;
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶-, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können;
mit dem Stickstoffatom verbunden zu einem gesättigten oder ungesättigten, 5-bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
- m: 0, 1 oder 2;
- n: 0, 2 oder 4 für m = 0 oder 1;
0, 4 oder 6 für m = 2;
- p: 0, 2 oder 4 für m = 0, wobei n + p = 2 oder 4 ist oder auch 0 sein kann, wenn einer der beiden Reste Y einen Rest der Formel (y1) oder (y2) und der andere Rest Wasserstoff bedeutet, wobei mindestens einer der beiden Reste R¹ oder R² im Rest (y1) einen der als Substituenten für die Reste R genannten (Het)Arylreste (iii) bedeutet, wenn L für eine chemische Bindung steht; 0, 2 oder 4 für m = 1, wobei n + p = 0, 2 oder 4 ist; 0, 4 oder 6 für m = 2, wobei n + p = 0, 4 oder 6 ist.

Bei den Rylenderivaten I handelt es sich um unsymmetrisch aufgebaute Perylen-, Terrylen- und Quaterrylentetra- und -dicarbonsäurederivate. Besonders bevorzugt sind dabei die Perylen- und Terrylenderivate.

Die Rylenderivate I sind an einem Molekülende (3,4-Position) sauer funktionalisiert. Die Reste X sind dabei miteinander unter Ausbildung eines Sechsrings verbunden zu einem Rest der Formel (x1), (x2) oder (x3)

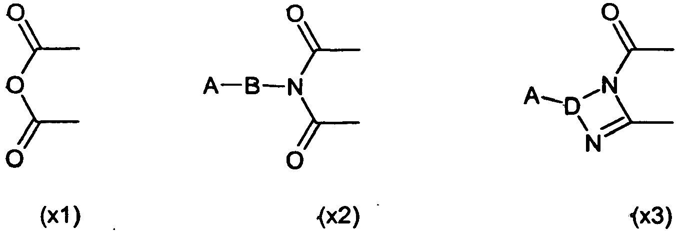

Die Rylenderivate I können nicht nur in Anhydridform (x1) sondern auch als freie Säuren oder als Salze (beide Reste X ein Rest -COOM) vorliegen. Geeignete Salze sind Salze abgeleitet von ein- oder zweiwertigen Metallionenund deren Komplexe, insbesondere neben den Guanadiniumsalzen, die Heterocyclicammoniumsalze, Ammoniumsalze (sowohl NH₄⁺ als auch Tetraalkyl/arylammonium [NR⁵₄]⁺ mit gleichen oder verschiedenen Resten R⁵) insbesondere die Alkalimetallsalze und Erdalkalimetallsalze.

Bei den Imid resten (x2) sind Säuregruppen A über ein Brückenglied B an das Imidstickstoffatom gebunden.

Geeignete Brückenglieder B sind C₁-C₆-Alkylenreste und Phenylenreste und Kombinationen dieser Reste, z.B. Alkylenphenylen-, Phenylenalkylen- und Alkylenphenylenalkylenreste. Die Phenylenreste können dabei ein- oder mehrfach durch C₁-C₁₂-Alkyl, C₁-C₆-Alkoxy, Hydroxy, Nitro, Cyano und/oder Halogen substituiert sein, bevorzugt sind sie jedoch unsubstituiert.

Bei den Säuregruppen A handelt es sich um Carboxyl-, Sulfo- oder Phosphonsäuregruppen, die ebenfalls als freie Säure oder in Salzform vorliegen können.

Bei den Kondensatresten (x3), die durch Kondensation des Dicarbonsäureanhydrids mit einem säuregruppenhaltigen o-Phenylendiamin, 1,8-Diaminonaphthalin oder auch 3,4-Diaminopyridin entstehen, ist die Säuregruppe A, die wiederum in Salzform vorliegen kann, an das aromatische Ringsystem D gebunden. Das Ringsystem D ist ansonsten vorzugsweise unsubstituiert, kann aber auch C₁-C₁₂-Alkyl, C₁-C₆-Alkoxy, Hydroxy, Nitro und/oder Halogen als Substituenten tragen.

Besonders bevorzugte Rylenderivate I weisen in 3,4-Position einen Anhydridrest (x1) bzw. das entsprechende Dicarbonsäuresalz auf.

Die Rylenderivate I können am anderen Molekülende unsubstituiert sein (beide Reste Y Wasserstoff) oder in peri-Position durch einen Aminorest (y1)

-L-NR¹R² (y1)

oder einen (Thio)Etherrest (y2)

-L-Z-R³ (y2)

substituiert sein (der zweite Rest Y bedeutet dementsprechend Wasserstoff) oder als Imid (y3) oder Kondensat (y4) 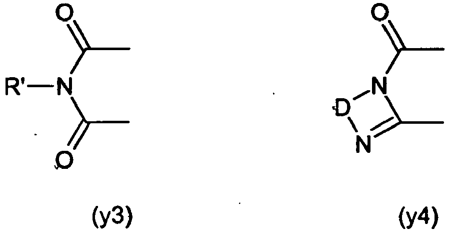 vorliegen (die Reste Y sind hier miteinander unter Ausbildung eines Sechsrings verbunden).

Bei den Resten Aminoresten (y1) und den (Thio)Etherresten (y2) ist die Aminofunktion bzw. die (Thio)Etherfunktion über ein Brückenglied L an das Rylengerüst gebunden.

Das Brückenglied L kann dabei eine chemische Bindung sein, d.h. die Aminogruppe ist direkt an das Rylengerüst gebunden, oder ein direkt oder über Ethenylen oder Ethinylen an das Rylengerüst gebundener (Het)Arylenrest der Formeln

-Ar- -Ar-E-Ar-

sein.

Die (Het)Arylenreste Ar können Heteroatome als Ringatome enthalten und/oder annelierte gesättigte oder ungesättigte 5- bis 7-gliedrige Ringe, die ebenfalls Heteroatome enthalten können, aufweisen. Handelt es sich um kondensierte Ringsysteme Ar, so können die Bindungen zum Rylengerüst und zur funktionellen Gruppe beide von demselben Ring oder von verschiedenen Ringen ausgehen. Das ganze Ringsystem kann zusätzlich ein- oder mehrfach durch Phenyl, C₁-C₁₂-Alkyl, C₁-C₁₂-Alkoxy, C₁-C₁₂-Alkylthio und/oder -NR⁵R⁶ substituiert sein, wobei C₁-C₁₂-Alkyl, C₁-C₁₂-Alkoxy und/oder -NR⁵R⁶ als Substituenten bevorzugt sind.

Enthält das Brückenglied L zwei (Het)Arylenreste Ar, so sind diese vorzugsweise gleich, sie können jedoch auch verschieden sein. Die beiden Reste Ar können direkt aneinander gebunden sein oder über eine Gruppierung -O-, -S-, -NR⁴-, -C≡C-, -CR⁴=CR⁴- oder C₁-C₆-Alkylen miteinander verknüpft sein. Das Bindeglied E bedeutet dabei bevorzugt eine chemische Bindung oder eine Gruppierung -O-, -S-, -NR⁴- oder -C≡C-.

Als Beispiele für geeignete Brückenglieder L seien genannt:

1,4-, 1,3- und 1,2-Phenylen, 1,4- und 1,8-Naphthylen, 1,4- und 2,3-Pyrrylen, 2,5-, 2,4- und 2,3-Thienylen, 2,5-, 2,4- und 2,3-Furanylen, 2,3-, 2,4-, 2,5-, 2,6-, 3,4- und 3,5-Pyridinylen, 2,3-, 2,5-, 2,6-, 3,7-, 4,8-, 5,8- und 6,7-Chinolinylen, 2,7-, 3,6-, 4,5-, 2,6-, 3,7-, 4,7- und 4,8-Isochinolinylen, 4,4'-, 3,3'- und 2,2'-Biphenylen, 3,3'- und 2,2'-Bithienylen, 1,4-[2,5-Di(tert.-butyl)]phenylen, 1,4-(2,5-Dihexyl)phenylen, 1,4-[2,5-Di(tert.-octyl)]phenylen, 1,4-(2,5-Didodecyl)phenylen, 1,4-[2,5-Di(2-dodecyl)]phenylen, 4,4'-Di(2,2',6,6'-tetramethyl)phenylen, 4,4'-Di(2,2',6,6'-tetraethyl)phenylen, 4,4'-Di(2,2',6,6'-tetraisopropyl)phenylen, 4,4'-Di(2,2',6,6'-tetrahexyl)phenylen, 4,4'-Di[2,2',6,6'-tetra(2-hexyl)]phenylen, 4,4'-Di[2,2',6,6'-tetra(tert.-octyl)]phenylen, 4,4'-Di(2,2',6,6'-tetra-dodecyl)phenylen und 4,4'-Di[2,2',6,6'-tetra(2-dodecyl)]phenylen sowie 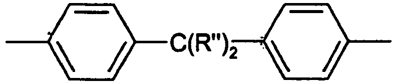 wobei R" Wasserstoff, Methyl, Ethyl oder Phenyl bedeutet.

Ganz besonders bevorzugte Brückenglieder L sind eine chemische Bindung, 1,4-Phenylen, 2,3-Thienylen und 4,4'-Di(2,2',6,6'-tetramethyl)phenylen.

Die Reste R¹ und R² im Aminorest (y1) können unabhängig voneinander einen der eingangs bei der Definition der Variable R als Substituenten genannten Alkylreste (i), Cycloalkylreste (ii) oder (Het)Arylreste (iii) bedeuten. Bevorzugt bedeuten die Reste R¹ und R² insbesondere gleiche Phenylreste, die C₁-C₁₈-Alkyl, C₁-C₁₂-Alkoxy, C₁-C₆-Alkylthio, -NR⁵R⁶ und/oder Phenoxy und/oder Phenylthio, das jeweils ein- oder mehrfach durch C₁-C₁₈-Alkyl, C₁-C₁₂-Alkoxy, C₁-C₆-Alkylthio, -NR⁵R⁶ substituiert sein kann, als Substituenten tragen können. Vorzugsweise sind sie in para-Position durch C₄-C₁₈-Alkyl, insbesondere verzweigtes C₄-C₁₈-Alkyl, z.B. tert.-Octyl, C₁-C₁₈-Alkoxy, z.B. Methoxy, oder Di(C₁-C₁₈-alkyl)amino, z.B. Dimethylamino, substituiert oder unsubstituiert.

Die Reste R¹ und R² können auch miteinander verbunden sein zu einem das Stickstoffatom des Aminorests (y1) enthaltenden, gesättigten oder ungesättigten, 5- bis 7-gliedrigen Ring, dessen Kohlenstoffgerüst durch eine oder mehrere Gruppierungen -O-, -S- und/oder -NR⁴- (R⁴: H oder C₁-C₁₈-Alkyl, vorzugsweise H oder C₁-C₆-Alkyl) unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkyithio und/oder -NR⁵R⁶, wobei C₄-C₁₈-Alkyl, C₁-C₁₈-Alkoxy und -NR⁵R⁶ als Substituenten bevorzugt sind.

Als Beispiele für bevorzugte unsubstituierte cyclische Aminoreste seien Piperidyl, Pyrrolidyl, Piperazyl, Morpholinyl, Thiomorpholinyl, Pyrryl, Dibenzopyrryl (Carbazyl), Dibenzo-1,4-oxiranyl (Phenoxazinyl), Dibenzo-1,4-thiazinyl (Phenothiazinyl), Dibenzo-1,4-pyrazyl (Phenazinyl) und Dibenzopiperidyl genannt, wobei Piperidyl, Pyrrolidinyl, Dibenzopyrryl, Dibenzo-1,4-oxiranyl, Dibenzo-1,4-thiazinyl, Dibenzo-1,4-pyrazyl und Dibenzopiperidyl besonders bevorzugt sind Phenothiazinyl, Piperidyl und Pyrrolidyl ganz besonders bevorzugt sind.

Als Edukte für diese cyclischen Aminoreste dienen die entsprechenden cyclischen Amine bzw. deren Salze. Als Beispiele für geeignete substituierte und unsubstituierte Amine seien genannt:
Piperidin, 2- oder 3-Methylpiperidin, 6-Ethylpiperidin, 2,6- oder 3,5-Dimethylpiperidin, 2,2,6,6-Tetramethylpiperidin, 4-Benzylpiperidin, 4-Phenylpiperidin, Piperidin-4-ol, 2,2,6,6-Tetramethylpiperidin-4-ylamin, Decahydrochinolin und Decahydroisochinolin;
Pyrrolidin, 2-Methylpyrrolidin, 2,5-Dimethylpyrrolidin, 2,5-Diethylpyrrolidin, Tropanol, Pyrrolidin-3-ylamin, (2,6-Dimethylphenyl)pyrrolidin-2-ylmethylamin, (2,6-Diisopropylphenyl)pyrrolidin-2-ylmethylamin und Dodecahydrocarbazol;
Piperazin, Diketopiperazin, 1-Benzylpiperazin, 1-Phenethylpiperazin, 1-Cyclohexylpiperazin, 1-Phenylpiperazin, 1-(2,4-Dimethylphenyl)piperazin, 1-(2-, 3- oder 4-Methoxyphenyl)piperazin, 1-(2-, 3- oder 4-Ethoxyphenyl)piperazin, 1-(2-, 3- oder 4-Fluorphenyl)piperazin, 1-(2-, 3- oder 4-Chlorphenyl)piperazin, 1-(2-, 3- oder 4-Bromphenyl)-piperazin, 1-, 2- oder 3-Pyridin-2-ylpiperazin und 1-Benzo[1,3]dioxol-4-ylmethylpiperazin;
Morpholin, 2,6-Dimethylmorpholin, 3,3,5,5-Tetramethylmorpholin, Morpholin-2- oder -3-ylmethanol, 3-Benzylmorpholin, 3-Methyl-2-phenylmorpholin, 2- oder 3-Phenylmorpholin, 2-(4-Methoxyphenyl)morpholin, 2-(4-Trifluoromethylphenyl)morpholin, 2-(4-Chlorphenyl)morpholin, 2-(3,5-Dichlorphenyl)morpholin, 3-Pyridin-3-ylmorpholin, 5-Phenylmorpholin-2-on, 2-Morpholin-2-ylethylamin und Phenoxazin;
Thiomorpholin, 2- oder 3-Phenylthiomorpholin, 2- oder 3-(4-Methoxyphenyl)thiomorpholin, 2- oder 3-(4-Fluorphenyl)thiomorpholin, 2- oder 3-(4-Trifluormethylphenyl)thiomorpholin, 2- oder 3-(2-Chlorphenyl)thiomorpholin, 4-(2-Aminoethyl)thiomorpholin, 3-Pyridin-3-ylthiomorpholin, 3-Thiomorpholinon und 2-Phenylthiomorpholin-3-on sowie die Thiomorpholinoxide und -dioxide.

Beispiele für besonders bevorzugte Reste (y1) sind: 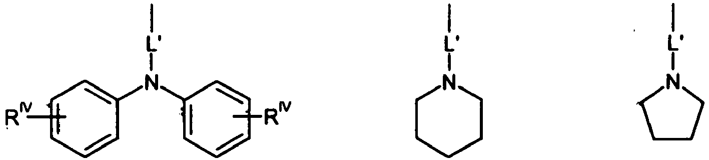 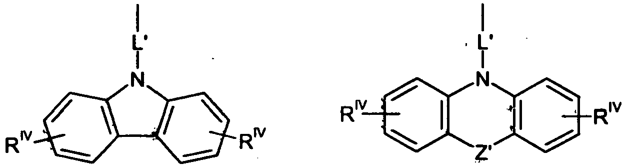 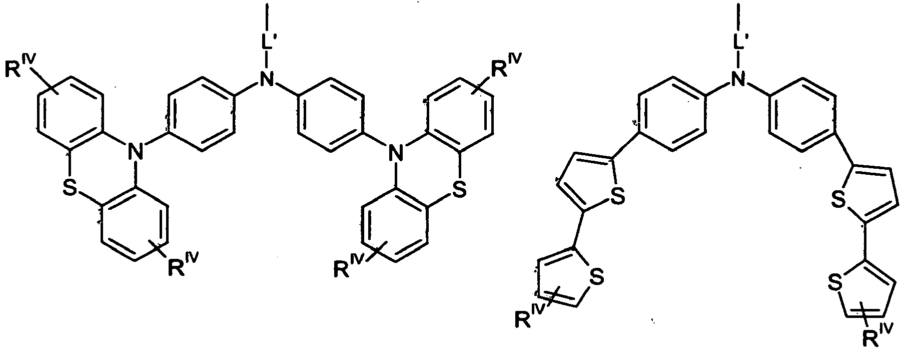 mit folgender Bedeutung der Variablen:
- L': chemische Bindung oder 1,4-Phenylen;
- Z': -O-, -S-, -NR^{8'}- oder -CH₂-, wobei R^{8'} C₁-C₁₈-Alykl bedeutet;
- R^{IV}: C₄-C₁₈-Alkyl, C₁-C₁₈-Alkoxy, (Hetero)aryl oder -NR⁵R⁶

Ganz besonders bevorzugte Aminoreste (y1) sind die oben aufgeführten Diphenylaminophenylen- und insbesondere die Diphenylaminoreste.

Für den (Thio)Etherrest (y2) sind als Brückenglieder L eine chemische Bindung, 1,4-Phenylen und 2,5-Thienylen besonders bevorzugt. Ganz besonders bevorzugtes Brückenglied L ist die chemische Bindung.

Der Rest R³ im (Thio)Etherrest (y2) kann einen der eingangs bei der Definition der Variable R als Substituenten genannten Alkylreste (i) oder (Het)Arylreste (iii) bedeuten. Bevorzugt bedeutet R³:
C₁-C₃₀-Alkyl, dessen Kohlenstoffkette durch eine oder mehrere Gruppierungen -O-, -S- und/oder -NR⁴- unterbrochen sein kann und das ein- oder mehrfach substituiert sein kann durch: C₁-C₁₂-Alkoxy, Hydroxy und/oder Aryl, das ein- oder mehrfach durch C₁-C₁₈-Alkyl oder C₁-C₆-Alkoxy substituiert sein kann;
Phenyl, das ein- oder mehrfach substituiert sein kann durch: C₁-C₁₈-Alkyl, C₁-C₁₂-Alkoxy, C₁-C₆-Alkylthio, -NR⁵R⁶ und/oder Phenoxy und/oder Phenylthio, das jeweils ein- oder mehrfach durch C₁-C₁₈-Alkyl, C₁-C₁₂-Alkoxy, C₁-C₆-Alkylthio und/oder -NR⁵R⁶ substituiert sein kann.

Beispiele für besonders bevorzugte Reste (y2) sind:
Phenoxy, Phenylthio, Naphthyloxy oder Naphthylthio, das jeweils ein- oder mehrfach durch C₄-C₁₈-Alkyl, C₁-C₁₈-Alkoxy und/oder -NR⁵R⁶ substituiert sein kann.

In den Imidresten (y3) kann R' neben Wasserstoff die eingangs definierten Alkylreste (i), Cycloalkylreste (ii) oder (Het)Arylreste (iii) bedeuten.

Bevorzugt hat R' folgende Bedeutung:
C₆-C₃₀-Alkyl, dessen Kohlenstoffkette durch eine oder mehrere Gruppierungen -O-, -S-und/oder -NR⁴- unterbrochen sein kann und das ein-oder mehrfach substituiert sein kann durch: C₁-C₆-Alkoxy, C₁-C₆-Alkylthio, -NR⁹R¹⁰ und/oder Aryl, das ein- oder mehrfach durch C₁-C₁₈-Alkyl oder C₁-C₆-Alkoxy substituiert sein kann, wobei C₆-C₃₀-Alkyl, das in ω-Position durch -NR⁹R¹⁰ substituiert ist, besonders bevorzugt ist;
(Het)Aryl, insbesondere Phenyl, Naphthyl, Pyridyl oder Pyrimidyl, vor allem Phenyl, das jeweils ein- oder mehrfach substituiert sein kann durch: C₁-C₁₈-Alkyl, C₁-C₆-Alkoxy, Halogen, Cyano, Nitro, -NR⁹R¹⁰, -CONR⁵R⁶, -SO₂NR⁵R⁶ und/oder Phenoxy, Pheylthio, Phenylazo und/oder Naphthylazo, das jeweils durch C₁-C₁₀-Alkyl, C₁-C₆-Alkoxy und/oder Cyano substituiert sein kann.

Ganz besonders bevorzugt bedeutet R' einen Phenylrest, der ein- oder mehrfach durch C₁-C₁₈-Alkyl oder -NR⁹R¹⁰ substituiert ist.

Die Reste R⁵ und R⁵ haben dabei die eingangs angegebene Bedeutung. Bevorzugt bedeuten sie unabhängig voneinander:
Wasserstoff;
   C₁-C₁₈-Alkyl, das ein- oder mehrfach durch C₁-C₆-Alkoxy, Hydroxy, Halogen und/oder Cyano substituiert sein kann;
Aryl oder Hetaryl, das jewelis ein- oder mehrfach durch C₁-C₆-Alkyl und/oder die vorstehenden, als Substituenten für Alkyl genannten Reste substituiert sein kann.

Besonders geeignete Substituenten sind dabei die Alkylreste und vor allem die Aminogruppen -NR⁹R¹⁰.

Die Bedeutung der Reste R⁹ und R¹⁰ ist ebenfalls eingangs angegeben. Bevorzugt bedeuten sie unabhängig voneinander:
C₁-C₃₀-Alkyl, dessen Kohlenstoffkette durch eine oder mehrere Gruppierungen -O-, -S-, -NR⁴-, -N=CR⁴-, -C=C- und/oder -CR⁴=CR⁴- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶ und/oder (Het)Aryl, das ein- oder mehrfach durch C₁-C₁₈-Alkyl und/oder die vorstehenden, als Substituenten für Alkyl genannten Reste substituiert sein kann;
Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können;
mit dem Stickstoffatom verbunden zu einem Piperidyl-, Pyrrolidinyl-, Dibenzopyrryl-, Dibenzo-1,4-oxiranyl-, Dibenzo-1,4-thiazinyl-, Dibenzo-1,4-pyrazyl- oder Dibenzopiperidylringsystem, das jeweils ein- oder mehrfach substituiert sein kann durch C₁-C₂₄-Alkyl, das durch C₁-C₁₈-Alkoxy, C₁-C₁₈-Alkylthio und/oder -NR⁵R⁶ substituiert sein kann.

Bei den Aminogruppen -NR⁹R¹⁰ handelt es sich bevorzugt um Di(het)arylaminogruppen oder cyclische Aminogruppen. Besonders bevorzugt sind Diphenylaminogruppen, bei denen die Phenylreste unsubstituiert sein können oder die vorstehenden Substituenten, insbesondere die Alkylreste, vorzugsweise in p-Position aufweisen können.

Bevorzugte Substitutionsmuster für die Phenylreste R' sind ortho,ortho'-Disubstitution (z.B. Alkylreste mit sekundärem Kohlenstoffatom in 1-Position) und para-Substitution (z.B. Alkylreste mit tertiärem Kohlenstoffatom in 1-Position und mindestens 5 Kohlenstoffatomen oder Aminogruppen -NR⁹R¹⁰).

Beispiele für besonders bevorzugte Reste R' sind: 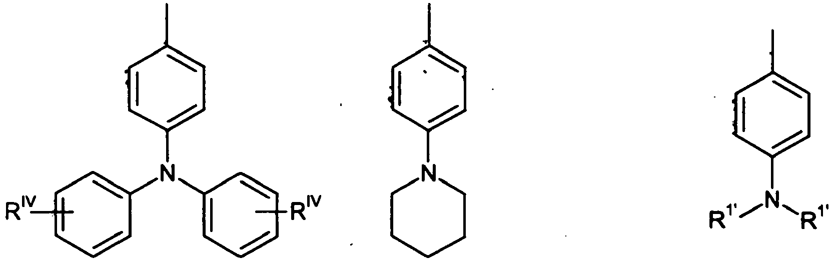 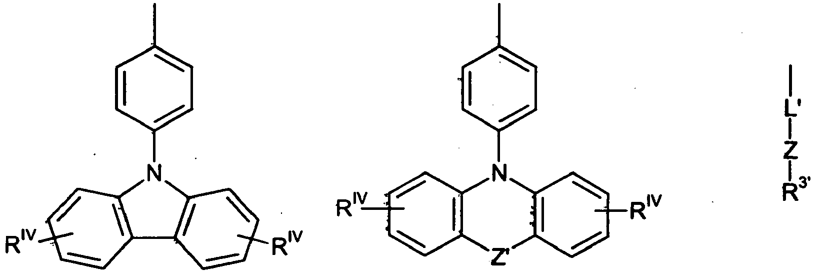 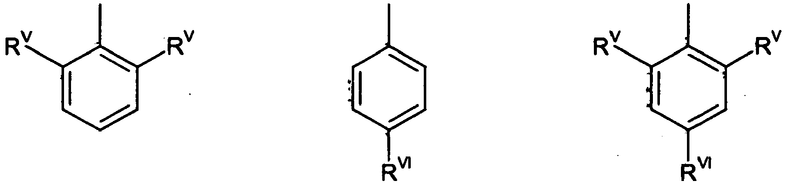 mit folgender Bedeutung der Variablen:
- R^{IV}: C₄-C₁₈-Alkyl oder C₁-C₁₈-Alkoxy;
- R^{V}: C₃-C₈-Alkyl mit sekundärem Kohlenstoffatom in 1-Position;
- R^{VI}: C₄-C₁₈-Alkyl mit tertiärem Kohlenstoffatom in 1-Position oder -NR⁹R¹⁰;
- R¹': C₁-C₁₈-Alkyl;
- R³': Phenyl, wenn L' eine chemische Bindung bedeutet; C₄-C₁₈-Alkyl, wenn L' 1,4-Phenylen bedeutet;
- L': chemische Bindung, 1,4-Phenylen oder 2,5-Thienylen;
- Z': -O-, -S-, -NR⁸- oder -CH₂-, wobei R⁸ C₁-C₁₈-Alkyl bedeutet;
- Z: -O- oder -S-

Ganz besonders bevorzugte Reste R' sind die Diphenylaminophenylenreste.

Die Kondensatreste (y4) werden durch Kondensation des Anhydrids mit gewünschtenfalls substituierten aromatischen Diaminen, insbesondere mit o-Phenylendiamin oder 1,8-Diaminonaphthalin oder auch 3,4-Diaminopyridin, gebildet.

Bevorzugt sind Rylenderivate I, bei denen einer der beiden Reste Y einen Rest (y1) oder (y2) und der andere Rest Wasserstoff bedeutet oder beide Reste Y einen Imidrest (y3) bedeuten, wobei die oben genannten weiteren Bevorzugungen gelten.

Dabei sind Rylenderivate I besonders bevorzugt, bei denen einer der beiden Reste Y einen Rest (y1) oder (y2) und der andere Rest Wasserstoff bedeutet.

Vorzugsweise sind die Rylenderivate I zusätzlich im Rylengerüst substituiert. Bevorzugt ist eine Tetrasubstitution in 1,6,7,12-Position bei den Perylenderivaten, 1,6,9,14-Position bei den Terrylenderivaten und 1,6,11,16-Position bei den Quaterrylenderivaten. Bei den Perylen- und Terrylenderivaten ist auch eine Disubstitution in 1,6- und/oder 1,7-Position bzw. 1,6- oder 9,14-Position und bei den Quaterrylenderivaten eine Hexasubstitution in 1,6,8,11,16,18,19-Position möglich. Die Zählung beginnt hierbei immer am Molekülende mit den Resten X.

In der Regel liegen die Rylenderivate I in Form von Mischungen von Produkten mit unterschiedlichem Substitutionsgrad vor, in denen die tetra- bzw. di- oder hexasubstituierten Produkte den Hauptbestandteil ausmachen. Da die Substituenten üblicherweise durch nucleophile Substitution von halogenierten, insbesondere bromierten, Rylenderivaten I oder entsprechend halogenierten Vorprodukten in das Rylengerüst eingeführt werden, können die Rylenderivate I noch Spuren Halogen enthalten, die gewünschtenfalls durch übergangsmetallkatalysierte reduktive oder baseninduzierte Enthalogenierung entfernt werden können.

Als Substituenten eignen sich insbesondere die eingangs definierten (Het)Aryloxy- und (Het)Arylthioreste R. Besonders geeignet sind dabei Phenoxy-, Thiophenoxy-, Pyridyloxy-, Primidylthio-, Pyridylthio- und Pyrimidylthioreste. Die Reste R können Resten der Formel (y2) entsprechen.

Bevorzugte Reste R sind Phenoxy- oder Thiophenoxyreste, die jeweils ein- oder mehrfach durch gleiche oder verschiedene Reste (i), (ii), (iii), (iv) und/oder (v) substituiert sein können:
(i) C₁-C₃₀-Alkyl, dessen Kohlenstoffkette durch eine oder mehrere Gruppierungen -O-, -S-, -NR⁴-, -C≡C-, -CR⁴=CR⁴- und/oder -CO- und/oder -SO₂- unterbrochen sein kann und das ein- oder mehrfach substituiert sein kann durch: C₁-C₁₂-Alkoxy, Hydroxy, Halogen, Cyano und/oder Aryl, das ein- oder mehrfach durch C₁-C₁₈-Alkyl oder C₁-C₆-Alkoxy substituiert sein kann;
(ii) C₃-C₈-Cycloalkyl, dessen Kohlenstoffgerüst durch eine oder mehrere Gruppierungen -O-, -S-, -NR⁴-, -CR⁴=CR⁴- und/oder -CO- unterbrochen sein kann und das ein-oder mehrfach durch C₁-C₁₈-Alkyl, C₁-C₁₂-Alkoxy und/oder C₁-C₆-Alkylthio substituiert sein kann;
(iii) Aryl oder Hetaryl, an das jeweils weitere 5- bis 7-gliedrige gesättigte oder ungesättigte Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, -C=CR⁴₂, -CR⁴=CR⁴₂, Hydroxy, Halogen, Cyano, -NR⁹R¹⁰, -NR⁵COR⁶, -CONR⁵R⁶, -SO₂NR⁵R⁶, -COOR⁷, -SO₃R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₈-Alkoxy und/oder Cyano substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR⁴-, -CO-, -SO- oder -SO₂- bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷ oder -SO₃R⁷.

Die (Thio)Phenoxyreste R können unsubstituiert oder einfach in ortho-, meta- oder vorzugsweise para-Position substituiert sein. Sie können auch zwei-, drei-, vier- oder fünffach substituiert sein, wobei alle Substitutionsmuster denkbar sind.

Weiterhin kann das Rylengerüst der Rylenderivate I auch durch Reste P substituiert sein. Dabei handelt es sich um Aminoreste -NR¹R². Die Reste P entsprechen daher Resten (y1), bei denen L eine chemische Bindung bedeutet.

Die Rylenderivate I können gleichzeitig durch (Het)Aryloxy- bzw. -thioreste R und cyclische Aminogruppen P oder entweder durch Reste R oder durch Reste P substituiert sein. Bevorzugt sind sie jedoch nur durch Reste R substituiert.

Die vorstehend beschriebenen bevorzugten Bedeutungen für die in Formel I auftretenden Variablen gelten ebenso für die neuen Rylenderivate la bzw. Ib.

Als Beispiele für die in erfindungsgemäßen Formeln auftretenden Reste R, R' bis R^{VI} sowie R¹ bis R¹⁰ bzw. deren Substituenten seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Ethenyl, 1- und 2-Propenyl, 1-, 2- und 3-Butenyl, 1-, 2-, 3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16- und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl; Hexoxycarbonyl, Dodecyloxycarbonyl, Octadecyloxycarbonyl, Phenoxycarbonyl, (4-tert.-Butyl-phenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Chlor, Brom und Iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methylisoindolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroisochinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethylchinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl,2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- and 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Die erfindungsgemäßen Rylenderivate I, die einen Anhydridrest (x1) und einen Imidrest (y3) bzw. einen Kondensatrest (y4) aufweisen, sind z.T. bekannt (WO-A-02/66438, Chem. Eur. J. 11, S. 1-9 (2005) sowie die älteren deutschen Patentanmeldungen 10 2005 021 362.6 und 10 2005 032 583.1).

Die Herstellung der neuen Rylenderivate la und Ib kann vorteilhaft nach den im folgenden beschriebenen Verfahren vorgenommen werden.

Die dabei verwendeten Hilfsstoffe, wie Lösungsmittel, Basen, Katalysatoren usw., können selbstverständlich, auch ohne daß explizit darauf hingewiesen wird, stets auch in Form von Mischungen zum Einsatz kommen.

Die dabei jeweils erhaltenen Rohprodukte können gewünschtenfalls einer zusätzlichen Reinigung durch Säulenfiltration oder Säulenchromatographie an Kieselgel mit unpolaren organischen Lösungsmitteln, wie Hexan oder Pentan, oder polaren organischen Lösungsmitteln, insbesondere halogenierten Kohlenwasserstoffen, wie Methylenchlorid und Chloroform, oder vor allem mit Mischungen unpolarer und polarer Lösungsmittel unterzogen werden.

### A. Herstellung von Rylenderivaten la

Zur Herstellung der Rylenderivate Ia, bei denen ein Rest Y einen Aminorest (y1) oder einen (Thio)Etherrest (y2) und der andere Rest Y Wasserstoff bedeutet, kann man peri-Halogenrylendicarbonsäureanhydride der Formel IIa 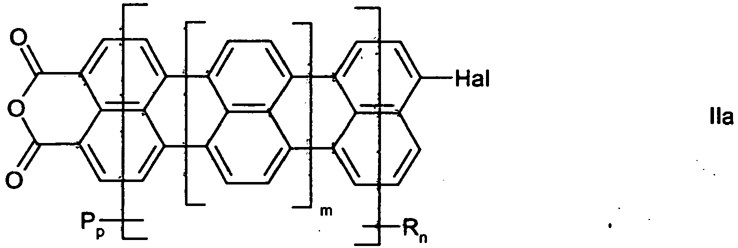 oder peri-Halogenrylendicarbonsäureimide der Formel IIb 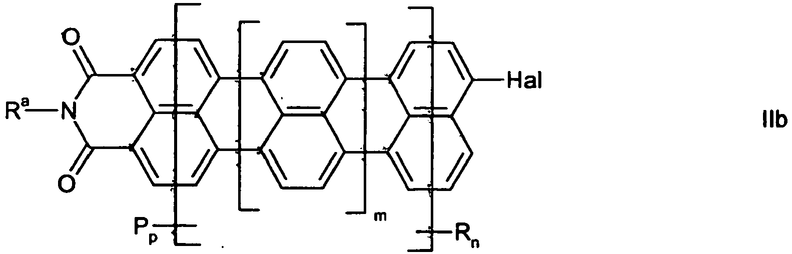 in der R^{a} einen der eingangs als Substituenten für die Reste R genannten Alkylreste (i) oder Cycloalkylreste (ii) und Hal Iod, bevorzugt Chlor und besonders bevorzugt Brom bedeutet, mit den den jeweiligen Rest (y1) oder (y2) enthaltenden Edukten (III) umsetzen.

Die Reaktionsbedingungen hängen dabei von dem jeweils in den Resten (y1) und (y2) enthaltenen Brückenglied L ab und werden unten separat beschrieben.

Werden für die Umsetzung mit dem Edukt (III) peri-Halogenrylendicarbonsäureimide IIb eingesetzt, was aufgrund ihrer besseren Löslichkeit die Umsetzung erleichtern kann, so müssen die jeweils erhaltenen peri-substituierten Rylendicarbonsäureimide der Formel IIb' 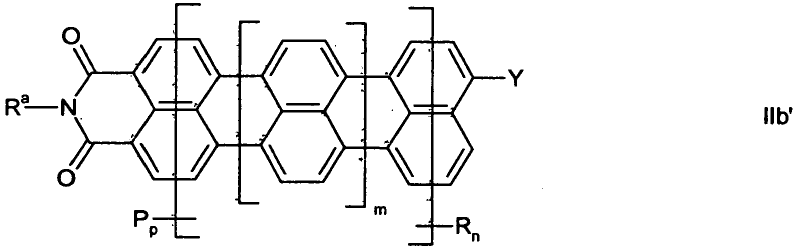 noch zum Rylendicarbonsäureanhydrid Ia verseift werden.

Die alkalische Verseifung kann für alle Rylendicarbonsäureimide IIb' unter vergleichbaren Bedingungen in polaren organischen Lösungsmitteln durchgeführt werden.

Als Reaktionsmedium sind insbesondere protische polare organische Lösungsmittel geeignet. Besonders geeignet sind aliphatische Alkohole, die 3 bis 8 Kohlenstoffatome aufweisen und unverzweigt sein können, vorzugsweise aber verzweigt sind. Beispielhaft seien neben n-Propanol und n-Butanol insbesondere Isopropanol, sec. und tert.-Butanol und 2-Methyl-2-butanol genannt.

Im allgemeinen kommen 5 bis 500 ml, vorzugsweise 20 bis 100 ml, Lösungsmittel pro g IIb' zum Einsatz.

Als Basen eignen sich alkalimetall- und erdalkalimetallhaltige Basen, wobei die alkalimetallhaltigen Basen bevorzugt und die natrium- und kaliumhaltigen Basen besonders bevorzugt sind. Dabei sind sowohl anorganische Basen, vor allem die Hydroxide, wie Natriumhydroxid und Kaliumhydroxid, als auch organische Basen, vor allem die Alkoholate, wie Natriummethylat, Kaliummethylat, Kaliumisopropylat und Kalium-tert.-butylat, geeignet, die üblicherweise in wasserfreier Form eingesetzt werden. Ganz besonders bevorzugt ist Kaliumhydroxid.

In der Regel werden 10 bis 200 mol, bevorzugt 30 bis 70 mol, Base, pro mol IIb' benötigt.

Insbesondere bei der Verseifung der Terrylendicarbonsäureimide IIb' hat es sich als vorteilhaft erwiesen, zusätzlich ein Metallfluorid, insbesondere ein Alkalimetallfluorid, z.B. Kaliumfluorid, Natriumfluorid oder Lithiumfluorid, als Hilfsmittel einzusetzen.

Geeignete Hilfsmittelmengen liegen in der Regel bei 0,1 bis 2 mol, insbesondere bei 0,7 bis 1,3 mol, pro mol Base.

Die Reaktionstemperatur beträgt im allgemeinen 50 bis 120°C, vorzugsweise 60 bis 100°C.

Übliche Reaktionsdauern sind 0,5 bis 24 h, vor allem 2 bis 10 h.

Verfahrenstechnisch geht man zweckmäßigerweise wie folgt vor:
Man erhitzt eine Mischung von Base, gegebenenfalls Hilfsmittel und Lösungsmittel unter starkem Rühren auf die Reaktionstemperatur und gibt dann das Rylendicarbonsäureimid IIb' zu. Nach der gewünschten Reaktionszeit tropft man so lange eine Säure, z.B. eine anorganische Säure wie Salzsäure oder vorzugsweise eine organische Säure wie Essigsäure, zu, bis ein pH-Wert von etwa 1 bis 4 erreicht ist, und rührt weitere 1 bis 4 h bei der Reaktionstemperatur. Das nach Abkühlen auf Raumtemperatur durch Verdünnen mit Wasser gefällte Reaktionsprodukt wird abfiltriert, mit heißem Wasser gewaschen und im Vakuum bei etwa 100°C getrocknet.

Wenn anstelle des jeweiligen Anhydrids das entsprechende Carbonsäuresalz isoliert werden soll, geht man zweckmäßigerweise so vor, daß man das Reaktionsgemisch nach der Verseifung nicht sauer stellt, sondern nur auf Raumtemperatur abkühlt, das ausgefallene Produkt abfiltriert, mit einem niederen aliphatischen Alkohol wie Isopropanol wäscht und bei etwa 100°C im Vakuum trocknet.

### A.1. Herstellung von Rylenderivaten der Formel Ia1

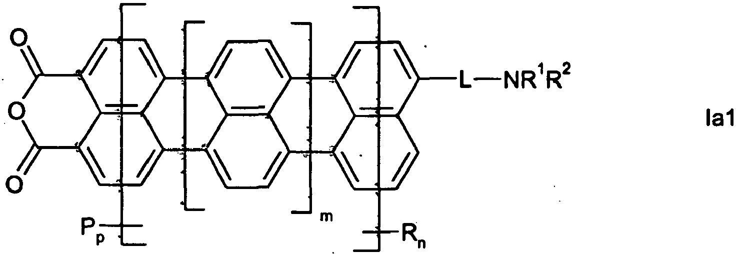

Die Herstellung der Rylenderivate Ia1 wird im folgenden in Abhängigkeit vom jeweiligen Brückenglied L abschnittsweise beschrieben.

### A.1.1. Herstellung von Rylenderivaten der Formel Ia11 (L = chemische Bindung)

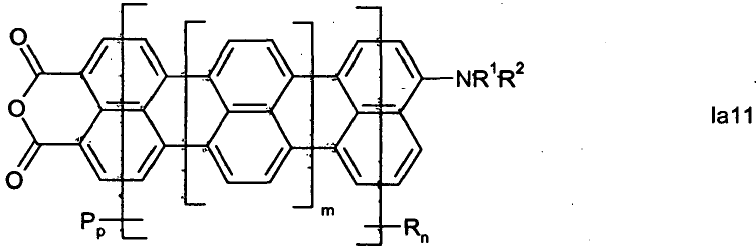

Zur Herstellung der Rylenderivate Ia11 kann man a) das peri-Halogenrylendicarbonsäureanhydrid IIa oder b) das peri-Halogenrylendicarbonsäureimid IIb in Gegenwart eines aprotischen organischen Lösungsmittels und gewünschtenfalls einer Base oder einer Kombination eines Übergangsmetallkatalysators und einer Base mit einem Amin der Formel IIIa

H-NR¹R² IIIa

umsetzen, wobei im Fall b) die oben beschriebene Verseifung anzuschließen ist. Die Verseifung und die nukleophile Substitution kann auch in einer Eintopf-Reaktion durchgeführt werden.

Wird die Umsetzung in Gegenwart des Übergangsmetallkatalysators vorgenommen, so kann in der Regel bei niedrigeren Reaktionstemperaturen und mit geringeren Mengen an Aminedukt IIIa gearbeitet werden.

Als organisches Lösungsmittel sind grundsätzlich alle unter den Reaktionsbedingungen stabilen aprotischen Lösungsmittel mit einem Siedepunkt oberhalb der gewählten Reaktionstemperatur geeignete, in den sich die Rylenedukte IIa bzw. IIb und das Amin IIIa vollständig und die gegebenenfalls verwendeten Katalysatoren und Basen zumindest partiell lösen, so daß weitgehend homogene Reaktionsbedingungen vorliegen. Es können sowohl polare als auch unpolare aprotische Lösungsmittel eingesetzt werden, wobei polare Lösungsmittel insbesondere dann bevorzugt sind, wenn kein Übergangsmetallkatalysator verwendet wird. Ist das eingesetzte Amin IIIa bei der Reaktionstemperatur flüssig, so kann es auch selbst als Reaktionsmedium dienen, und der Einsatz eines Lösungsmittels kann unterbleiben.

Beispiele für geeignete polare aprotische Lösungsmittel sind vor allem N,N-disubstituierte aliphatische Carbonsäureamide (insbesondere N,N-Di-C₁-C₄-alkyl-C₁-C₄-carbonsäureamide), stickstoffhaltige Heterocyclen ohne NH-Funktionen, Dimethylsulfoxid und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für besonders geeignete Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Dimethylsulfoxid; Tetrahydrofuran, Di- und Tetramethyltetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether, wobei Dimethylformamid und Tetrahydrofuran bevorzugt sind.

Beispiele für geeignete unpolare aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Undecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exxsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Im allgemeinen werden 10 bis 1000 ml, vorzugsweise 10 bis 500 ml, Lösungsmittel pro g Rylenedukt IIa bzw. IIb eingesetzt.

Als Basen eignen sich alkalimetall- und erdalkalimetallhaltige Basen, wobei die alkalimetallhaltigen Basen bevorzugt und die natrium- und kallumhaltigen Basen besonders bevorzugt sind. Dabei sind sowohl anorganische Basen, vor allem die Hydroxide, wie Natriumhydroxid und Kaliumhydroxid, und die Salze schwacher anorganischer Säuren, vor allem die Carbonate und Hydrogencarbonate, als auch organische Basen, vor allem die Alkoholate, wie Natriummethylat, Natrium-tert.-butylat, Kaliummethylat, Kaliumisopropylat und Kalium-tert.-butylat, und die Salze schwacher organischer Säuren, vor allem die Acetate, geeignet, die üblicherweise in wasserfreier Form eingesetzt werden.

Bei Reaktionsführung in Gegenwart eines Übergangsmetallkatalysators sind starke Basen, insbesondere die Alkoholate, wie Natrium- und Kalium-tert.-butylat, bevorzugt, während bei Abwesenheit des Katalysators schwache nicht-nucleophile Basen, vor allem die Salz schwacher Säuren, vorzugsweise die Carbonate, wie Natriumcarbonat, besonders geeignet sind.

In der Regel kommen 0,1 bis 10 mol, vorzugsweise 0,5 bis 3 mol mol, Base pro mol Rylenedukt IIa bzw. IIb zum Einsatz.

Als Übergangsmetallkatalysator eignen sich insbesondere Palladiumkomplexe, die in Kombination mit freien Ligandmolekülen eingesetzt werden können, wie Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)ferrocen]-palladium(II)chlorid, Bis(triethylphosphin)palladium(II)chlorid, Bis(tricyclohexylphosphin)palladium(II)acetat, (2,2'-Bipyridyl)palladium(II)chlorid, Bis(triphenylphosphin)-palladium(II)chlorid, Tris(dibenzylidenaceton)dipalladium(0), 1,5-Cyclooctadienpalladium(II)chlorid, Bis(acetonitril)palladium(II)chlorid und Bis(benzonitril)palladium(II)-chlorid, wobei [1,1'-Bis(diphenylphosphino)ferrocen]palladium(II)chlorid, Tetrakis(triphenylphosphin)palladium(0) und Tris(dibenzylidenaceton)dipalladium(0) bevorzugt sind.

Üblicherweise wird der Übergangsmetallkatalysator in einer Menge von 1 bis 20 mol-%, vor allem 2 bis 10 mol-%, bezogen auf IIa oder IIb, eingesetzt.

In der Regel empfiehlt sich die gleichzeitige Anwesenheit freier Ligandmoleküle, z.B. von Tri(tert.-butyl)phosphin, Triphenylphosphin und Tris(o-tolyl)phosphin und 1,1'-(2,2'-Diphenylphosphino)binaphthalin (BINAP). Übliche Mengen liegen bei 80 bis 500 mol-%, bevorzugt 100 bis 300 mol-%, bezogen auf den Übergangsmetallkatalysator.

Das Molverhältnis von Aminedukt IIIa zu Rylenedukt IIa bzw. IIb beträgt bei Anwesenheit eines Übergangsmetallkatalysators im allgemeinen 5 : 1 bis 1 : 1, insbesondere 3 : 1 bis 2 : 1, und bei seiner Abwesenheit in der Regel 200 : 1 bis 1 : 1, vor allem 50 : 1 bis 20 : 1. Dient das Amindedukt IIIa gleichzeitig als Reaktionsmedium, entfällt diese mengenmäßige Beschränkung.

Die Reaktionstemperatur liegt in Gegenwart des Katalysators üblicherweise bei 25 bis 100°C, vorzugsweise bei 70 bis 90°C, und bei seiner Abwesenheit bei 25 bis 200°C, bevorzugt bei 70 bis 170°C.

Insbesondere bei Einsatz des Übergangskatalysators empfiehlt es sich, die Umsetzung unter Schutzgas vorzunehmen.

Die Reaktionszeiten liegen bei Anwesenheit des Katalysators üblicherweise bei 1 bis 48 h, insbesondere bei 10 bis 20 h, und in Abwesenheit des Katalysators in der Regel bei 0,5 bis 24 h, vor allem bei 1 bis 3 h.

### A.1.2. Herstellung von Rylenderivaten der Formel Ia 12 (L = -Ar- oder -Ar-E-Ar-)

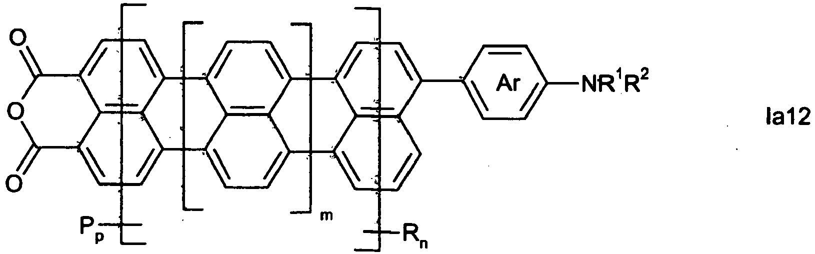

Der Phenylenring Ar stellt hierbei den Platzhalter für die (Het)Arylenreste -Ar- und -Ar-E-Ar- dar.

Zur Herstellung der Rylenderivate Ia12 kann man a) das peri-Halogenrylendicarbonsäureanhydrid IIa oder b) das peri-Halogenrylendicarbonsäureimid IIb in Gegenwart eines organischen Lösungsmittels, gewünschtenfalls im Gemisch mit Wasser, sowie eines Übergangsmetallkatalysators und einer Base einer Suzuki-Kupplungsreaktion mit einem dioxaborolanylsubstitutierten Amin der Formel IIIb 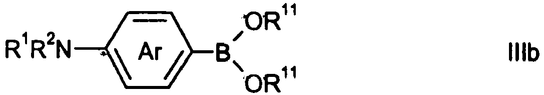 in der die Reste R¹¹ gleich oder verschieden sind und unabhängig voneinander und unabhängig voneinander Wasserstoff, C₁-C₃₀-Alkyl, C₅-C₈-Cycloalkyl oder (Het)Aryl bedeuten, wobei die an jeweils einem Boratom befindlichen Reste R¹¹ auch unter Ausbildung eines die beiden Sauerstoffatome sowie das Boratom enthaltenden Fünf- oder Sechsrings, der an den Kohlenstoffatomen durch bis zu 4 C₁-C₃₀-Alkyl-, C₅-C₈-Cycloalkyl- oder (Het)Arylgruppen substituiert sein kann, miteinander verbunden sein können,
umsetzen, wobei im Fall b) die oben beschriebene Verseifung anzuschließen ist.

Die hierbei als Aminedukt eingesetzten dioxaborolanylsubstitutierten Amine IIIb sind durch Umsetzung der entsprechenden bromierten Amine IIIc 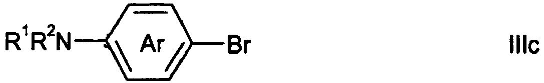 mit Diboranen der Formel IV 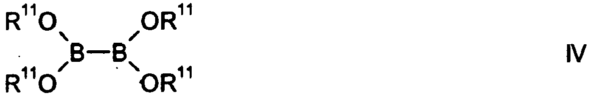 in Gegenwart eines aprotischen organischen Lösungsmittels, eines Übergangsmetallkatalysators und einer Base erhältlich.

Man kann jedoch auch das peri-Halogenrylendicarbonsäureimid IIb durch analoge Umsetzung mit dem Diboran IV in das peri-Dioxaborolanylderivat IIb" 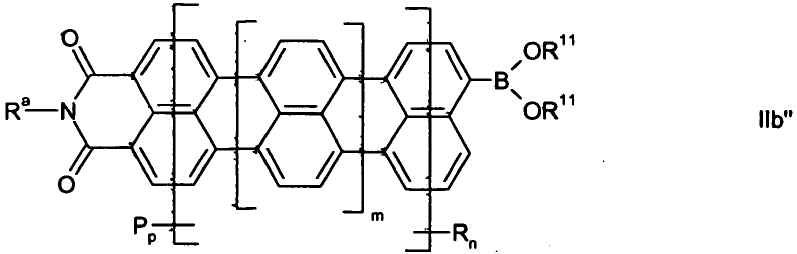 überführen und dieses dann der Suzuki-Kupplung mit dem bromierten Amin IIIc unterziehen.

Für die Herstellung der Dioxaboranylderivate IIIb bzw. IIb' eignen sich als Diborane IV insbesondere Bis(1,2- und 1,3-diolato)diborane, Tetraalkoxydiborane, Tetracycloalkoxydiborane und Tetra(het)aryloxydiborane sowie deren Mischformen. Als Beispiele für diese Verbindungen seien genannt: Bis(pinacolato)diboran, Bis(1,2-benzoldiolato)-diboran, Bis(2,2-dimethyl-1,3-propandiolato)diboran, Bis(1,1,3,3-tetramethyl-1,3-propandiolato)diboran, Bis(4,5-pinandiolato)diboran, Bis(tetramethoxy)diboran, Bis(tetracyclopentoxy)diboran, Bis(tetraphenoxy)diboran und Bis(4-pyridiyloxy)diboran.

Bevorzugt sind Diborane IV, bei denen die beiden an einem Boratom befindlichen Reste R¹¹ unter Ausbildung eines die beiden Sauerstoffatome sowie das Boratom enthaltenden Fünf- oder Sechsrings miteinander verbunden sind. An die gebildeten Fünf- oder Sechsringe können aromatische oder gesättigte, auch bicyclische, Ringe, mit 5 bis 7 C-Atomen als Ringgliedern anneliert sein. Alle Ringe bzw. Ringsysteme können durch bis zu 4 C₁-C₃₀-Alkyl-, C₅-C₈-Cycloalkyl-, Aryl- und/oder Hetarylreste substituiert sein, vorzugsweise sind sie durch bis zu 4 C₁-C₄-Alkylreste substituiert. Beispiele für diese bevorzugten Diborane sind die bereits oben genannten Bis(1,2- und 1,3-diolato)-diborane, wobei Bis(pinacolato)diboran besonders bevorzugt ist.

Das Molverhältnis Diboran IV zum Edukt IIIc bzw. IIb liegt dabei im allgemeinen bei 0,8 : 1 bis 3 : 1, insbesondere bei 1,5 : 1 bis 2 : 1.

Als Lösungsmittel sind grundsätzlich alle unter den Reaktionsbedingungen gegen Basen stabilen aprotischen Lösungsmittel mit einem Siedepunkt oberhalb der gewählten Reaktionstemperatur geeignet, in denen sich die Edukte bei Reaktionstemperatur vollständig und die verwendeten Katalysatoren und Basen zumindest partiell lösen, so daß weitgehend homogene Reaktionsbedingungen vorliegen. Es können sowohl unpolare aprotische als auch polare aprotische Lösungsmittel eingesetzt werden, wobei die unpolaren aprotischen Lösungsmittel, insbesondere Toluol, bevorzugt sind.

Beispiele für weitere besonders geeignete unpolare und polare aprotische Lösungsmittel sind die in Abschnitt A.1.1 aufgeführten Lösungsmittel.

Die Lösungsmittelmenge beträgt in der Regel 10 bis 1000 ml, bevorzugt 20 bis 300 ml, pro g IIIc bzw. IIb.

Als Übergangsmetallkatalysator eignen sich insbesondere die in Abschnitt A.1.1 genannten Palladiumkomplexe, die wiederum in der Regel in Mengen von 1 bis 20 mol-%, vor allem 2 bis 10 mol-%, bezogen auf IIIc bzw. IIb, eingesetzt werden. Die zusätzliche Anwesenheit von freien Ligandmolekülen ist üblicherweise nicht erforderlich.

Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonat, zum Einsatz. Bevorzugte Basen sind die Acetate, vor allem Kaliumacetat.

Im allgemeinen werden 1 bis 5 mol, bevorzugt 2 bis 4 mol, Base pro mol IIIc bzw. IIb verwendet.

Die Reaktionstemperatur liegt üblicherweise bei 20 bis 180°C, vor allem bei 60 bis 120°C.

Die Reaktionszeit beträgt in der Regel 0,5 bis 30 h, insbesondere 1 bis 20 h.

Verfahrenstechnisch geht man bei der Herstellung der Dioxaboranylderivate zweckmäßigerweise wie folgt vor:
Man legt Edukt IIIc bzw. IIb und Lösungsmittel vor, gibt Diboran IV, den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Suzuki-Kupplung zwischen dem peri-Halogenrylendicarbonsäurederivat IIa bzw. IIb und dem dioxaborolanylsubstitutierten Amin IIIb bzw. zwischen dem Dioxaborolanylrylenderivat IIb' und dem p-Bromphenylamin IIIc wird in Gegenwart eines organischen Lösungsmittels, gewünschtenfalls im Gemisch mit Wasser, sowie eines Übergangsmetallkatalysators und einer Base durchgeführt.

Das Molverhältnis von IIIb zu IIa bzw. IIb beträgt dabei in der Regel 0,8 : 1 bis 3 : 1, vorzugsweise 0,9 : 1 bis 2 : 1. Das Molverhältnis von IIb' zu IIIc liegt im allgemeinen bei 0,8 : 1 bis 3 : 1, bevorzugt bei 1,5 : 1 bis 2,5 : 1.

Als Lösungsmittel eignen sich für die Suzuki-Kupplung alle Lösungsmittel, in denen sich die Edukte bei Reaktionstemperatur vollständig und die verwendeten Katalysatoren und Basen zumindest partiell lösen, so daß weitgehend homogene Reaktionsbedingungen vorliegen. Insbesondere geeignet sind die vorstehend für die Herstellung der Dioxaborolanylderivate genannten Lösungsmittel, wobei auch hier die alkylsubstituierten Benzole bevorzugt sind. Die Lösungsmittelmenge liegt üblicherweise bei 10 bis 1000 ml, vorzugsweise bei 20 bis 100 ml, pro g Rylenderivat.

Vorzugsweise setzt man Wasser als zusätzliches Lösungsmittel ein. In diesem Fall werden in der Regel 10 bis 1000 ml, insbesondere 250 bis 500 ml, Wasser pro I organisches Lösungsmittel verwendet.

Als Übergangsmetallkatalysatoren werden wiederum vorzugsweise Palladiumkomplexe eingesetzt, wobei dieselben Bevorzugungen gelten. Die Einsatzmenge Katalysator beträgt üblicherweise 1 bis 20 mol-%, insbesondere 1,5 bis 5 mol-%, bezogen auf das Rylenderivat.

Als Base sind wiederum die Alkalimetallsalze schwacher Säuren bevorzugt, wobei die Carbonate, wie Natriumcarbonat und vor allem Kaliumcarbonat besonders bevorzugt sind. In der Regel liegt die Basenmenge bei 0,1 bis 10 mol, insbesondere bei 0,2 bis 5 mol, pro mol Rylenderivat.

Die Reaktionstemperatur beträgt im allgemeinen 20 bis 180°C, bevorzugt 60 bis 120°C. Wird in Schritt b) Wasser eingesetzt, so empfiehlt es sich, die Umsetzung nicht bei Temperaturen über 100°C vorzunehmen, da ansonsten unter Druck gearbeitet werden müßte.

Die Reaktion ist üblicherweise in 0,5 bis 48 h, insbesondere in 5 bis 20 h, beendet.

Verfahrenstechnisch geht man bei der Suzuki-Kupplung zweckmäßigerweise wie folgt vor:
Man legt die Edukte sowie Lösungsmittel vor, gibt Übergangsmetallkatalysator und die vorzugsweise in Wasser oder einem Wasser/Alkohol-Gemisch gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

### A.1.3. Herstellung von Rylenderivaten der Formel Ia13(L = über Ethenylen verknüpfter (Het)Arylenrest -Ar- oder -Ar-E-Ar-)

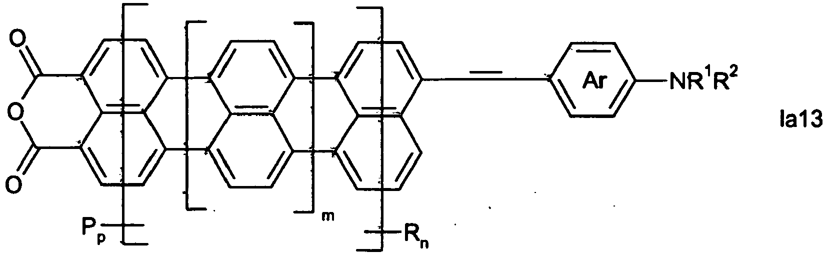

Der Phenylenring Ar stellt hierbei wiederum den Platzhalter für die (Het)Arylenreste -Ar- und -Ar-E-Ar- dar.

Zur Herstellung der Rylenderivate Ia13 kann man a) das peri-Halogenrylendicarbonsäureanhydrid IIa oder b) das peri-Halogenrylendicarbonsäureimid IIb in Gegenwart eines aprotischen organischen Lösungsmittels, eines Übergangsmetallkatalysators, einer Base und gewünschtenfalls eines Cokatalysators in einer Heck-Reaktion mit einem Vinylamin der Formel IIId 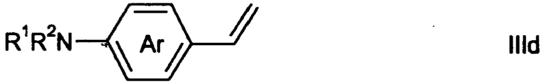 umsetzen, wobei im Fall b) die oben beschriebene Verseifung anzuschließen ist.

Als Lösungsmittel eignen sich für diese Umsetzung insbesondere polare, aber auch unpolare aprotische organische Lösungsmittel. Neben den in Abschnitt A.1.1 aufgeführten Lösungsmitteln sind z.B. auch halogenierte Kohlenwasserstoffe und aliphatische Amine als Lösungsmittel geeignet. Als Beispiele für besonderes geeignete Lösungsmittel seien im einzelnen genannt: Diethylamin, Piperidin, Tetrahydrofuran, Dimethylformamid, N-Methylpyrrolidon, Methylenchlorid, Dimethylsulfoxid und Toluol.

Als Übergangsmetallkatalysator sind die ebenfalls in Abschnitt A.1.1 aufgeführten Palladiumverbindungen geeignet, die auch in situ aus Palladiumsalzen, wie Palladium(II)-acetat, und Ligandmolekülen hergestellt werden können. Generell empfiehlt sich auch bei direktem Einsatz von Palladiumkomplexen die gleichzeitige Anwesenheit freier Ligandmoleküle. Beispiele für besonders bevorzugte Übergangskatalysatorsysteme sind Tetrakis(triphenylphosphin)palladium(0), Tris(dibenzylidenaceton)dipalladium(0) und Palladium(II)acetat sowie Triphenylphosphin und Tris(o-tolyl)phosphin.

Als Cokatalysatoren können Alkalimetallhalogenide und quaternäre Ammoniumsalze zum Einsatz kommen. Beispielhaft genannt seien Lithiumchlorid, Tetrabutylammoniumchlorid, -bromid und -hydrogensulfat.

Als Basen eignen sich neben den Alkalimetallsalzen, insbesondere den Natrium- und Kaliumsalzen, schwacher anorganischer und organischer Säuren auch tertiäre Amine. Beispiele für besonders geeignete Basen sind Natriumhydrogencarbonat, Natriumcarbonat, Kaliumcarbonat, Natriumacetat, Kaliumacetat und Tri(C₂-C₄-alkyl)amine, wie Triethylamin.

Die Reaktionstemperaturen liegen üblicherweise bei 0 bis 150°C, vorzugsweise 25 bis 80°C.

Es empfiehlt sich, unter Schutzgas zu arbeiten.

Die Umsetzung ist in der Regel in 1 bis 24 h beendet.

Weitere Einzelheiten zu dieser Umsetzung sind Chem. Rev. 100, S. 3009-3068 (2000) zu entnehmen.

### A.1.4. Herstellung von Rylenderivaten der Formel Ia14 (L = über Ethinylen verknüpfter (Het)Arylenrest -Ar- oder -Ar-E-Ar-)

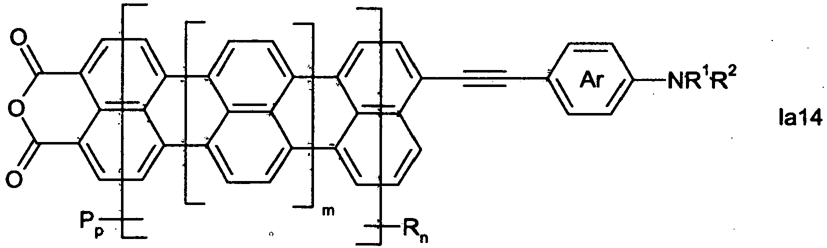

Der Phenylenring Ar stellt hierbei wiederum den Platzhalter für die (Het)Arylenreste -Ar- und -Ar-E-Ar- dar.

Zur Herstellung der Rylenderivate Ia14 kann man a) das peri-Halogenrylendicarbonsäureanhydrid IIa oder b) das peri-Halogenrylendicarbonsäureimid IIb in Gegenwart eines aprotischen organischen Lösungsmittels, eines Übergangsmetallkatalysators, einer Base und gewünschtenfalls eines Kupfer-Cokatalysators mit einem Ethinylamin der Formel IIIe 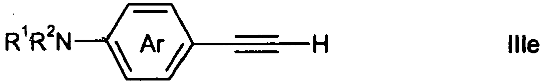 umsetzen, wobei im Fall b) die oben beschriebene Verseifung anzuschließen ist.

Als Lösungsmittel eignen sich für diese Umsetzung insbesondere polare, aber auch unpolare organische Lösungsmittel. Neben den in Abschnitt A.1.1 aufgeführten Lösungsmitteln sind z.B. auch halogenierte Kohlenwasserstoffe und Amine als Lösungsmittel geeignet. Als Beispiele für besonderes geeignete Lösungsmittel seien im einzelnen genannt: Diethylamin, Piperidin, Tetrahydrofuran, Dimethylformamid, Methylenchlorid und Toluol.

Als Übergangsmetallkatalysator sind die ebenfalls in Abschnitt A.1.1 aufgeführten Palladiumkomplexe geeignet, wobei Tetrakis(triphenylphosphin)palladium(0) und Bis(triphenylphosphin)palladium(II)chlorid bevorzugt sind.

Als Cokatalysatoren können Kupfer(I)- und Kupfer(II)verbindungen eingesetzt werden. Neben den Oxiden eignen sich Kupfersalze organischer und insbesondere anorganischer Säuren, vor allem Kupfer(I)iodid.

Als Basen eignen sich neben den Alkalimetallsalzen, insbesondere den Caesiumsalzen, schwacher anorganischer und organischer Säuren auch sekundäre und teriäre Amine. Beispiele für besonders geeignete Basen sind Caesiumcarbonat, Di(C₂-C₄-alkyl)amine, wie Diethylamin und Diisopropylamin, Tri(C₂-C₄-alkyl)amine, wie Triethylamin, und cyclische Amine, wie Piperidin.

Die Reaktionstemperaturen liegen üblicherweise bei 0 bis 150°C, vorzugsweise 25 bis 80°C.

Es empfiehlt sich, unter Schutzgas zu arbeiten.

Die Umsetzung ist in der Regel in 1 bis 24 h beendet.

Weitere Einzelheiten zu dieser Umsetzung sind Chem. Rev. 103, S. 1979-2017 (2003) zu entnehmen.

### A.2. Herstellung von Rylenderivaten der Formet Ia2

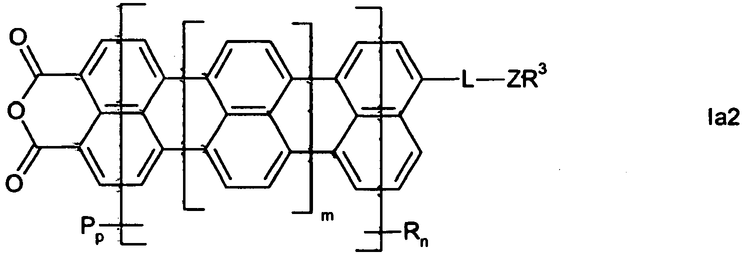

Die Herstellung der Rylenderivate Ia2 wird im folgenden in Abhängigkeit vom jeweiligen Brückenglied L abschnittsweise beschrieben.

### A.2.1. Herstellung von Rylenderivaten der Formel Ia21 (L = chemische Bindung)

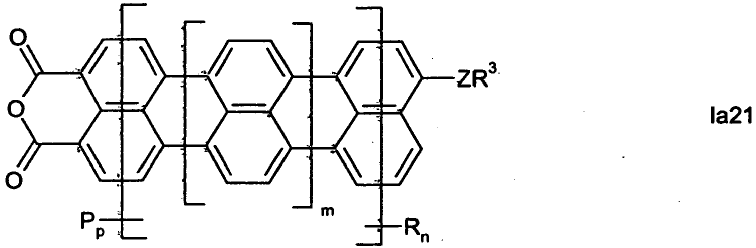

Zur Herstellung der Rylenderivate Ia21 kann man a) das peri-Halogenrylendicarbonsäureanhydrid IIa oder b) das peri-Halogenrylendicarbonsäureimid IIb in Gegenwart eines nichtnucleophilen Lösungsmittels und einer Base mit einem (Thio)Alkohol der Formel Va

H-ZR³ Va

umsetzen, wobei im Fall b) die oben beschriebene Verseifung anzuschließen ist.

Als nichtnucleophile Lösungsmittel eignen sich für diese Umsetzung vor allem polare aprotische Lösungsmittel, insbesondere aliphatische Carbonsäureamide (vorzugsweise N,N-Di-C₁-C₄-alkyl-C₁-C₄-carbonsäureamide) und Lactame, wie Dimethylformamid, Diethylformamid, Dimethylacetamid, Dimethylbutyramid und N-Methylpyrrolidon.

Als nichtnucleophile Lösungsmittel können auch unpolare aprotische Lösungsmittel eingesetzt werden, diese Lösungsmittel sind jedoch nicht bevorzugt. Beispielhaft seien aromatische Kohlenwasserstoffe, wie Benzol, Toluol und Xylol, genannt.

Die Lösungsmittelmenge hängt von der Löslichkeit des halogenierten Rylenderivats ab. In der Regel werden 2 bis 200 ml, insbesondere 3 bis 150 ml Lösungsmittel, pro g Rylenedukt IIa bzw. IIb benötigt.

Als Base eignen sich vor allem anorganische und organische alkali- oder erdalkalimetallhaltige Basen, wobei die alkalimetallhaltigen Basen besonders geeignet sind. Beispiele für anorganische Basen sind Alkali- und Erdalkalimetallcarbonate und -hydrogencarbonate, -hydroxide, -hydride und -amide, Beispiele für organische Basen sind Alkali- und Erdalkalimetallalkoholate (insbesondere die C₁-C₁₀-Alkoholate, vor allem tert.-C₄-C₆-Alkoholate), -(phenyl)alkylamide (insbesondere die Bis(C₁-C₄-alkyl)amide) und Triphenylmethylmetallate. Bevorzugte Basen sind die Carbonate und Hydrogencarbonate, wobei die Carbonate besonders bevorzugt sind. Bevorzugte Alkalimetalle sind Lithium, Natrium, Kalium und Caesium, besonders geeignete Erdalkalimetalle sind Magnesium und Calcium.

Als Beispiele für die metallhaltigen Basen seien im einzelnen genannt: Lithiumcarbonat, Natriumcarbonat, Kaliumcarbonat und Caesiumcarbonat; Natriumhydrogencarbonat und Kaliumhydrogencarbonat; Lithiumhydroxid, Natriumhydroxid, Kaliumhydroxid und Caesiumhydroxid; Lithiumhydrid, Natriumhydrid und Kaliumhydrid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat, Kalium-tert.-butylat, Lithium-(1,1-dimethyl)octylat, Natrium-(1,1-dimethyl)octylat und Kalium-(1,1-dimethyl)octylat; Lithiumdimethylamid, Lithiumdiethylamid, Lithiumdiisopropylamid, Natriumdiisopropylamid, Lithiumhexamethyldisilazid, Natriumhexamethyldisilazid, Kaliumhexamethyldisilazid Triphenylmethyllithium, Triphenylmethylnatrium und Triphenylmethylkalium.

Neben diesen metallhaltigen Basen eignen sich auch rein organische stickstoffhaltige Basen.

Geeignete Beispiele hierfür sind Alkylamine, insbesondere Tri(C₂-C₆-alkyl)amine, wie Triethylamin, Tripropylamin und Tributylamin, Alkoholamine, insbesondere Mono-, Oi- und Tri(C₂-C₄-alkohol)amine, wie Mono-, Di- und Triethanolamin, und heterocyclische Basen, wie Pyridin, 4-(N,N-Dimethylamino)pyridin, (4-Pyrrolidino)pyridin, N-Methylpiperidin, N-Methylpiperidon, N-Methylmorpholin, N-Methyl-2-pyrrolidon, Pyrimidin, Chinolin, Isochinolin, Chinaldin, Diazabicyclooctan (DABCrO), Diazabicyclononen (DBN) und Diazabicycloundecen (DBU).

Ganz besonders bevorzugte Basen sind Lithiumcarbonat, Natriumcarbonat, Kaliumcarbonat und Caesiumcarbonat.

In der Regel werden mindestens 0,4 Äquivalente Base pro mol (Thio)Alkohol Va benötigt. Besonders geeignete Einsatzmengen betragen für die Metallbasen 0,4 bis 3, insbesondere 0,4 bis 1,2, Äquivalente pro mol Va. Bei den rein organischen Basen liegt die Einsatzmenge bevorzugt bei 0,4 bis 10, besonders bevorzugt bei 0,4 bis 3, Äquivalenten pro mol Va. Wird die organische Base gleichzeitig als Lösungsmittel eingesetzt, was insbesondere bei den heterocyclischen Basen der Fall sein kann, ist eine mengenmäßige Beschränkung selbstverständlich überflüssig.

Die Umsetzung kann in Gegenwart von Phasentransferkatalysatoren vorgenommen werden.

Als Phasentransferkatalysatoren sind vor allem quartäre Ammoniumsalze und Phosphoniumsalze, wie Tetra(C₁-C₁₈-alkyl)ammoniumhalogenide und -tetrafluoroborate, Benzyltri(C₁-C₁₈-alkyl)ammoniumhalogenide und -tetrafluoroborate und Tetra(C₁-C₁₈-alkyl)- und Tetraphenylphosphoniumhalogenide, und Kronenether geeignet. Bei den Halogeniden handelt es sich in der Regel um die Fluoride, Chloride, Bromide und Iodide, wobei die Chloride und Bromide bevorzugt sind. Besonders geeignete Beispiele sind im einzelnen: Tetraethylammoniumchlorid, Tetrabutylammoniumbromid, Tetrabutylammoniumiodid, Tetrabutylammoniumtetrafluoroborat und Benzyltriethylammmoniumchlorid; Tetrabutylphosphoniumbromid und Tetraphenylphosphoniumchlorid und -bromid; 18-Krone-6, 12-Krone-4 und 15-Krone-5.

Wird ein Phasentransferkatalysator verwendet, so liegt seine Einsatzmenge üblicherweise bei 0,4 bis 10, insbesondere 0,4 bis 3, Äquivalenten pro mol (Thio)Alkohol Va. In der Regel werden 1 bis 10 mol, vorzugsweise 1 bis 5 mol, (Thio)Alkohol Va pro mol Rylenedukt IIa bzw. IIb eingesetzt.

Die Reaktionstemperatur hängt von der Reaktivität des Substrats ab und liegt im allgemeinen im Bereich von 30 bis 150°C.

Die Reaktionszeit beträgt üblicherweise 0,5 bis 96 h, insbesondere 2 bis 72 h.

### A.2.2. Herstellung von Rylenderivaten der Formel Ia22 (L = -Ar- oder -Ar-E-Ar-)

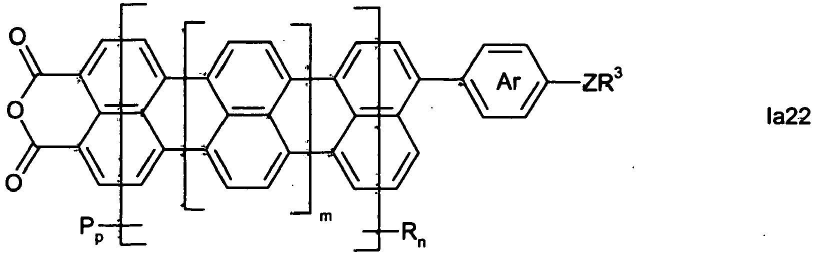

Der Phenylenring Ar stellt hierbei den Platzhalter für die (Het)Arylenreste -Ar- und -Ar-E-Ar- dar.

Die Herstellung der Rylenderivate Ia22 kann analog zu der in Abschnitt A.1.2 beschriebenen Herstellung der Rylenderivate Ia12 vorgenommen werden.

Dementsprechend kann man a) das peri-Halogenrylendicarbonsäureanhydrid IIa oder b) das peri-Halogenrylendicarbonsäureimid IIb in Gegenwart eines organischen Lösungsmittels, gewünschtenfalls im Gemisch mit Wasser, sowie eines Übergangsmetallkatalysators und einer Base einer Suzuki-Kupplungsreaktion mit einem dioxaborolanylsubstitutierten (Thio)Alkohol der Formel Vb 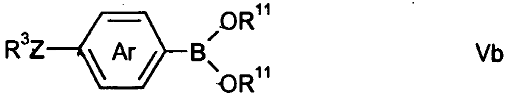 in der die Reste R¹¹ gleich oder verschieden sind und unabhängig voneinander und unabhängig voneinander Wasserstoff, C₁-C₃₀-Alkyl, C₅-C₈-Cycloalkyl oder (Het)Aryl bedeuten, wobei die an jeweils einem Boratom befindlichen Reste R¹¹ auch unter Ausbildung eines die beiden Sauerstoffatome sowie das Boratom enthaltenden Fünf- oder Sechsrings, der an den Kohlenstoffatomen durch bis zu 4 C₁-C₃₀-Alkyl-, C₅-C₈-Cycloalkyl- oder (Het)Arylgruppen substituiert sein kann, miteinander verbunden sein können,
umsetzen, wobei im Fall b) die oben beschriebene Verseifung anzuschließen ist.

Die hierbei als Aminedukt eingesetzten dioxaborolanylsubstitutierten (Thio)Alkohole Vb sind analog zu den dioxaborolanylsubstituierten Aminen IIIc durch Umsetzung der entsprechenden bromierten (Thio)Alkohole Vc 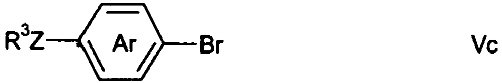 mit Diboranen der Formel IV 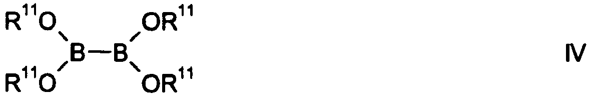 in Gegenwart eines aprotischen organischen Lösungsmittels, eines Übergangsmetallkatalysators und einer Base erhältlich.

Man kann jedoch auch hier das peri-Halogenrylendicarbonsäureimid IIb durch analoge Umsetzung mit dem Diboran IV in das peri-Dioxaborolanylderivat IIb" 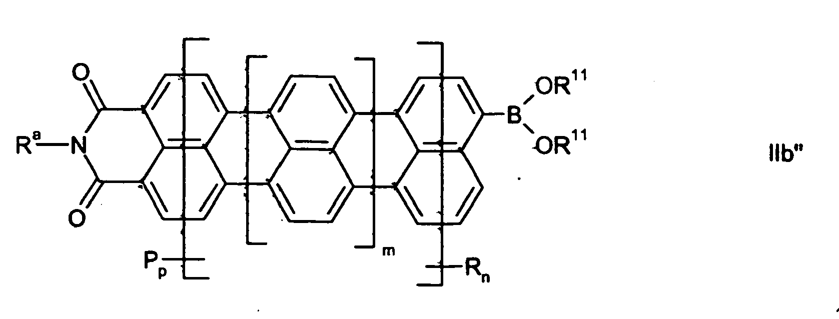 überführen und dieses dann der Suzuki-Kupplung mit dem bromierten (Thio)Alkohol Vc unterziehen.

Weitere Einzelheiten zu diesen Umsetzungen können der Beschreibung der analogen Umsetzungen in Abschnitt A.1.2 entnommen werden.

### A.2.3. Herstellung von Rylenderivaten der Formel Ia23 (L = über Ethenylen verknüpfter (Het)Arylenrest -Ar- oder -Ar-E-Ar-)

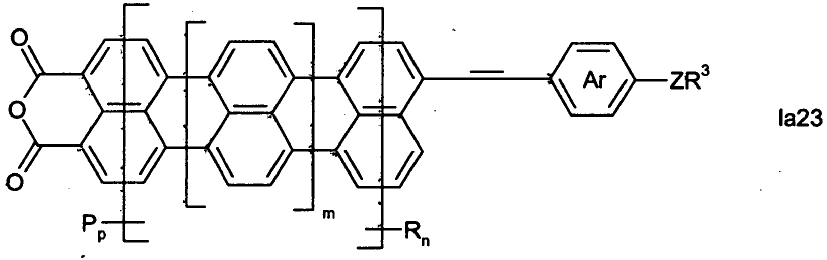

Der Phenylenring Ar stellt hierbei wiederum den Platzhalter für die (Het)Arylenreste -Ar- und -Ar-E-Ar- dar.

Die Herstellung der Rylenderivate Ia23 kann analog zu der in Abschnitt A.1.3 beschriebenen Herstellung der Rylenderivate Ia13 vorgenommen werden.

Dementsprechend kann man a) das peri-Halogenrylendicarbonsäureanhydrid IIa oder b) das peri-Halogenrylendicarbonsäureimid IIb in Gegenwart eines aprotischen organischen Lösungsmittels, eines Übergangsmetallkatalysators, einer Base und gewünschtenfalls eines Cokatalysators in einer Heck-Reaktion mit einem Vinyl(thio)alkohol der Formel Vd 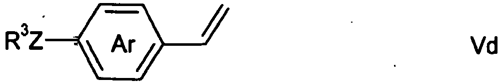 umsetzen, wobei im Fall b) die oben beschriebene Verseifung anzuschließen ist.

Weitere Einzelheiten zu dieser Umsetzung sind Abschnitt A.1.3 zu entnehmen.

### A.2.4. Herstellung von Rylenderivaten Ia24 (L = über Ethinylen verknüpfter (Het)Arylenrest -Ar- oder -Ar-E-Ar-)

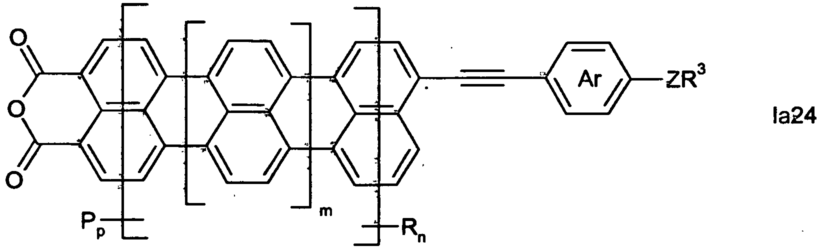

Der Phenylenring Ar stellt hierbei wiederum den Platzhalter für die (Het)Arylenreste -Ar- und -Ar-E-Ar- dar.

Die Herstellung der Rylenderivate Ia24 kann wiederum analog zu der in Abschnitt A.1.4 beschriebenen Herstellung der Rylenderivate Ia14 vorgenommen werden.

Dementsprechend kann man a) das peri-Halogenrylendicarbonsäureanhydrid IIa oder b) das peri-Halogenrylendicarbonsäureimid IIb in Gegenwart eines aprotischen organischen Lösungsmittels, eines Übergangsmetallkatalysators, einer Base und gewünschtenfalls eines Kupfer-Cokatalysators mit einem Ethinyl(thio)alkohol der Formel Ve 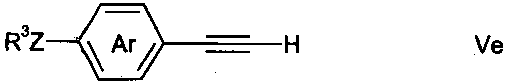 umsetzen, wobei im Fall b) die oben beschriebene Verseifung anzuschließen ist.

Weitere Einzelheiten zu dieser Umsetzung sind Abschnitt A.1.4 zu entnehmen.

### A.3. Herstellung von Rylenderivaten der Formel Ia3

Zur Herstellung der Rylentetracarbonsäuremonoimidmonoanhydride Ia3 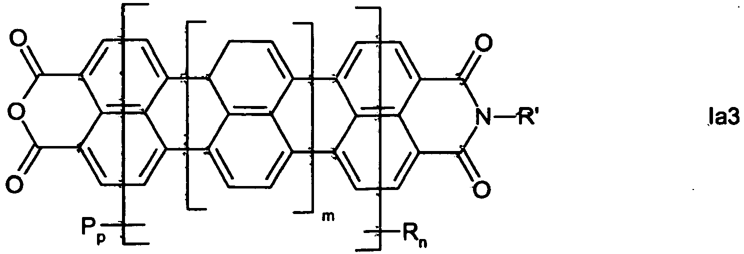 kann man ein Rylentetracarbonsäuredianhydrid der Formel IIc 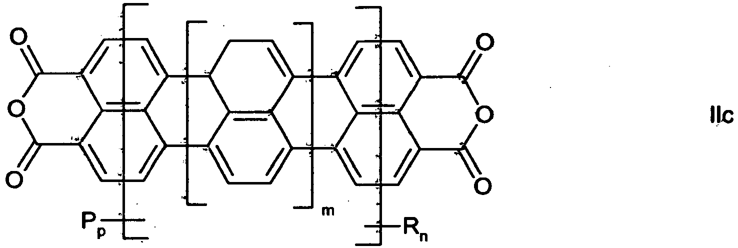 in Gegenwart eines polaren aprotischen Lösungsmittels und eines Imidierungskatalysators mit einem primären Amin der Formel VI

R'-NH₂ VI

umsetzen.

Als polares aprotisches Lösungsmittel eignen sich dabei vor allem aprotische Stickstoffheterocyclen, wie Pyridin, Pyrimidin, Imidazol, Chinolin, Isochinolin, Chinaldin, N-Methylpiperidin, N-Methylpiperidon und N-Methylpyrrolidon, Carbonsäureamide, wie Dimethylformamid und Dimethylacetamid, und Tetraalkylharnstoffe, wie Tetramethylharnstoff, wobei N-Methylpyrrolidon besonders bevorzugt ist.

In der Regel werden 2 bis 500 ml, vorzugsweise 2 bis 50 ml, Lösungsmittel pro g IIc eingesetzt.

Als Imidierungskatalysatoren eignen sich insbesondere Lewis-saure Salze organischer und anorganischer Säuren mit Metallen, wie Zink, Eisen, Kupfer und Magnesium, sowie die Oxide dieser Metalle, z.B. Zinkacetat, Zinkpropionat, Zinkoxid, Eisen(II)acetat, Eisen(III)chlorid, Eisen(II)sulfat, Kupfer(II)acetat, Kupfer(II)oxid und Magnesiumacetat, wobei Zinkacetat besonders bevorzugt ist. Die Salze werden vorzugsweise in wasserfreier Form eingesetzt.

Üblicherweise kommen 20 bis 500 mol-%, insbesondere 80 bis 120 mol-% Katalysator, bezogen auf IIc, zum Einsatz.

Man kann auch die Säuren selbst, also z.B. organische Säuren, insbesondere C₁-C₃-Carbonsäuren, wie Ameisensäure, Essigsäure und Propionsäure, und anorganische Säuren, wie Phosphorsäure, jeweils bevorzugt in möglichst konzentrierter Form, als Imidierungskatalysatoren einsetzen. Die Säuren dienen dabei gleichzeitig als Lösungsmittel oder als Cosolvens und kommen daher üblicherweise im Überschuß zum Einsatz.

Üblicherweise liegt das Molverhältnis von primärem Amin VI zu Rylentetracarbonsäuredianhydrid IIc bei etwa 0,1 : 1,5 bis 0,5 : 1,2, vor allem bei 0,8 : 1,5 bis 0,8 : 1,2.

Die Reaktionstemperatur beträgt im allgemeinen 60 bis 250°C, bevorzugt 100 bis 230°C, besonders bevorzugt 90 bis 170°C.

Es empfiehlt sich, die Umsetzung unter Schutzgas durchzuführen.

Die Reaktion ist üblicherweise in 1 bis 4 h abgeschlossen.

### B. Herstellung von Rylenderivaten Ib

Bei der Herstellung der Rylenderivate Ib, bei denen die beiden Reste X zu einem säuregruppenhaltigen Imid rest (x2) oder Kondensatrest (x3) verbunden sind, können vorteilhaft die Rylenderivate la als Edukt eingesetzt werden, die die jeweils gewünschten Reste Y aufweisen.

### B.1. Herstellung von Rylenderivaten der Formel Ib1

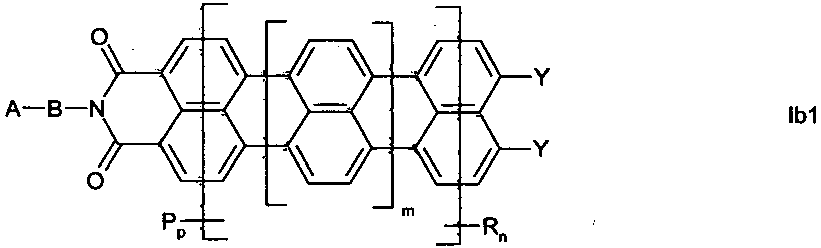

Die Herstellung der Rylenderivate Ib1 wird im folgenden in Abhängigkeit von den Substituenten Y abschnittsweise beschrieben.

### B.1.1. Herstellung von Rylenderivaten Ib11

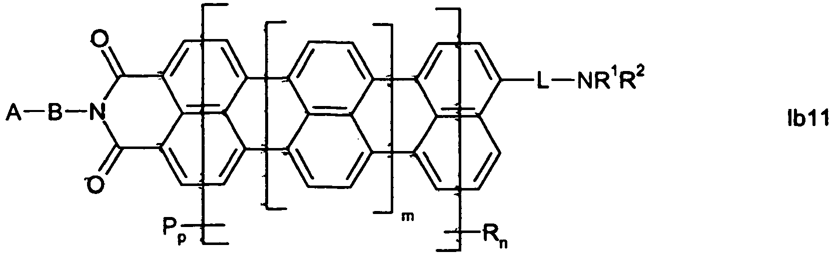

Die Herstellung der Rylenderivate Ib11 kann durch Umsetzung der entsprechenden Rylenderivate Ia1 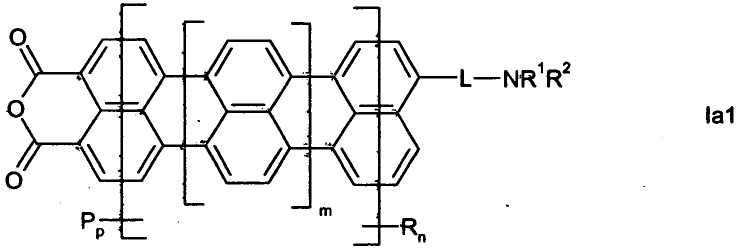 mit einem primären Amin der Formel VII

A-B-NH₂ VII

in Gegenwart eines polaren aprotischen Lösungsmittels und eines Imidierungskatalysators erfolgen.

Diese Imidierungsreaktion kann analog zu der in Abschnitt A.3 beschriebenen Imidierungsreaktion zur Herstellung der Rylentetracarbonsäuremonoimidmonoanhydride Ia3 vorgenommen werden.

Alternativ kann die Imidierung auch mit größeren Mengen an primärem Amin VI (in der Regel 1 bis 10 ml, vorzugsweise 2 bis 6 mol, pro mol Ia1) in Gegenwart des polaren aprotischen Lösungsmittels und einer alkalimetall- oder erdalkalimetallhaltigen Base durchgeführt werden.

Bevorzugt sind hierbei die alkalimetallhaltigen Basen, insbesondere die natrium- und kaliumhaltigen Basen. Es sind sowohl anorganische Basen, vor allem die Hydroxide, wie Natriumhydroxid und Kaliumhydroxid, als auch organische Basen, vor allem die Alkoholate, wie Natriummethylat, Kaliummethylat, Kaliumisopropylat und Kalium-tert.-butylat, geeignet, die üblicherweise in wasserfreier Form eingesetzt werden. Ganz besonders bevorzugt ist Kaliumhydroxid.

In der Regel werden 1 bis 10 mol, bevorzugt 2 bis 4 mol, Base, pro mol 1a1 benötigt.

Die Reaktionstemperatur beträgt im allgemeinen 50 bis 200°C, vorzugsweise 60 bis 150°C.

Übliche Reaktionsdauern sind 0,5 bis 24 h, vor allem 2 bis 19 h.

### B.1.2. Herstellung von Rylenderivaten der Formel Ib12

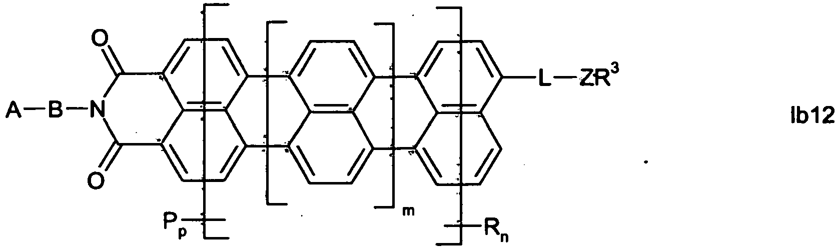

Die Herstellung der Rylenderivate Ib12 kann analog durch Umsetzung der entsprechenden Rylenderivate Ia2 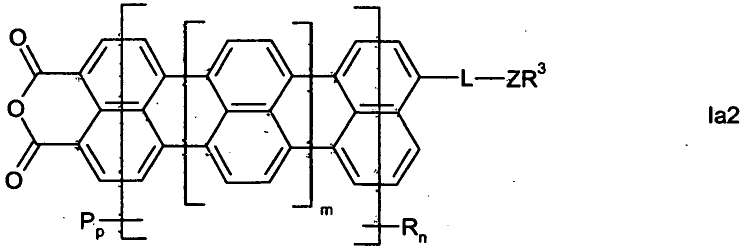 mit einem primären Amin der Formel VII

A-B-NH₂ VII

in Gegenwart eines polaren aprotischen Lösungsmittels und eines Imidierungskatalysators erfolgen.

Weitere Einzelheiten zur Durchführung dieser Imidierungsreaktion können wiederum Abschnitt A.3 entnommen werden.

Alternativ kann die Imidierung auch, wie in Abschnitt B.1.1 beschrieben, mit gößeren Mengen Amin VII in Gegenwart des polaren aprotischen Lösungsmittels und einer (erd)alkalimetallhaltigen Base vorgenommen werden.

### B.1.3. Herstellung von Rylenderivaten der Formel Ib13

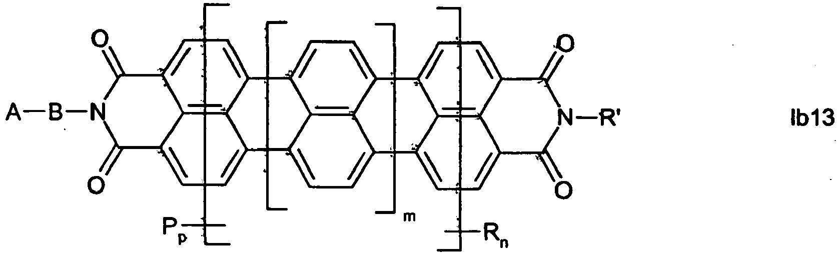

Auch die Rylenderivate Ib13 sind analog durch Umsetzung der entsprechenden Rylenderivate Ia3 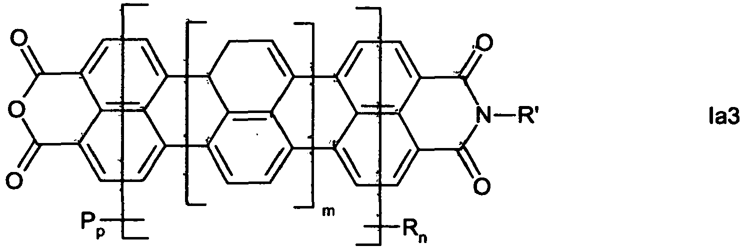 mit einem primären Amin der Formel VII

A-B-NH₂ VII

in Gegenwart eines polaren aprotischen Lösungsmittels und eines Imidierungskatalysators zu erhalten.

Weitere Einzelheiten zur Durchführung dieser Imidierungsreaktion können wiederum Abschnitt A.3 entnommen werden.

Die Imidierung kann auch, wie in Abschnitt B.1.1 beschrieben, mit gößeren Mengen Amin VII in Gegenwart des polaren aprotischen Lösungsmittels und einer (erd)alkalimetallhaltigen Base vorgenommen werden.

Alternativ kann man die Terrylen- und Quaterrylenderivate Ib13 (m = 1 bzw. 2) auch herstellen, indem man
a) Naphthalin- oder Perylendicarbonsäureanhydrid VIII 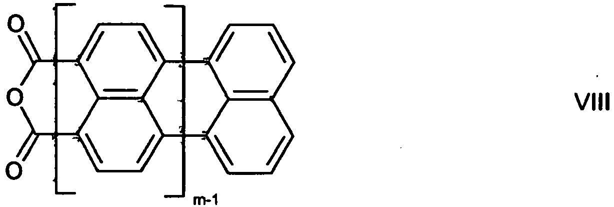 zunächst in Gegenwart eines polaren aprotischen Lösungsmittels und eines Imidierungskatalysators mit einem primärem Amin der Formel VII

   A-B-NH₂ VII

   umsetzt,
b) das hierbei erhaltene Naphthalin- bzw. Perylendicarbonsäureimid der Formel VIIIa 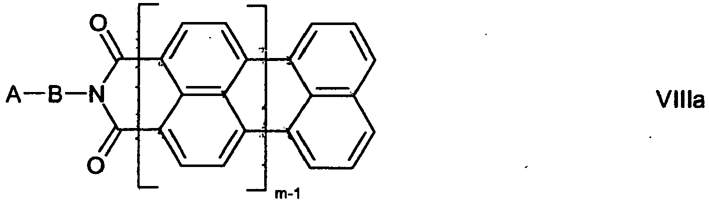 dann in Gegenwart eines basenstabilen hochsiedenden organischen Lösungsmittels und einer alkali- oder erdalkalimetallhaltigen Base und einer stickstoffhaltigen Hilfsbase mit einem Perylendicarbonsäureimid der Formel VIIIb 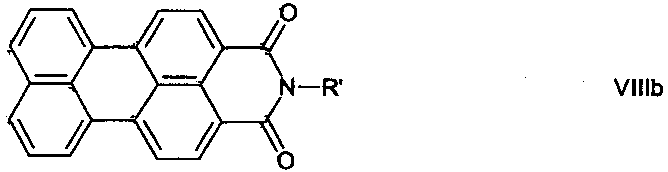 umsetzt und
c) das erhaltene im Rylengerüst unsubstituierte Terrylen- bzw. Quaterrylentetracarbonsäuremonoimidmonoanhydrid Ib13 (n= p=0) gewünschtenfalls
c1) in Gegenwart eines inerten Verdünnungsmittels mit elementarem Brom umsetzt und
c2) das bromierte Terrylen- bzw. Quaterrylenderivat Ib13' 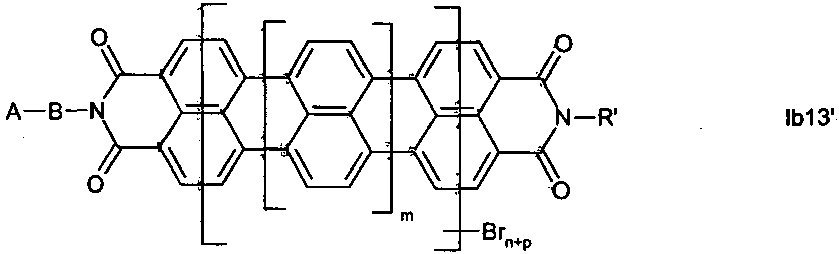 dann in Gegenwart eines nichtnucleophilen Lösungsmittels und einer Base mit einem (Thio)Alkohol der Formel IX

   H-ZR IX

   und/oder einem Amin der Formel IIIa

   H-NR¹R² IIIa

   zum substituierten Rylenderivat Ib13 (n und/oder p ≠ 0) umsetzt.

Im Imidierungsschritt a) kann man wiederum analog zu der in Abschnitt A.3 beschriebenen Imidierung vorgehen.

In Schritt b) wird durch Umsetzung des säurefunktionalisierten Naphthalin- oder Perylendicarbonsäureimids VIIIa mit dem Perylendicarbonsäureimid VIIIb das im Rylengerüst unsubstituierte Terrylen- bzw. Quaterrylenderivat Ib13 gebildet.

Als Lösungsmittel eignen sich für Schritt b) grundsätzlich alle unter den Reaktionsbedingungen gegen Basen stabilen hochsiedenden (Siedepunkt > 100°C und oberhalb der Reaktionstemperatur) Lösungsmittel, in denen sich die Edukte VIIIa und Vlllb bei Reaktionstemperatur vollständig und die verwendeten Basen zumindest partiell lösen, so daß weitgehend homogene Reaktionsbedingungen vorliegen.

Besonders geeignet sind dabei aprotische Lösungsmittel, die unpolar oder polar sein können, vorzugsweise jedoch polar sind.

Beispiele für diese Lösungsmittel sind die in Abschnitt A.1.1 genannten Lösungsmittel, wobei als polares aprotisches Lösungsmittel zusätzlich auch Trialkylamine, insbesondere Tri(C₃-C₆-alkyl)amine, wie Tripropyl- und Tributylamin, eingesetzt werden können.

Besonders bevorzugte Lösungsmittel sind Diphenylether und vor allem Dialkylether von Diethylenglykol, insbesondere Diethylenglykoldimethyl- und -diethylether.

Daneben können auch protische Lösungsmittel, insbesondere solche, die Amino- und Hydroxyfunktionen aufweisen, z.B. Alkoholamine, vor allem Mono-, Di- und Tri-C₂-C₄-alkoholamine, wie Mono-, Di- und Triethanolamin, eingesetzt werden.

In der Regel werden 10 bis 50 ml polares aprotisches Lösungsmittel, 50 bis 250 ml unpolares aprotisches Lösungsmittel bzw. 3 bis 50 ml protisches Lösungsmittel pro g Edukt auf Perylenbasisverwendet.

Als Base sind starke anorganische und organische alkali- oder erdalkalimetallhaltige Basen geeignet, wobei die alkalimetallhaltigen Basen besonders geeignet sind.

Beispiele für diese Basen sind in Abschnitt A.2.1 genannt. Besonders bevorzugt sind dabei Lithiumpropylamid, Natriumhydroxid, Kaliumhydroxid, Natriummethylat, Natrium-tert.-butylat, Kaliummethylat und Kalium-tert.-butylat, wobei die tert.-Butylate ganz besonders bevorzugt sind.

Zur Erleichterung der Umsetzung empfiehlt sich der Zusatz einer stickstoffhaltigen Hilfsbase mit geringer nucleophiler Wirkung. Geeignete Basen sind bei den Reaktionstemperaturen flüssige Alkylamine, insbesondere Tri-C₃-C₆-alkyl-amine, wie Tripropylamin und Tributylamin, Alkoholamine, insbesondere Mono-, Di- und Tri-C₂-C₄-alkohol-amine, wie Mono-, Di- und Triethanolamin, und insbesondere heterocyclische Basen, wie Pyridin, N-Methylpiperidin, N-Methylpiperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU).

Geeignete Einsatzmengen liegen für die Metallbase bei 2 bis 20 mol, vorzugsweise bei 8 bis 20 mol, pro mol Perylenedukt und für die Hilfsbase bei 1 bis 60 g, bevorzugt bei 5 bis 30 g, pro g Perylenedukt.

Die Metallbase kann in fester oder in gelöster form eingesetzt werden. Wenn die Metallbase in Kombination mit einem unpolaren aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 50 bis 210°C, bevorzugt bei 70 bis 180°C.

Die Umsetzung ist in der Regel in 2 bis 12 h abgeschlossen.

Es ist auch möglich, ein peri-Halogenderivat, insbesondere ein peri-Chlorderivat und vor allem ein peri-Bromderivat, eines der beiden Edukte VIIIa oder Vlllb einzusetzen und so die Umsetzung zu erleichtern.

In diesem Fall kann z.B. bei der Herstellung der Terrylenderivate Ib3 ein Molverhältnis von halogenierten Naphthalinedukt VIIIa zu Perylenedukt Vlllb von 4 : 1 bis 1 : 1, insbesondere 2 : 1 bis 1 : 1 verwendet werden, während das Molverhältnis im Fall des nichthalogenierten Naphthalinedukts Villa in der Regel bei 8 : 1 bis 1 : 1, vor allem bei 6 : 1 bis 2 : 1, liegt.

Bei Einsatz eines halogenierten Edukts sind neben den auf Ethern basierenden polaren aprotischen Lösungsmitteln auch unpolare aprotische Lösungsmittel, vorzugsweise Xylole, Mesitylen und insbesondere Toluol und Dekalin, als Reaktionsmedium besonders geeignet.

Die Basenmenge kann ebenfalls reduziert werden und die Anwesenheit der stickstoffhaltigen Hilfsbase empfiehlt sich insbesondere nur bei der Verwendung der Alkoholate und der Hydroxide. So beträgt die Einsatzmenge für die Metallbase im allgemeinen 5 bis 20 mol, insbesondere 5 bis 10 mol, pro mol Perylenedukt und für die Hilfsbase in der Regel 1 bis 15 g, vor allem 1 bis 5 g, pro g Perylenedukt.

Auch die Reaktionszeiten erniedrigen sich bei Verwendung von einem halogenierten Edukt üblicherweise auf 1 bis 3 h.

Die Bromierung im optionalen Schritt c2) kann auf allgemein bekannte Weise vorgenommen werden.

Als inerte Verdünnungsmittel eignen sich neben halogenierten Aromaten, wie Chlorbenzol und Di- und Trichlorbenzolen, insbesondere aliphatische Halogenkohlenwasserstoffe, vor allem Methane und Ethane, wie Tribrom-, Tetrachlor- und Tetrabrommethan, 1,2-Dichlor-, 1,1-Dibrom- und 1,2-Dibromethan, 1,1,1-Trichlor-, 1,1,2-Trichlor-, 1,1,1-Tribrom- und 1,1,2-Tribromethan und 1,1,1,2-Tetrachlor-, 1,1,2,2-Tetrachlor-, 1,1,1,2-Tetrabrom- und 1,1,2,2-Tetrabromethan, wobei Dichlormethan (Methylenchlorid) und Trichlormethan (Chloroform) bevorzugt sind.

In der Regel werden 30 bis 200 g, vorzugsweise 100 bis 150 g, Lösungsmittel pro g Rylenedukt eingesetzt.

Das Molverhältnis Brom zu Rylenedukt liegt im allgemeinen bei 10 : 1 bis 100 : 1, vor allem bei 40 : 1 bis 60 : 1.

Die Reaktionstemperatur liegt üblicherweise bei 40 bis 140°C, insbesondere 40 bis 90°C.

Liegt die Reaktionstemperatur über dem Siedepunkt des eingesetzten Lösungsmittels, so empfiehlt es sich, die Bromierung unter Druck vorzunehmen.

Die anschließende nucleophile Substitution der Bromatome durch (Het)Aryloxy- oder -thioreste -ZR oder Aminoreste -NR¹R² im optionalen Schritt c2) kann schließlich analog zu der in Abschnitt A.2.1 beschriebenen Herstellung der Rylenderivate Ia21 erfolgen.

### B.1.4. Herstellung von Rylenderivaten der Formel Ib14

Als Edukt für die Herstellung der Rylenderivate Ib14 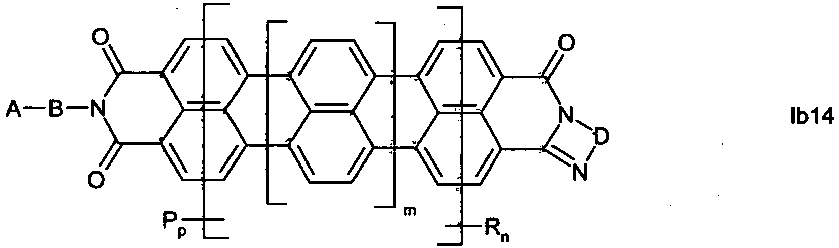 können die entsprechenden Rylenderivate I' 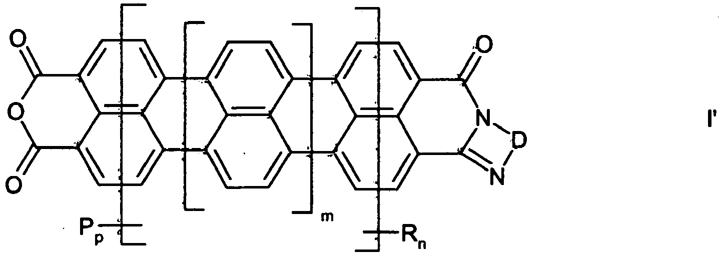 dienen, die wiederum in Gegenwart eines polaren aprotischen Lösungsmittels und eines Imidierungskatalysators mit einem primären Amin der Formel VII

A-B-NH₂ VII

umgesetzt werden können.

Weitere Einzelheiten zur Durchführung dieser Imidierungsreaktion sind auch hier Abschnitt A.3 zu entnehmen.

Alternativ kann die Imidierung auch hier, wie in Abschnitt B.1.1 beschrieben, mit gößeren Mengen Amin VII in Gegenwart des polaren aprotischen Lösungsmittels und einer (erd)alkalimetallhaltigen Base durchgeführt werden.

Die Herstellung des als Edukt dienenden Semikondensats I' kann in üblicher Weise durch Umsetzung des Rylentetracarbonsäuredianhydrids IIc 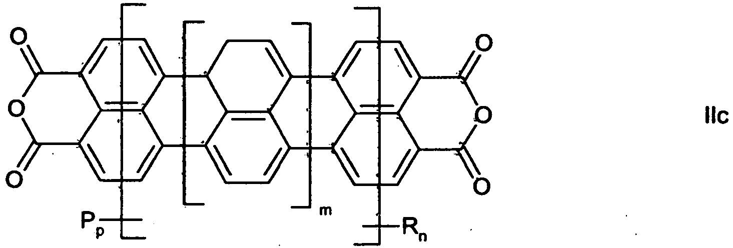 mit einem aromatischen Diamin der Formel X

H₂N-D-NH₂ X

in Gegenwart einer stickstoffbasischen Verbindung oder von Phenol als-Lösungsmittel und einer Lewis-Säure oder von Piperidin als Katalysator durchgeführt werden.

Das Molverhältnis von aromatischem Diamin X zu Rylentetracarbonsäuredianhydrid IIc beträgt dabei üblicherweise 1,5 : 1 bis 1 : 1, insbesondere 1,2 : 1 bis 1,05 : 1.

Als stickstoffbasische Verbindung eignen sich vor allem stickstoffhaltige Heterocyclen, die vorzugsweise nicht weiter funktionalisiert sind, wie Chinolin, Isochinolin, Chinaldin, Pyrimidin, N-Methylpiperidin, Pyridin, Pyrrol, Pyrazol, Triazol, Tetrazol, Imidazol und Methylimidazol. Bevorzugt sind tertiäre stickstoffbasische Verbindungen, vor allem Chinolin.

In der Regel kommen 5 bis 200 ml, vorzugsweise 10 bis 50 ml, Lösungsmittel pro g Rylenedukt IIc zum Einsatz.

Als Katalysator eignen sich Lewis-Säuren, z.B. Zinkverbindungen, vor allem Zinksalze, wie Zinkacetat und Zinkchlorid, und Zinkoxid, anorganische und organische Säuren, wie Salzsäure, Essigsäure und p-Toluolsulfonsäure, wobei Zinkacetat bevorzugt ist.

Ebenfalls als Katalysator geeignet ist Piperazin, das vorzugsweise in Kombination mit Phenol als Lösungsmittel zum Einsatz kommt.

Üblicherweise werden 0,25 bis 5,0 mol, insbesondere 1,0 bis 2,0 mol, Katalysator pro mol IIc eingesetzt.

Die Reaktionstemperatur beträgt im allgemeinen 100 bis 240°C, vorzugsweise 160 bis 240°C.

Es empfiehlt sich, unter Schutzgas, z.B. Stickstoff oder Argon, zu arbeiten.

Im allgemeinen ist die Kondensation in 0,5 bis 24 h, vor allem 2 bis 6 h, beendet.

### B.2. Herstellung von Rylenderivaten der Formel Ib2

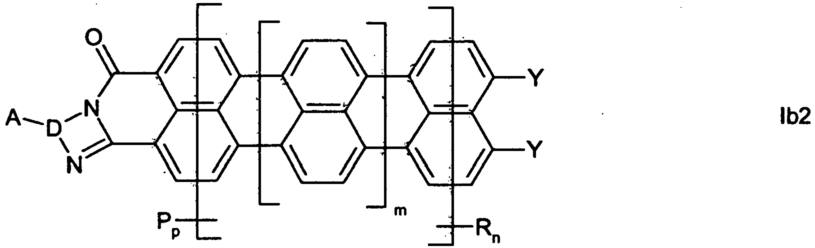

Die Herstellung der Rylenderivate Ib2 wird im folgenden ebenfalls in Abhängigkeit von den Substituenten Y abschnittsweise beschrieben.

### B.2.1. Herstellung von Rylenderivaten Ib21

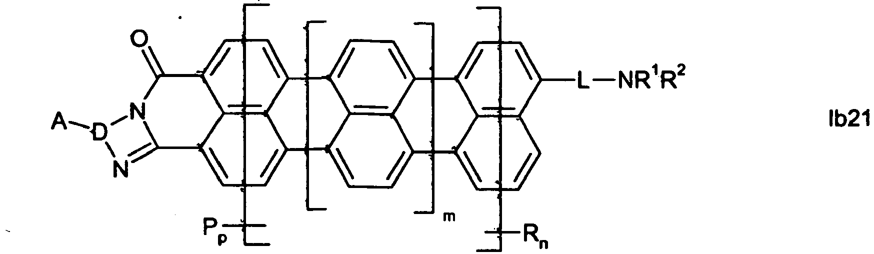

Die Herstellung der Rylenderivate Ib21 kann durch Umsetzung der entsprechenden Rylenderivate Ia1 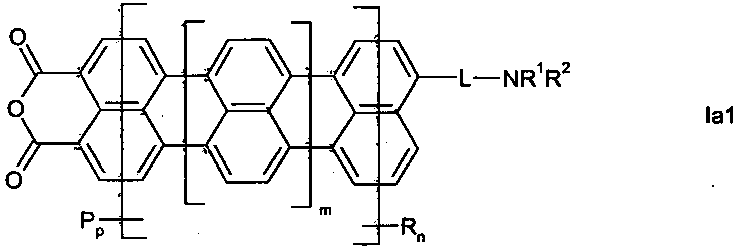 mit einem aromatischen Diamin der Formel XI 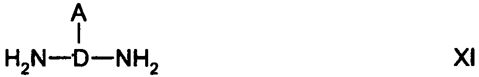 in Gegenwart einer stickstoffbasischen Verbindung oder von Phenol als Lösungsmittel und einer Lewis-Säure oder von Piperidin als Katalysator erfolgen.

Diese Kondensationsreaktion kann analog zu der in Abschnitt B.1.4 beschriebenen Kondensationsreaktion zur Herstellung der Rylenedukte I' vorgenommen werden.

### B.2.2. Herstellung von Rylenderivaten der Formel Ib22

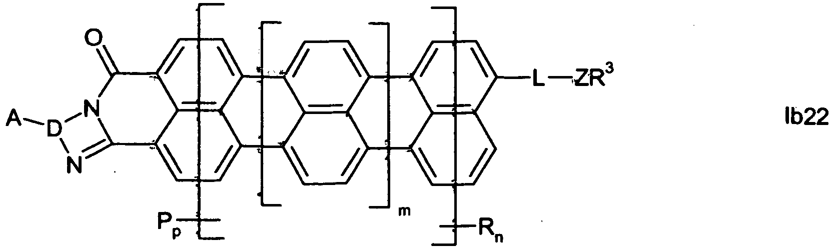

Die Herstellung der Rylenderivate Ib22 kann analog durch Umsetzung der entsprechenden Rylenderivate Ia2 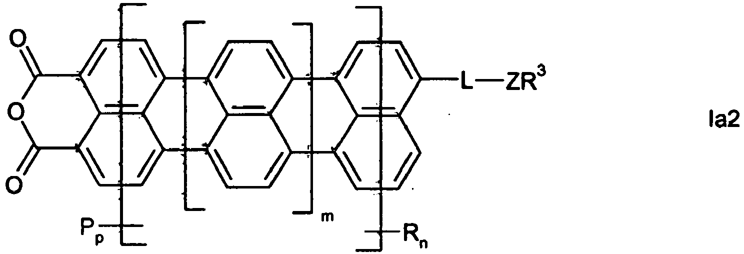 mit einem aromatischen Diamin der Formel XI 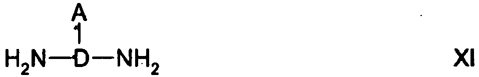 in Gegenwart einer stickstoffbasischen Verbindung oder von Phenol als Lösungsmittel und einer Lewis-Säure oder von Piperidin als Katalysator erfolgen.

Weitere Einzelheiten zur Durchführung dieser Kondensationsreaktion können wiederum Abschnitt B.1.4 entnommen werden.

### B.2.3. Herstellung von Rylenderivaten der Formel Ib23

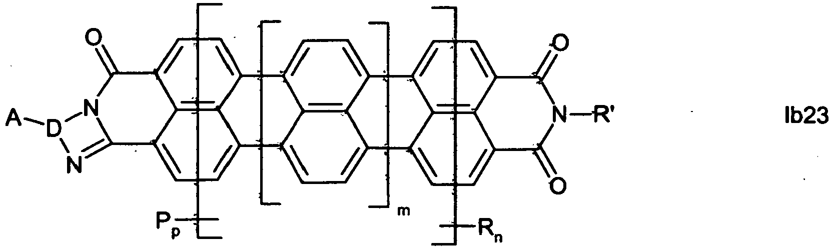

Auch die Rylenderivate Ib23 sind analog durch Umsetzung der entsprechenden Rylenderivate Ia3 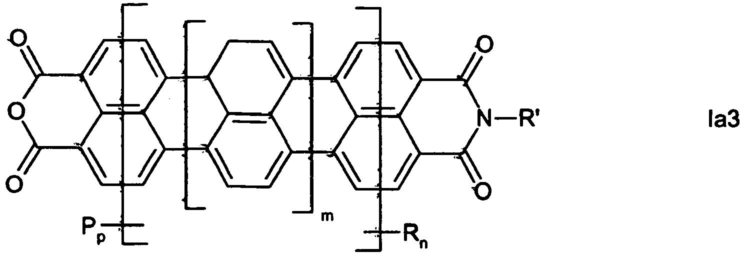 mit einem aromatischen Diamin der Formel XI 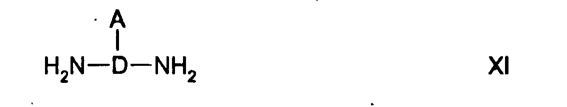 in Gegenwart einer stickstoffbasischen Verbindung oder von Phenol als Lösungsmittel und einer Lewis-Säure oder von Piperidin als Katalysator zu erhalten.

Weitere Einzelheiten zur Durchführung dieser Kondensationsreaktion sind auch hier Abschnitt B.1.4 zu entnehmen.

### B.2.4. Herstellung von Rylenderivaten der Formel Ib24

Als Edukt für die Herstellung der Rylenderivate Ib24 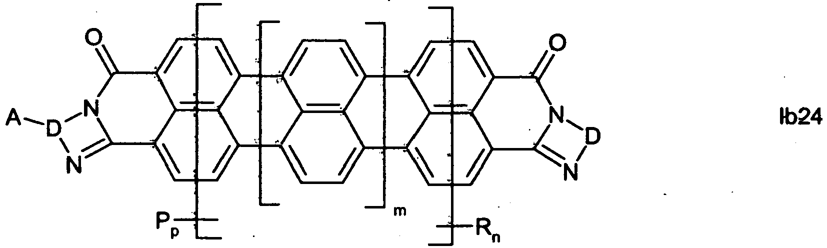 können die entsprechenden, bereits in Abschnitt B.1.4 beschriebenen Rylenderivate I' 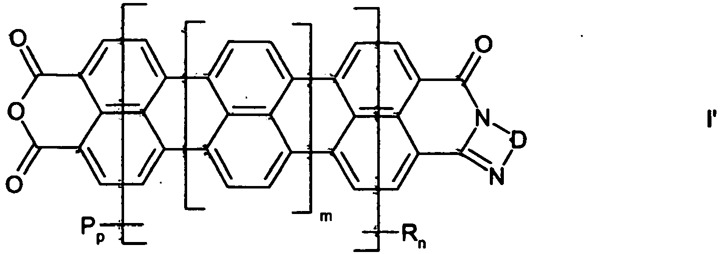 dienen, die einer weiteren Kondensationsreaktion mit einem aromatischen Diamin der Formel XI 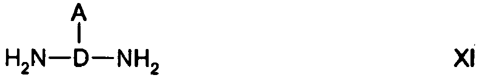 in Gegenwart einer stickstoffbasischen Verbindung oder von Phenol als Lösungsmittel und einer Lewis-Säure oder von Piperidin als Katalysator unterzogen werden können.

Diese Kondensationsreaktion kann wiederum analog zur Herstellung der Rylenderivate I' vorgenommen werden.

Die erfindungsgemäßen Rylenderivate I eignen sich hervorragend für den Einsatz in farbstoffsensibilisierten Solarzellen.

Sie zeigen starke Absorption im Wellenlängenbereich des Sonnenlichts und können dabei in Abhängigkeit von der Länge des konjugierten Systems einen Bereich von etwa 400 nm (Perylenderivate I) bis zu etwa 900 nm (Quaterrylenderivate I) abdecken. Rylenderivate I auf Terrylenbasis absorbieren je nach ihrer Zusammensetzung in festem, an Titandioxid adsorbiertem Zustand in einem Bereich von etwa 400 bis 800 nm. Um eine möglichst weitgehende Nutzung des eingestrahlten Sonnenlichts vom sichtbaren bis in den nahinfraroten Bereich zu erreichen, ist es vorteilhaft, Mischungen verschiedener Rylenderivate I einzusetzen. Gelegentlich kann es sich auch empfehlen, dabei auch verschiedenen Rylenhomologe zu verwenden.

Außerdem können die Rylenderivate vorteilhaft mit allen n-Halbleitem, die üblicherweise in diesen Solarzellen Verwendung finden, kombiniert werden. Als bevorzugte Beispiele seien in der Keramik eingesetzte Metalloxide, wie Titandioxid, Zinkoxid, Zinn(IV)-oxid, Wolfram(VI)oxid, Tantal(V)oxid, Niob(V)oxid, Caesiumoxid, Strontiumtitanat, Zinkstannat, komplexe Oxide vom Perowskit-Typ, z.B. Bariumtitanat, und binäre und ternäre Eisenoxide genannt, die in nanokristalliner oder amorpher Form vorliegen können.

Besonders bevorzugte Halbleiter sind Zinkoxid und Titandioxid in der Anatasmodifikation, das vorzugsweise in nanokristalliner Form eingesetzt wird.

Die Metalloxidhalbleiter können allein oder in Form von Mischungen eingesetzt werden. Es ist auch möglich, ein Metalloxid mit einem oder mehreren anderen Metalloxiden zu beschichten. Weiterhin können die Metalloxide auch als Beschichtung auf einem anderen Halbleiter, z.B. GaP, ZnP oder ZnS, aufgebracht sein.

Das nanopartikuläre Titandioxid wird üblicherweise,aufgepresst oder durch einen Sinterprozeß als dünner poröser Film mit großer Oberfläche auf ein leitfähiges Substrat aufgebracht. Als Substrat eignen sich neben Metallfolien vor allem Kunststoffplatten oder -folien und insbesondere Glasplatten, die mit einem leitfähigen Material, z.B. transparenten leitfähigen Oxiden (TCO), wie mit Fluor oder Indium dotiertes Zinnoxid (FTO bzw. ITO) und aluminiumdotiertes Zinkoxid (AZO), Kohlenstoffnanoröhren oder Metallfilmen, belegt sind.

Die Rylenderivate I können leicht und dauerhaft auf dem Metalloxidfilm fixiert werden. Die Bindung erfolgt dabei über die Anhydridfunktion (x1) bzw. die in situ gebildeten Carboxylgruppen -COOH bzw. -COO- oder über die in den Imid- oder Kondensatresten ((x2) bzw. (x3)) enthaltenen Säuregruppen A.

Das Dicarbonsäuresalz der Rylenderivate I kann vorteilhaft aus der Anhydridform (x1) erhalten werden, indem man das Anyhdrid in Tetrahydrofuran löst, mit einer wäßrigen Tetraalkylammoniumhydroxid- bzw. Alkalimetallcarbonatlösung alkalisch stellt, etwa 1 h unter Rückfluß kocht, in einem Gemisch von Methylenchlorid und Wasser aufnimmt, die organische Phase von der wäßrigen Phase abtrennt und das Methylenchlorid im Vakuum entfernt.

Aufgrund der starken Absorption der Rylenderivate I reichen bereits dünne Metalloxidfilme aus, um die erforderliche Menge Rylenderivat aufzunehmen. Dünne Metalloxidfilme haben den Vorteil, daß die Wahrscheinlichkeit unerwünschter Rekombinationsprozesse und der innere Widerstand der Zelle reduziert werden. Daher eignen sie sich besonders für die Herstellung von festen Farbstoffsolarzellen.

Die Fixierung der Rylenderivate I auf den Metalloxidfilmen kann in einfacher Weise erfolgen, indem die Metalloxidfilme in frisch gesintertem (noch warmen) Zustand über einen ausreichenden Zeitraum (etwa 0,5 bis 24 h) mit einer Lösung des jeweiligen Rylenderivats I in einem geeigneten organischen Lösungsmittel in Kontakt gebracht werden. Dies kann beispielsweise durch Eintauchen des mit dem Metalloxid beschichteten Substrats in die Lösung des Rylenderivats geschehen. Diese einfache Vorgehensweise eignet sich aufgrund ihrer ausgezeichneten Löslichkeit insbesondere für die durch (Het)Aryloxy- oder -thioreste R substituierten Rylenderivate I. Sollen Kombinationen verschiedener Rylenderivate I eingesetzt werden, so können diese aus einer alle Rylenderivate enthaltender Lösung oder nacheinander aus verschiedenen Lösungen aufgebracht werden. Die zweckmäßigste Methode kann im Einzelfall leicht ermittelt werden.

Die Rylenderivate I weisen, wie bereits beschrieben, eine Funktionalität auf, die ihre Fixierung am n-Halbleiterfilm gewährleisten. Am anderen Molekülende enthalten sie vorzugsweise Elektronendonatoren Y, die die Regeneration des Rylenderivats nach der Elektronenabgabe an den n-Halbleiter erleichtern und außerdem die Rekombination mit bereits an den Halbleiter abgebenen Elektronen verhindern.

Als elektronendonierende Gruppen Y sind dabei neben den (Thio)Etherresten (y2) insbesondere die Aminoreste (y1) oder Imid- oder Kondensatreste (y3) bzw. (y4) bevorzugt, die (Thio)Ethergruppen oder Aminogruppen als Substituenten tragen. Besonders bevorzugt sind die durch Aminogruppen -NR⁷R⁸ substituierte Imidreste (y3) und ganz besonders bevorzugt sind die Aminoreste (y1).

Diese Substituenten bewirken zusätzlich eine bathochrome Verschiebung der Absorption und erweitern damit den durch die Rylenderivate nutzbaren Spektralbereich.

Die Regeneration der Rylenderivate I kann schließlich sowohl mit flüssigen Elektrolyten als auch mit festen p-Leitern erfolgen.

Als Beispiele für flüssige Elektrolyte seien Redoxsysteme, wie Iod/Iodid, Brom/Bromid und Hydrochinon/Chinon sowie Übergangsmetallkomplexe, die in einem polaren organischen Lösungsmittel gelöst, in einer ionischer Flüssigkeit oder in einer Gelmatrix vorliegen können, genannt.

Flüssig oder Flüssigkrystaline p-Leiter wie Triphenylaminderivate können auch als p-Leiter eingesetzt werden.

Beispiele für feste p-Leiter sind anorganische Festkörper, wie Kupfer(I)iodid und Kupfer(I)thiocyanat, und vor allem organische p-Leiter auf Basis von Polymeren, wie Polythiophen und Polyarylaminen, oder von amorphen, reversibel oxidierbaren, nichtpolymeren organischen Verbindungen, wie den eingangs erwähnten Spirobifluorenen oder andere p-leitende Moleküle.

Feste p-Leiter können in den erfindungsgemäßen farbstoffsensibilisierten Solarzellen auch ohne Erhöhung des Zellenwiderstands zum Einsatz kommen, da die Rylenderivate I stark absorbieren und deshalb nur dünne n-Halbleiterschichten erfordern.

Jede Verbindung I hat verschiedene elektronische Eigenschaften und die anderen Komponenten der Farbstoffsolarzelle (n- und p-Leiter, Additive usw.) müssen deswegen an jede Verbindung angepasst werden.

Die erfindungsgemäßen farbstoffsensibilisierten Solarzellen sind ansonsten wie üblich aufgebaut, so daß weitere Erklärungen hier nicht erforderlich ist.

Die erfindungsgemäßen farbstoffsensibilisierten Solarzellen können vorteilhaft als Energiequelle für eine Reihe von Anwendungen eingesetzt werden. Als Beispiel für eine besonders interessante Einsatzmöglichkeit sei die Gewinnung von Wasserstoff und Sauerstoff durch elektrolytische Spaltung von Wasser genannt.

Durch Verwendung von Kombinationen der erfindungsgemäßen Verbindungen mit verschiedenen Absorptionseigenschaften lassen sich Tandemzellen herstellen.

Die erfindungsgemäßen farbstoffsensibilisierten Solarzellen sind ansonsten wie üblich aufgebaut, so daß weitere Erklärungen hier nicht erforderlich ist.

Die erfindungsgemäßen farbstoffsensibilisierten Solarzellen können vorteilhaft als Energiequelle für eine Reihe von Anwendungen eingesetzt werden. Als Beispiel für eine besonders interessante Einsatzmöglichkeit sei die Gewinnung von Wasserstoff und Sauerstoff durch elektrolytische Spaltung von Wasser genannt.

### Beispiele

### I. Herstellung von Rylenderivaten I

### Beispiel 1

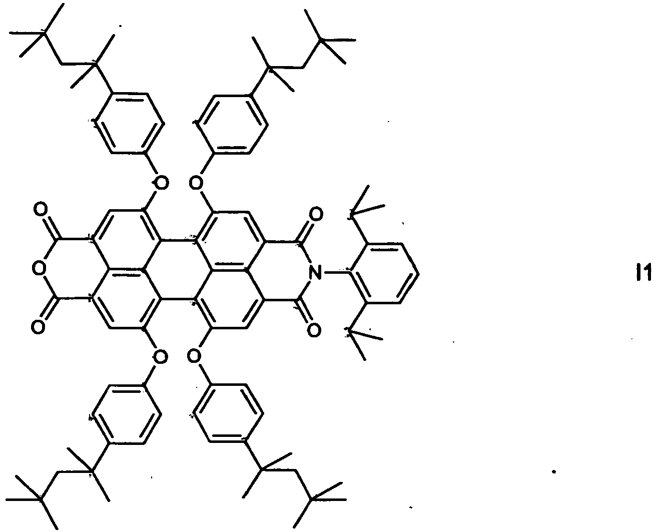

Eine Mischung von 50,0 g (0,033 mol) N-(2,6-Diisopropylphenyl)-1,6,7,12-tetra[(4-tert.-octyl)phenoxy]perylen-3,4:9,10-tetracarbonsäurediimid und 800 ml 2-Methyl-2-butanol wurde auf 60°C erhitzt. Nach 0,5 h wurden 55,2 g (0,98 mol) Kaliumhydroxid und 56,9 g (0,98 mol) Kaliumfluorid zugegeben. Dann wurde auf leichten Rückfluß (etwa 80°C) erhitzt und 72 h bei dieser Temperatur gerührt. Die dünnschichtchromatographische Untersuchung einer Probe mit Toluol zeigte nur noch eine Spur Edukt.

Nach Abkühlen auf 50°C, Ansäuern mit 50 gew.-%iger Essigsäure und weiterem zweistündigen Rühren bei 80°C wurde das Reaktionsprodukt auf Wasser gefällt, abfiltriert, mit heißem Wasser gewaschen, im Vakuum bei 70°C getrocknet und danach einer Säulenchromatographie an Kieselgel mit einem Methylenchlorid/Hexan-Gemisch (1:1) als Eluens unterzogen.

Es wurden 19,4 g I1 in Form eines roten Feststoffs erhalten, was einer Ausbeute von 49% entspricht.

Analytische Daten von I1:
¹H-NMR (500 MHz, CD₂Cl₂, 25°C): δ = 8,21 (d, 4H); 7,48-7,41 (m, 1 H); 7,39-7,27 (m, 10H); 6,90 (d, 8H); 2,70-2,61 (m, 2H); 1,76 (s, 4H); 1,74 (s, 4H); 1,40 (s, 12H); 1,38 (s, 12H); 1,10 (d, 12H); 0,77 (s, 18H); 0,70 (s, 18H) ppm; UV-Vis (CHCl₃): λₘₐₓ (ε) = 584 (47 200), 544 (27 800), 450 (18 600) (M⁻¹cm⁻¹); MS (FD): m/z (rel. Int.) = 1367,7 (100%) [M⁺].

Außerdem wurden 12,2 g (27%) des analog substituierten Perylen-3,4:9,10-tetracarbonsäuredianhydrids erhalten, das folgende analytische Daten aufwies:
¹H-NMR (500 MHz, CDCl₃, 25°C): δ = 8,11 (s, 4H); 7,62 (d, 8H); 6,77 (d, 8H); 1,75 (s, 8H); 1,38 (s, 24H); 0,78 (s, 36H) ppm;
UV-Vis (CHCl₃): λₘₐₓ (ε) = 584 (42 500), 542 (25 000), 450 (19 000) (M⁻¹cm⁻¹); MS (FD): m/z (rel. Int.) = 1208,5 (100%) [M⁺].

### Beispiel 2

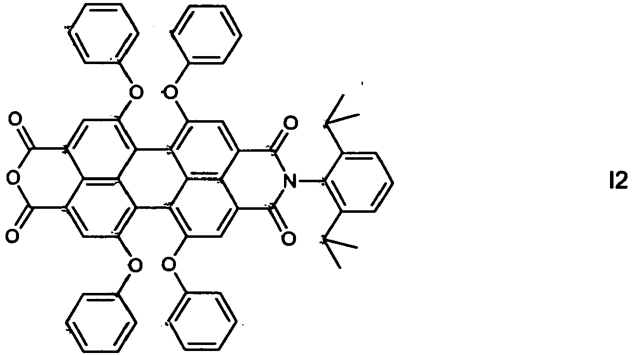

Eine Mischung von 50,0 g (0,046 mol) N-(2,6-Diisopropylphenyl)-1,6,7,12-tetraphenoxyperylen-3,4:9,10-tetracarbonsäurediimid und 800 ml 2-Methyl-2-butanol wurde auf 60°C erhitzt. Nach 0,5 h wurden 55,2 g (0,98 mol) Kaliumhydroxid und 56,9 g (0,98 mol) Kaliumfluorid zugegeben. Dann wurde auf leichten Rückfluß (etwa 80°C) erhitzt und 72 h bei dieser Temperatur gerührt. Die dünnschichtchromatographische Untersuchung einer Probe mit Toluol zeigte nur noch eine Spur Edukt.

Nach Abkühlen auf 50°C, Ansäuern mit 50 gew.-%iger Essigsäure und weiterem zweistündigen Rühren bei 60°C wurde das Reaktionsprodukt auf Wasser gefällt, abfiltriert, mit heißem Wasser gewaschen, im Vakuum bei 70°C getrocknet und danach einer Säulenchromatographie an Kieselgel mit Methylenchlorid als Eluens unterzogen.

Es wurden 16,0 g I2 in Form eines roten Feststoffs erhalten, was einer Ausbeute von 38% entspricht.

Analytische Daten von I2:
¹H-NMR (250 MHz, CO₂Cl₂, 25°C): δ = 8,17 (s, 2H); 8,16 (s, 2H); 7,46 (t, 1 H); 7,35 (d, 2H); 7,33-7,28 (m, 8H); 7,21-7,13 (m, 4H); 7,03-6,97 (m, 8H); 2,73-2,62 (sept, 2H); 1,08 (d, 12H) ppm;
UV-Vis (CHCl₃): = 611 nm;
MS (FD): m/z (rel. Int.) = 920 (100%) [M⁺].

### Beispiel 3

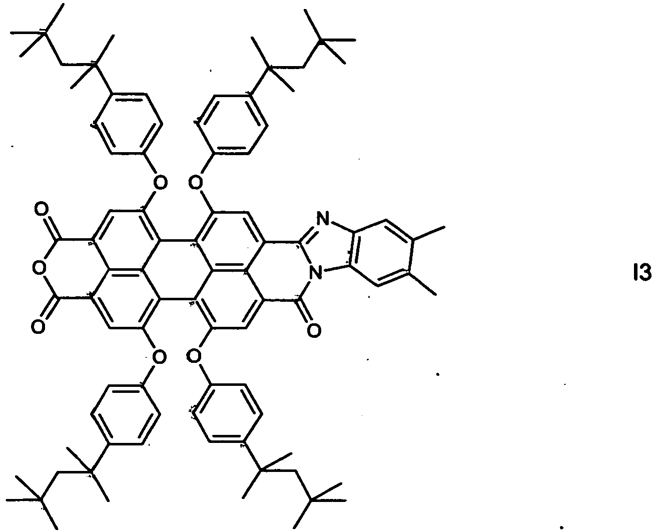

Eine Lösung von 0,72 g (0,60 mmol) N-(2,6-Diisopropylphenyl)-1,6,7,12-tetra[(4-tert.-octyl)phenoxy]perylen-3,4:9,10-tetracarbonsäuredianhydrid in 75 ml Chinolin wurde unter Stickstoff in einem 250 ml-Schlenckrohr vorgelegt, dann wurden ebenfalls unter Stickstoff nacheinander 0,074 g (0,54 mmol) 4,5-Dimethylphenylen-1,2-diamin und 0,28 g (1,5 mmol) wasserfreies Zinkacetat zugegeben. Die Mischung wurde dann unter Stickstoff auf 215°C erhitzt und unter mit Abtrennen des entstehenden Wassers 40 min bei dieser Temperatur gehalten. Während der Reaktion wurde ein Farbumschlag von tiefrot nach dunkelgrün beobachtet.

Nach Abkühlen auf Raumtemperatur wurde das Reaktionsgemisch in 300 ml 10 gew.-%ige Salzsäure gegeben und ca. 12 h gerührt. Das auf diese Weise ausgefällte Produkt wurde abfiltriert, zunächst mit heißem Wasser, dann mit einem Wasser/Methanol-Gemisch (1:1) und abschließend mit Methanol bis zum klaren Ablauf gewaschen und danach einer Säulenfiltration an Kieselgel mit Chloroform und anschließend mit Ethanol als Eluens unterzogen.

Es wurden 0,295 g I3 in Form eines blauen Feststoffs erhalten, was einer Ausbeute von 38% entspricht.

Analytische Daten von I3:
UV-Vis (CHCl₃): λₘₐₓ (ε) = 614 (49 700) nm (M⁻¹cm⁻¹);
MS (FD): m/z (rel. Int.) = 1308,6 (100%) [M⁺].

### Beispiel 4

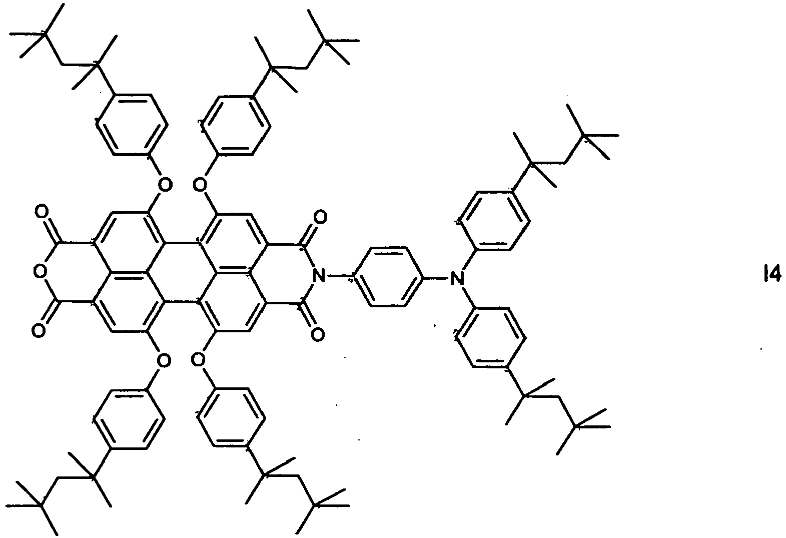

Das bei der Herstellung des Rylenderivats 14 eingesetzte 4-[Bis(4'-tert.-octylphenyl)-amino]anilin wurde dabei in einer zweistufigen Reaktion ausgehend von Bis(4-tert.-octylphenyl)amin wie folgt hergestellt:

### 1) Bis(4-tert.-octylphenyl)(4-nitrophenyl)amin:

Eine Mischung von 0,70 g (1,0 mol) Tris(dibenzylidenaceton)dipalladium(0), 0,70 g (0,8 mmol) Bis(diphenylphosphino)ferrocen und 7,0 g (70 mmol) Natrium-tert-butylat wurde bei Raumtemperatur gerührt. Nach 15 min wurden 41,0 g (0,2 mol) 4-Bromnitro-benzol und nach weiteren 15 min 20 g (50 mmol) Bis(4-tert.-octylphenyl)amin zugegeben. Dann wurde die Mischung auf 90°C erhitzt und 16 h bei dieser Temperatur gerührt.

Das auf Raumtemperatur abgekühlte Reaktionsgemisch wurde auf 500 ml Wasser gegeben. Die durch Extraktion mit Toluol abgetrennte organische Phase wurde mit Wasser gewaschen, dann wurde das Lösungsmittel unter Vakuum entfernt. Das Rohprodukt wurde einer Säulenchromatographie an Kieselgel mit einem Methylenchlorid/Hexan-Gemisch (3:1) als Eluens unterzogen. Das hierbei erhaltene Produkt wurde in Methanol umkristallisiert.

Es wurden 11,7 g Bis(4-tert.-octyldiphenyl)(4-nitrophenyl)amin in Form eines gelben Feststoffs erhalten, was einer Ausbeute von 45% entspricht.

Analytische Daten:
¹H-NMR (500 MHz, CD₂Cl₂, 25°C): δ = 7,97 (d, 2H); 7,19 (d, 4H); 7,10 (d, 4H); 6,82 (d, 2H); 1,75 (s, 4H); 1,38 (s, 12 H); 0,76 (s, 18 H) ppm; MS (FD): m/z (rel. Int.) = 515 (100%) [M⁺].

### 2) 4-[Bis(4'-tert.-octylphenyl)amino]anilin:

Eine Mischung von 5 g (10 mmol) Bis(4-tert.-octyldiphenyl)(4-nitrophenyl)amin, 10 g (0,15 mol) Zinkstaub und 50 ml Ethanol wurde mit einem Eisbad auf 5°C gekühlt. Dann wurden 15 g Eisessig unter Einhaltung einer Temperatur von 5°C langsam zugegeben. Die Mischung wurde anschließend 5 h bei Raumtemperatur gerührt.

Der Zinkstaub wurde abfiltriert und mit Ethanol gewaschen. Nach Entfernen des Ethanols unter Vakuum wurde das Produkt in Methylenchlorid aufgenommen, und die Methylenchloridphase wurde mehrmals mit Wasser gewaschen. Nach Entfernendes Methylenchlorids unter Vakuum wurde das Produkt einer Säulenfiltration an Kieselgel mit einem Toluol/Hexan-Gemisch (1:1) als Eluens unterzogen.

Es wurden 3,6 g 4-[Bis(4'-tert.-octylphenyl)amino]anilin in Form eines gelbraunen Öls erhalten, was einer Ausbeute von 75% entspricht.

Analytische Daten:
¹H-NMR (500 MHz, CD₂Cl₂, 25°C): 8 = 7,21 (d, 4H); 6,89 (m, 6H); 6;62 (d, 2H); 6,82 (d, 2H), 1,72 (s, 4H); 1,38 (s, 12 H); 0,76 (s, 18 H) ppm; MS (FD): m/z (rel. Int.) = 484,5 (100%) [M⁺].

Eine Lösung von 0,70 g (0,56 mmol) N-(2,6-Dilsopropylphenyl)-1,6,7,12-tetra[(4-tert.octyl)phenoxy]perylen-3,4:9,10-tetracarbonsäuredianhydrid (gemäß Beispiel 1 erhalten) in 75 ml wasserfreiem N-Methylpyrrolidon wurde unter Stickstoff in einem 250 ml-Schlenckrohr vorgelegt, dann wurden ebenfalls unter Stickstoff nacheinander 0,816 g (1,35 mmol) 4-[Bis(4'-tert.-octylphenyl)amino]anilin und 0,103 g (0,56 mmol) wasserfreies Zinkacetat zugegeben. Die Mischung wurde dann unter Stickstoff auf 130°C erhitzt und 24 h bei dieser Temperatur gehalten.

Nach Abkühlen auf Raumtemperatur wurde das Produkt durch leichtes Ansäuern des Reaktionsgemischs mit 10 gew.-%iger Salzsäure ausgefällt. Die Mischung wurden 0,5 h unter Rückfluß erhitzt. Das Rohprodukt wurde abfiltriert, mit heißem Wasser gewaschen und dann einer Säulenfiltration an Kieselgel mit einem Methylenchlorid/Hexan-Gemisch (1:1) als Eluens unterzogen.

Es wurden 0,250 g I4 in Form eines roten Feststoffs erhalten, was einer Ausbeute von 27% entspricht.

Analytische Daten von I4:
¹H-NMR (500 MHz, CDCl₃, 25°C): δ = 8,20 (s, 2H); 8,09 (s, 2H); 7,30-7,25 (m, 8H); 7,23-7,20 (d, 4H); 7,10-7,00 (m, 8H); 6,91 (d, 4H); 6,84 (d, 4H); 1,71-1,67 (m, 12H); 1,33-1,30 (m, 36H); 0,76 (d, 54H) ppm;
UV-Vis (CHCl₃): λₘₐₓ = 577, 537, 444 nm;
MS (FD): m/z (rel. Int.) = 1674,2 (100%) [M⁺].

### Beispiel 5

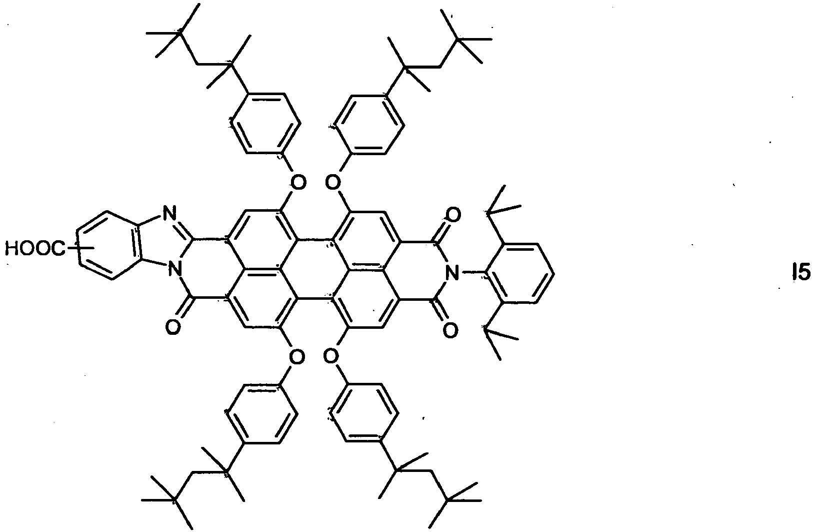

Bei dem Rylenderivat I5 handelt es sich um ein Gemisch zweier Isomere mit der Carboxylgruppe in 3- bzw. 4-Position am Phenylenring.

Eine Lösung von 0,50 g (0,37 mmol) N-(2,6-Diisopropylphenyl)-1,6,7,12-tetra[(4-tert.-octyl)phenoxy]perylen-3,4:9,10-tetracarbonsäuremonoimidmonoanhydrid (gemäß Beispiel 1 erhalten) in 25 ml Chinolin wurde unter Stickstoff in einem 50 ml-Schlenckrohr vorgelegt, dann wurden ebenfalls unter Stickstoff nacheinander 0,172 g (1,10 mmol) 3,4-Diaminobenzoesäure und 0,20 g (1,10 mmol) wasserfreies Zinkacetat zugegeben. Die Mischung wurde dann unter Stickstoff auf 215°C erhitzt und unter Abtrennen des entstehenden Wassers 3 h bei dieser Temperatur gehalten. Während der Reaktion wurde ein Farbumschlag von tiefrot nach dunkelblau beobachtet.

Nach Abkühlen auf Raumtemperatur wurde das Reaktionsgemisch in eine Mischung von 300 ml 10 gew.-%iger Salzsäure und 25 ml Ethanol gegeben und ca. 12 h gerührt. Das auf diese Weise ausgefällte Produkt wurde abfiltriert, zunächst mit heißem Wasser und dann mit einem Wasser/Methanol-Gemisch (95:5) gewaschen, im Vakuum bei 70°C getrocknet und danach einer Säulenfiltration an Kieselgel mit Chloroform und anschließend mit Ethanol als Eluens unterzogen.

Es wurden 0,080 g I5 in Form eines blauen Feststoffs erhalten, was einer Ausbeute von 15% entspricht.

Analytische Daten des Isomerengemischs I5:
UV-Vis (CHCl₃): λₘₐₓ (ε) = 612 (52 000) nm (M⁻¹cm⁻¹);
MS (FD): m/z (rel. Int.) = 1483,7 (100%) [M⁺].

### Beispiel 6

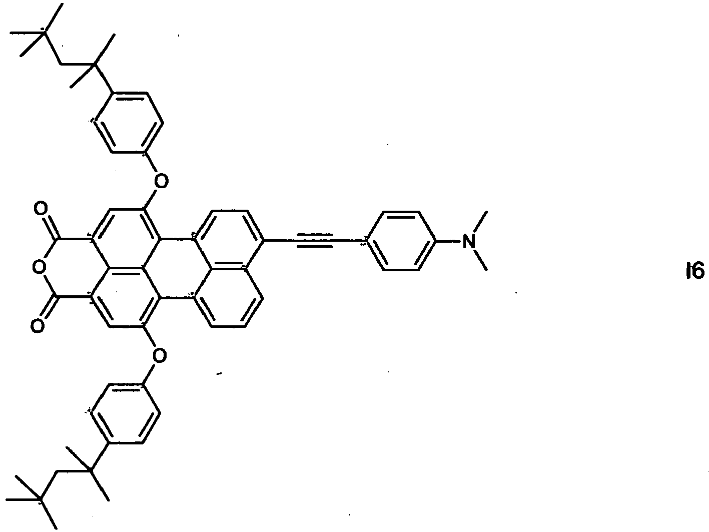

Die Herstellung des Rylenderivats I6 erfolgte ausgehend von N-(2,6-Diisopropylphenyl)-9-brom-1,6-bis[(4-tert.-octyl)phenoxy]perylen-3,4-dicarbonsäureimid, das zunächst unter Bromaustausch mit (4-Ethinylphenyl)dimethylamin zum aminosubstituierten Dicarbonsäureimid umgesetzt wurde (Schritt a), welches anschließend alkalisch zum Dicarbonsäureanhydrid I6 verseift wurde (Schritt b).

### Schritt a):

Zu einer unter Argon gerührten Mischung von 0,3 g (0,31 mmol) N-(2,6-Diisopropylphenyl)-9-brom-1,6-bis[(4-tert.-octyl)phenoxy]perylen-3,4-dicarbonsäureimid, 25 ml Triethylamin und 25 ml trockenem Tetrahydrofuran wurden zunächst 34,7 mg (0,03 mmol) Tetrakis(triphenylphosphin)palladium(0) und 14,6 mg (0,024 mmol) Kupfer(I)-iodid und dann 0,09 g (0,62 mmol) (4-Ethinylphenyl)dimethylamin gegeben. Dann wurde die Mischung auf 60°C erhitzt und 12 h bei dieser Temperatur gerührt.

Das auf Raumtemperatur abgekühlte Reaktionsgemisch wurde auf 100 ml Wasser gegeben. Die organische Phase wurde durch Extraktion mit Methylenchlorid abgetrennt, dann wurde das organische Lösungsmittel durch Verdampfen entfernt. Das Rohprodukt wurde einer Säulenchromatographie an Kieselgel mit einem Methylenchlorid/Pentan-Gemisch (2:3) alsy Eluens unterzogen.

Es wurden 100 mg N-(2,6-Diisopropylphenyl)-9-(4-dimethylaminophenyl)ethinyl-1,6-bis[(4-tert.-octyl)phenoxy]perylen-3,4-dicarbonsäureimid in Form eines violetten Feststoffs erhalten, was einer Ausbeute von 31% entspricht.

Analytische Daten:
¹H-NMR (250 MHz, CD₂Cl₂, 25°C): δ = 9,35 (d, J = 8 Hz, 1H); 9,26 (d, J = 7 Hz, 1H); 8,53 (d, J = 8 Hz, 1H); 8,.13 (s, 2H); 7,71 (m, 2H); 7,47 (d, J = 8 Hz, 2H); 7,36 (m, 5H); 7,23 (d, J = 7 Hz, 2H); 7.02 (d, J = 8 Hz, 4H); 6,66 (d, J = 8 Hz, 2H); 2,94 (m, 6H); 2,60 (m, 2H); 1,65 (s, 4H); 1,30 (s, 12H); 1,02 (d, J = 6 Hz, 12H); 0,65 (s, 18H) ppm; MS (FD): m/z (rel. Int.) = 1033,2 (100%) [M⁺].

### Schritt b):

Eine Mischung von 250 mg (0,25 mmol) N-(2,6-Diisopropylphenyl)-9-(4-dimethylaminophenyl)ethinyl-1,6-bis[(4-tert.-octyl)phenoxy]perylen-3,4-dicarbonsäureimid, 2 g (0,036 mol) Kaliumhydroxid und 500 ml Isopropanol wurde 2 d auf Rückflußtemperatur (etwa 82°C) erhitzt.

Nach Abkühlen auf Raumtemperatur wurde das Reaktionsgemisch auf 1 I Wasser gegeben, mit 25 gew.-%iger Salzsäure neutralisiert, auf 100°C erhitzt und 0,5 h bei dieser Temperatur gerührt. Das auf diese Weise ausgefällte Produkt wurde abfiltriert, mit Wasser neutral gewaschen, getrocknet und einer Säulenchromatographie an Kieselgel mit einem Methylenchlorid/-Pentan-Gemisch (1:1) als Eluens unterzogen.

Es wurden 100 mg I6 in Form eines violetten Feststoffs erhalten, was einer Ausbeute von 50% entspricht.

Analytische Daten von I6:
¹H-NMR (250 MHz, CD₂Cl₂, 25°C): δ = 9,29 (d, J = 8 Hz, 1 H); 9,14 (d, J = 8 Hz, 1 H); 8,39 (d, J = 8 Hz, 1 H); 8,.00(s, 1 H); 7,98 (s, 1 H); 7,54 (m, 2H); 7,40 (d, J = 9 Hz, 2H); 7,.35 (d, J = 9 Hz, 2H); 7,02 (d, J = 9 Hz, 4H); 6,60 (d, J = 9 Hz, 4H); 2,94 (s, 6H); 2,60 (m, 2H); 1,69 (s, 4H); 1,32 (s, 12H); 0,69 (s, 18H) ppm;
UV-Vis (CH₂Cl₂): λₘₐₓ (ε) = 570 (35 000) nm (M⁻¹cm⁻¹);
MS (FD): m/z (rel. Int.) = 874,5 (100%) [M⁺].

### Beispiel 7

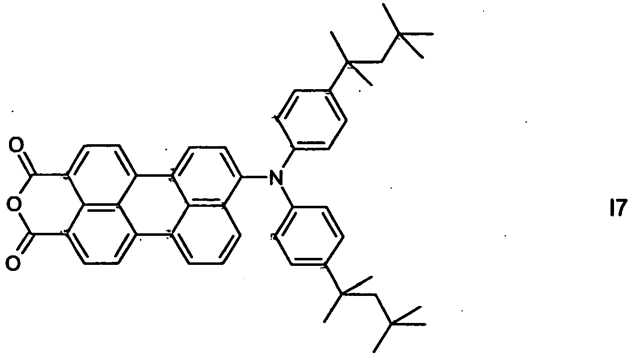

Die Herstellung des Rylenderivats I7 erfolgte ausgehend von N-(2,6-Diisopropylphenyl)-9-bromperylen-3,4-dicarbonsäureimid, das zunächst unter Bromaustausch mit Bis[(4-tert.-octyl)phenyl]amin zum aminosubstituierten Dicarbonsäureimid umgesetzt wurde (Schritt a), welches anschließend alkalisch zum Dicarbonsäureanhydrid I7 verseift wurde (Schritt b).

### Schritt a):

Eine Mischung von 0,5 g (0,89 mmol) N-(2,6-Diisopropylphenyl)-9-bromperylen-3,4dicarbonsäureimid, 0,39 g (0,99 mmol) Bis[(4-tert.-octyl)phenyl]amin, 40 mg (0,043 mmol) Tris(dibenzylidenaceton)dipalladium(0), 32 mg (0,15 mmol) Tris(tert.-butyl)-phosphin, 130 mg (0,13 mmol) Natrium-tert.-butylat und 100 ml trockenem Toluol wurde unter Argon auf 80°C erhitzt und 1 d bei dieser Temperatur gerührt.

Nach Abdestillieren des Lösungsmittels wurde das Rohprodukt einer Säulenchromatographie an Kieselgel mit einem Methylenchlorid/Pentan-Gemisch (1:2) als Eluens unterzogen.

Es wurden 0,59 mg N-(2,6-Diisopropylphenyl)-9-[bis(4-tert.-octyl)phenyl]aminoperylen-3,4-dicarbonsäureimid in Form eines blauen Feststoffs erhalten, was einer Ausbeute von 77% entspricht.

Analytische Daten:
¹H-NMR (250 MHz, CD₂Cl₂, 25°C): δ = 8,55 (m, 2H); 8,42 (m, 4H); 7,97 (d, J = 8 Hz, 1 H); 7,45(m, 2H); 7,28 (d, J = 7 Hz, 3H); 7,20 (d, J = 8 Hz, 4H); 6,92 (d, J = 8 Hz, 2H); 2,70 (m, 2H); 1,63 (s, 4H); 1,27 (s, 12H); 1,06 (d, J = 6 Hz, 12H); 0.67 (s, 18H) ppm; MS (FD): m/z (rel. Int.) = 873,8 (100%) [M⁺].

### Schritt b):

Eine Mischung von 250 mg (0,29 mmol) N-(2,6-Diisopropylphenyl)-9-[bis(4-tert.-octyl)-phenyl]aminoperylen-3,4-dicarbonsäureimid, 2 g (0,036 mol) Kaliumhydroxid und 500 ml Isopropanol wurde 2 d auf Rückflußtemperatur (etwa 82°C) erhitzt.

Nach Abkühlen auf Raumtemperatur wurde das Reaktionsgemisch auf 1 I Wasser gegeben, mit 25 gew.-%iger Salzsäure neutralisiert, auf 100°C erhitzt und 0,5 h bei dieser Temperatur gerührt. Das auf diese Weise ausgefällte Produkt wurde abfiltriert, mit Wasser neutral gewaschen, getrocknet und einer Säulenchromatographie an Kieselgel mit einem Methylenchlorid/Pentan-Gemisch (1:1) als Eluens unterzogen.

Es wurden 40 mg I7 in Form eines blauen Feststoffs erhalten, was einer Ausbeute von 18% entspricht.

Analytische Daten von I7:
¹H-NMR (250 MHz, CD₂Cl₂, 25°C): δ = 8,50 (m, 2H); 8,37 (m, 4H); 7,99 (d, J = 9 Hz, 1H); 7,34 (m, 2H); 7,21(d, J = 9 Hz, 4H); 6,91 (d, J = 9 Hz,4H); 1,63 (s, 4H); 1,27 (s, 12H); 0,67 (s, 18H) ppm;
UV-Vis (CH₂Cl₂): λₘₐₓ (ε) = 605 (21 000) nm (M⁻¹cm⁻¹);
MS (FD): m/z (rel. Int.) = 714,0 (100%) [M⁺].

### Beispiel 8

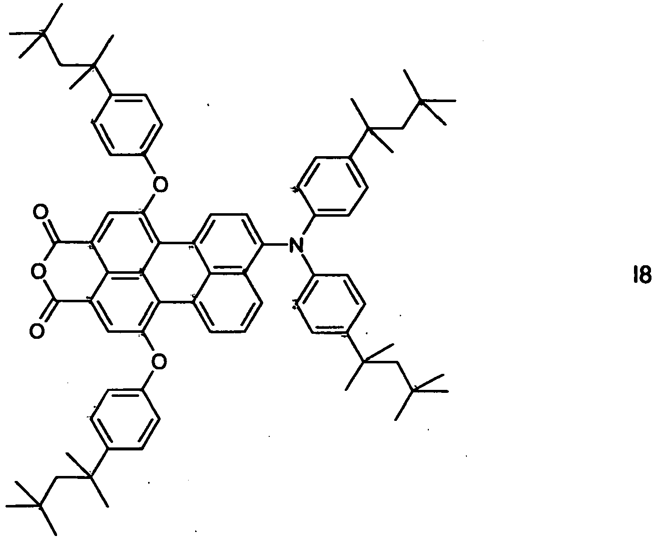

Die Herstellung des Rylenderivats I8 erfolgte ausgehend von N-(2,6-Diisopropyl-phenyl)-1,6-bis[(4-tert.-octyl)phenoxy]-9-bromperylen-3,4-dicarbonsäureimid, das zunächst unter Bromaustausch mit Bis[(4-tert.-octyl)phenyl]amin zum aminosubstituierten Dicarbonsäureimid umgesetzt wurde (Schritt a), welches anschließend alkalisch zum Dicarbonsäureanhydrid I8 verseift wurde (Schritt b).

### Schritt a):

Eine Mischung von 0,86 g (0,89 mmol) N-(2,6-Diisopropylphenyl)-1,6-bis[(4-tert.-octyl)-phenoxy]-9-bromperylen-3,4-dicarbonsäureimid, 0,39 g (0,99 mmol) Bis(4-tert.-octyl-phenyl)amin, 130 mg (0,13 mmol) Tris(dibenzylidenaceton)dipalladium(0), 32 mg (0,15 mmol) Tris(tert.-butyl)phosphin, 130 mg (0,13 mmol) Natrium-tert.-butylat und 100 ml trockenem Toluol wurde unter Argon auf 80°C erhitzt und 1 d bei dieser Temperatur gerührt.

Nach Abdestillieren des Lösungsmittels wurde das Rohprodukt einer Säulenchromatographie an Kieselgel mit einem Methylenchlorid/Pentan-Gemisch (1:2) als Eluens unterzogen.

Es wurde 1 g N-(2,6-Diisopropylphenyl)-1,6-bis[(4-tert.-octyl)phenoxy]-9-[bis(4-tert.-octyl)phenyl]aminoperylen-3,4-dicarbonsäureimid in Form eines blauen Feststoffs erhalten, was einer Ausbeute von 88% entspricht.

Analytische Daten:
¹H-NMR (250 MHz, CD₂Cl₂, 25°C): δ = 9,34 (m, 2H); 8,21 (s, 2H); 8,09 (d, J = 8 Hz, 1 H); 7,44(m, 5H); 7,33 (m, 8H); 7,08 (m, 4H); 6,97 (d, J = 8 Hz, 4H); 2,72 (m, 2H); 1,75 (d, J = 9 Hz, 8H); 1,39 (d, J = 10 Hz, 24H); 1,13 (d, J = 7 Hz, 12H); 0.74 (s, 36H) ppm; MS (FD): m/z (rel. Int.) = 1291,3 (100%) [M⁺].

### Schritt b):

Eine Mischung von 250 mg (0,20 mmol) N-(2,6-Diisopropylphenyl)-1,6-bis[(4-tert.-octyl)phenoxy]-9-[bis(4-tert.-octyl)phenyl]aminoperylen-3,4-dicarbonsäureimid, 2 g (0,036 mol) Kaliumhydroxid, und 500 ml Isopropanol wurde 2 d auf Rückflußtemperatur (etwa 82°C) erhitzt.

Nach Abkühlen auf Raumtemperatur wurde das Reaktionsgemisch auf 1 I Wasssr gegeben, mit 25 gew.-%iger Salzsäure neutralisiert, auf 100°C erhitzt und 0,5 h bei dieser Temperatur gerührt. Das auf diese Weise ausgefällte Produkt wurde abfiltriert, mit Wasser neutral gewaschen, getrocknet und einer Säulenchromatographie an Kieselgel mit einem Methylenchlorid/Pentan-Gemisch (1:1) als Eluens unterzogen.

Es wurden 100 mg I8 in Form eines blauen Feststoffs erhalten, was einer Ausbeute von 40% entspricht.

Analytische Daten von I8:
¹H-NMR (250 MHz, CD₂Cl₂, 25°C): δ = 9,33 (m, 2H); 8,14 (s, 2H); 8,09 (d, J = 8 Hz, 1H); 7,44 (m, 4H); 7,22 (m, 6H); 7,08 (m, 4H); 6,95 (d, J = 8 Hz, 4H); 1,76 (d, J = 16 Hz, 8H); 1,39 (d, J = 16 Hz, 24H); 0,73 (m, 36H) ppm;
UV-Vis (CH₂Cl₂): λₘₐₓ (ε) = 600 (32 000) nm (M⁻¹cm⁻¹);
MS (FD): m/z (rel. Int.) = 1122,4 (100%) [M⁺].

### Beispiel 9

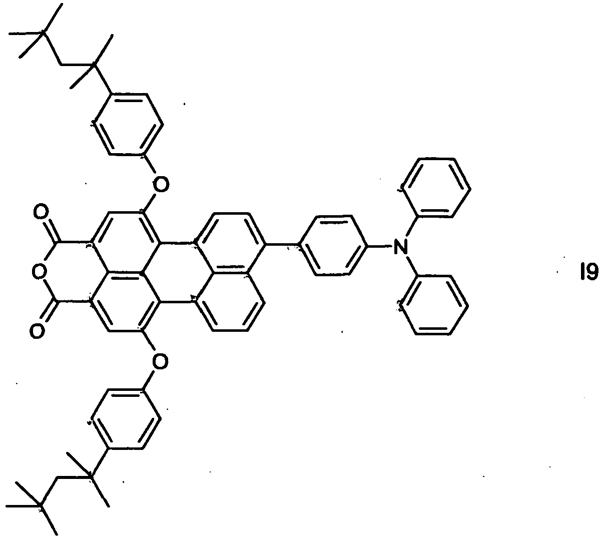

Die Herstellung des Rylenderivats I9 erfolgte ausgehend von N-(2,6-Diisopropyl-phenyl)-1,6-bis[(4-tert.-octyl)phenoxy]-9-(4,4,5,5-tetramethyl-1,3,2-dioxaboran-2-yl)-perylen-3,4-dicarbonsäureimid, das zunächst einer Suzuki-Kupplung mit N,N-Diphenyl-4-bromanilin zum aminophenylsubstituierten-Dicarbonsäureimid unterzogen wurde (Schritt a), welches anschließend alkalisch zum Dicarbonsäureanhydrid I9 verseift wurde (Schritt b).

### Schritt a):

Eine Mischung von 400 mg (0,4 mmol) N-(2,6-Diisopropylphenyl)-1,6-bis[(4-tert.-octyl)-phenoxy]-9-(4,4,5,5-tetramethyl-1,3,2-dioxaboran-2-yl)perylen-3,4-dicarbonsäureimid, 193 mg (0,6 mmol) N,N-Diphenyl-4-bromanilin und 70 ml trockenem Toluol wurde unter Argon in einem 250 ml-Schleckrohr vorgelegt, dann wurden ebenfalls unter Argon 15 ml einer 1 m wäßrigen Kaliumcarbonatlösung und 80 mg (0,07 mmol) Tetrakis(tri-phenylphosphin)palladium(0) zugegeben. Die Mischung wurde unter Argon dann auf 90°C erhitzt und 16 h bei dieser Temperatur gehalten.

Nach Abkühlen auf Raumtemperatur wurde die organische Phase abgetrennt, dann wurde das Lösungsmittel unter Vakuum entfernt. Das erhaltene Rohprodukt wurde einer Säulenchromatographie an Kieselgel mit Methylenchlorid als Eluens unterzogen.

Es wurden 200 mg N-(2,6-Diisopropylphenyl)-1,6-bis[(4-tert.-octyl)phenoxy]-9-[(4-di-phenylamino)phenyl]perylen-3,4-dicarbonsäureimid in Form eines violetten Feststoffs erhalten, was einer Ausbeute von 45% entspricht.

Analytische Daten:
¹H-NMR (250 MHz, CD₂Cl₂, 25°C): δ = 9,37 (m, 2H); 8,23 (s, 2H); 8,14 (d, J = 8 Hz, 1H); 7,59 (m, 2H); 7,44 (m, 7H); 7,34 (m, 6H); 7,20 (d, J = 8 Hz, 6H); 7,08 (m, 6H); 2,70 (m, 2H); 1,73 (s, 4H); 1,37 (s, 12H); 1,11 (d, J = 7 Hz, 12H); 0,72 (s, 18H) ppm; MS (FD): m/z (rel. Int.) = 1133,1 (100%) [M⁺].

### Schritt b):

Eine Mischung von 200 mg (0,18 mmol) N-(2,6-Diisopropylphenyl)-1,6-bis[(4-tert.-octyl)phenoxy]-9-[(4-di-phenylamino)phenyl]perylen-3,4-dicarbonsäureimid, 2 g (0,036 mol) Kaliumhydroxid, 1 g (0,017 mol) Kaliumfluorid und 500 ml Isopropanol wurde 1 d auf Rückflußtemperatur (etwa 82°C) erhitzt.

Nach Abkühlen auf Raumtemperatur wurde das Reaktionsgemisch auf 1 I Wasser gegeben, mit 25 gew.-%iger Salzsäure neutralisiert, auf 100°C erhitzt und 0,5 h bei dieser Temperatur gerührt. Das auf diese Weise ausgefällte Produkt wurde abfiltriert, mit Wasser neutral gewaschen, getrocknet und einer Säulenchromatographie an Kieselgel mit einem Methylenchlorid/Pentan-Gemisch (1:1) als Eluens unterzogen.

Es wurden 100 mg 19 in Form eines blauen Feststoffs erhalten, was einer Ausbeute von 61% entspricht.

Analytische Daten von I9:
¹H-NMR (500 MHz, CD₂Cl₂, 25°C): δ = 9,36 (m, 2H); 8,15 (d, J = 8 Hz, 1H); 8,08 (s, 2H); 7,60 (m, 2H); 7,42 (m, 6H); 7,31 (m, 4H); 7,20 (d, J = 8 Hz, 6H); 7,04 (m, 6H); 1,76 (s, 4H); 1,40 (s, 12H); 0,77 (s, 18H) ppm;
UV-Vis (CH₂Cl₂): λₘₐₓ (ε) = 541 (35 000) nm (M⁻¹cm⁻¹);
MS (FD): m/z (rel. Int.) = 974,8 (100%) [M⁺].

### Beispiel 10

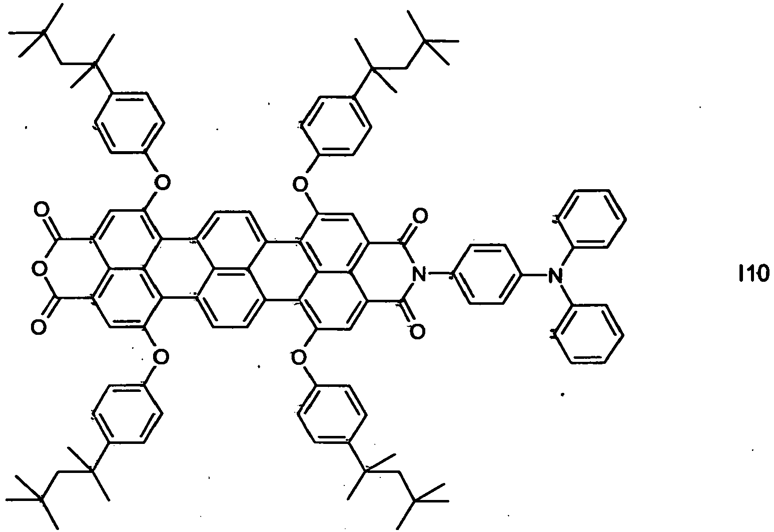

Zu einer Mischung von 0,250 g (0,19 mmol) 1,6,9,14-Tetra[(4-tert.-octyl)phenoxy]-terrylen-3,4:11,12-tetracarbonsäuredianhydrid und 25 ml wasserfreiem N-Methylpyrrolidon wurden unter Stickstoff 0,028 g, (0,15 mmol) wasserfreies Zinkacetat und 0,039 g (0,15 mmol) 4-Diphenylaminoanilin gegeben. Die Mischung wurde dann unter Stickstoff auf 130°C erhitzt und 24 h bei dieser Temperatur gerührt.

Nach Abkühlen auf Raumtemperatur wurde das Produkt durch Zugabe von 10 gew.-%iger Salzsäure ausgefällt, abfiltriert, zunächst mit 10 gew.-%iger Salzsäure und anschließend mit heißem Wasser gewaschen und dann einer Säulenchromatographie an Kieselgel mit Toluol als Eluens unterzogen.

Es wurden 0,03 g 110 in Form eines blaugrünen Feststoffs erhalten, was einer Ausbeute von 10% entspricht.

Analytische Daten von I10:
¹H-NMR (400 MHz, COCl₃, 25°C): δ = 9,51 (s, 4H); 8,27 (s, 2H); 8,15 (s, 2H); 7,49-7,40 (m, 8H); 7,29-7,21 (m, 6H); 2,70 (m, 2H); 1,79 (d, 8H, J = 15,0 Hz); 1,40 (d, 24H, J = 11,0 Hz); 1,02 (d, 12 H, J = 6,8 Hz); 0,75 (d, 36H, J = 24,0 Hz) ppm;
UV-Vis (Toluol): λₘₐₓ = 670, 616 nm;
MS (FD): m/z (rel. Int.) = 1575,7 (100%) [M⁺].

### Beispiel 11

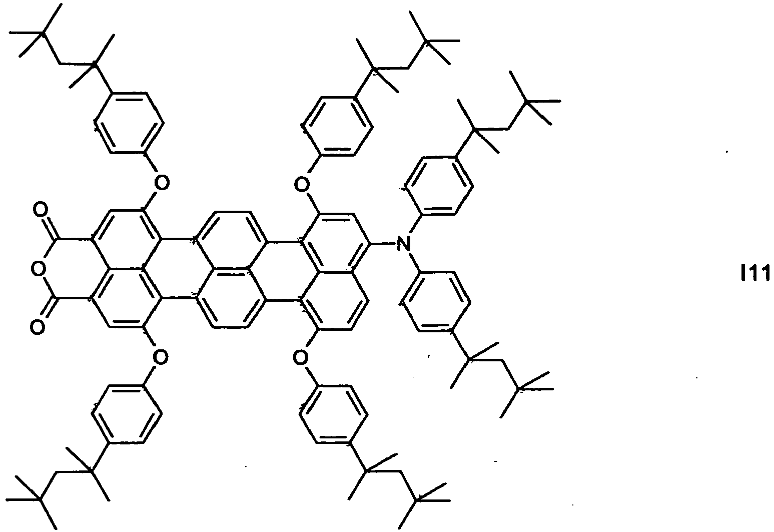

Die Herstellung des Rylenderivats I11 erfolgte ausgehend von N-(2,6-Diisopropylphenyl)-1,6,9,14-tetra[(4-tert.-octyl)phenoxy]-11-bromterrylen-3,4-dicarbonsäureimid, das zunächst unter Bromaustausch mit Bis[(4-tert.-octyl)phenyl]amin zum aminosubstituierten Dicarbonsäureimid umgesetzt wurde (Schritt a), welches anschließend alkalisch zum Dicarbonsäureanhydrid I11 verseift wurde (Schritt b).

### Schritt a):

Eine Mischung von 0,39 g (0,26 mmol) N-(2,6-Diisopropylphenyl)-1,6,9,14-tetra[(4-tert.-octyl)phenoxy]-11-bromterrylen-3,4-dicarbonsäureimid, 0,119 g (0,28 mmol) Bis(4-tert.-octylphenyl)amin, 0,01 g (0,012 mmol) Tris(dibenzylidenaceton)dipalladium(0), 0,1 g (0,11 mmol) Tris(tert.-butyl)phosphin, 0,01 g (0,05 mmol) Natrium-tert.-butylat und 20 ml trockenem Toluol wurde unter Argon auf 80°C erhitzt und 16 h bei dieser Temperatur gerührt.

Nach Abdestillieren des Lösungsmittels wurde das Rohprodukt einer Säulenchromatographie an Kieselgel mit einem Hexan/Ethylacetat-Gemisch (10:1) als Eluens unter-zogen.

Es wurden 0,29 g N-(2,6-Diisopropylphenyl)-1,6,9,14-tetra[(4-tert.-octyl)phenoxy]-9-[bis(4-tert.-octyl)phenyl]aminoterrylen-3,4-dicarbonsäureimid in Form eines blauen Feststoffs erhalten, was einer Ausbeute von 61 % entspricht.

Analytische Daten:
MS (FD): m/z (rel. Int.) = 1653,9 (100%) [M⁺].

### Schritt b):

Eine Mischung von 0,30 g (0,17 mmol) N-(2,6-Diisopropylphenyl)-1,6,9,14-tetra[(4-tert.-octyl)phenoxy]-9-[bis(4-tert.-octyl)phenyl]aminoterrylen-3,4-dicarbonsäureimid und 30 ml 2-Methyl-2-butanol wurde auf 60°C erhitzt. Nach 0,5 h wurden 0,28 (5 mmol) Kaliumhydroxid und 0,29 g (5 mmol) Kaliumfluorid zugegen. Dann wurde die Mischung auf leichten Rückfluß (etwa 118°C) erhitzt und 16 h bei dieser Temperatur gerührt.

Nach Abkühlen auf 50°C, Ansäuern mit 50 gew.-%iger Essigsäure und weiterem zweistündigen Rühren bei 80°C wurde das Reaktionsprodukt auf Wasser gefällt, abfiltriert, mit heißem Wasser gewaschen, im Vakuum bei 70°C getrocknet und danach einer Säulenchromatographie an Kieselgel mit einem Ethylacetat/Hexan-Gemisch (1:19) als Eluens unterzogen.

Es wurden 0,16 g I11 in Form eines blaugrünen Feststoffs erhalten, was einer Ausbeute von 59% entspricht.

Analytische Daten von I11:
¹H-NMR (500 MHz, CD₂Cl₂, 25°C): δ = 9,41-9,38 (m, 4H); 9,09 (dd, 2H); 8,10 (s, 2H); 7,83 (d, 1 H); 7,42 (d, 4H); 7,38 (d, 2H); 7,27 (d, 2H); 7,20 (d, 4H); 7,06 (d, 4H); 7,00-6,97 (m, 3H); 6,95-6,87 (m, 7H); 1,75-1,69 (m, 12H); 1,40-1,30 (m, 36H); 0,75-0,65 (m, 54H) ppm;
UV-Vis (CH₂Cl2): λₘₐₓ = 692 nm;
MS (FD): m/z (rel. Int.) = 1653,9 (100%) [M⁺].

### Beispiel 12

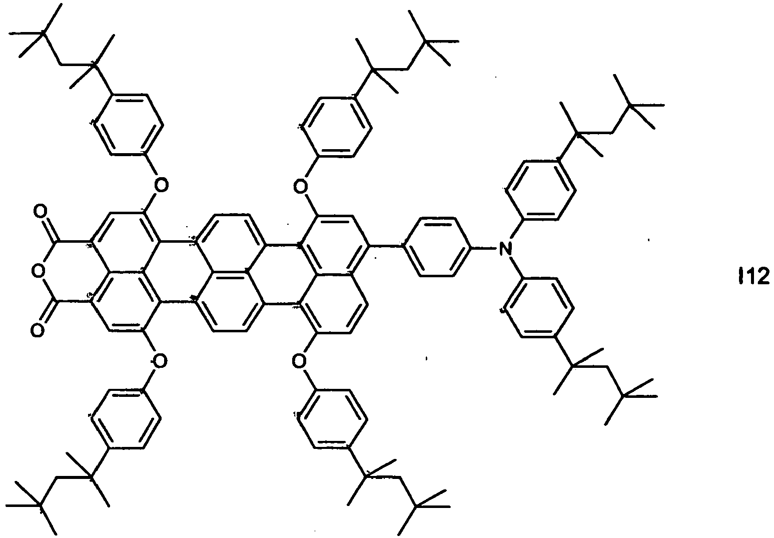

Die Herstellung des Rylenderivats I12 erfolgte ausgehend von N-(2,6-Diisopropyl-phenyl)-1,6,9,14-tetra[(4-tert.-octyl)phenoxy]-11-bromterrylen-3,4-dicarbonsäureimid, das zunächst einer Suzuki-Kupplung mit N,N-Bis[(4-tert.-octyl)phenyl]-4-(4,4,5,5-tetramethyl-1,3,2-dioxaboran-2-yl)anilin zum aminophenylsubstituierten Dicarbonsäureimid unterzogen wurde (Schritt a), welches anschließend alkalisch zum Dicarbonsäureanhydrid I12 verseift wurde (Schritt b).

Das in Schritt a) als Edukteingesetzte N,N-Bis[(4-tert.-octyl)phenyl]-4-(4,4,5,5-tetramethyl-1,3,2-dioxaboran-2-yl)anilin wurde in einer zweistufigen Reaktion ausgehend von Bis(4-tert.-octylphenyl)amin wie folgt hergestellt:

### 1) Bis(4-tert.-octylphenyl)(4-bromphenyl)amin:

Eine Mischung von 12 g (30 mol) Bis(4-tert.-octylphenyl)amin, 9,4 g (0,04 mmol) 1,4-Dibrombenzol und 20 ml wasserfreiem Toluol wurde bei Raumtemperatur gerührt. Nach 10 min wurden 0,14 g (0,16 mmol) Tris(dibenzylidenaceton)dipalladium(0), 0,15g (0,24 mmol) BINAP und 1,2 g (12 mmol) Natrium-tert-butylat zugegeben. Dann wurde die Mischung auf 90°C erhitzt und 72 h bei dieser Temperatur gerührt.

Das auf Raumtemperatur abgekühlte Reaktionsgemisch wurde auf 100 ml Wasser gegeben. Die durch Extraktion mit Toluol abgetrennte organische Phase wurde mit Wasser gewaschen, dann wurde das Lösungsmittel unter Vakuum entfernt. Das Rohprodukt wurde einer Säulenchromatographie an Kieselgel mit Hexan als Eluens unterzogen.

Es wurden 5,9 g in Form eines weißen Feststoffs erhalten, was einer Ausbeute von 36% entspricht.

Analytische Daten:
¹H-NMR (500 MHz, CDCl₃, 25°C): 8 = 7,29-7.27 (m, 6H); 6.95 (d, 4H); 6.85 (d, 2H); 6,82 (d, 2H); 1,75 (s, 4H); 1,38 (s, 12 H); 0,76 (s, 18 H) ppm;
MS (FD): m/z (rel. Int.) = 322 (100%) [M⁺].

### 2) N,N-Bis[(4-tert.-octyl)phenyl]-4-(4,4,5,5-tetramethyl-1,3,2-dioxaboran-2-yl)anilin:

Eine Mischung von 3 g (5 mmol) Bis(4-tert.-octylphenyl)(4-bromphenyl)amin, 1,3 g (14 mmol) Kaliumacetat, 3,7 g (15 mmol) Bis(pinacolato)diboran und 0,2 g (0,3 mmol) [1,1'-Bis(diphenylphosphino)ferrocen]palladium(II)chlorid und 45 ml Dioxan wurde unter Stickstoff auf 70°C erhitzt und 16 h bei dieser Temperatur gerührt.

Nach Abkühlen auf Raumtemperatur wurde das Lösungsmittel unter Vakuum entfernt. Das Rohprodukt wurde einer Säulenchromatographie an Kieselgel mit einem Methylenchlorid/Hexan-Gemisch (1:1) als Eluens unterzogen.

Es wurden 1,6 g N,N-Bis[(4-tert.-octyl)phenyl]-4-(4,4,5,5-tetramethyl-1,3,2-dioxaboran-2-yl)anilin in Form eines weißen Feststoffs erhalten, was einer Ausbeute von 54% entspricht.

Analytische Daten:
¹H-NMR (500 MHz, CDCl₃, 25°C): δ = 7,57 (d, 2H); 7.28 (d, 4H); 7,00 (d, 4H); 6,92 (d, 2H); 1,74 (s, 4H); 1,38 (s, 12 H); 0,76 (s, 18 H) ppm;
MS (FD): m/z (rel. Int.) = 596,4 (100%) [M⁺].

### Schritt a):

Zu einer Mischung von 0,3 g (0,20 mmol) N-(2,6-Diisopropylphenyl)-1,6,9,14-tetra[(4-tert.-octyl)phenoxy]-11-bromterrylen-3,4-dicarbonsäureimid, 0,525 g (0,88 mmol) N,N-Bis[(4-tert.-octyl)phenyl]-4-(4,4,5,5-tetramethyl-1,3,2-dioxaboran-2-yl)anilin und 25 ml trockenem Toluol wurden unter Stickstoff 0,021 g (0,01 mmol) Tetrakis(triphenylphosphin)palladium(0) und 0,1 g (0,072 mol) Kaliumcarbonat, gelöst in 1 ml eines Wasser/Etha-nol-Gemischs (10:1), gegeben. Die Mischung wurde dann unter Stickstoff auf 80°C erhitzt und 16 h bei dieser Temperatur gehalten.

Nach Abkühlen auf Raumtemperatur wurde das Lösungsmittel unter Vakuum entfernt. Das erhaltene Rohprodukt wurde einer Säulenchromatographie an Kieselgel mit einem Methylenchlorid/Hexan-Gemisch (1:1) als Eluens unterzogen.

Es wurden 0,22 g N-(2,6-Diisopropylphenyl)-1,6,9,14-tetra[(4-tert.-octyl)phenoxy]-11[4-(N,N-bis(4-tert.-octylphenyl)amino)phenyl]terrylen-3,4-dicarbonsäureimid in Form eines blauen Feststoffs erhalten, was einer Ausbeute von 58% entspricht.

Analytische Daten:
¹H-NMR (500 MHz, CDCl₃, 25°C): δ = 9,42-9,38 (m, 2H); 9,18-9,12 (m, 2H);, 8,27 (s, 2H); 7,95-7,91 (m, 1 H); 7,43-7,40 (m, 1 H); 7,40 -7,32 (m, 8H); 7,30-7,32(m, 9H); 7,10-7,00 (m, 15H); 2,72-2,68 (m, 2H); 1,75-1,69 (m, 12H); 1,40-1,25 (m, 36H); 0,80-0,70 (m, 54H) ppm;
MS (FD): m/z (rel. Int.) = 1889,2 (100%) [M⁺].

### Schritt b):

Eine Mischung von 0,10 g (0,053 mmol) N-(2,6-Diisopropylphenyl)-1,6,9,14-tetra[(4-tert.-octyl)phenoxy]-11-[4-(N,N-bis(4-tert.-octylphenyl)amino)phenyl]terrylen-3,4-dicar-bonsäureimid und 18 ml 2-Methyl-2-butanol wurde auf 60°C erhitzt. Nach 0,5 h wurden 0,089 g (1,59 mmol) Kaliumhydroxid und 0,093 g (1,59 mmol) Kaliumfluorid zugegen. Dann wurde die Mischung auf leichten Rückfluß (etwa 118°C) erhitzt und 21 h bei dieser Temperatur gerührt.

Nach Abkühlen auf 50°C wurde das Reaktionsgemisch mit 50 gew.%iger Essigsäure leicht angesäuert und 1 h bei dieser Temperatur gerührt. Das Produkt wurde auf 250 ml Wasser gefällt, abfiltriert, mit Wasser neutral gewaschen, im Vakuum bei 70°C getrocknet und dann einer Säulenchromatographie an Kieselgel mit einem Chloroform/- Ethanol-Gemisch (19:1) als Eluens unterzogen.

Es wurden 0,8 g 112 in Form eines blaugrünen Feststoffs erhalten, was einer Ausbeute von 87% entspricht.

Analytische Daten von I12:
UV-Vis (CH₂Cl₂): λₘₐₓ = 676 nm;
MS (FD): m/z (rel. Int.) = 1730,0 (100%) [M⁺].

### Beispiel 13

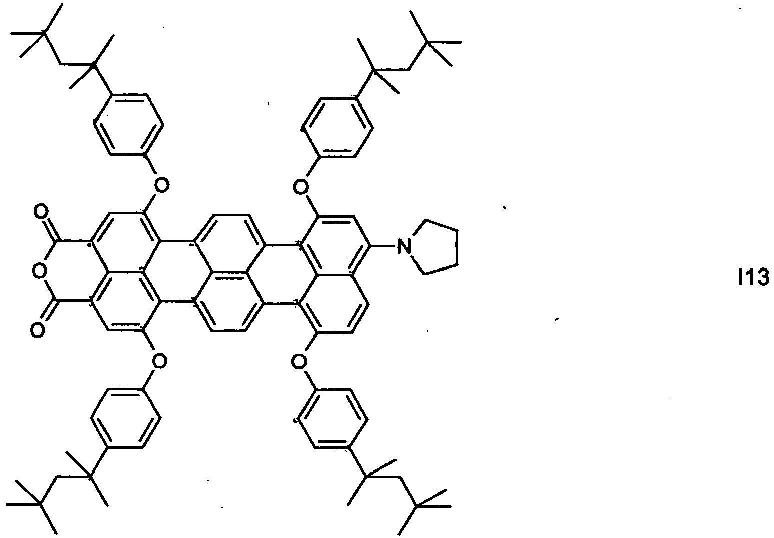

Die Herstellung des Rylenderivats 113 erfolgte ausgehend von N-(2,6-Diisopropyl-phenyl)-1,6,9,14-tetra[(4-tert.-octyl)phenoxy]-11-bromterrylen-3,4-dicarbonsäureimid, das zunächst unter Bromaustausch mit Pyrrolidin zum pyrrolidylsubstituierten Dicarbonsäureimid umgesetzt wurde (Schritt a), welches anschließend alkalisch zum Dicarbonsäureanhydrid I13 verseift wurde (Schritt b).

### Schritt a):

Zu einer Lösung von 0,10 g(0,07 mmol) N-(2,6-Diisopropylphenyl)1,6,9,14-tetra[(4-tert.-octyl)phenoxy]-11-bromterrylen-3,4-dicarbonsäureimid in 10 ml Dimethylformamid wurden unter Stickstoff 0,51 ml (6 mmol) Pyrrolidin gegeben, dann wurde die Mischung unter Stickstoff auf 90°C erhitzt. Nach 3 h war ein Farbumschlag von blau nach grün zu beobachten.

Nach Abkühlen auf Raumtemperatur wurde das Lösungsmittel unter Vakuum abdestilliert. Das Rohprodukt wurde einer Säulenchromatographie an Kieselgel mit Chloroform als Eluens unterzogen.

Es wurden 0,065 g N-(2,6-Diisopropylphenyl)-1,6,9,14-tetra[(4-tert.-octy))phenoxyl]-9-pyrrolidylterrylen-3,4-dicarbonsäureimid in Form eines blauen Feststoffs erhalten, was einer Ausbeute von 65% entspricht.

Analytische Daten:
UV-Vis (CHCl₃): λₘₐₓ (ε) = 706 (83 000) nm (M⁻¹cm⁻¹);
MS (FD): m/z (rel. Int.) = 1490,7 (100%) [M⁺].

### Schritt b):

Eine Mischung von 0,30 g (0,17 mmol) N-(2,6-Diisopropylphenyl)-1,6,9,14-tetra[(4-tert.-octyl)phenoxy]-9-[bis(4-tert.-octyl)phenyl]aminoterrylen-3,4-dicarbonsäureimid und 30 ml 2-Methyl-2-butanol wurde auf 60°C erhitzt. Nach 0,5 h wurden 0,28 g(5 mmol) Kaliumhydroxid und 0,29 g (5 mmol) Kaliumfluorid zugegen. Dann wurde die Mischung auf leichten Rückfluß (etwa 118°C) erhitzt und 16 h bei dieser Temperatur gerührt.

Nach Abkühlen auf 50°C, Ansäuern mit 50 gew.-%iger Essigsäure und weiterem zweistündigen Rühren bei 80°C wurde das Reaktionsprodukt auf Wasser gefällt, abfiltriert, mit heißem Wasser gewaschen; im Vakuum bei 70°C getrocknet und danach einer Säulenchromatographie an Kieselgel mit einem Ethylacetat/Hexan-Gemisch (1:19) als Eluens unterzogen.

Es wurden 0,16 g I13 in Form eines blaugrünen Feststoffs erhalten, was einer Ausbeute von 59% entspricht.

Analytische Daten von I13:
¹H-NMR (500 MHz, CD₂Cl₂, 25°C): δ = 9,41-9,38 (m, 4H); 9,09 (dd, 2H); 8,10 (s, 2H); 7,83 (d, 1 H); 7,42 (d, 4H); 7,38 (d, 2H); 7,27 (d, 2H); 7,20 (d, 4H); 7,06 (d, 4H); 7,00-6,97 (m, 3H); 6,95-6,87 (m, 7H); 1,75-1,69 (m, 12H); 1,40-1,30 (m, 36H); 0,75-0,65 (m, 54H) ppm;
MS (FD): m/z (rel. Int.) = 1653,9 (100%) [M⁺].

### Beispiel 14

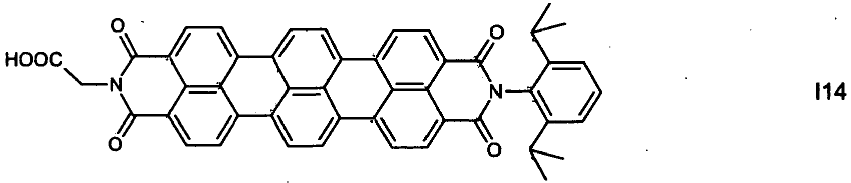

Eine Mischung von 69,9 g (0,71 mol) Natrium-tert.-butylat, 68,4 ml Diethylenglykoldiethylether und 126,5 ml (0,85 mol) DBU wurde in einem Schlenckrohr unter Rühren und unter Stickstoff auf 60°C erhitzt. Nach 20 min wurden 17,6 g (0,035 mol) N-(2,6-Diisopropylphenyl)perylen-3,4-dicarbonsäureimid und nach weiteren 5 min 18,0 g (0,071 mol) N-(2-Carboxyethyl)naphthalin-1,8-dicarbonsäureimid unter Stickstoff zugegeben. Die Mischung wurde dann unter Stickstoff auf 120°C erhitzt und 6 h bei dieser Temperatur gerührt.

Nach Abkühlen auf Raumtemperatur wurde das Reaktionsgemisch etwa 12 h an Luft stehen gelassen und dann in 3 I Wasser gegeben. Das auf diese Weise ausgefällte Produkt wurde abfiltriert, mit Wasser gewaschen und getrocknet, dann mit 13 gew.-%iger Salzsäure angesäuert, erneut mit Wasser gewaschen und getrocknet und abschließend zunächst in Methanol und dann in Ethanol aufgekocht, abfiltriert, mit heiβem Wasser gewaschen und getrocknet.

Es wurden 4,34 g 114 in Form eines blauen Feststoffs erhalten, was einer Ausbeute von 13% entspricht.

Analytische Daten von I14:
MS (FD): m/z (rel. Int.) = 733,2 (100%) [M⁺].

### Beispiel 15

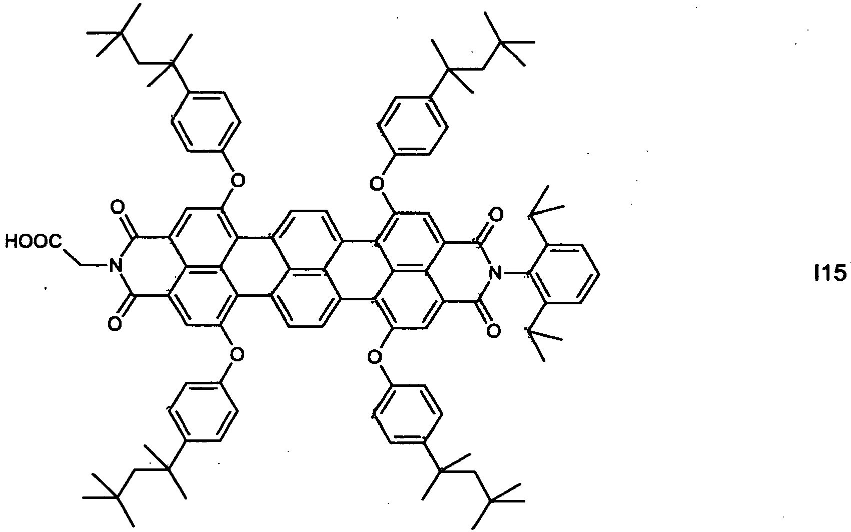

Zur Herstellung des Rylenderivats I15 wurde das im Rylengerüst unsubstituierte Rylenderivat 113 zunächst durch Umsetzung mit Brom in das Tetrabromderivat überführt (Schritt a), das dann mit 4-(tert.-Octyl)phenol zum Rylenderivat I15 umgesetzt wurde (Schritt b).

### Schritt a):

Eine Mischung von 0,3 g (4 mmol) I13 in 40 ml Chloroform wurde auf 60°C erhitzt und 10 min bei dieser Temperatur gerührt. Nach Abkühlen auf 40°C wurden zunächst 80 ml Wasser und dann 16,0 g (0,10 mol) Brom zugegeben. Die Mischung wurde dann 10 h bei 40°C gerührt. Der Reaktionsverlauf wurde dabei dünnschichtchromatographisch verfolgt (Laufmittel: Methylenchlorid/Aceton 3:1).

Nach Abkühlen auf Raumtemperatur und Entfernen des überschüssigen Broms mit Stickstoff wurde das Reaktionsgemisch nach Zugabe einer wäßrigen Lösung von Na-triumdisulfit und Methylenchlorid weiter gerührt. Die abgetrennte organische Phase wurde mit Magnesiumsulfat getrocknet, dann wurde das Lösungsmittel im Vakuum entfernt.

Es wurden 3,5 g 1,6,9,14-tetrabromiertes Rylenderivat 113 erhalten, was einer Ausbeute von 83% entspricht.

Analytische Daten:
MS (FD): m/z (rel. Int.) = 1048,6 (100%) [M⁺].

### Schritt b):

Eine Mischung von 3,0 g (2,9 mmol) 1,6,9,14-tetrabromiertem Rylenderivat I13 und 200 ml wasserfreiem N-Methylpyrrolidon wurde mit Stickstoff gespült, anschließend wurden 4,4 g (21,8 mmol) tert.-Octylphenol und 2,2 g (16,0 mmol) Kaliumcarbonat zugegeben. Die Mischung wurde dann auf 80°C erhitzt und 16 h bei dieser Temperatur gerührt.

Nach Abkühlen auf Raumtemperatur wurde das Produkt durch Zugabe von 100 ml verdünnter Salzsäure ausgefällt, abfiltriert, mit Wasser neutral gewaschen und getrocknet.

Es wurden 2,0 g I15 in Form eines blauen Feststoffs erhalten, was einer Ausbeute von 43% entspricht.

Analytische Daten von I15:
UV-Vis (Ethanol): λₘₐₓ = 665, 623 nm;
MS (FD): m/z (rel. Int.) = 1550,3 (100%) [M⁺].

### Beispiel 16

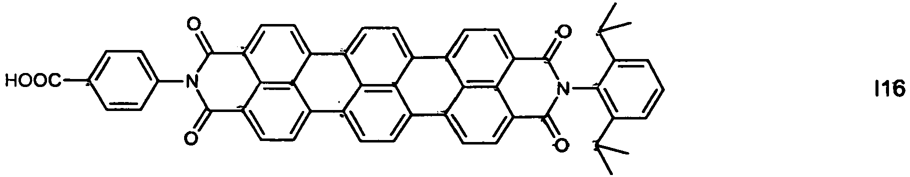

Das bei der Herstellung des Rylenderivats I16 eingesetzte N-(4-Carboxyphenyl)naph-thalin-1,8-dicarbonsäureimid wurde wie folgt hergestellt.

Zu einer unter Stickstoff vorgelegten Mischung von 8,24 g (0,04 mmol) Naphthalin-1,8-dicarbonsäureanhydrid und 40 ml wasserfreiem N-Methylpyrrolidon wurde unter Stickstoff wurden 7,34 g (0,04 mmol) wasserfreies Zinkacetat und 21,94 g (0,16 mmol) 4-Aminobenzoesäure gegeben. Die Mischung wurde dann auf 160°C erhitzt und 15 h bei dieser Temperatur gerührt.

### Nach Abkühlen auf Raumtemperatur wurde das Produkt durch Zugabe von 1 I5 gew.-%iger Salzsäure ausgefällt, abfiltriert, zunächst mit 5 gew.-%iger Salzsäure und dann mit heißem Wasser gewaschen und getrocknet.

Es wurden 12,7 g N-(4-Carboxyphenyl)naphthalin-1,8-dicarbonsäureimid in Form eines weißen Feststoffs erhalten, was einer Ausbeute von 100% entspricht.

Analytische Daten:
UV-Vis (CH₂Cl₂): λₘₐₓ = 640, 595 nm;
¹H-NMR (400 MHz, THF, 25°C): δ = 8,58 (d, 2H); 8,39 (d, 2H); 8,17 (d, 2H); 7,91 (t, 2H); 7,07 (d, 2H) ppm.

Eine Mischung von 49,9 g (0,50 mol) Natrium-tert.-butylat, 47,4 ml Diethylenglykoldiethylether und 90,3 ml (0,61 mol) DBU wurde in einem Schlenckrohr unter Rühren und unter Stickstoff auf 60°C erhitzt. Nach 20 min wurden 12,6 g (0,023 mol) N-(2,6-Diisopropylphenyl)perylen-3,4-dicarbonsäureimid und nach weiteren 5 min 16,0 g (0,050 mol) N-(4-Carboxyphenyl)naphthalin-1,8-dicarbonsäureimid unter Stickstoff zugegeben. Die Mischung wurde dann unter Stickstoff auf 120°C erhitzt und 6 h bei dieser Temperatur gerührt.

Nach Abkühlen auf 60°C und Zugabe von 150 ml Wasser wurde das Reaktionsgemisch etwa 12 h an Luft stehen gelassen und dann in 1 I Wasser gegeben. Das auf diese Weise ausgefällte Produkt wurde abfiltriert, zunächst mit Wasser und dann mit Chloroform gewaschen und getrocknet, dann mit 13 gew.-%iger Salzsäure angesäuert, erneut mit Wasser gewaschen und getrocknet und abschließend durch eine Soxhlett-Extraktion mit Methanol gereinigt.

Es wurden 5,1 g I16 in Form eines blauen Feststoffs erhalten, was einer Ausbeute von 25% entspricht.

Analytische Daten von I16:
UV-Vis (CHCl₃): λₘₐₓ (ε) = 658 (89 000), 600 (45 000) nm (M⁻¹cm⁻¹);
MS (FD): m/z (rel. Int.) = 795,2 (100%) [M⁺].

### Beispiel 17

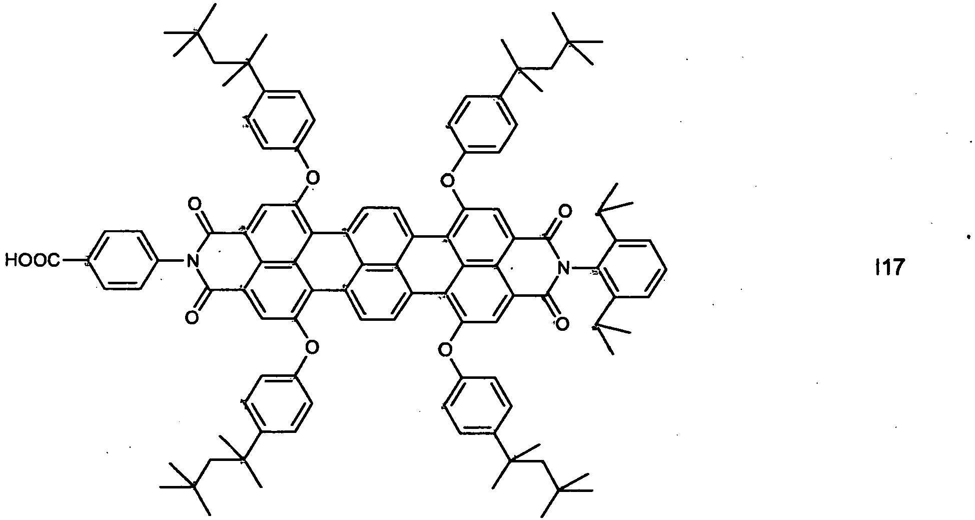

Zur Herstellung des Rylenderivats I17 wurde das im Rylengerüst unsubstituierte Rylenderivat I15 zunächst durch Umsetzung mit Brom in das Tetrabromderivat überführt (Schritt a), das dann mit 4-(tert.-Octyl)phenol zum Rylenderivat I17 umgesetzt wurde (Schritt b).

### Schritt a):

Eine Mischung von 2,0 g (2,5 mmol) I15 in 26 ml Chloroform wurde auf 60°C erhitzt und 10 min bei dieser Temperatur gerührt. Nach Abkühlen auf 40°C wurden zunächst 52 ml Wasser und dann 12,8 g (80,0 mmol) Brom zugegeben. Die Mischung wurde dann 10 h bei 40°C gerührt. Der Reaktionsverlauf wurde dabei dünnschichtchromatographisch verfolgt (Laufmittel: Methylenchlorid/Aceton 3:1).

Nach Abkühlen auf Raumtemperatur und Entfernen des überschüssigen Broms mit Stickstoff wurde das Reaktionsgemisch nach Zugabe einer wäßrigen Lösung von Na-triumdisulfit und Methylenchlorid weiter gerührt. Die abgetrennte organische Phase wurde mit Magnesiumsulfat getrocknet, dann wurde das Lösungsmittel im Vakuum entfernt.

Es wurden 2,4 g 1,6,9,14-tetrabromiertes Rylenderivat 115 erhalten, was einer Ausbeute von 88% entspricht.

Analytische Daten:
MS (FD): m/z (rel. Int.) = 1110,7 (100%) [M⁺].

### Schritt b):

Eine Mischung von 3,0 g (2,7 mmol) 1,6,9,14-tetrabromiertem Rylenderivat I15 und 200 ml wasserfreiem N-Methylpyrrolidon wurde mit Stickstoff gespült, anschließend wurden 4,7 g (23,0 mmol) tert.-Octylphenol und 2,3 g (17,0 mmol) Kaliumcarbonat zugegeben. Die Mischung wurde dann auf 80°C erhitzt und 16 h bei dieser Temperatur gerührt.

Nach Abkühlen auf Raumtemperatur wurde das Produkt durch Zugabe von 100 ml verdünnter Salzsäure ausgefällt, abfiltriert, mit Wasser neutral gewaschen und getrocknet.

Es wurden 2,0 g I17 in Form eines blauen Feststoffs erhalten, was einer Ausbeute von 46% entspricht.

Analytische Daten von I17:
UV-Vis (C₂H₅OH): λₘₐₓ = 670 nm;
MS (FD): m/z (rel. Int.) = 1612,3 (100%) [M⁺].

### Beispiel 18

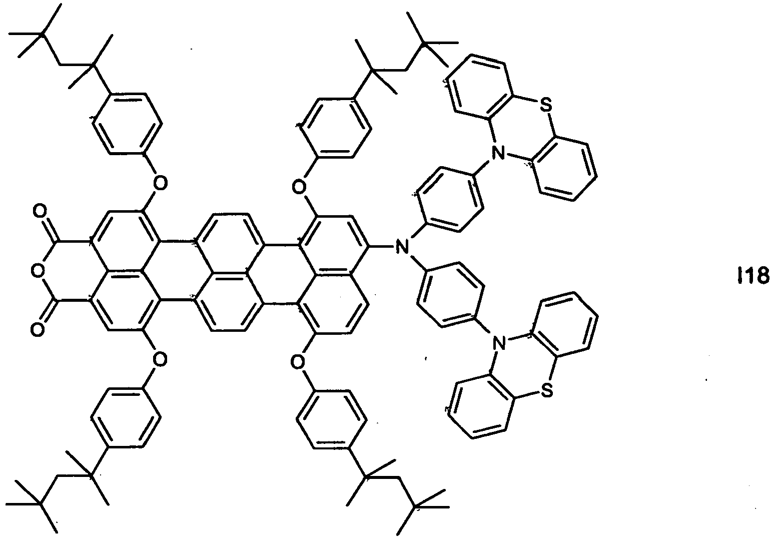

Die Herstellung des Rylenderivats 118 erfolgte ausgehend von N-(2,6-Diisopropyl-phenyl)-1,6,9,14-tetra[(4-tert.-octyl)phenoxy]-11-bromterrylen-3,4-dicarbonsäureimid, das zunächst unter Bromaustausch mit Bis(4-(Phenothiazinal)phenyl)-amin zum aminosubstituierten Dicarbonsäureimid umgesetzt wurde (Schritt a), welches anschließend alkalisch zum Dicarbonsäureanhydrid I18 verseift wurde (Schritt b).

### Schritt a):

Eine Mischung von 0,20 g (0,13 mmol) N-(2,6-Diisopropyiphenyl)-1,6,9,14-tetra((4-tert.-octyl)phenoxy]-11-bromterrylen-3,4-dicarbonsäureimid, 0,147 g (0,26 mmol) Bis(4-(Phenothiazinal)phenyl)-amin, 0,007 g (0,007 mmol) Tris(dibenzylidenaceton)dipalladium(0), 0,132 g (0,065 mmol), Tris(tert.-butyl)phosphin (Lsg 10%ig/Toluol), 0,016 g (0,13 mmol) Kalium-tert.-butylat und
10 ml trockenem Toluol wurde unter Stickstoff auf 75°C erhitzt und 19 h bei dieser Temperatur gerührt.

Nach Abdestillieren des Lösungsmittels wurde das Rohprodukt einer Säulenchromatographie an Kieselgel mit einem Hexan/Ethylacetat-Gemisch (10:1) als Eluens unterzogen.

Es wurden 0,04 g N-(2,6-Diisopropylphenyl)-1,6,9,14-tetra[(4-tert.-octyl)phenoxy]-9-[bis(4-(Phenothiazinal)phenyl)-aminoterrylen-3,4-dicarbonsäureimid in Form eines blauen Feststoffs erhalten, was einer Ausbeute von 16% (nur die saubersten Fraktionen genommen) entspricht.

Analytische Daten:
UV-Vis (CH₂Cl₂): λₘₐₓ = 654 nm;
MS (FD): m/z (rel. Int.) = 1982,9 (100%) [M⁺].

### Schritt b):

Eine Mischung von 0,04 g (0,02 mmol) N-(2,6-Diisopropy)phenyl)-1,6,9,14-tetra[(4-tert.-octyl)phenoxy]-9-[bis(4-(Phenothiazinal)phenyl)-aminoterrylen-3,4-dicarbonsäureimid und
8 ml 2-Methyl-2-butanol wurde auf 60°C erhitzt. Nach 0,5 h wurden 0,034 g (0,6 mmol) Kaliumhydroxid und 0,035 g (0,60 mmol) Kaliumfluorid zugegen. Dann wurde die Mischung auf leichten Rückfluß (etwa 118°C) erhitzt und 17 h bei dieser Temperatur gerührt.

Nach Abkühlen auf 40°C, Ansäuern mit 50 gew.-%iger Essigsäure und weiterem einstündigen Rühren bei 40°C wurde das Reaktionsprodukt auf Wasser gefällt, abfiltriert, mit heißem,Wasser gewaschen, Rückstand erneut in Wasser suspendiert, mit 50% iger Essigsäure angesäuert, 2h bei Rückfluss gekocht, abfiltriert, mit heißem Wasser gewaschen und getrocknet. Danach wurde das Rohprodukt einer Säulenchromatographie an Kieselgel mit Dichlormethan als Eluens unterzogen.

Es wurden 0,033 g 118 in Form eines blaugrünen Feststoffs erhalten, was einer Ausbeute von 90% entspricht.

Analytische Daten von I18:
MS (FD): m/z (rel. Int.) = 1823,7 (100%) [M⁺].

### Beispiel 19

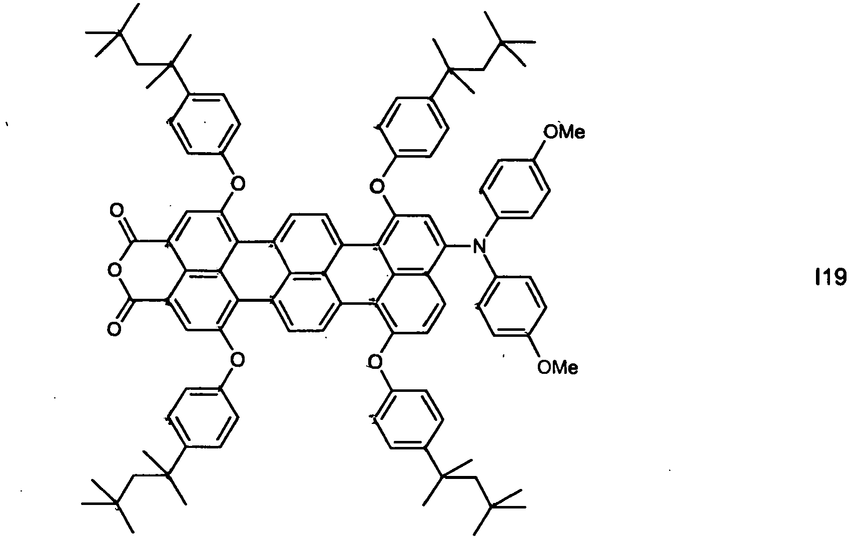

Die Herstellung des Rylenderivats 119 erfolgte ausgehend von N-(2,6-Diisopropyl-phenyl)-1,6,9,14-tetra[(4-tert.-octyl)phenoxy]-11-bromterrylen-3,4-dicarbonsäureimid, das zunächst unter Bromaustausch mit Bis[(4-methoxy)phenyl]amin zum aminosubstituierten Dicarbonsäureimid umgesetzt wurde (Schritt a), welches anschließend alkalisch zum Dicarbonsäureanhydrid I19 verseift wurde (Schritt b).

### Schritt a):

Eine Mischung von 0,090 g (0,06 mmol) N-(2,6-Diisopropylphenyl)-1,6,9,14-tetra[(4-tert.-octyl)phenoxy]-11-bromterrylen-3,4-dicarbonsäureimid, 0,028 g (0,12 mmol) Bis(4-methoxy)phenyl-amin, 0,003 g (0,003 mmol) Tris(dibenzylidenaceton)dipalladium(0), 0,06 g (0,03 mmol) Tris(tert.-butyl)phosphin (Lsg 10%ig/Toluol), 0,006 g (0,6 mmol) Natrium-tert.-butylat und
5 ml trockenem Toluol wurde unter Stickstoff auf 70°C erhitzt und 20 h bei dieser Temperatur gerührt.

Nach Abdestillieren des Lösungsmittels wurde das Rohprodukt einer Säulenchroma-tographie an Kieselgel mit einem Tolul/Hexans-Gemisch (2:1) als Eluens unterzogen.

Es wurden 0,115 g N-(2,6-Diisopropylphenyl)-1,6,9,14-tetra[(4-tert.-octyt)phenoxy]-9-[bis(4-(methoxy)phenyl)-aminoterrylen-3,4-dicarbonsäureimid in Form eines blauen Feststoffs erhalten, was einer Ausbeute von 16% entspricht.

Analytische Daten:
UV-Vis (CH₂Cl₂): λₘₐₓ = 678 nm;
MS (FD): m/z (rel. Int.) = 1649,1 (100%) [M⁺].

### Schritt b):

Eine Mischung von 0,105 g (0,064 mmol) N-(2,8-Diisopropylphenyl)-1,6,9,14-tetra[(4-tert.-octyl)phenoxy]-9-[bis(4-(methoxy)phenyl)-aminoterrylen-3,4-dicarbonsäureimid und
20 ml 2-Methyl-2-butanol wurde auf 60°C erhitzt. Nach 0,5 h wurden 0,108 g (1,92 mmol) Kaliumhydroxid und 0,112 g (1,92 mmol) Kaliumfluorid zugegen. Dann wurde die Mischung auf leichten Rückfluß (etwa 118°C) erhitzt und 17 h bei dieser Temperatur gerührt.

Nach Abkühlen auf 40°C, Ansäuern mit 50 gew.-%iger Essigsäure und weiterem einstündigen Rühren bei 40°C wurde das Reaktionsprodukt auf Wasser gefällt, abfiltriert, mit heißem Wasser/Methanol gewaschen, Rückstand erneut in Wasser suspendiert, mit 50% iger Essigsäure angesäuert, 2h bei Rückfluss gekocht, abfiltriert und mit heißem Wasser gewaschen und getrocknet. Danach wurde das Rohprodukt einer Säulenchromatographie an Kieselgel mit einem Toluol/Eisessig-Gemisch (97:3) als Eluens unterzogen. Danach wurde das Produkt neutral gestellt.

Es wurden 0,050 g 119 in Form eines blaugrünen Feststoffs erhalten, was einer Ausbeute von 90% entspricht.

Analytische Daten von I19:
UV-Vis (CH₂Cl₂): λₘₐₓ = 684 nm;
MS (FD): m/z (rel. Int.) = 1489,9 (100%) [M⁺].

Beispiel 20

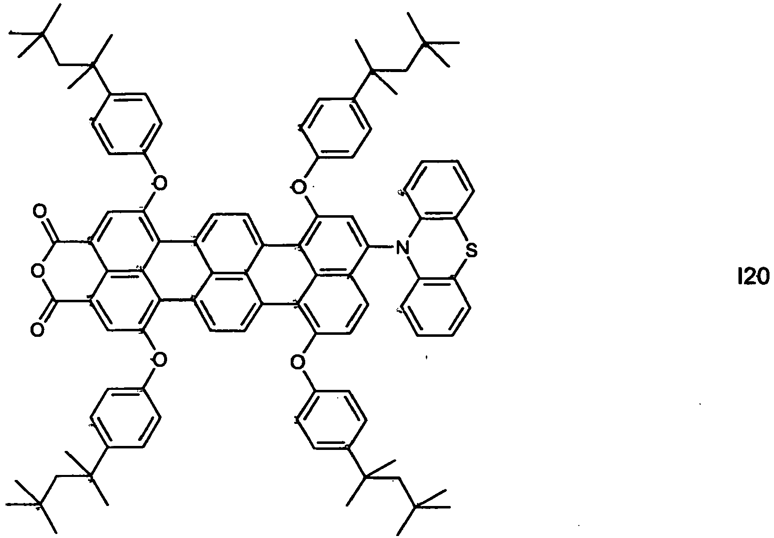

Die Herstellung des Rylenderivats I20 erfolgte ausgehend von N-(2,6-Diisopropylphenyl)-1,6,9,14-tetra[(4-tert.-octyl)phenoxy]-11-bromterrylen-3,4-dicarbonsäureimid, das zunächst unter Bromaustausch mit Phenothiazin zum phenothiazinolsubstituierten Dicarbonsäureimid umgesetzt wurde (Schritt a), welches anschließend alkalisch zum Dicarbonsäureanhydrid I20 verseift wurde (Schritt b).

### Schritt a):

Eine Mischung von 0,20 g (0,13 mmol) N-(2,6-Diisopropylphenyl)-1,6,9,14-tetra[(4-tert.-octyl)phenoxy]-11-bromterrylen-3,4-dicarbonsäureimid, 0,053 g (0,26 mmol) Phenothiazin, 0,007 g (0,007 mmol) Tris(dibenzylidenaceton)dipalladium(0), 0,132 g (0,065 mmol) Tris(tert.-butyl)phosphin (Lsg 10%ig/Toluol), 0,016 g (0,13 mmol) Kalium-tert.-butylat und
10 ml trockenem Toluol wurde unter Stickstoff auf 90°C erhitzt und 22 h bei dieser Temperatur gerührt.

Nach Abdestillieren des Lösungsmittels wurde das Rohprodukt einer Säulenchromatographie an Kieselgel mit einem Hexan/Ethylacetat-Gemisch (15:1) als Eluens unter-zogen.

Es wurden 0,125 g N-(2,6-Diisopropylphenyl)-1,6,9,14-tetra[(4-tert.-octyl)phenoxy]-9-phenothiazinolterrylen-3,4-dicarbonsäureimid in Form eines blauen Feststoffs erhalten, was einer Ausbeute von 59% entspricht.

Analytische Daten:
MS (FD): m/z (rel. Int.) = 1616,8 (100%) [M⁺].

### Schritt b):

Eine Mischung von 0,070 g (0,043 mmol) N-(2,6-Diisopropylphenyl)-1,6,9,14-tetra[(4-tert.-octyl)phenoxy]-9-[bis(4-(methoxy)phenyl)-aminoterrylen-3,4-dicarbonsäureimid und
13 ml 2-Methyl-2-butanol wurde auf 60°C erhitzt. Nach 0,5 h wurden 0,075 g (1,29 mmol) Kaliumhydroxid und 0,073 g (1,29 mmol) Kaliumfluorid zugegen. Dann wurde die Mischung auf leichten Rückfluß (etwa 118°C) erhitzt und 16 h bei dieser Temperatur gerührt.

Nach Abkühlen auf 40°C, Ansäuern mit 50 gew.-%iger Essigsäure und weiterem einstündigen Rühren bei 40°C wurde das Reaktionsprodukt auf Wasser gefällt, abfiltriert, mit Wasser/Methanol gewaschen, Rückstand erneut in Wasser suspendiert, mit 50% iger Essigsäure angesäuert, 2h bei Rückfluss gekocht, abfiltriert, mit heißem Wasser gewaschen und getrocknet. Danach wurde das Rohprodukt einer Säulenchromatographie an Kieselgel mit einem Dichlormethan/Hexan/Eisessig-Gemisch(47,5:47,5:5) als Eluens unterzogen. Danach wurde das Produkt neutral gestellt.

Es wurden 0,040 g I20 in Form eines blauen Feststoffs erhalten, was einer Ausbeute von 60% entspricht.

Analytische Daten von I20:
UV-Vis (CH₂Cl₂): λₘₐₓ = 646 nm;
MS (FD): m/z (rel. Int.) = 1459,5 (100%) [M⁺].

### Beispiel 21

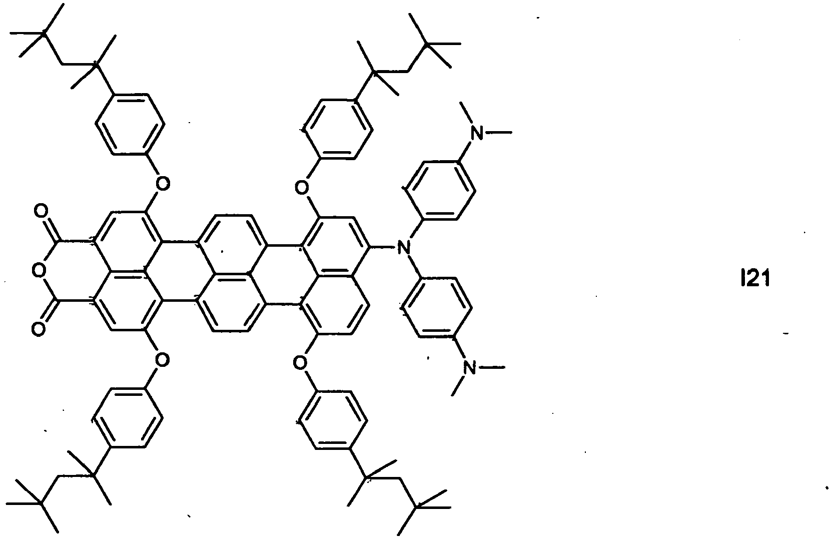

Die Herstellung des Rylenderivats I21 erfolgte ausgehend von N-(2,6-Diisopropyl-phenyl)-1,6,9,14-tetra[(4-tert.-octyl)phenoxy]-11-bromterrylen-3,4-dicarbonsäureimid, das zunächst unter Bromaustausch mit Bis[(4-bismethylamin)phenyl]amin zum aminosubstituierten Dicarbonsäureimid umgesetzt wurde (Schritt a), welches anschließen alkalisch zum Dicarbonsäureanhydrid 121 verseift wurde (Schritt b).

### Schritt a):

Eine Mischung von 0,400 g (0,27 mmol) N-(2,6-Diisopropylphenyl)-1,6,9,14-tetra[(4-tert.-octyl)phenoxy]-11-bromterrylen-3,4-dicarbonsäureimid, 0,141 g (0,54 mmol) 1,4-Benzenediamin, N'-(4-(dimethylamino)phenyl)-N,N-dimethyl, 0,013 g (0,014 mmol) Tris(dibenzylidenaceton)dipalladium(0), 0,273 g (0,135 mmol) Tris(tert.-butyl)phosphin (Lsg 10%ig/Toluol), 0,026 g (0,27 mmol) Natrium-tert.-butylat und
20 ml trockenem Toluol wurde unter Stickstoff auf 80°C erhitzt und 13 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,280 g eines blaugrünen Feststoffs erhalten, was einer Ausbeute von 62% entspricht.

Analytische Daten:
MS (FD): m/z (rel. Int.) = 1675,0 (100%) [M⁺].

### Schritt b):

Eine Mischung von 0,195 g (0,12 mmol) N-(2,6-Diisopropylphenyl)-1,6,9,14-tetra[(4-tert.-octyl)phenoxy]-9-[bis(4-(methoxy)phenyl)-aminoterrylen-3,4-dicarbonsäureimid und
36 ml 2-Methyl-2-butanol wurde auf 60°C erhitzt. Nach 0,5 h wurden 0,213 g (3,60 mmol) Kaliumhydroxid und 0,209 g (3,60 mmol) Kaliumfluorid zugegen. Dann wurde die Mischung auf leichten Rückfluß (etwa 118°C) erhitzt und 13 h bei dieser Temperatur gerührt.

Nach Abkühlen auf 40°C, Ansäuern mit 50 gew.-%iger Essigsäure und weiterem einstündigen Rühren bei 40°C wurde das Reaktionsprodukt auf Wasser gefällt, abfiltriert, mit Wasser/Methanol gewaschen, Rückstand erneut in Wasser suspendiert, mit 50% iger Essigsäure angesäuert, 2h bei Rückfluss gekocht, abfiltriert, mit heißem Wasser gewaschen und getrocknet. Danach wurde das Rohprodukt einer Säulenchromatographie an Kieselgel mit einem Dichlorethan/ Essigsäure -Gemisch (95:5) als Eluens unterzogen. Danach wurde das Produkt neutral gestellt.

Es wurden 0,018 g 121 in Form eines grünblauen Feststoffs erhalten, was einer Ausbeute von 10% entspricht.

Analytische Daten von I21:
UV-Vis (CH₂Cl₂): λₘₐₓ = 682 nm;
MS (FD): m/z (rel. Int.) = 1515,7 (100%) [M⁺].
Analytische Daten von I11:

### Beispiel 22

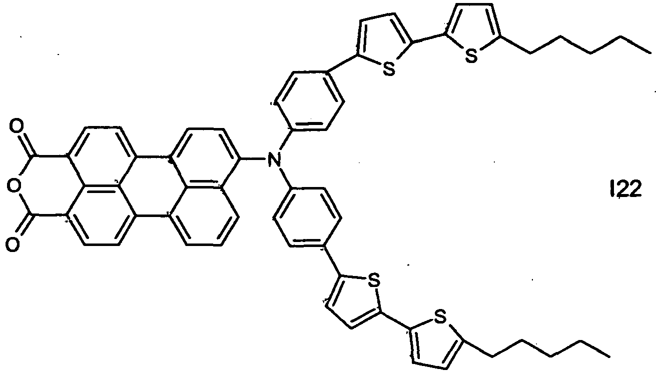

Die Herstellung des Rylenderivats I22 erfolgte ausgehend von N-(2,6-Diisopropyl-phenyl)-9-bromperylen-3,4-dicarbonsäureimid, das zunächst unter Bromaustausch mit Bis-[4-(5'-hexyl-[2,2']bithiophenyl-5-yl)-phenyl]-amine zum aminosubstituierten Dicar-bonsäureimid umgesetzt wurde (Schritt a), welches anschließend alkalisch zum Dicar-bonsäureanhydrid 122 verseift wurde (Schritt b).

### Schritt a):

### Buchwald Reaction (allgemein)

Eine Mischung von 9-Bromperylenderivate (1 Äq.), Amin (1,1-1,5 Äq), Tris(dibenzylidenaceton)dipalladium(0) (2 mol%), Tris(tert.-butyl)-phosphin (3 mol%) und Natrium-tert.-butylat (1,5 Äq) in trockenem Toluol wurde unter Argon auf 80°C erhitzt und 1 d bei dieser Temperatur gerührt.

Nach Abdestillieren des Lösungsmittels wurde das Rohprodukt einer Säulenchromatographie an Kieselgel unterzogen.

Es wurden 450 mg des Perylenaminderivats in Form eines grünen Feststoffs erhalten, was einer Ausbeute von 55% entspricht.

### Schritt b):

### Verseifung (allgemein)

Eine Mischung Perylenmonoimid (Produkt von Schritt a), Kaliumhydroxid (300-500 Äq) und Isopropanol wurde 16 h auf Rückflußtemperatur (etwa 82°C) erhitzt.

Nach Abkühlen auf Raumtemperatur wurde das Reaktionsgemisch auf Essigsäure gegeben, auf 40°C erhitzt und 4 h bei dieser Temperatur gerührt. Die Essigsäure wurde abdestilliert und das Produkt mit Wasser neutral gewaschen, getrocknet und einer Säulenchromatographie an Kieselgel unterzogen.

I22 wurde keiner Säulenchromatographie unterzogen, stattdessen wurde das Produkt in THF gelöst und zweimal in Ethanol ausgefällt.

Es wurden 150 mg I22 in Form eines blauen Feststoffs erhalten, was einer Ausbeute von 45% entspricht.

Analytische Daten:
UV-Vis (CH₂Cl₂): λₘₐₓ (ε) = 398 (40 400), 602 (14 900) nm (M⁻¹cm⁻¹);
MS (FD): m/z (rel. Int.) = 985,2 (100%) [M⁺].

### Schritt b):

### Beispiel 23

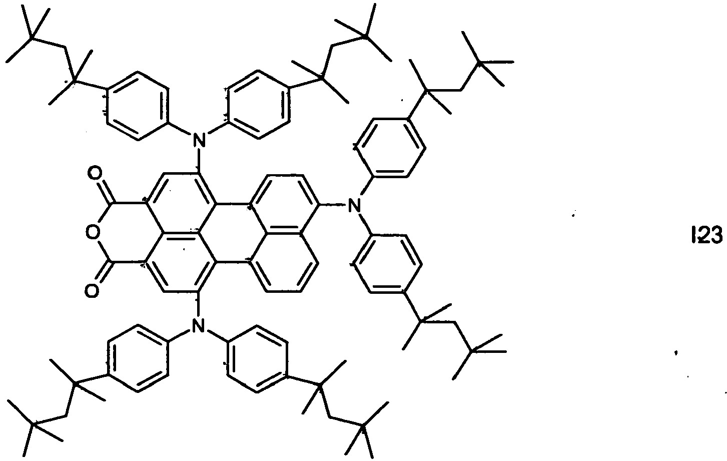

Die Herstellung des Rylenderivats I23 erfolgte ausgehend von N-(2, 6-diisopropylphenyl)-1, 6, 9-tribromo-perylene-3, 4-dicarboximide, das zunächst unter Bromaustausch mit Bis[(4-tert.-octyl)phenyl]amin zum trisaminosubstituierten Dicarbonsäureimid umgesetzt wurde (Schritt a), welches anschließend alkalisch zum Dicarbonsäureanhydrid I23 verseift wurde (Schritt b).

### Schritt a):

Eine Mischung von 0,8 g (1,1 mmol) N-(2,6-diisopropylphenyl)-1, 6, 9-tribromoperylene-3, 4-dicarboximide, 2,6 g (6,6 mmol) Bis[(4-tert.-octyl)phenyl]amin, 160 mg (0,14 mmol) Tris(dibenzylidenaceton)dipalladium(0), 40 mg (0,16 mmol) Tris(tert.-butyl)-phosphin, 160 mg (0,18 mmol) Natrium-tert.-butylat und 100 ml trockenem Toluol wurde unter Argon auf 80°C erhitzt und 2 d bei dieser Temperatur gerührt.

Nach Abdestillieren des Lösungsmittels wurde das Rohprodukt einer Säulenchromatographie an Kieselgel mit Toluol als Eluens unterzogen.

Es wurden 0,20 mg des Perylenimids in Form eines blauen Feststoffs erhalten, was einer Ausbeute von 10% entspricht.

Analytische Daten:
UV-Vis (CH₂Cl₂): λₘₐₓ (ε) = 681 (19 000) nm (M⁻¹cm⁻¹);

### Schritt b):

Verseifung analog Beispiel 22 (Schritt b)

Es wurden 20 mg I23 in Form eines violeten Feststoffs erhalten, was einer Ausbeute von 10% entspricht.

Analytische Daten von I23:
UV-Vis (CH₂Cl₂): λₘₐₓ (ε) = 698 (13 000) nm (M⁻¹cm⁻¹);
MS-(FD): m/z (rel. Int.) = 1494,1 (100%) [M⁺].

### Beispiel 24

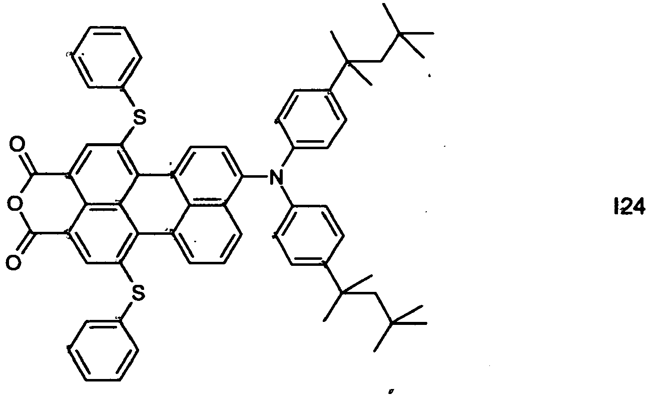

Die Herstellung des Rylenderivats I24 erfolgte ausgehend von N-(2,6-diisopropylphenyl)-1, 6, 9-tribromo-perylene-3, 4-dicarboximide, das zunächst unter Bromaustausch mit thiophenol zum N-(2,6-diisopropylphenyl)-1,6-thiophenyl]-9-bromo-perylene-3, 4-dicarboximide umgesetzt wurde (Schritt a), welches danach einen weiteren Bromaustausch mit Bis[(4-tert.-octyl)phenyl]amin zum N-(2,6-diisopropylphenyl)-9-(bis-p-t-octylphenyl)amino-1,6-dithiophenyl-perylene-3, 4-dicarboximide umgesetzt wurde (Schritt b), welches anschließend alkalisch zum Dicarbonsäureanhydrid I24 verseift wurde (Schritt c).

### Schritt a):

Eine Mischung von 1,0 g (1,99 mmol) N-(2,6-diisopropylphenyl)-1, 6, 9-tribromo-perylene-3, 4-dicarboximide, 0,229 g (1,39 mmol) Thiophenol, und 0,256g (2,78 mmol) Kaliumhydroxid und 80 ml NMP wurde unter Argon auf 80°C erhitzt und 3 h bei dieser Temperatur gerührt.

Nach Abkühlen wurde das Produkt in 25 gew.-%iger Salzsäure gegeben. Das auf diese Weise ausgefällte Produkt wurde abfiltriert, mit Wasser gewaschen, getrocknet und einer Säulenchromatographie an Kieselgel mit einem Methylenchlorid Eluens unterzogen.

Es wurde 600 mg N-(2, 6-diisopropylphenyl)-1,6-thiophenyl]-9-bromo-perylene-3,4-dicarboximide in Form eines roten Feststoffs erhalten, was einer Ausbeute von 56% entspricht.

### Schritt b):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 350 mg N-(2,6-diisopropylphenyl)-9-(bis-p-t-octylphenyl)amino-1,6-dithiophenyl-perylene-3, 4-dicarboximide in Form eines blauen Feststoffs erhalten, was einer Ausbeute von 84% entspricht.

Analytische Daten:
UV-Vis (CH₂Cl₂): λₘₐₓ (ε) = 606 (13898), 462 (7690) (M⁻¹cm⁻¹);
MS (FD): m/z (rel. Int.) = 929,5

### Schritt c):

Verseifung analog Beispiel 22 (Schritt b)
Einer Säulenchromatographie an Kieselgel mit einem Toluol als Eluens wurde unterzogen.

Es wurden 150 mg I24 in Form eines blauen Feststoffs erhalten, was einer Ausbeute von 59% entspricht.

Analytische Daten:
UV-Vis (CH₂Cl₂): λₘₐₓ (ε) = 620 (22 800), 462 (13 700) nm (M⁻¹cm⁻¹); MS (FD): m/z (rel. Int.) = 929,5 (100%) [M⁺].

### Beispiel 25

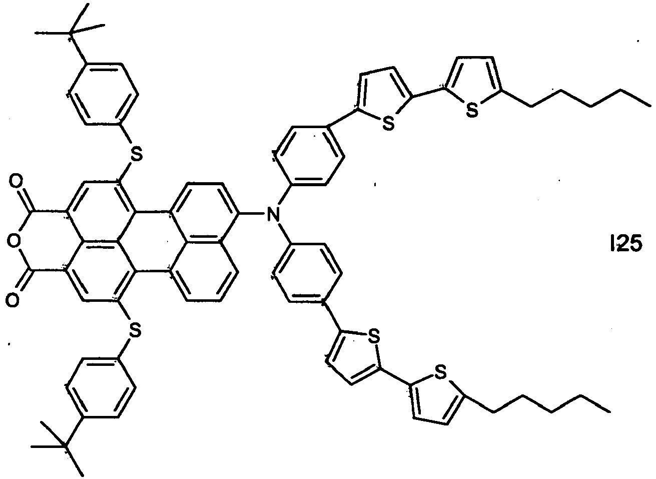

Die Herstellung des Rylenderivats I25 erfolgte ausgehend von N-(2, 6-diisopropylphenyl)-1, 6, 9-tribromo-perylene-3, 4-dicarboximide, das zunächst unter Bromaustausch mit tert-butyl-thiophenol zum N-(2, 6-diisopropylphenyl) - 1, 6- p-tert.-butyl-thiophenyl]-9-bromo-perylene-3, 4-dicarboximide umgesetzt wurde (Schritt a), welches danach einen weiteren Bromaustausch mit Bis-[4-(5'-hexyl-[2,2']bithiopheny)-5-yl)-phenyl]-amine N-(2, 6-diisopropylphenyl)- 1, 6- di-p-tert.-butyl-thiophenyl 9-bis-[4-(5'-hexyl- [2,2']bithiophenyl-5-yl)-phenyl]-amino -perylene-3, 4-dicarboximide umgesetzt wurde (Schritt b), welches anschließend alkalisch zum Dicarbonsäureanhydrid I25 ver seift wurde (Schritt c).

### Schritt a):

Eine Mischung von 5,0 g (6,96 mmol) N-(2, 6-diisopropylphenyl)-1, 6, 9-tribromo-perylene-3, 4-dicarboximide, 1,16 g (6,96 mmol) tert-butyl-Thiophenol, und 0,96 g (6,99 mmol) Kaliumhydroxid und 80 ml NMP wurde auf 50°C erhitzt und 1,5 h bei dieser Temperatur gerührt. Danach wurde noch 0,58 g (3,48 mmol) tert-butyl-Thiophenol und 0,48 g (3,48 mmol) Kaliumhydroxid dazugegeben und weiter 1,5 h bei dieser Temperatur gerührt.

Nach Abkühlen wurde das Produkt in 25 gew.-%iger Salzsäure gegeben. Das auf diese Weise ausgefällte Produkt wurde abfiltriert, mit Wasser gewaschen, getrocknet und einer Säulenchromatographie an Kieselgel mit Toluol als Eluens unterzogen.

Es wurde 3,0 g N-(2, 6-diisopropylphenyl) - 1, 6- p-tert.-butyl-thiophenyl]-9-bromo-perylene-3, 4-dicarboximide in Form eines roten Feststoffs erhalten, was einer Ausbeute von 48% entspricht.
MS (FD): m/z (rel. Int.) = 888,6 (100%) [M⁺].

### Schritt b):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 500 mg N-(2, 6-diisopropylphenyl)- 1, 6- di-p-tert.-butyl-thiophenyl 9-bis-[4-(5'-hexyl- [2,2']bithiophenyl-5-yl)-phenyl]-amino -perylene-3, 4-dicarboximide in Form eines grünen Feststoffs erhalten, was einer Ausbeute von 75% entspricht.

### Schritt c):

Verseifung analog Beispiel 22 (Schritt b)
Einer Säulenchromatographie an Kieselgel mit Toluol als Eluens wurde unterzogen.

Es wurden 200 mg I25 in Form eines blauen Feststoffs erhalten, was einer Ausbeute von 57% entspricht.

Analytische Daten:
UV-Vis (CH₂Cl₂): λₘₐₓ (ε) = 602 (19 200) nm (M⁻¹cm⁻¹);
MS (FD): m/z (rel. Int.) = 1316,3 (100%) [M⁺].

### Beispiel 26

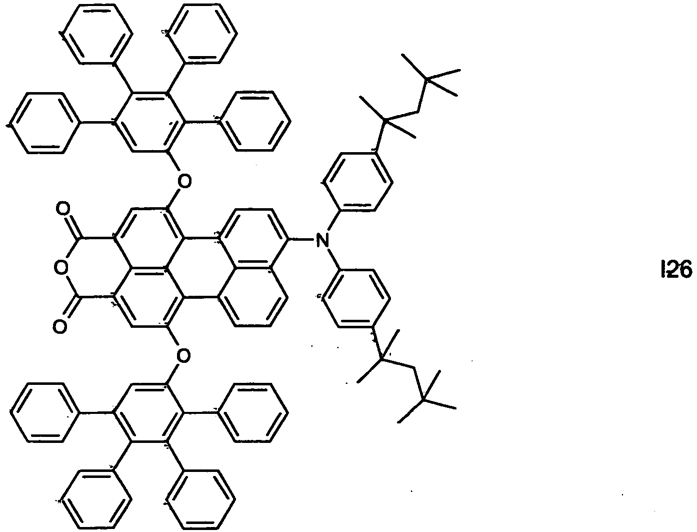

Die Herstellung des Rylenderivats I26 erfolgte ausgehend von N-(2, 6-diisopropylphenyl)-1, 6, 9-tribromo-perylene-3, 4-dicarboximide unter ähnlichen Bedingungen wie I25. Die Beschreibung wie man das tetraphenyl-Phenyl-substituierte Perylenderivat herstellt ist in Qu et al, Chem.Eur.J. 2004, 10, 528-537 zu finden,

### Schritt a):

Buchwald analog Beispiel 22 (Schritt a)
Nach Abdestillieren des Lösungsmittels wurde das Rohprodukt einer Säulenchromatographie an Kieselgel mit Dichlormethan als Eluens unterzogen.

Es wurden 500 mg N-(2, 6-diisopropylphenyl)-9-(bis-p-tert-octylphenyl)amino-1,6-di(2,3,4,5-tetra- phenyl-phenoxy)-perylene-3, 4-dicarboximide in Form eines blauen Feststoffs erhalten, was einer Ausbeute von 80% entspricht.

Analytische Daten:
MS (FD): m/z (rel. Int.) = 1656,5 (100%) [M⁺].

### Schritt b):

Verseifung analog Beispiel 22 (Schritt b)
Einer Säulenchromatographie an Kieselgel mit Dichlormethan als Eluens wurde unterzogen.

Es wurden 120 mg I26 in Form eines blauen Feststoffs erhalten, was einer Ausbeute von 66% entspricht.

Analytische Daten von I26:
UV-Vis (CH₂Cl₂): λₘₐₓ = 603, 486 nm.

### Beispiel 27

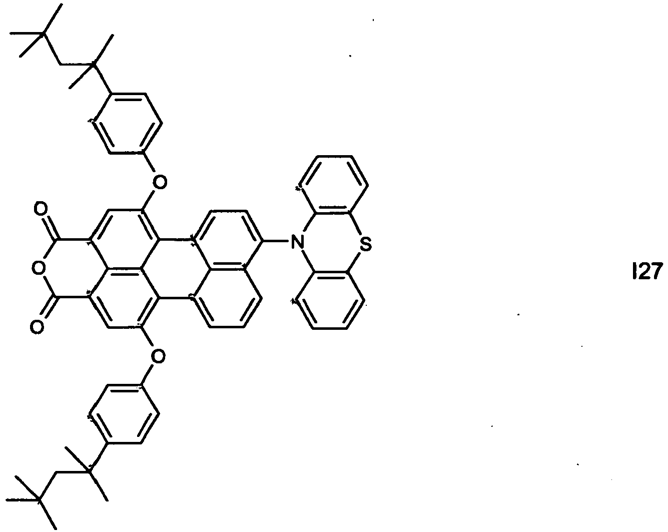

Die Herstellung des Rylenderivats I27 erfolgte ausgehend von N-(2,6-Diisopropylphenyl)-1,6-bis[(4-tert.-octyl)phenoxy]-9-bromperylen-3,4-dicarbonsäureimid, das zunächst unter Bromaustausch mit Phenothiazin zum substituierten Dicarbonsäureimid umgesetzt wurde (Schritt a), welches anschließend alkalisch zum Dicarbonsäureanhydrid I27 verseift wurde (Schritt b).

### Schritt a):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 330 mg -(2, 6-diisopropylphenyl)-9-phenothiazine-1,6-di(p-tert-octylphenoxy)-perylene- 3, 4-dicarboximide in Form eines braunen Feststoffs erhalten, was einer Ausbeute von 95% entspricht.

Analytische Daten:
UV-Vis (CH₂Cl₂): λₘₐₓ (ε) = 516 (24 000) nm (M⁻¹cm⁻¹);

### Schritt b):

Verseifung analog Beispiel 22 {Schritt b)
Einer Säulenchromatographie an Kieselgel mit Toluol als Eluens wurde unterzogen.

Es wurden 40 mg 127 in Form eines brauen Feststoffs erhalten, was einer Ausbeute von 15% entspricht.

Analytische Daten:
UV-Vis (CH₂Cl₂): λₘₐₓ (ε) = 518 (22 000) nm (M⁻¹cm⁻¹);

### Beispiel 28

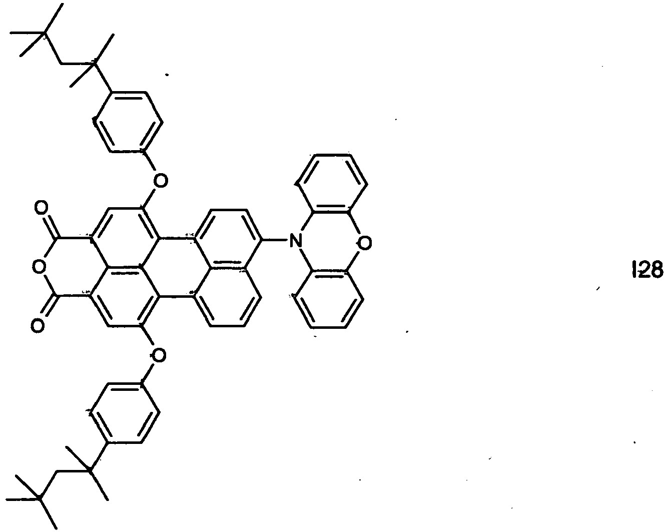

Die Herstellung des Rylenderivats I28 erfolgte ausgehend von N-(2,6-Diisopropyl-phenyl)-1,6-bis[(4-tert.-octyl)phenoxy]-9-bromperyten-3,4-dicarbonsäureimid, das zunächst unter Bromaustausch mit Phenoxazine zum substituierten Dicarbonsäureimid umgesetzt wurde (Schritt a), welches anschließend alkalisch zum Dicarbonsäureanhydrid I28 verseift wurde (Schritt b).

### Schritt a):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 300 mg N-(2, 6-diisopropylphenyl)-9-phenoxazine-1,6-di(p-tert-octylpheno-xy)- perylene-3, 4-dicarboximide in Form eines braunen Feststoffs erhalten, was einer Ausbeute von 90% entspricht.

Analytische Daten:
UV-Vis (CH₂Cl₂): λₘₐₓ (ε) = 513 (24 000) nm (M⁻¹cm⁻¹);

### Schritt b):

Verseifung analog Beispiel 22 (Schritt b)
Einer Säulenchromatographie an Kieselgel mit Toluol als Eluens wurde unterzogen.

Es wurden 50 mg I28 in Form eines brauen Feststoffs erhalten, was einer Ausbeute von 20% entspricht.

Analytische Daten:
UV-Vis (CH₂Cl₂): λₘₐₓ (ε) = 514 (14 000) nm (M⁻¹cm⁻¹);

### Beispiel 29

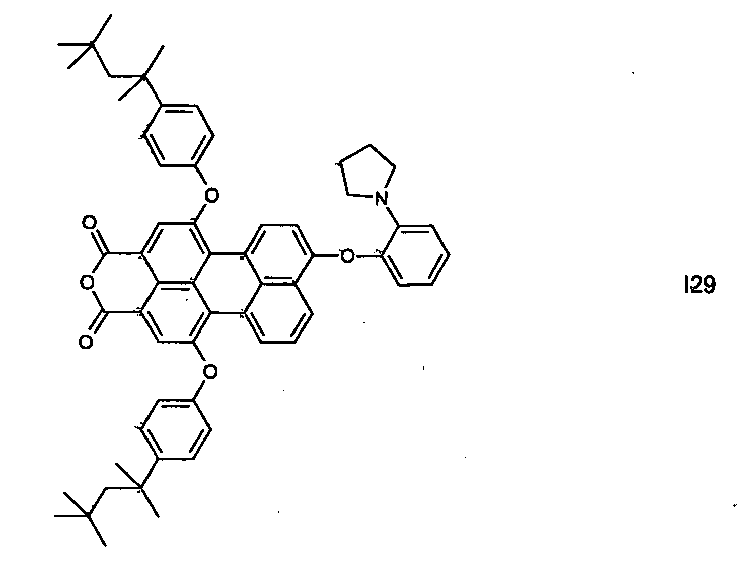

Die Herstellung des Rylenderivats 129 erfolgte ausgehend von N-(2,6-Diisopropylphenyl)-1,6-bis[(4-tert.-octyl)phenoxy]-9-bromperylen-3,4-dicarbonsäureimid, das zunächst unter Bromaustausch mit o-pyrrolidino-phenol zum substituierten Dicarbonsäureimid umgesetzt wurde (Schritt a), welches anschließend alkalisch zum Dicarbonsäureanhydrid I29 verseift wurde (Schritt b).

### Schritt a):

Eine Mischung von 0,3 g (0,31 mmol) N-(2, 6-diisopropylphenyl)-1, 6, 9-tribromo-perylene-3, 4-dicarboximide, 0,076 g (0,46 mmol) *o*-pyrrolidino-phenol, und 0,043 g (0,31 mmol) Kaliumhydroxid und 80 ml NMP wurde unter Argon auf 80°C erhitzt und 12 h bei dieser Temperatur gerührt.

Nach Abkühlen wurde das Produkt in 25 gew.-%iger Salzsäure gegeben. Das auf diese Weise ausgefällte Produkt wurde abfiltriert, mit Wasser gewaschen, getrocknet und einer Säulenchromatographie an Kieselgel mit einem Methylenchlorid/Pentan-Gemisch (1:2) als Eluens unterzogen.

Es wurde 150 mg N-(2, 6-diisopropylphenyl) - 1, 6- bis[4-(1,1,3,3-tetramethylbutyl) phenoxy] -9-(p-pyrrolidino)phenoxy-perylene-3, 4-dicarboximide in Form eines roten Feststoffs erhalten, was einer Ausbeute von 92% entspricht.
UV-Vis (CH₂Cl₂): λₘₐₓ = 541 nm.

### Schritt b):

Verseifung analog Beispiel 22 (Schritt b)
Einer Säulenchromatographie an Kieselgel mit einem Methylenchlorid/Pentan-Gemisch (1:2) als Eluens wurde unterzogen.

Es wurden 35 mg I29 in Form eines violeten Feststoffs erhalten, was einer Ausbeute von 20% entspricht.

Analytische Daten:
UV-Vis (CH₂Cl₂): λₘₐₓ = 548 ;
MS (FD): m/z (rel. Int.) = 892,4 (100%) [M⁺].

### Beispiel 30

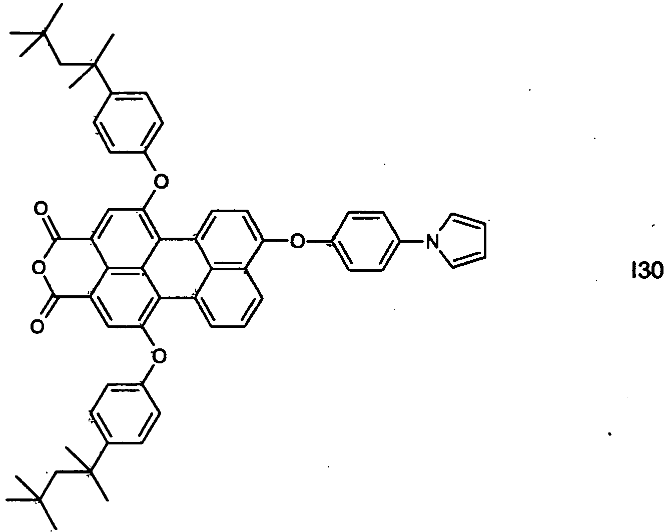

Die Herstellung des Rylenderivats I30 erfolgte ausgehend von N-(2,6-Diisopropyl-phenyl)-1,6-bis[(4-tert.-octyl)phenoxy]-9-bromperylen-3,4-dicarbonsäureimid, das zunächst unter Bromaustausch mit p-pyrrol-phenol zum substituierten Dicarbonsäureimid umgesetzt wurde (Schritt a), welches anschließend alkalisch zum Dicarbonsäureanhydrid I30 verseift wurde (Schritt b).

### Schritt a):

Eine Mischung von 0,3 g (0,31 mmol) N-(2, 6-diisopropylphenyl)-1, 6, 9-tribromo-perylene-3, 4-dicarboximide, 0,074 g (0,46 mmol) o-pyrrolidino-phenol, und 0,070 g (0,46 mmol) Kaliumhydroxid und 80 ml NMP wurde unter Argon auf 80°C erhitzt und 12 h bei dieser Temperatur gerührt.

Nach Abkühlen wurde das Produkt in 25 gew.-%iger Salzsäure gegeben. Das auf diese Weise ausgefällte Produkt wurde abfiltriert, mit Wasser gewaschen, getrocknet und einer Säulenchromatographie an Kieselgel mit einem Methylenchlorid/Pentan-Gemisch (1:2) als Eluens unterzogen.

Es wurde 300 mg N-(2, 6-diisopropylphenyl) 1, 6- bis[4-(1,1,3,3-tetramethylbutyl) phenoxy] -9-(p-pyrrol)phenoxy-perylene-3, 4-dicarboximide in Form eines roten Feststoffs erhalten, was einer Ausbeute von 92% entspricht.
UV-Vis (CH₂Cl₂): λₘₐₓ = 536, 415 nm;

### Schritt b):

Verseifung analog Beispiel 22 (Schritt b)
Einer Säulenchromatographie an Kieselgel mit einem Methylenchlorid/Pentan-Gemisch (1:2) als Eluens wurde unterzogen.

Es wurden 50 mg I30 in Form eines violeten Feststoffs erhalten, was einer Ausbeute von 15% entspricht.

Analytische Daten:
UV-Vis (CH₂Cl₂): λₘₐₓ = 536 ;
MS (FD): m/z (rel. Int.) = 866,6 (100%) [M⁺].

### Beispiel 31

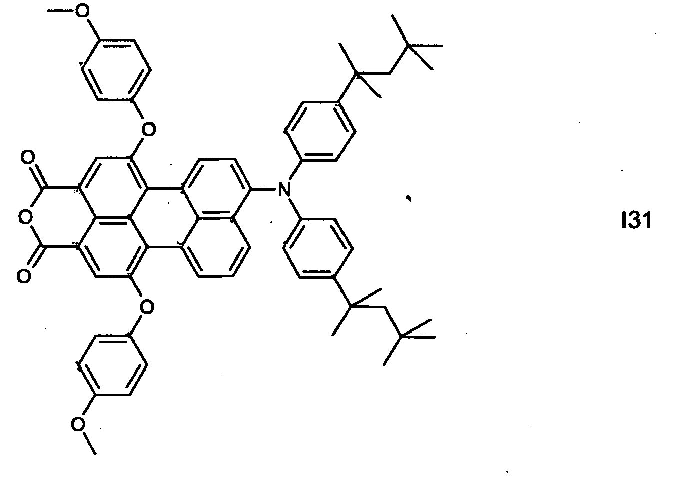

Die Herstellung des Rylenderivats I31 erfolgte ausgehend von N-(2, 6-diisopropylphenyl)-1, 6, 9-tribromo-perylene-3, 4-dicarboximide, das zunächst unter Bromaustausch mit 4-Methoxyphenol zum N-(2,6-Diisopropyi-phenyl)-1,6-bis[phenoxy]-9-bromperylen-3,4-dicarbonsäureimid umgesetzt wurde (Schritt a), welches danach einen weiteren Bromaustausch mit Bis[(4-tert.-octyl)phenyl]amin zum aminderivat umgesetzt wurde (Schritt b), welches anschließend alkalisch zum Dicarbonsäureanhydrid I31 verseift wurde (Schritt c).

### Schritt a):

Phenoxylierung Reaktion (allgemein)
Eine Mischung von N-(2, 6-diisopropylphenyl)-1, 6, 9-tribromo-perylene-3, 4-dicarboximide (1 Äq.), Phenolderivat (1 Äq.), Kaliumhydroxid (1 Äq.) wurde in NMP gel öst, auf 80°C erhitzt und 1,5 h bei dieser Temperatur gerührt. Danach wurden noch Phenolderivat (1 Äq.) und Kaliumhydroxid (1 Äq.) zugegeben und weitere 2 h bei dieser Temperatur gerührt.

Nach Abkühlen wurde das Produkt in 25 gew.-%ige Salzsäure gegeben. Das auf diese Weise ausgefälltes Produkt wurde abfiltriert, mit Wasser gewaschen, getrocknet und einer Säulenchromatographie an Kieselgel unterzogen.

Es wurden 640 mg N-(2, 6-diisopropylphenyl) -1, 6- diphenoxy-9-bromo-perylene-3, 4-dicarboximide in Form eines roten Feststoffs erhalten, was einer Ausbeute von 55% entspricht.

### Schritt b):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 400 mg N-(2, 6-diisopropylphenyl)-9-(bis-p-tert-octylphenyl)amino-1,6-diphenoxy-peryl- ene-3, 4-dicarboximide in Form eines blauen Feststoffs erhalten, was einer Ausbeute von 80% entspricht.

### Schritt c):

Verseifung analog Beispiel 22 (Schritt b)
Einer Säulenchromatographie an Kieselgel mit Toluol als Eluens wurde unterzogen.

Es wurden 200 mg I31 in Form eines blauen Feststoffs erhalten, was einer Ausbeute von 60% entspricht.

Analytische Daten:
UV-Vis (CH₂Cl₂): λₘₐₓ (ε) = 602, 487 nm;
MS (FD): m/z (rel. Int.) = 957,4 (100%) [M⁺].

### Beispiel 32

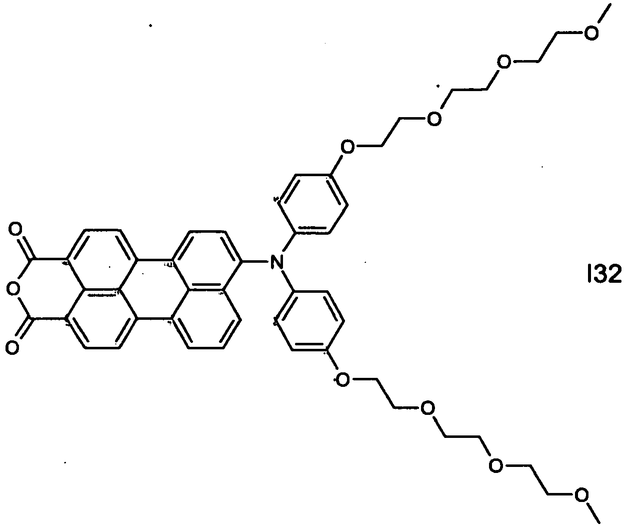

Die Herstellung des Rylenderivats I32 erfolgte ausgehend von N-(2, 6-diisopropylphenyl)-9-(bis-p-methyloxyphenyl) amino -perylene-3, 4-dicarboximide (Beispiel 49), das zunächst zu N-(2, 6-diisopropylphenyl)-9-(bis-p-hydroxyphenyl) amino -perylene-3, 4-dicarboximide umgewandelt wird (Schritt a), welches danach mit TEG-tosy umgesetzt wird(Schritt b), welches anschließend alkalisch zum Oicarbonsäureanhydrid I32 verseift wurde (Schritt c).

### Schritt a):

(0,4 mL, 4,2 mmol) BBr₃ wurde zu einer Lösung von N-(2, 6-diisoprapylphenyl)-9-(bis-p- methyloxyphenyl) amino -perylene-3, 4-dicarboximide in 50 mL Dichtormethan bei 0°C gegeben. Nach 3 h wurde 50 mL Wasser dazugegeben und die Phasen wurden getrennt.

Nach Abdestillieren des Lösungsmittels wurde N-(2, 6-diisopropylphenyl)-9-(bis-p-hydroxyphenyl) amino -perylene-3, 4-dicarboximide isoliert als blaues Feststoff (48mg, 99%).

### Schritt b):

Eine Mischung von 480 mg (0,70 mmol) N-(2, 6-diisopropylphenyl)-9-(bis-p-hydroxyphenyl) amino -perylene-3, 4-dicarboximide, (890 mg, 2.8 mmol) TEG-tosy, (400 mg, 2,8 mmol) Kaliumcarbonat und 100 mL wasserfreies Toluol 0,45 auf 90°C erhitzt und 1 d bei dieser Temperatur gehalten.
Kaliumhydroxid und 500 ml tert-Butanol wurde 16 h auf Rückflußtemperatur erhitzt.

Nach Abdestillieren des Lösungsmittels wurde das Rohprodukt einer Säulenchromatographie an Kieselgel mit einem Methylenchlorid/Aceton-Gemisch (10:1) als Eluens unterzogen.

Es wurden 440 mg N-(2, 6-diisopropylphenyl)-9-(bis-p-triethylene glycolphenyl) amino-perylene-3, 4-dicarboximide in Form eines blauen Feststoffs erhalten, was einer Ausbeute von 65% entspricht.

Analytische Daten:
MS (FD): m/z (rel. Int.) = 974,4

### Schritt c):

Verseifung analog Beispiel 22 (Schritt c)
Einer Säulenchromatographie an Kieselgel wurde einem Methylenchlorid/Aceton-Gemisch (10:1) als Eluens unterzogen.

Es wurden 170 mg I32 in Form eines blauen Feststoffs erhalten, was einer Ausbeute von 54% entspricht.

Analytische Daten:
UV-Vis (CH₂Cl₂): λₘₐₓ (ε) = 612, 466 nm;
MS (FD): m/z (rel. Int.) = 813,3 (100%) [M⁺].

### Beispiel 33

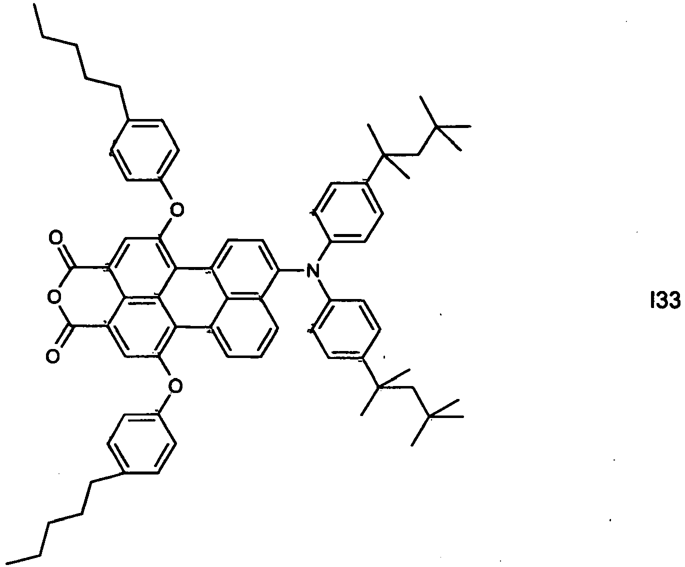

Die Herstellung des Rylenderivats I33 erfolgte ausgehend von N-(2, 6-diisopropylphenyl)-1, 6, 9-tribromo-perylene-3, 4-dicarboximide, das zunächst unter Bromaustausch mit 4-(n-pentyl) phenol zum N-(2,6-Diisopropyl-phenyl)-1,6-bis[phenoxy]-9-bromperylen-3,4-dicarbonsäureimid umgesetzt wurde (Schritt a), welches danach einen weiteren Bromaustausch mit Bis[(4-tert.-octyl)phenyl]amin zum aminderivat umgesetzt wurde (Schritt b), welches anschließend alkalisch zum Dicarbonsäureanhydrid I33 verseift wurde (Schritt c).

### Schritt a):

Phenoxylierung analog Beispiel 31
Eine Säulenchromatographie wurde an Kieselgel mit einem Methylenchlorid/Pentan-Gemisch (1:2) als Eluens unterzogen.

Es wurde 800 mg N-(2, 6-diisopropylphenyl) - 1, 6- bis[4-(n-pentyl) phenoxy]-9-bromo-perylene-3, 4-dicarboximide in Form eines roten Feststoffs erhalten, was einer Ausbeute von 45% entspricht.

### Schritt b):

Buchwald analog Beispiel 22 (Schritt a)
Nach Abdestillieren des Lösungsmittels wurde das Rohprodukt einer Säulenchromatographie an Kieselgel mit Toluol als Eluens unterzogen.

Es wurden 500 mg N-(2, 6-diisopropylphenyl)-9-(bis-p-tert-octylphenyl)amino-1,6-di(p-n- pentylphenoxy)-perylene-3, 4-dicarboximide in Form eines blauen Feststoffs erhalten, was einer Ausbeute von 70% entspricht.

### Schritt c):

Verseifung analog Beispiel 22 (Schritt b)
Einer Säulenchromatographie an Kieselgel mit Toluol als Eluens wurde unterzogen.

Es wurden 150 mg I33 in Form eines blauen Feststoffs erhalten, was einer Ausbeute von 50% entspricht.

Analytische Daten:
UV-Vis (CH₂Cl₂): λₘₐₓ (ε) = 603, 486 nm;
MS (FD): m/z (rel. Int.) = 1040,5 (100%) [M⁺].

### Beispiel 34

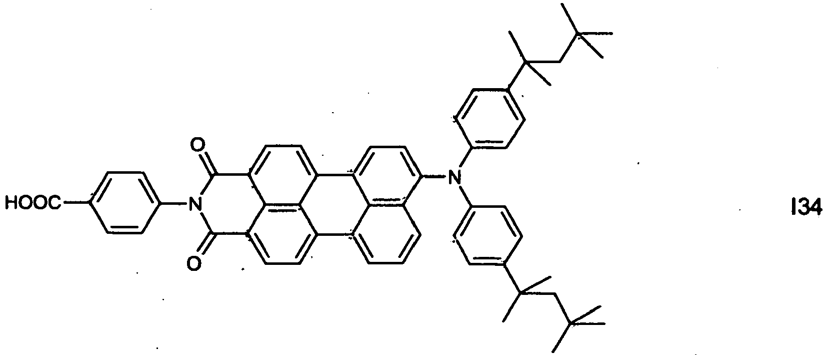

Zu einer unter Stickstoff vorgelegten Mischung von 0,20 g (0,28 mmol) N-(2,6-Diisopropylphenyl)-9-[bis(4-tert.-octyl)phenyl]aminoperylen-3,4-dicarbonsäureimid (Beispiel 7) und 10 ml wasserfreiem N-Methylpyrrolidon wurde unter Stickstoff wurden 0,05 g (0,28 mmol) wasserfreies Zinkacetat und 0,16 g (1,1 mmol) 4-Aminobenzoesäure gegeben. Die Mischung wurde dann auf 160°C erhitzt und 48 h bei dieser Temperatur gerührt.

Nach Abkühlen auf Raumtemperatur wurde das Produkt auf Wasser gegeben, abfiltriert, mit Wasser gewaschen und getrocknet. Es wurde einer Säulenchromatographie an Kieselgel mit einem Aceton/Methanol-Gemisch (1:1) und anschliessend mit reinem Methanol als Eluens unterzogen

Es wurden 0,038 g eines blauen Feststoffs erhalten, was einer Ausbeute von 16% entspricht.

Analytische Daten von I34:
UV-Vis (CH₂Cl₂): λₘₐₓ = 640, 595 nm;

### Beispiel 35

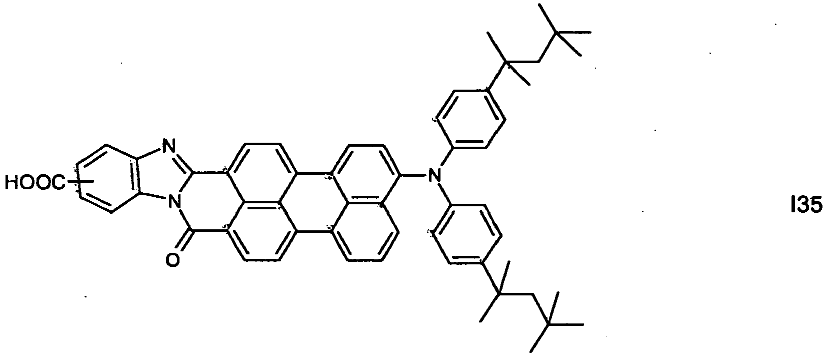

Bei dem Rylenderivat I35 handelt es sich um ein Gemisch zweier Isomere mit der Carb-oxylgruppe in 3- bzw. 4-Position am Phenylenring.

Eine Lösung von 0,10 g (0,14 mmol) N-(2,6-Diisopropylphenyl)-9-[bis(4-tert.-octyl)phenyl]aminoperylen-3,4-dicarbonsäureimid (Beispiel 7) in 6 ml Chinolin wurde unter Stickstoff in einem 50 ml-Schlenckrohr vorgelegt, dann wurden ebenfalls unter Stickstoff nacheinander 0,060 g (0,42 mmol) 3,4-Diaminobenzoesäure und 0,02 g wasserfreies Zinkacetat zugegeben. Die Mischung wurde dann unter Stickstoff auf 220°C erhitzt und unter Abtrennen des entstehenden Wassers 4 h bei dieser Temperatur gehalten.

Nach Abkühlen auf Raumtemperatur wurde das Reaktionsgemisch in eine Mischung von 100 ml 6gew.-%iger Salzsäure gegeben und ca. 12 h gerührt. Das auf diese Weise ausgefällte Produkt wurde abfiltriert, mit heißem Wasser gewaschen und im Vakuum bei 70°C getrocknet.

Es wurden 0,11 g I35 in Form eines blauen Feststoffs erhalten, was einer Ausbeute von 95% entspricht.

Analytische Daten des Isomerengemischs I35:
UV-Vis (CHCl₃): λₘₐₓ (ε) = 606 (12 000) nm (M⁻¹cm⁻¹);

### Beispiel 36

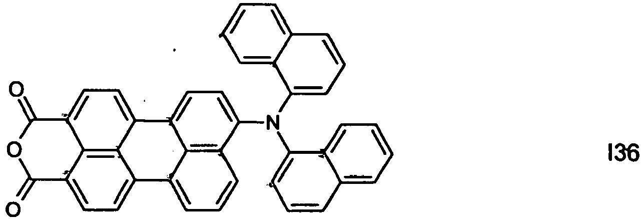

Die Herstellung des Rylenderivats I36 erfolgte ausgehend von N-(2,6-Diisopropyl-phenyl)-9-bromperylen-3,4-dicarbonsäureimid, das zunächst unter Bromaustausch mit N,N-Di(naphth-1-yl)amin zum aminosubstituierten Dicarbonsäureimid umgesetzt wurde (Schritt a), welches anschließend alkalisch zum Dicarbonsäureanhydrid I36 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,38 g (1,30 mmol) N,N-Di(naphth-1-yl)amin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Argon auf 80°C erhitzt und 16 h bei dieser Temperatur gerührt.

Nach Abdestillieren des Lösungsmittels wurde das Rohprodukt einer Säulenchromatographie an Kieselgel mit einem Methylenchlorid/Hexan-Gemisch (10:1) als Eluens unterzogen.

Es wurden 0,21 g N-(2,6-Diisopropylphenyl)-9-N,N-Di(naphth-1-yl)aminoperyien-3,4-dicarbonsäureimid in Form eines violeten Feststoffs erhalten, was einer Ausbeute von 32% entspricht.

Analytische Daten:
UV-Vis (CH₂Cl₂): λₘₐₓ = 523 nm ;
MS (FD): m/z (rel. Int.) = 784,3 (100%) [M⁺].

### Schritt b):

Eine Mischung von 200 mg (0,27 mmol) N-(2,6-Diisopropylphenyl)-9-N,N-Di(naphth-1-yl)aminoperylen-3,4-dicarbonsäureimid, 0,45 g (8,0 mol) Kaliumhydroxid und 15 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefällt, abfiltriert, mit Wasser neutral gewaschen, getrocknet und einer Säulenchromatographie an Kieselgel mit Methylenchlorid als Eluens unterzogen.

Es wurden 30 mg I36 in Form eines violeten Feststoffs erhalten, was einer Ausbeute von 19% entspricht.

Analytische Daten von I36:
UV-Vis (CH₂Cl₂): λₘₐₓ (ε) = 447 (21 000) nm (M⁻¹cm⁻¹);
MS (FD): m/z (rel. Int.) = 589,1 (100%) [M⁺].

### Beispiel 37

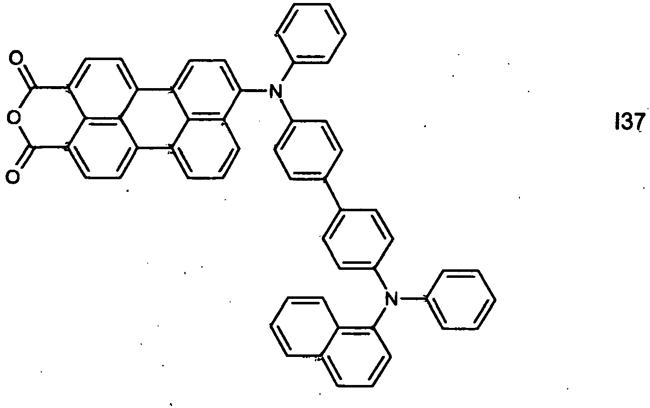

Die Herstellung des Rylenderivats I37 erfolgte ausgehend von N-(2,6-Diisopropyl-phenyl)-9-bromperylen-3,4-dicarbonsäureimid, das zunächst unter Bromaustausch mit N,N-Diphenyl-N'-naphth-1-yl-benzidine zum aminosubstituierten Dicarbonsäureimid umgesetzt wurde (Schritt a), welches anschließend alkalisch zum Dicarbonsäureanhydrid I37 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,62 g (1,30 mmol) N,N,N'-Triphenyl-p-phenylendiamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Stickstoff auf 80°C erhitzt und 16 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,650 g des aminsubstitutierten Perylens in Form eines blauen Feststoffs erhalten, was einer Ausbeute von 79% entspricht.

Analytische Daten:
UV-Vis (CH₂Cl₂): λₘₐₓ = 573 nm ;
MS (FD): m/z (rel. Int.) = 941,3 (100%) [M⁺].

### Schritt b):

Eine Mischung von 650 mg Perylenaminderivat (von Schritt a), 1,2 g (21,0 mol) Kaliumhydroxid und 15 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde gegen Dichlormethan ausgeschüttelt, einrotiert, getrocknet und einer Säulenchromatographie an Kieselgel mit Methylenchlorid als Eluens unterzogen.

Es wurden 310 mg I37 in Form eines blauen Feststoffs erhalten, was einer Ausbeute von 54% entspricht.

Analytische Daten von I37:
UV-Vis (CH₂Cl₂): λₘₐₓ (ε) = 593 (24 000) nm ;
MS (FD): m/z (rel. Int.) = 782,0 (100%) [M⁺].

### Beispiel 38

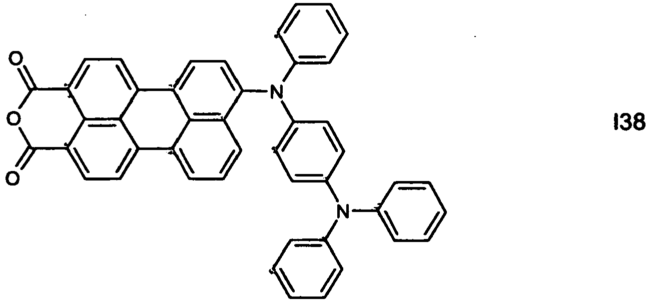

Die Herstellung des Rylenderivats I38 erfolgte ausgehend von N-(2,6-Diisopropylphenyl)-9-bromperylen-3,4-dicarbonsäureimid, das zunächst unter Bromaustausch mit N,N,N'-Triphenyl-p-phenylendiamin zum aminosubstituierten Dicarbonsäureimid umgesetzt wurde (Schritt a), welches anschließend alkalisch zum Dicarbonsäureanhydrid I38 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,46 g (1,30 mmol) N,N,N'-Triphenyl-p-phenylendiamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Stickstoff auf 80°C erhitzt und 16 h bei dieser Temperatur gerührt.

Nach Abdestillieren des Lösungsmittels wurde das Rohprodukt einer Säulenchromatographie an Kieselgel mit Chloroform als Eluens unterzogen.

Es wurden 0,285 g des aminsubstitutierten Perylens in Form eines violeten Feststoffs erhalten, was einer Ausbeute von 39% entspricht.

Analytische Daten:
UV-Vis (CH₂Cl₂): λₘₐₓ = 598 nm ;
MS (FD): m/z (rel. Int.) = 815,3 (100%) [M⁺].

### Schritt b):

Eine Mischung von 450 mg (0,55 mmol) Perylenaminderivat (von Schritt a), 0,93 g (17,0 mol) Kaliumhydroxid und 15 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 72 mg 138 in Form eines blauen Feststoffs erhalten, was einer Ausbeute von 20% entspricht.

Analytische Daten von I38:
UV-Vis (CH₂Cl₂): λₘₐₓ (ε) = 568 nm (25 000)
MS (FD): m/z (rel. Int.) = 656,1 (100%) [M⁺].

### Beispiel 39

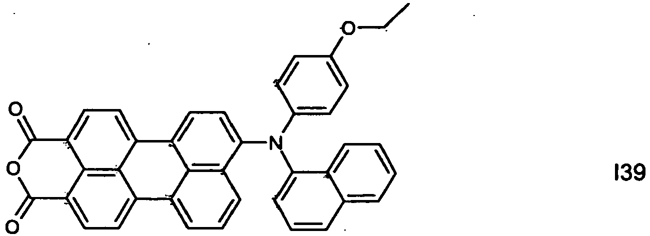

Die Herstellung des Rylenderivats I39 erfolgte ausgehend von N-(2,6-Diisopropyl-phenyl)-9-bromperylen-3,4-dicarbonsäureimid, das zunächst unter Bromaustausch mit N-(4-Ethoxy-phenyl)-1-Naphthylamin zum aminosubstituierten Dicarbonsäureimid umgesetzt wurde (Schritt a), welches anschließend alkalisch zum Dicarbonsäureanhydrid I39 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,40 g (1,30 mmol) N-(4-Ethoxy-phenyl)-1-Naphthylamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Argon auf 80°C erhitzt und 5 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,13 g N-(2,6-Diisopropylphenyl)-9-N-(4-Ethoxy-phenyl)-1-Naphthyl amino-perylen-3,4-dicarbonsäureimid in Form eines blauen Feststoffs erhalten, was einer Ausbeute von 20% entspricht.

Analytische Daten:
UV-Vis (CH₂Cl₂): λₘₐₓ = 575 nm ;
MS (FD): m/z (rel. Int.) = 742,3 (100%) [M⁺].

### Schritt b):

Eine Mischung von 120 mg (0,16 mmol) N-(2,6-Diisopropylphenyl)-9-)N-(4-Ethoxy-phenyl)-1-Naphthyl-aminoperylen-3,4-dicarbonsäureimid,0,27 g (4,9mol) Kaliumhydroxid und 10 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 20 mg I39 in Form eines blauen Feststoffs erhalten, was einer Ausbeute von 22% entspricht.

Analytische Daten von I39:
UV-Vis (CH₂Cl₂): λₘₐₓ (ε) = 457 (21 000) nm (M⁻¹cm⁻¹);
MS (FD): m/z (rel. Int.) = 583,3 (100%) [M⁺].

### Beispiel 40

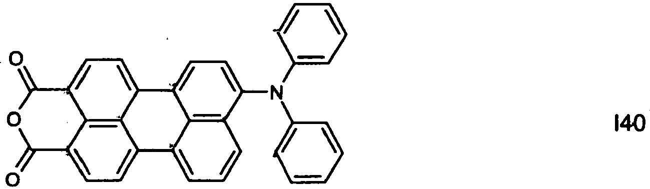

Die Herstellung des Rylenderivats I40 erfolgte ausgehend von N-(2,6-Diisopropyl-phenyl)-9-bromperylen-3,4-dicarbonsäureimid, das zunächst unter Bromaustausch mit Diphenylamin zum aminosubstituierten Dicarbonsäureimid umgesetzt wurde (Schritt a), welches anschließend alkalisch zum Dicarbonsäureanhydrid I40 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,20 g (1,30 mmol) Diphenylamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Argon auf 80°C erhitzt und 16 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,48 g des aminsubstituierten Perylens in Form eines violeten Feststoffs erhalten, was einer Ausbeute von 83% entspricht.

Analytische Daten:
UV-Vis (CH₂Cl₂): λₘₐₓ = 557 nm ;
MS (FD): m/z (rel. Int.) = 648,2 (100%) [M⁺].

### Schritt b):

Eine Mischung von 450 mg (0,70 mmol) Perylenamins (von Schritt a), 1,2 g (21,0 mol) Kaliumhydroxid und 10 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 60 mg I40 in Form eines blauen Feststoffs erhalten, was einer Ausbeute von 18% entspricht.

Analytische Daten von I40:
UV-Vis (CH₂Cl₂): λₘₐₓ (ε) = 445 (21 000) nm (M⁻¹cm⁻¹);
MS (FD): m/z (rel. Int.) = 489,0 (100%) [M⁺].

### Beispiel 41

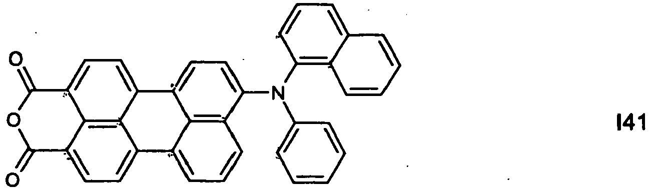

Die Herstellung des Rylenderivats 141 erfolgte ausgehend von N-(2,6-Diisopropyl-phenyl)-9-bromperylen-3,4-dicarbonsäureimid, das zunächst unter Bromaustausch mit N-Phenyl-1-naphtylamin zum aminosubstituierten Dicarbonsäureimid umgesetzt wurde (Schritt a), welches anschließend alkalisch zum Dicarbonsäureanhydrid 141 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,30 g (1,30 mmol) N-Phenyl-1-naphtylamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Stickstoff auf 80°C erhitzt und 16 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,39 g des aminsubstituierten Perylens in Form eines violeten Feststoffs erhalten, was einer Ausbeute von 62% entspricht.

Analytische Daten:
MS (FD): m/z (rel. Int.) = 698,2 (100%) [M⁺].

### Schritt b):

Eine Mischung von 350 mg (0,50 mmol) Perylenaminderivat (von Schritt a), 0,84 g (15,0 mol) Kaliumhydroxid und 20 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 79 mg I41 in Form eines blau-violeten Feststoffs erhalten, was einer Ausbeute von 30% entspricht.

Analytische Daten von I41:
UV-Vis (CH₂Cl₂): λₘₐₓ (ε) = 569 (21 000) nm (M⁻¹cm⁻¹);
MS (FD): m/z (rel. Int.) = 539,0 (100%) [M⁺].

### Beispiel 42

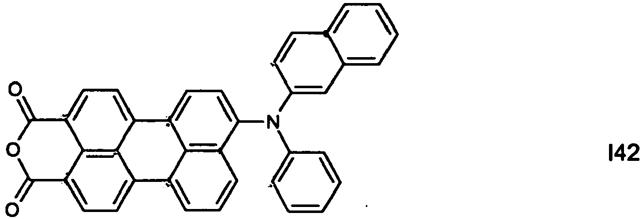

Die Herstellung des Rylenderivats 142 erfolgte ausgehend von N-(2,6-Diisopropyl-phenyl)-9-bromperylen-3,4-dicarbonsäureimid, das zunächst unter Bromaustausch mit N-Phenyl-2-naphtylamin zum aminosubstituierten Dicarbonsäureimid umgesetzt wurde (Schritt a), welches anschließend alkalisch zum Dicarbonsäureanhydrid I42 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,30 g (1,30 mmol) N-Phenyl-2-naphtylamin, 20 mg (0,018 mmol) Tris(dibenzylidenaceton)dipalladium(0), 90 mg (0,045 mmol) Tris(tert.-butyl)-phosphin, 130 mg (1,3 mmol) Natrium-tert.-butylat und 10 ml trockenem Toluol wurde unter Argon auf 80°C erhitzt und 16 h bei dieser Temperatur gerührt.

Nach Abdestillieren des Lösungsmittels wurde das Rohprodukt einer Säulenchromatographie an Kieselgel mit Chloroform als Eluens unterzogen.

Es wurden 0,41 g des aminsubstituierten Perylens in Form eines blaue-violeten Feststoffs erhalten, was einer Ausbeute von 65% entspricht.

Analytische Daten:
UV-Vis (CH₂Cl₂): λₘₐₓ = 561 nm;
MS (FD): m/z (rel. Int.) = 698,2 (100%) [M⁺].

### Schritt b):

Eine Mischung von 350 mg (0,50 mmol) Perylenaminderivat (von Schritt a), 0,84 g (15,0 mol) Kaliumhydroxid und 20 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 120 mg I42 in Form eines blau-violeten Feststoffs erhalten, was einer Ausbeute von 44% entspricht.

Analytische Daten von I42:
UV-Vis (CH₂Cl₂): λₘₐₓ (ε) = 576 (21 000) nm (M⁻¹cm⁻¹);
MS (FD): m/z (rel. Int.) = 539,1 (100%) [M⁺].

### Beispiel 43

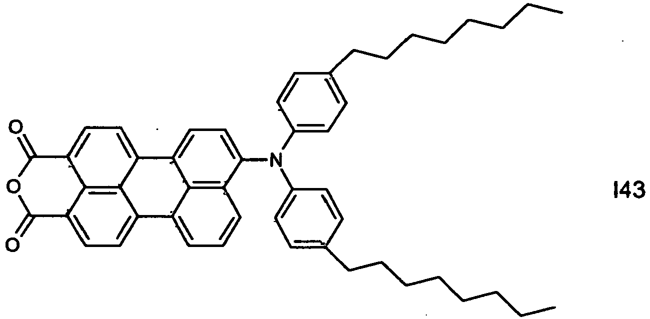

Die Herstellung des Rylenderivats I43 erfolgte ausgehend von N-(2,6-Diisopropylphenyl)-1,6-bis[(4-tert.-octyl)phenoxy]-9-bromperylen-3,4-dicarbonsäureimid, das zunächst unter Bromaustausch mit Bis[(4-tert.-octyl)phenyl]amin zum substituierten Dicarbonsäureimid umgesetzt wurde (Schritt a), welches anschließend alkalisch zum Dicarbonsäureanhydrid I43 verseift wurde (Schritt b).

### Schritt a):

Buchwald analog Beispiel 22 (Schritt a)
Nach Abdestillieren des Lösungsmittels wurde das Rohprodukt einer Säulenchromatographie an Kieselgel mit Dichlormethan als Eluens unterzogen.

Es wurden 700 mg N-(2, 6-diisopropylphenyl)-9-Bis-(4-octyl-phenyl)-amino-perylene-3, 4-dicarboximide in Form eines violeten Feststoffs erhalten, was einer Ausbeute von 90% entspricht.

Analytische Daten:
UV-Vis (CH₂Cl₂): λₘₐₓ (ε) = 582 (33 200) nm (M⁻¹cm⁻¹);
MS (FD): m/z (rel. Int.) = 873,9 (100%) [M⁺].

### Schritt b):

Verseifung analog Beispiel 22 (Schritt b)
Einer Säulenchromatographie an Kieselgel mit Toluol als Eluens wurde unterzogen.

Es wurden 350 mg I43 in Form eines violeten Feststoffs erhalten, was einer Ausbeute von 72% entspricht.

Analytische Daten:
UV-Vis (CH₂Cl₂): λₘₐₓ (ε) = 596 (16 000) nm (M⁻¹cm⁻¹);
MS (FD): m/z (rel. Int.) = 713,4 (100%) [M⁺].

### Beispiel 44

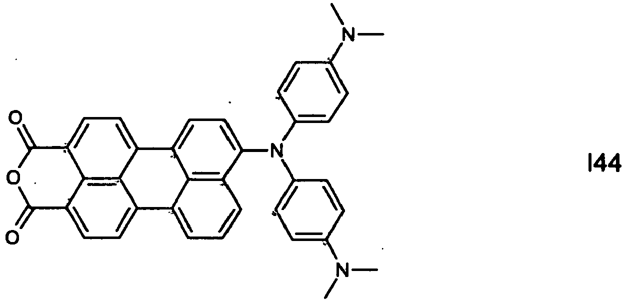

Die Herstellung des Rylenderivats I44 erfolgte ausgehend von N-(2,6-Diisopropyl-phenyl)-9-bromperylen-3,4-dicarbonsäureimid, das zunächst unter Bromaustausch mit Bindschedler's Green zum aminosubstituierten Dicarbonsäureimid umgesetzt wurde (Schritt a), welches anschließend alkalisch zum Dicarbonsäureanhydrid I44 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,34 g (1,30 mmol) Binschedler's Green, 40 mg (0,045 mmol) Tris(dibenzylidenaceton)dipalladium(0), 0,45ml (0,45 mmol) Tris(tert.-butyl)-phosphin, 120 mg (1,3 mmol) Natrium-tert.-butylat und 30 ml trockenem Toluol wurde unter Argon auf 80°C erhitzt und 16 h bei dieser Temperatur gerührt.

Nach Abdestillieren des Lösungsmittels wurde das Rohprodukt einer Säulenchromatographie an Kieselgel mit einem Methylenchlorid/Ethanol-Gemisch (50:1) als Eluens unterzogen.

Es wurden 0,35 g Perylenamindeivats in Form eines grünen Feststoffs erhalten, was einer Ausbeute von 53% entspricht.

Analytische Daten:
UV-Vis (CH₂Cl₂): λₘₐₓ = 667 nm ;
MS (FD): m/z (rel. Int.) = 734,2 (100%) [M⁺].

### Schritt b):

N-(2,6-Diisopropylphenyl)-9-[bis(4-tert.-octyl)phenyl]aminoperylen-3,4-dicarbonsäureimid
Eine Mischung von 330 mg (0,45 mmol) N-(2,6-Diisopropylphenyl)-9-[bis(4-bismethylamino)phenyl]aminoperylen-3,4-dicarbonsäureimid, 0,76 g (13.5mmol) Kaliumhydroxid und 50 ml tert-Butanol wurde 16 h auf Rückflußtemperatur erhitzt.

Nach Abkühlen auf 70°C wurde zum Reaktionsgemisch 50 gew.%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid/Ethanol 50:1 als Eluens unterzogen.

Es wurden 30 mg I44 in Form eines blauen Feststoffs erhalten, was einer Ausbeute von 11% entspricht.

Analytische Daten von I44:
UV-Vis (CH₂Cl₂): λₘₐₓ (ε) = 689 (21 000) nm (M⁻¹cm⁻¹);
MS (FD): m/z (rel. Int.) = 575,1 (100%) [M⁺].

### Beispiel 45

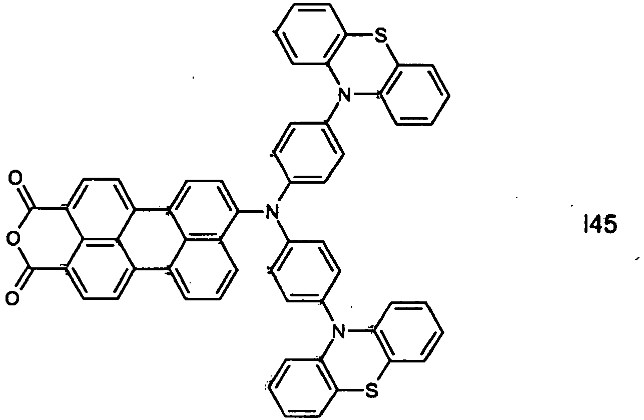

Die Herstellung des Rylenderivats I45 erfolgte ausgehend von N-(2,6-Diisopropyl-phenyl)-1,6-bis[(4-tert.-octyl)phenoxy]-9-bromperylen-3,4-dicarbonsäureimid, das zunächst unter Bromaustausch mit Bis(4-(Phenothiazinal)phenyl)-amin zum substituierten Dicarbonsäureimid umgesetzt wurde (Schritt a), welches anschließend alkalisch zum Dicarbonsäureanhydrid I45 verseift wurde (Schritt b).

### Schritt a):

Buchwald analog Beispiel 22 (Schritt a)

Nach Abdestillieren des Lösungsmittels wurde das Rohprodukt einer Säulenchromatographie an Kieselgel mit Dichlormethan als Eluens unterzogen.

Es wurden 1,2 g N-(2, 6-diisopropylphenyl)-9-bis-(phenothiazin-yl-phenyl) amino - perylene-3, 4-dicarboximide in Form eines violeten Feststoffs erhalten, was einer Ausbeute von 81 % entspricht.

Analytische Daten:
UV-Vis (CH₂Cl₂): λₘₐₓ = 544 nm;
MS (FD): m/z (rel. Int.) = 1045,3 (100%) [M⁺].

### Schritt b):

Verseifung analog Beispiel 22 (Schritt b)
Einer Säulenchromatographie an Kieselgel mit Dichlormethan als Eluens wurde unterzogen.

Es wurden 400 mg 145 in Form eines violeten Feststoffs erhalten, was einer Ausbeute von 59% entspricht.

Analytische Daten:
UV-Vis (CH₂Cl₂): λₘₐₓ (ε) = 556 (6000) nm (M⁻¹cm⁻¹);
MS (FD): m/z (rel. Int.) = 884,5 (100%) [M⁺].

### Beispiel 46

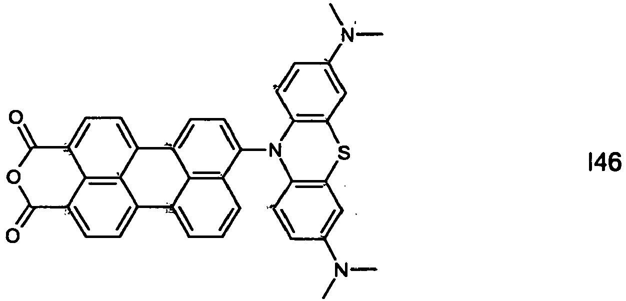

Die Herstellung des Rylenderivats I46 erfolgte ausgehend von N-(2,6-Diisopropyl-phenyl)-9-bromperylen-3,4-dicarbonsäureimid, das zunächst unter Bromaustausch mit 3,7-Bis-dimethylamino-phenothiazin (zuerst nach der Entschutsung von (3,7-Bis-dimethylamino-phenothiazin-10-yl)-phenyl-methanone) zum phenothiazinsubstituierten Dicarbonsäureimid umgesetzt wurde (Schritt a), welches anschließend alkalisch zum Dicarbonsäureanhydrid I46 verseift wurde (Schritt b).

### Schritt a):

Eine Mischung von (3,7-Bis-dimethylamino-phenothiazin-10-yl)-phenyl-methanone (1,5 g, 3,8 mmol), Natriumhydroxid (310 mg, 3,8 mmol), THF (5 mL) und Methanol (15 mL) wurde 12 h auf Rückflußtemperatur erhitzt. Nach Ankülen wurde die Lösungsmittel mit Argon entfernt.

Eine Mischung von 1,5 g (3,1 mmol) N-(2,6-Diisopropylphenyl)-9-bromperylen-3,4-dicarbonsäureimid, 0,123 g (0,13 mmol) Tris(dibenzylidenaceton)dipalladium(0), 27 mg (0,13 mmol) Tris(tert.-butyl)-phosphin, 12 mg (0,13 mmol) Natrium-tert.-butylat und 100 ml trockenem Toluol wurde unter Argon dazugegeben, auf 80°C erhitzt und 16 h bei dieser Temperatur gerührt.

Nach Abdestillieren des Lösungsmittels wurde das Rohprodukt einer Säulenchromatographie an Kieselgel mit einem Methylenchlorid/Methanol-Gemisch (20:1) als Eluens unterzogen.

Es wurden 0,500 g N-(2,6-diisopropylphenyl)-9-(3,7-bis-dimethylamino-phenothiazine-10-yl)-perylene-3, 4-dicarboximide in Form eines braunen Feststoffs erhalten, was einer Ausbeute von 17% entspricht.

Analytische Daten:
UV-Vis (CH₂Cl₂): λₘₐₓ = 510, 486 nm;
MS (FD): m/z (rel. Int.) = 765,0 (100%) [M⁺].

### Schritt b):

Verseifung analog Beispiel 22 (Schritt b)
Einer Säulenchromatographie an Kieselgel mit mit einem Methylenchlorid/Methanol-Gemisch (20:1) als Eluens unterzogen.

Es wurden 200 mg I46 in Form eines braunen Feststoffs erhalten, was einer Ausbeute von 72% entspricht.

Analytische Daten:
MS (FD): m/z (rel. Int.) = 606,0 (100%) [M⁺].

### Beispiel 47

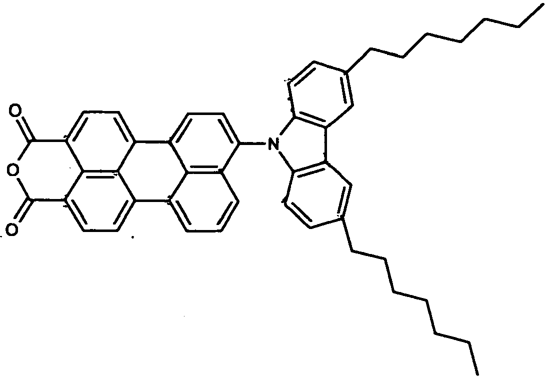

Die Herstellung des Rylenderivats I47 erfolgte ausgehend von N-(2,6-Diisopropyl-phenyl)-1,6-bis[(4-tert.-octyl)phenoxy]-9-bromperylen-3,4-dicarbonsäureimid, das zunächst unter Bromaustausch mit 3,6-Bisheptylcarbazol zum substituierten Dicarbonsäureimid umgesetzt wurde (Schritt a), welches anschließend alkalisch zum Dicarbonsäureanhydrid I47 verseift wurde (Schritt b).

### Schritt a):

Buchwald analog Beispiel 22 (Schritt a)
Nach Abdestillieren des Lösungsmittels wurde das Rohprodukt einer Säulenchromatographie an Kieselgel mit Dichlormethan als Eluens unterzogen.

Es wurden 400 mg N-(2, 6-diisopropylphenyl)-9-(3,6-diheptyl-carbazol-9-yl)-perylene-3, 4-dicarb- oximide in Form eines violeten Feststoffs erhalten, was einer Ausbeute von 85% entspricht.

Analytische Daten:
UV-Vis (CH₂Cl₂): λₘₐₓ = 514 nm;

### Schritt b):

Verseifung analog Beispiel 22 (Schritt b)
Einer Säulenchromatographie an Kieselgel mit Dichlormethan als Eluens wurde unterzogen.

Es wurden 80 mg I46 in Form eines violeten Feststoffs erhalten, was einer Ausbeute von 49% entspricht.

Analytische Daten:
UV-Vis (CH₂Cl₂): λₘₐₓ (ε) = 596 (16 000) nm (M⁻¹cm⁻¹);
MS (FD): m/z (rel. Int.) = 713,4 (100%) [M⁺].

### Beispiel 48

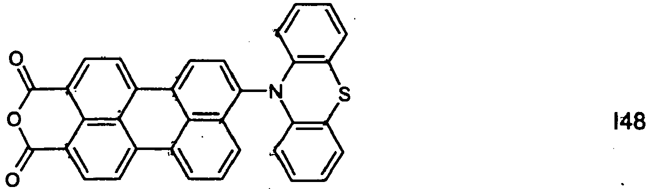

Die Herstellung des Rylenderivats I48 erfolgte ausgehend von N-(2,6-Diisopropyl-phenyl)-9-bromperylen-3,4-dicarbonsäureimid, das zunächst unter Bromaustausch mit Phenothiazin zum aminosubstituierten Dicarbonsäureimid umgesetzt wurde (Schritt a), welches anschließend alkalisch zum Dicarbonsäureanhydrid I48 verseift wurde (Schritt b).

### Schritt a):

Eine Mischung von 0,50 g (0,89 mmol) N-(2,6-Diisopropylphenyl)-9-bromperylen-3,4-dicarbonsäureimid, 0,40 g (1,30 mmol) Phenothiazin, 70 mg (0,08 mmol) Tris(dibenzylidenaceton)dipalladium(0), 65 mg (0,9 mmol) BINAP, 340 mg (3,6 mmol) Natrium-tert.-butylat und 40 ml trockenem Toluol wurde unter Stickstoff auf 80°C erhitzt und 3 d bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,5 g des phenothiazinylsubstituierten Perylens als roter Feststoff erhalten, was einer Ausbeute von 41% entspricht.

Analytische Daten:
MS (FD): m/z (rel. Int.) = 678,2 (100%) [M⁺].

### Schritt b):

Eine Mischung von 500 mg (0,7 mmol) Perylenphenothiazinderivats, 2,0 g (35,0 mol) Kaliumhydroxid und 20 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 90 mg I48 in Form eines roten Feststoffs erhalten, was einer Ausbeute von 25% entspricht.

Analytische Daten von I48:
UV-Vis (CH₂Cl₂): λₘₐₓ (ε) = 432 (21000) nm (M⁻¹cm⁻¹);
MS (FD): m/z (rel. Int.) = 519,0 (100%) [M⁺].

### Beispiel 49

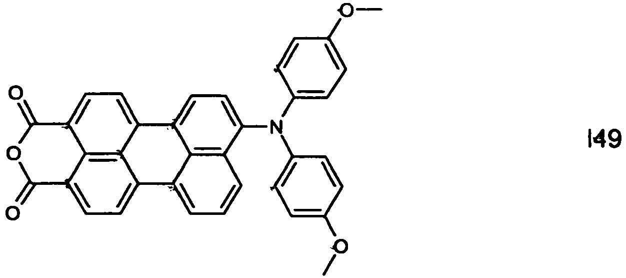

Die Herstellung des Rylenderivats I49 erfolgte ausgehend von N-(2,6-Diisopropyl-phenyl)-9-bromperylen-3,4-dicarbonsäureimid, das zunächst unter Bromaustausch mit 4,4-Dimethoxydiphenylamin zum aminosubstituierten Dicarbonsäureimid umgesetzt wurde (Schritt a), welches anschließend alkalisch zum Dicarbonsäureanhydrid I49 verseift wurde (Schritt b).

### Schritt a):

Eine Mischung von 1,0 g (1,8 mmol) N-(2,6-Diisopropylphenyl)-9-bromperylen-3,4-dicarbonsäureimid, 0,8 g (3,6 mmol) 4,4-Dimethoxydiphenylamin, 80 mg (0,08 mmol) Tris(dibenzylidenaceton)dipalladium(0), 560 mg (0,9 mmol) BINAP, 340 mg (3,6 mmol) Natrium-tert.-butylat und 40 ml trockenem Toluol wurde unter Stickstoff auf 80°C erhitzt und 16 h bei dieser Temperatur gerührt.

Nach Abdestillieren des Lösungsmittels wurde das Rohprodukt einer Säulenchromatographie an Kieselgel mit Dichlormethan als Eluens unterzogen.

Es wurden 0,2 g N-(2,6-Diisopropylphenyl)-9-[bis(4-methoxy)-phenyl]aminoperylen-3,4-dicarbonsäureimid in Form eines blauen Feststoffs erhalten, was einer Ausbeute von 15% entspricht.

Analytische Daten:
UV-Vis (CH₂Cl₂): λₘₐₓ = 597 nm ;
MS (FD): m/z (rel. Int.) = 709,1 (100%) [M⁺].

### Schritt b):

Eine Mischung von 150 mg (0,2 mmol) N-(2,6-Diisopropylphenyl)-9-[bis(4-methoxy)-phenyl]aminoperylen-3,4-dicarbonsäureimid, 0,65 g (10,0 mol) Kaliumhydroxid und 10 ml tert-Butanol wurde 16 h auf Rückflußtemperatur erhitzt.

Nach Abkühlen auf 40°C wurde zum Reaktionsgemisch 50 gew.-%iger Essigsäure gegeben und 1 h bei dieser Temperatur gerührt. Das Produkt wurde auf Wasser ausgefallen, abfiltriert, mit Wasser neutral gewaschen, getrocknet und einer Säulenchromatographie an Kieselgel mit Methylenchlorid als Eluens unterzogen.

Es wurden 25 mg I49 in Form eines blauen Feststoffs erhalten, was einer Ausbeute von 23% entspricht.

Analytische Daten von I49:
UV-Vis (CH₂Cl₂): λₘₐₓ (ε) = 474 (21 000) nm (M⁻¹cm⁻¹);
MS (FD): m/z (rel. Int.) = 549,1 (100%) [M⁺].

### II. Anwendung von Rylenderivaten I

Um die Eignung der Rylenderivate I als Farbstoffsensibilisatoren in Solarzellen zu testen, wurden wie folgt Solarzellen hergestellt.

Als Basismaterial wurden mit fluordotiertem Zinnoxid (FTO) beschichtete Glasplatten der Abmessung 12 mm x 14 mm x 3 mm oder 25 mm x 15 mm x 3 mm (Pilkington TEC 8) eingesetzt, die nacheinander mit Glasreiniger, Aceton und Ethanol jeweils 15 min im Ultraschallbad behandelt, dann in Ethanol gelagert und vor dem Einsatz im Stickstoffstrom getrocknet worden waren.

Zur Herstellung der Gegenelektrode wurden 20µl einer 5 mM Lösung von H₂PtCl₆ in Isopropanol gleichmäßig auf der beschichteten Seite einer FTO-Glasplatte mit Bohrlöchern verteilt und nach kurzem Trocknen an Luft 30 min bei 380°C gesintert und anschließend staubfrei gelagert.

Zur Herstellung der Arbeitselektrode wurde folgendermaßen vorgegangen:
Aus einem Klebeband wurde ein rundes Loch von 10 mm Durchmesser ausgestanzt. Danach wurde das Klebeband als Schablone auf die beschichtete Seite einer FTO-Glasplatte ohne Bohrlöcher aufgeklebt. Dann wurde mit einem Rakel eine vorwiegend aus dem jeweiligen Halbleiter-Metalloxid bestehende Paste ein- oder mehrfach aufgezogen.

Nach Entfernen des Klebebands und kurzem Trocknen bei 80°C wurde die metalloxidbeschichtete Glasplatte 30 min bei 450°C gesintert, dann auf 80°C abkühlen gelassen und 1 bis 24 h in eine 5 x 10⁻⁴ bis1 x 10⁻⁵ M Lösung des jeweiligen Rylenderivats I in einem organischen Lösungsmittel gelegt. Die aus der Lösung herausgenommene Glasplatte wurde dem entsprechenden Lösungsmittel abgespült und im Stickstoffstrom getrocknet.

Nähere Einzelheiten zur Herstellung der Arbeitselektrode sind den Beispielen zu entnehmen.

Die Glasplatten wurden anschließend so mit einer 50 µm dicken Heißklebefolie (Surlyn^{®} 1702; DuPont) versiegelt. Der Zwischenraum zwischen den beiden Elektroden wurde danach über die Bohrlöcher mit dem jeweils in den Beispielen angegebenen Elektrolyt befüllt.

Nach Versiegelung der Bohrlöcher mit weiterer Heißklebefolie wurden die Kontaktflächen von Arbeits- und Gegenelektrode mit Silberleitlack bestrichen und mit Kupferklebeband (3M) beklebt.

Die so hergestellten Zellen hatten eine aktive Fläche von 0,32 cm² bzw. 0,502 cm².

Die Quanteneffizienz (IPCE = Incident Photon-to-current Conversion Efficiency) wurde dann mit einer 75 Watt-Xenon-Bogenlampe (LOT-Oriel), einem 1/8 m Monochromator (SpectraPro-2150i; Acton Research Corporation), einem Transimpedanzverstärker (Aescusoft GmbH Automation) und einem Lock-in-Verstärker 7265 (Signal Recovery) gemessen.

Zur Bestimmung des Wirkungsgrads η wurde die jeweilige Strom/Spannungs-Kennlinie mit einem Source Meter Model 2400 (Keithley Instruments Inc.) unter Bestrahlung mit einem Halogen-Lampenfeld (Xenophot^{®} 64629; Osram) (Beispiele 1-3) oder mit einem einem Xenon-Lampe (300W Xe Arc Lamp, LSN252, LOT Oriel Gruppe) (Beispiele 4-5) als Sonnensimulator gemessen.

Die folgenden Rylenderivate I wurden getestet:
- Farbstoff A:: Rylenderivat I2 aus Beispiel 2
- Farbstoff B:: N-(2,6-Diisopropylphenyl)-1,6,9,14-tetra[(4-tert.-octyl)phenoxy]terrylen-3,4:11,12-tetracarbonsäuremonoimidmonoanhydrid, hergestellt gemäß Beispiel 1 der älteren deutschen Patentanmeldung 10 2005 021 362.6
- Farbstoff C:: N-(2,6-Diisopropylphenyl)-1,6,11,16-tetra[(4-tert.-octyl)phenoxy]quaterry-len-3,4:13,14-tetracarbonsäuremonoimidmonoanhydrid, hergestellt gemäß Beispiel 8 der älteren deutschen Patentanmeldung 10 2005 021 362.6
- Farbstoff D:: Rylenderivat I6 aus Beispiel 6
- Farbstoff E:: Rylenderivat I7 aus Beispiel 7
- Farbstoff F:: Rylenderivat 18 aus Beispiel 8
- Farbstoff G:: Rylenderivat I9 aus Beispiel 9
- Farbstoff H:: Rylenderivat I32 aus Beispiel 24
- Farbstoff I:: Rylenderivat I34 aus Beispiel 34
- Farbstoff J:: Rylenderivat I35 aus Beispiel 35

Zum Vergleich wurde der Ruthenium-Komplex N-179 (Farbstoff V), dessen Struktur z.B. in J. Chem. Phys. B 107, S. 13280-13285 (2003) beschrieben ist, getestet.

### Beispiel 1

Der Farbstoff B wurde in einer TiO₂-Solarzelle eingesetzt.

Beschreibung der Zelle sowie der Einsatzstoffe:
FTO-beschichtete Glasplatten der Abmessung 25 mm x 15 mm x 3 mm
TiO₂-Paste Ti-Nanoxide HT (Solaronix SA)
TiO₂-Schichtdicke nach dem Sintern (450°C/30 min): 9 µm
0,5 mM Lösung des Farbstoffs B in Toluol (eingelegt bei 80°C), Einlegezeit 14 h bei Raumtemperatur
Elektrolyt: 0,5 M Lil, 0,25 M Tetrabutylammoniumiodid und 0,05 M I₂ in Methoxyproprionitril
aktive Fläche: 0,502 cm²

Zum Vergleich wurde der Farbstoff V in einer TiO₂-Solarzelle eingesetzt. Die Parameter stimmten, soweit nicht anders angegeben, mit den obengenannten Parametern überein.

TiO₂-Schichtdicke nach dem Sintern: 17 µm
0,5 mM Lösung des Farbstoffs V in Ethanol (eingelegt bei 80°C), Einlegezeit 14 h bei Raumtemperatur
Elektrolyt: 0,5 M LiI, 0,6 M Tetrabutylammoniumiodid, 0,05 M I₂ und 0,5 M 4-tert.-Butylpyridin in Acetonitril

Beide Solarzellen wurden unter 0,1 Sonne belichtet.

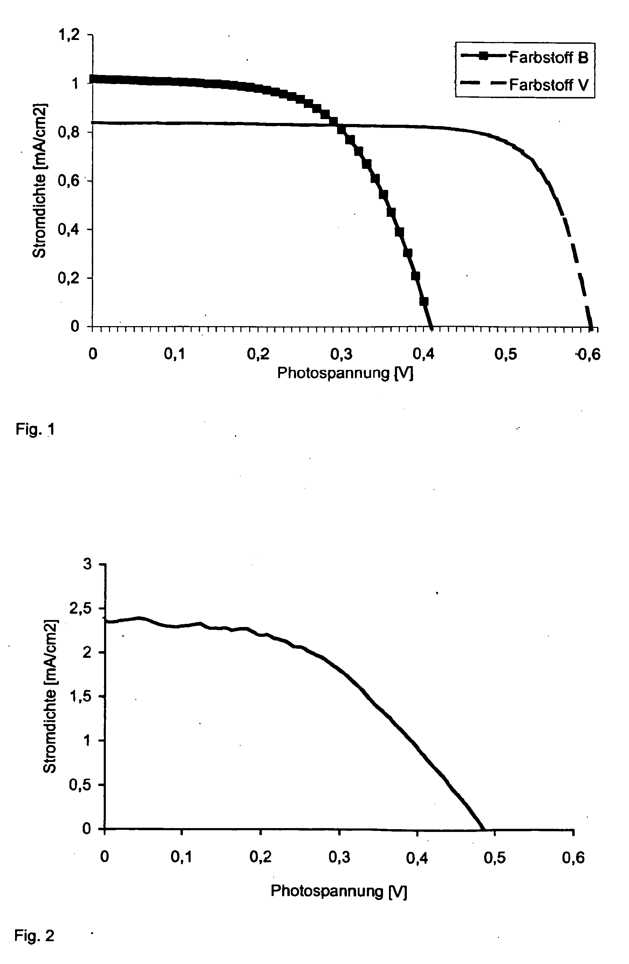

Die hierbei gemessenen Strom/Spannungs-Kurven sind in Fig. 1 abgebildet.

Die ermittelten Wirkungsgrade η betrugen 2,25% für den Farbstoff B und 5,06% für den Farbstoff V.

### Beispiel 2

Der Farbstoff B wurde in einer ZnO-Solarzelle eingesetzt.

Beschreibung der Zelle sowie der Einsatzstoffe:
FTO-beschichtete Glasplatten der Abmessung 12 mm x 14 mm x 3 mm
ZnO-Paste, hergestellt nach dem in J. Phys. Chem. B 105, S. 5585-5587 (2001) beschriebenen Rezept
ZnO-Schichtdicke nach dem Sintern (350°C/30 min): 0,9 µm
0,5 mM Lösung des Farbstoffs B in N-Methylpyrrolidon, Einlegezeit 14 h bei 50°C Elektrolyt: 0,6 M Tetrabutylammoniumiodid und 0,05 M I₂ in Methoxyproprionitril aktive Fläche: 0,32 cm²

Die Solarzelle wurde unter 1 Sonne belichtet.

Die hierbei gemessenen Strom/Spannungs-Kurve ist in Fig. 2 abgebildet.

Die ermittelte Wirkungsgrad η betrug 0,54%.

### Beispiel 3

Die Farbstoffe A, B und C wurden jeweils in einer TiO₂-Solarzelle eingesetzt.

Beschreibung der Zelle sowie der Einsatzstoffe:
FTO-beschichtete Glasplatten der Abmessung 25 mm x 15 mm x 3 mm
TiO₂-Paste mit einer TiO₂-Partikelgröße von 25 nm (ECN)
TiO₂-Schichtdicke nach dem Sintern (450°C/30 min): 4 µm
0,5 mM Lösung des jeweiligen Farbstoffs in Toluol (eingelegt bei 80°C), Einlegezeit 14 h bei Raumtemperatur
Elektrolyt: 0,5 M Lil, 0,05 M I₂ und 0,5 M 4-tert.-Butylpyridin in Methoxyproprionitril aktive Fläche: 0,502 cm²

Zum Vergleich wurde der Farbstoff V in einer TiO₂-Solarzelle eingesetzt. Die Parameter stimmten, soweit nicht anders angegeben, mit den obengenannten Parametern überein.

TiO₂-Schichtdicke nach dem Sintern: 17 µm
0,5 mM Lösung des Farbstoffs V in Ethanol (eingelegt bei 80°C), Einlegezeit 14 h bei Raumtemperatur
Elektrolyt: 0,5 M LiI, 0,6 M Tetrabutylammoniumiodid, 0,05 M I₂ und 0,5 M 4-tert.-Butylpyridin in Acetonitril

Alle Solarzellen wurden unter 0,1 Sonne belichtet.

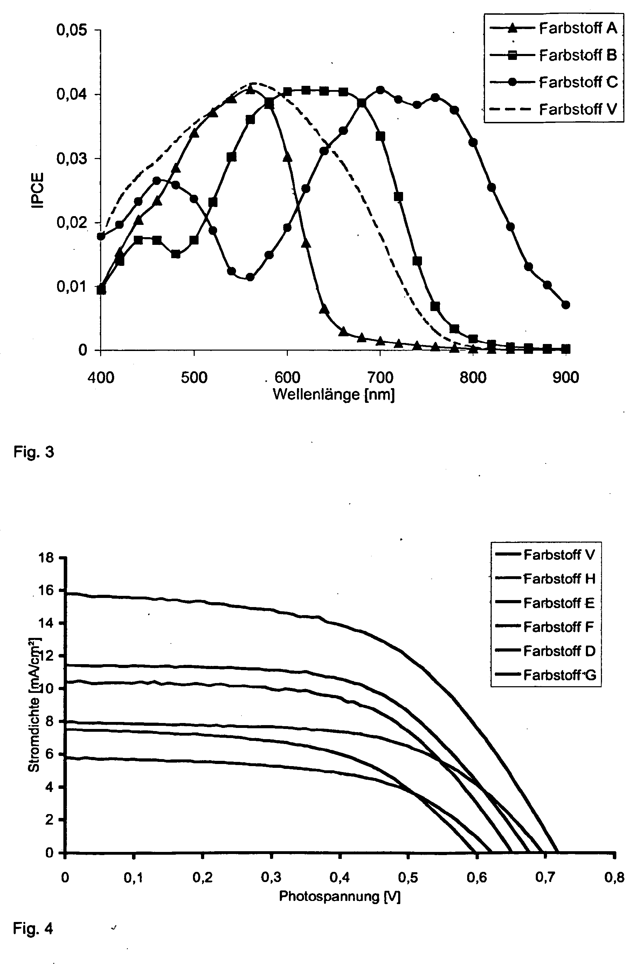

Die jeweils gemessenen IPCE-Kurven sind in Fig. 3 abgebildet. Die für den Farbstoff C erhaltene Kurve wurde dabei an die Kurven der anderen Farbstoffe angeglichen.

Die ermittelten Wirkungsgrade η betrugen 1,6% für den Farbstoff A, 2,2% für den Farbstoff B, 0,6% für den Farbstoff C und 5% für den Farbstoff V.

### Beispiel 4

Die Farbstoffe D, E, F, G und H wurden in TiO₂-Solarzellen eingesetzt.

Beschreibung der Zelle sowie der Einsatzstoffe:
FTO-beschichtete Glasplatten der Abmessung 25 mm x 15 mm x 3 mm
TiO₂-Paste Ti-Nanoxide HT (Solaronix SA)
TiO₂-Schichtdicke nach dem Sintern (450°C/30 min): 9-10µm
0,5 mM Lösung des Farbstoffs in Dichlormethan (eingelegt bei 80°C), Einlegezeit 14 h bei Raumtemperatur
Elektrolyt: 0,1 M Lil, 0,6 M Tetrabutylammoniumiodid , 0,05 M I₂ und 0.5 M 4-tert.-Butylpyridin in Acetonitril
aktive Fläche: 0,502 cm²

Zum Vergleich wurde der Farbstoff V in einer TiO₂-Solarzelle eingesetzt. Die Parameter stimmten, soweit nicht anders angegeben, mit den obengenannten Parametern überein.

TiO₂-Schichtdicke nach dem Sintern: 9-10µm
0,5 mM Lösung des Farbstoffs V in Ethanol (eingelegt bei 80°C), Einlegezeit 14 h bei Raumtemperatur
Elektrolyt: 0,1 M Lil, 0,6 M 2,3-dimethyl-1-propyl imidazolium iodid, 0,05 M I₂ und 0,5 M 4-tert.-Butyl-pyridin in Acetonitril

Beide Solarzellen wurden unter 1,0 Sonne belichtet.

Die hierbei gemessenen Strom/Spannungs-Kurven sind in Fig. 4 abgebildet.

Die ermittelten Wirkungsgrade η betrugen 6,0% für den Farbstoff V, 4,4% für den Farbstoff H, 3,9% für den Farbstoff E, 3,2% für den Farbstoff F, 2,4% für den Farbstoff D und 2,0% für den Farbstoff G.

### Beispiel 5

Die Farbstoffe I und J wurden jeweils in einer TiO₂-Solarzelle eingesetzt.

Beschreibung der Zelle sowie der Einsatzstoffe:
FTO-beschichtete Glasplatten der Abmessung 25 mm x 15 mm x 3 mm
TiO₂-Paste mit einer TiO₂-Partikelgröße von 25 nm (ECN)
TiO₂-Schichtdicke nach dem Sintern (450°C/30 min): 9-10µm
0,5 mM Lösung des jeweiligen Farbstoffs in (Farbstoff I) oder in Dichlormethan (Farbstoff J) (eingelegt bei 80°C), Einlegezeit 14 h bei Raumtemperatur
Elektrolyt: 0,5 M Lil, 0.25 Tetrabutylammoniumiodid und 0,05 M I₂ in 3-Methoxyproprionitril
aktive Fläche: 0,502 cm²

Beide Solarzellen wurden unter 0,1 Sonne belichtet.

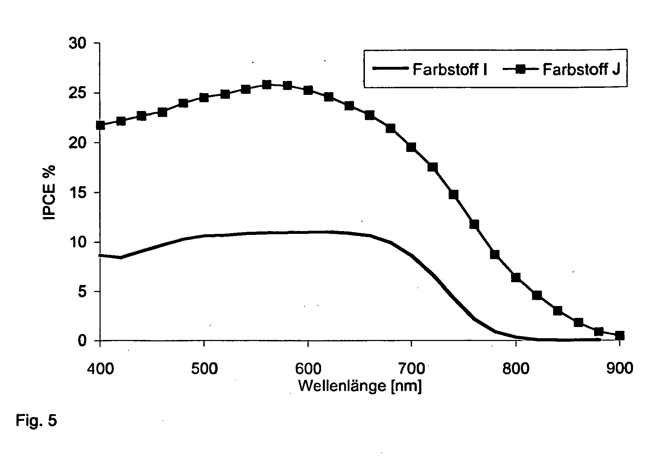

Die jeweils gemessenen IPCE-Kurven sind in Fig. 5 abgebildet

Die ermittelten Wirkungsgrade η betrugen 1,0 % für den Farbstoff I und 0,7% für den Farbstoff J.

## Patentansprüche

1. Verwendung von Rylenderivaten der allgemeinen Formel I 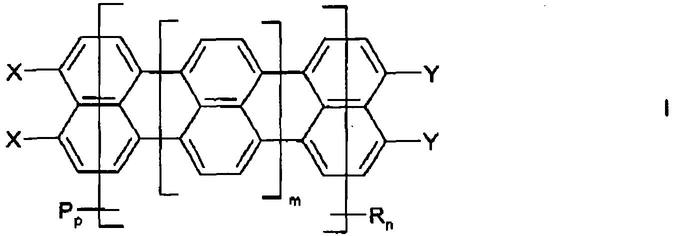 in der die Variablen folgende Bedeutung haben:
X miteinander unter Ausbildung eines Sechsrings verbunden zu einem Rest der Formel (x1), (x2) oder (x3) 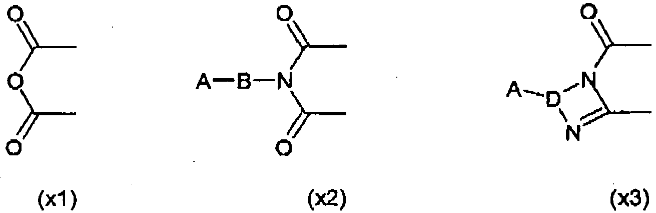 beide ein Rest -COOM;
Y einer der beiden Reste ein Rest der Formel (y1)
-L-NR¹R² (y1)
oder ein Rest der Formel (y2)
-L-Z-R³ (y2)
und der andere Rest jeweils Wasserstoff;
miteinander unter Ausbildung eines Sechsrings verbunden zu einem Rest der Formel (y3) oder (y4) 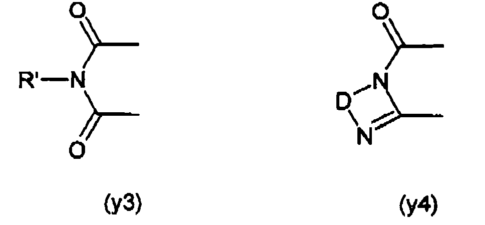 oder beide Wasserstoff:
R gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, annellert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO-und/oder -SO₂- unterbrochen sein kann und das ein- oder mehrfach sub-stituiert sein kann durch: C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂,-POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, wobei die (Het)Aryl- und Cyclo-alkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO-und/oder -SO₂- unterbrochen sein kann, anneliert sein können, wobei das gesamte Ringsystem ein- oder mehrfach substituiert sein kann durch: C₁-C₁₈-Alkyl, C₁-C₁₂₋Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶; -COOR⁷, -SO₃R⁷, -PR⁷₂ und/oder -POR⁷R⁷;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷. -SO₃R⁷, -PR⁷₂ ,-POR⁷R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, C₁-C₆-Alkylthio, Hydroxy, Mercapto, Halogen, Cyano, Nitro, -NR⁹R¹⁰, -NR⁵COR⁶, -CONR⁵R⁶, -SO₂NR⁵R⁶ ,-COOR⁷, -SO₃R⁷, -PR⁷₂ ,-POR⁷R⁷ substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR⁴-, -CO-, -SO- oder -SO₂-bedeutet:
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷:
P ein Aminorest -NR¹R²;
B C₁-C₆-Alkylen, Phenylen oder Kombinationen dieser Brückenglieder, wobei die Phenylenreste ein- oder mehrfach durch C₁-C₁₂-Alkyl, Nitro, Cyano und/oder Halogen substituiert sein können;
A -COOM, -SO₃M oder -PO₃M₂;
D Phenylen, Naphthylen oder Pyridylen, das jeweils ein- oder mehrfach durch C₁-C₁₂-Alkyl, C₁-C₁₂-Alkoxy, Hydroxy, Nitro und/oder Halogen substituiert sein kann;
M Wasserstoff, einweriges oder zweiwertiges Metallkation, insbesondere Erdalkali- oder Alkalimetallkation, Ammoniumsalze von cyclischen Aminen, Guanidiniumsalze oder [NR⁵₄]⁺;
L eine chemische Bindung oder ein direkt oder über Ethenylen oder Ethinylen an das Rylengerüst gebundener Arylen- oder Hetarylenrest der Formeln
-Ar- -Ar-E-Ar-
in denen die (Het)Arylenreste Ar gleich oder verschieden sein können, Heteroatome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl, C₁-C₁₂-Alkoxy, C₁-C₁₂-Alkylthio und/oder -NR⁵R⁶ substituiert sein kann:
E eine chemische Bindung oder eine Gruppierung -O-, -S-, -NR⁴-, -C≡C-, -CR⁴=CR⁴- oder C₁-C₆-Alkylen;
R¹, R² unabhängig voneinander einer der als Substituenten für die Reste R genannten Alkylreste (i), Cycloalkylreste (ii) oder (Het)Arylreste (iii); miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4-bis 8-gliedrige Ringe anneliert sein können, deren Kohlenstoffkette eben-falls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
Z -O- oder -S-;
R³ einer der als Substituenten für die Reste R genannten Alkylreste (i) oder (Het)Arylreste (iii);
R' Wasserstoff;
C₁-C₃₀-Alkyl, dessen Kohlenstoffkette durch eine oder mehrere Grupplerungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach durch die als Substituenten für die Reste R genannten Reste (ii), (iii), (iv) und/oder (v) substituiert sein kann; C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach durch die als Substituenten für die Reste R genannten Reste (i), (ii), (iii), (iv) und/oder (v) substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch die als Substituenten für die Reste R genannten Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
R⁴ Wasserstoff oder C₁-C₁₈-Alkyl, wobei die Reste R⁴ gleich oder verschieden sein können, wenn sie mehrfach auftreten;
R⁵, R⁶ unabhängig voneinander: Wasserstoff; C₁-C₁₈-Alkyl, dessen Kohlenstoffkette durch eine oder mehrere Gruppierungen -O-, -S-, -CO-, -SO- und/oder -SO₂- unterbrochen sein kann und
R⁷ das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁵ gleich oder verschieden sein können, wenn sie mehrfach auftreten; C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁷ gleich oder verschieden sein können, wenn sie mehrfach auftreten;
R⁸ C₁-C₁₈-Alkyl;
R⁹, R¹⁰ C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴- und/oder -CR⁴=CR⁴-unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch; C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
m 0, 1 oder 2;
n 0, 2 oder 4 für m = 0 oder 1; 0, 4 oder 6 für m = 2;
p 0, 2 oder 4 für m = 0, wobei n + p = 2 oder 4 ist oder auch 0 sein kann, wenn die beiden Reste X miteinander unter Ausbildung eines Sechsrings verbunden sind zu einem Rest der Formel (x1) oder (x2) oder beide einen Rest -COOM bedeuten und
einer der beiden Reste Y einen Rest der Formel (y1) oder (y2) und der andere Rest Wasserstoff bedeutet, wobei mindestens einer der beiden Reste R¹ oder R² im Rest (y1) einen der als Substituenten für die Reste R genannten (Het)Arylreste (iii) bedeutet, wenn L für eine chemische Bindung steht; 0, 2 oder 4 für m = 1, wobei n + p = 0, 2 oder 4 ist; 0, 4 oder 6 für m = 2, wobei n + p = 0, 4 oder 6 ist, oder von Mischungen der Rylenderivate als Photosensibilisatoren In Solarzellen.

2. Verwendung nach Anspruch 1, **dadurch gekennzeichnet, daß** die Variablen In Formel I folgende Bedeutung haben:
X miteinander unter Ausbildung eines Sechsrings verbunden zu einem Rest der Formel (x1) oder beide ein Rest -COOM;
Y einer der beiden Reste ein Rest der Formel (y1) oder (y2) und der andere Rest Wasserstoff oder miteinander unter Ausbildung eines Sechsrings verbunden zu einem Rest der Formel (y3);
R Phenoxy oder Thiophenoxy, das jeweils ein- oder mehrfach durch gleiche oder verschiedene Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -C≡C-, -CR⁴=CR⁴- und/oder -CO- und/oder -SO₂- unterbrochen sein kann und das ein- oder mehrfach substituiert sein kann durch: C₁-C₁₂-Alkoxy, Hydroxy, Halogen, Cyano und/oder Aryl, das ein- oder mehrfach durch C₁-C₁₈-Alkyl oder C₁-C₆-Alkoxy substituiert sein kann;
(ii) C₃-C₈-Cycloalkyl, dessen Kohlenstoffgerüst durch eine oder mehrere Gruppierungen -O-, -S-, -NR⁴-, -CR⁴=CR⁴- und/oder -CO- unterbrochen sein kann und das ein- oder mehrfach durch C₁-C₁₈-Alkyl, C₁-C₁₂-Alkoxy und/oder C₁-C₆-Alkylthio substituiert sein kann;
(iii) Aryl oder Hetaryl, an das jeweils weitere 5- bis 7-gliedrige gesättigte oder ungesättigte Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, -C=CR⁴₂, -CR⁴=CR⁴₂, Hydroxy, Halogen, Cyano, -NR⁹R¹⁰, -NR⁵COR⁶, -CONR⁵R⁶, -SO₂NR⁵R⁶, -COOR⁷, -SO₃R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₈-Alkoxy und/oder Cyano substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR⁴-, -CO-, -SO- oder -SO₂-bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷ und/oder -SO₃R⁷;
M Wasserstoff, einweriges oder zweiwertiges Metallkation, insbesondere Erdalkali- oder Alkalimetallkation, Ammoniumsalze von cyclischen Aminen, Guanidiniumsalze oder [NR⁵₄]⁺:
L eine chemische Bindung oder Phenylen;
R¹, R² gleiche oder verschiedene Phenylreste, die jeweils ein- oder mehrfach substituiert sein können durch C₁-C₁₈₋Alkyl, C₁-C₁₂-Alkoxy, C₁-C₆-Alkyl-thio, -NR⁵R⁶ und/oder Phenoxy und/oder Phenylthio, das jeweils ein- oder mehrfach durch C₁-C₁₈-Alkyl, C₁-C₁₂-Alkoxy, C₁-C₆-Alkylthio und/oder -NR⁵R⁶ substituiert sein kann; mit dem Stickstoffatom verbunden zu einem Piperidyl-, Pyrrolidinyl-, Di-benzopyrryl-, Dibenzo-1,4-oxiranyl-, Dibenzo-1,4-thiazinyl-, Dibenzo-1,4-pyrazyl- oder Dibenzopiperidylringsystem, das jeweils ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
Z -O- oder -S-;
R³ C₁-C₃₀-Alkyl, dessen Kohlenstoffkette durch eine oder mehrere Gruppierungen -O-, -S- und/oder -NR⁴- unterbrochen sein kann und das ein- oder mehrfach substituiert sein kann durch: C₁-C₁₂-Alkoxy, Hydroxy und/oder Aryl, das ein- oder mehrfach durch C₁-C₁₈-Alkyl oder C₁-C₆-Alkoxy substituiert sein kann;
Phenyl, das ein- oder mehrfach substituiert sein kann durch: C₁-C₁₈-Alkyl, C₁-C₁₂-Alkoxy, C₁-C₆-Alkylthio, -NR⁵R⁶ und/oder Phenoxy und/oder Phenylthio, das jeweils ein- oder mehrfach durch C₁-C₁₈-Alkyl, C₁-C₁₂-Alkoxy, C₁-C₆-Alkylthio und/oder -NR⁵R⁶ substituiert sein kann;
R' C₆-C₃₀-Alkyl, dessen Kohlenstoffkette durch eine oder mehrere Gruppierungen -O-, -S- und/oder -NR⁴- unterbrochen sein kann und das ein- oder mehrfach substituiert sein kann durch: C₁-C₆-Alkoxy, C₁-C₆-Alkylthio, -NR⁹R¹⁰ und/oder Aryl, das ein- oder mehrfach durch C₁-C₁₈-Alkyl oder C₁-C₆-Alkoxy substituiert sein kann;
Phenyl, Naphthyl, Pyridyl oder Pyrimidyl, das jeweils ein- oder mehrfach substituiert sein kann durch: C₁-C₁₈-Alkyl, C₁-C₆-Alkoxy, Halogen, Cyano, Nitro, -NR⁹R¹⁰, -CONR⁵R⁶, -SO₂NR⁵R⁶ und/oder Phenoxy, Phenylthio, Phenylazo und/oder Naphthylazo, das jeweils durch C₁-C₁₀-Alkyl, C₁-C₆-Alkoxy und/oder Cyano substituiert sein kann;
R⁴ Wasserstoff oder C₁-C₆-Alkyl;
R⁵, R⁶ unabhängig voneinander:
Wasserstoff;
C₁-C₁₈-Alkyl, das ein- oder mehrfach durch C₁-C₆-Alkoxy, Hydroxy, Halogen und/oder Cyano substituiert sein kann;
Aryl oder Hetaryl, das jeweils ein- oder mehrfach durch C₁-C₆-Alkyl und/oder die vorstehenden, als Substituenten für Alkyl genannten Reste substituiert sein kann,
wobei die Reste R⁵ gleich oder verschieden sein können, wenn sie mehrfach auftreten;
R⁷ C₁-C₁₈-Alkyl, das ein- oder mehrfach durch C₁-C₆-Alkoxy, Hydroxy, Halogen und/oder Cyano substituiert sein kann; Aryl oder Hetaryl, das jeweils ein- oder mehrfach durch C₁-C₆-Alkyl und/oder die vorstehenden, als Substituenten für Alkyl genannten Reste substituiert sein kann
R⁹, R¹⁰ C₁-C₃₀-Alkyl, dessen Kohlenstoffkette durch eine oder mehrere Gruppierungen -O-, -S-, -NR⁴-, -N=CR⁴-, -C≡C- und/oder -CR⁴=CR⁴- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶ und/oder (Het)Aryl, das ein- oder mehrfach durch C₁-C₁₈-Alkyl und/oder die vorstehenden, als Substituenten für Alkyl genannten Reste substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem Piperidyl-, Pyrrolidinyl-, Di-benzopyrryl-, Dibenzo-1,4-oxiranyl-, Dibenzo-1,4-thiazinyl-, Dibenzo-1,4-pyrazyl- oder Dibenzopiperidylringsystem, das jeweils ein- oder mehrfach substituiert sein kann durch C₁-C₂₄-Alkyl, das durch C₁-C₁₈-Alkoxy, C₁-C₁₈-Alkylthio und/oder -NR⁵R⁶ substituiert sein kann;
m 0, 1 oder 2;
n 4 für m = 0 oder 1; 4 oder 6 für m = 2;
p 0.

3. Farbstoffsensibilisierte Solarzellen, die mindestens ein Rylenderivat der Formel I gemäß Anspruch 1 oder 2 als Photosensibilisatoren enthalten.

4. Rylenderivate der allgemeinen Formel la 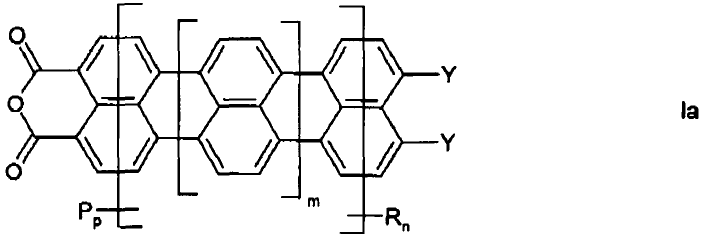 in der der Dicarbonsäureanhydridrest auch stellvertretend für zwei Carboxylreste -COOM stehen kann und die Variablen folgende Bedeutung haben:
Y einer der beiden Reste ein Rest der Formel (y1)
-L-NR¹R² (y1)
oder ein Rest der Formel (y2)
-L-Z-R³ (y2)
und der andere Rest jeweils Wasserstoff;
R gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO-und/oder -SO₂- unterbrochen sein kann und das ein- oder mehrfach substituiert sein kann durch: C₁-C₁₂-Alkoxy, C₁-C₈-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-,-NR⁴-, -N=CR⁴-, -CR⁴=CR⁴-, -CO-, -SO- und/oder -SO₂- unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO-und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁷R⁸, -NR⁵COR⁶, -CONR⁵R⁶, -SO₂NR⁵R⁶, -COOR⁷, -SO₃R⁷, -PR⁷₂, -POR⁷R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, C₁-C₆-Alkylthio, Hydroxy, Mercapto, Halogen, Cyano, Nitro, -NR⁷R⁸, -NR⁵COR⁶, -CONR⁵R⁶, -SO₂NR⁵R⁶. -COOR⁷, -SO₃R⁷, -PR⁷₂ und/oder -POR⁷R⁷ substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR³-, -CO-, -SO- oder -SO₂-bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁷R⁶, -NR⁵COR⁶, -CONR⁵R⁶, -SO₂NR⁵R⁶, -COOR⁷, -SO₃R⁷, -PR⁷₂ und/oder -POR⁷R⁷;
P ein Aminorest -NR¹R²;
M Wasserstoff, einweriges oder zweiwertiges Metallkation, insbesondere Erdalkali- oder Alkalimetallkation, Ammoniumsalze von cyclischen Aminen, Guanidiniumsalze oder [NR⁶₄]⁺;
L eine chemische Bindung oder ein direkt oder über Ethenylen oder Ethinylen an das Rylengerüst gebundener Arylen- oder Hetarylenrest der Formeln
-Ar- -Ar-E-Ar-
in denen die (Het)Arylenreste Ar gleich oder verschieden sein können, Heteroatome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl, C₁-C₁₂-Alkoxy, C₁-C₁₂-Alkylthio und/oder -NR⁵R⁶ substituiert sein kann;
E eine chemische Bindung oder eine Gruppierung -O-, -S-, -NR⁴-, -C≡C-, -CR⁴=CR⁴- oder C₁-C₆-Alkylen;
R¹, R² unabhängig voneinander einer der als Substituenten für die Reste R genannten Alkylreste (i), Cycloalkylreste (ii) oder (Het)Arylreste (iii); miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4-bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
Z -O- oder -S-;
R³ einer der als Substituenten für die Reste R genannten Alkylreste (i) oder (Het)Arylreste (iii);
R⁴ Wasserstoff oder C₁-C₁₈-Alkyl, wobei die Reste R⁴ gleich oder verschieden sein können, wenn sie mehrfach auftreten;
R⁵, R⁶ unabhängig voneinander.
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, annellert sein können, wobei das gesamte Ringsystem ein- oder mehrfach durch C₁-C₁₂-Alkyl und/oder die vorstehenden, als Substituenten für Alkyl genannten Reste substituiert sein kann,
wobei die Reste R⁵ gleich oder verschieden sein können, wenn sie mehrfach auftreten;
R⁷ C₁-C₁₆-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann,
wobei die Reste R⁷ gleich oder verschieden sein können, wenn sie mehrfach auftreten;
R⁶ C₁-C₁₈-Alkyl;
R⁹, R¹⁰ C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, -S-,-NR⁴-, -N=CR⁴- und/oder -CR⁴=CR⁴-unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
m 0, 1 oder 2;
n 0, 2 oder 4 für m = 0;
0, 2 oder 4 für m = 1;
0, 4 oder 6 für m = 2;
p 0, 2 oder 4 für m = 0, wobei n + p = 0, 2 oder 4 ist, wobei für den Fall, daß n + p = 0 ist und einer der beiden Reste Y einen Rest der Formel (y1) und der andere Rest Wasserstoff bedeutet, mindestens einer der beiden Reste R¹ oder R² einen der als Substituenten für die Reste R genannten (Het)Arylreste (iii) bedeutet, wenn L für eine chemische Bindung steht;
0, 2 oder 4 für m = 1, wobei n + p = 0, 2 oder 4 ist;
0, 4 oder 6 für m = 2, wobei n + p = 0, 4 oder 6 ist

5. Rylenderivate der allgemeinen Formel Ib 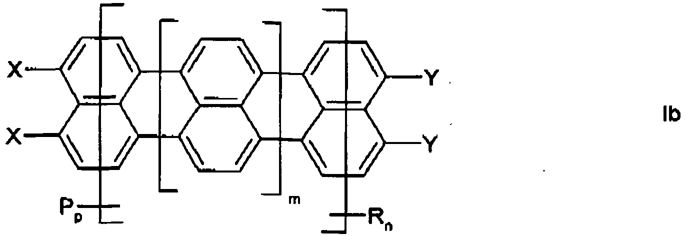 in der die Variablen folgende Bedeutung haben:
X miteinander unter Ausbildung eines Sechsrings verbunden zu einem Rest der Formel (x2) oder (x3) 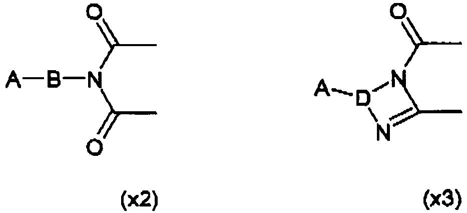
Y einer der beiden Reste ein Rest der Formel (y1)
-L-NR¹R² (y1)
oder ein Rest der Formel (y2)
-L-Z-R³ (y2)
oder ein Rest R und der andere Rest jeweils Wasserstoff; miteinander unter Ausbildung eines Sechsrings verbunden zu einem Rest der Formel (y3) oder (y4) 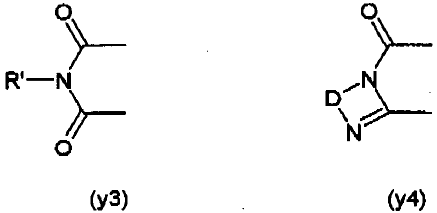 oder beide Wasserstoff;
R gleiche oder verschiedene Reste: Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CO-, -SO- und/oder -SO₂- unterbrochen sein kann, annellert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO-und/oder -SO₂- unterbrochen sein kann und das ein- oder mehrfach substituiert sein kann durch: C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, wobei die (Het)Aryl- und Cyclo-alkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO-und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, C₁-C₈-Alkylthio, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷, substituiert sein können;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR⁴-, -CO-, -SO- oder -SO₂-bedeutet;
(v) C₁-C₁₂-Alkoxy, C₁-C₆-Alkylthio, -C≡CR⁴, -CR⁴=CR⁴₂, Hydroxy, Mercapto, Halogen, Cyano, Nitro, -NR⁹R¹⁰, -NR⁵COR⁶, -CONR⁵R⁶, -SO₂NR⁵R⁶, -COOR⁷, -SO₃R⁷, -PR⁷₂ und/oder -POR⁷R⁷;
P ein Aminorest -NR¹R²;
B C₁-C₈-Alkylen, Phenylen oder Kombinationen dieser Brückenglieder, wobei die Phenylenreste ein- oder mehrfach durch C₁-C₁₂-Alkyl, Nitro, Cyano und/oder Halogen substituiert sein können;
A -COOM, -SO₃M oder -PO₃M₂;
D Phenylen, Naphthylen oder Pyridylen, das jeweils ein- oder mehrfach durch C₁-C₁₂-Alkyl, C₁-C₆-Alkoxy, Hydroxy, Nitro und/oder Halogen substituiert sein kann;
M Wasserstoff. einweriges oder zweiwertiges Metallkation, insbesondere Erdalkali- oder Alkalimetallkation, Ammoniumsalze von cyclischen Aminen, Guanidiniumsalze oder [NR⁵₄]⁺;
L eine chemische Bindung oder ein direkt oder über Ethenylen oder Ethinylen an das Rylengerüst gebundener Arylen- oder Hetarylenrest der Formeln
-Ar- -Ar-E-Ar-
in denen die (Het)Arylenreste Ar gleich oder verschieden sein können, Heteroatome als Ringatome enthalten können und/oder annelierte gesättigte oder ungesättigte 5- bis 7-gliedrige Ringe, die ebenfalls Heteroatome enthalten können, aufweisen können, wobei das gesamte Ringsystem ein- oder mehrfach durch Phenyl, C₁-C₁₂-Alkyl. C₁-C₁₂-Alkoxy, C₁-C₁₂-Alkylthio und/oder -NR⁵R⁶ substituiert sein kann;
E eine chemische Bindung oder eine Grupplerung -O-, -S-, -NR⁴-, -C≡C-, -CR⁴=CR⁴- oder C₁-C₆-Alkylen;
R¹, R² unabhängig voneinander einer der als Substituenten für die Reste R genannten Alkylreste (i), Cycloalkylreste (ii) oder (Het)Arylreste (iii): miteinander verbunden zu einem das Stickstoffatom enthaltenden, gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4-bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
Z -O- oder -S-;
R³ einer der als Substituenten für die Reste R genannten Alkylreste (i) oder (Het)Arylreste (iii);
R' Wasserstoff;
C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach durch die als Substituenten für die Reste R genannten Reste (ii), (iii), (iv) und/oder (v) substituiert sein kann;
C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach durch die als Substituenten für die Reste R genannten Reste (i), (ii), (iii), (iv) und/oder (v) substituiert sein kann; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch die als Substituenten für die Reste R genannten Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
R⁴ Wasserstoff oder C₁-C₁₈-Alkyl, wobei die Reste R⁴ gleich oder verschieden sein können, wenn sie mehrfach auftreten;
R⁵, R⁶ unabhängig voneinander:
Wasserstoff;
C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁵ gleich oder verschieden sein können, wenn sie mehrfach auftreten;
R⁷ C₁-C₁₈-Alkyl, dessen Kohlenstoffkette durch eine oder mehrere Gruppierungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR⁸ substituiert sein kann; Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, wobei die Reste R⁷ gleich oder verschieden sein können, wenn sie mehrfach auftreten;
R⁸ C₁-C₁₈-Alkyl;
R⁹, R¹⁰ C₁-C₃₀-Alkyl, dessen Kohlenstoffkette durch eine oder mehrere Gruppierungen -O-, -S-, -NR⁴-, -N=CR⁴-, -C≡C-, -CR⁴=CR⁴-, -CO-, -SO- und/oder -SO₂- unterbrochen sein kann und das ein- oder mehrfach substituiert sein kann durch: C₁-C₁₂-Alkoxy, C₁-C₈-Alkylthio, -C≡CR⁴, -CR⁴=CR⁴₂, Hydroxy, -NR⁵R⁶, -NR⁵COR⁶, (Het)Aryl und/oder gesättigtes oder ungesättigtes C₄-C₇-Cycloalkyl, dessen Kohlenstoffgerüst durch eine oder mehrere Gruppierungen -O-, -S-, -NR⁴-, -N=CR⁴- und/oder -CR⁴=CR⁴-unterbrochen sein kann, wobei die (Het)Aryl- und Cycloalkylreste jeweils ein- oder mehrfach durch C₁-C₁₈-Alkyl und/oder die vorstehenden, als Substituenten für Alkyl genannten Reste substituiert sein können; Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR⁴-, -N=CR⁴-, -CR⁴=CR⁴-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR⁴, -CR⁴=CR⁴₂, Hydroxy, -NR⁵R⁶, -NR⁵COR⁶, (Het)Aryl, (Het)Aryloxy und/oder (Het)Arylthio, wobei die (Het)Arylreste jeweils ein- oder mehrfach durch C₁-C₁₈-Alkyl, C₁-C₁₂-Alkoxy, Hydroxy, -NR⁵R⁶ und/oder -NR⁵COR⁶ substituiert sein können; mit dem Stickstoffatom verbunden zu einem gesättigten oder ungesättigten, 5- bis 7-gliedrigen Ring, dessen Kohlenstoffkette durch eine oder mehrere Gruppierungen -O-, -S- und/oder -NR⁴- unterbrochen sein kann, an den ein oder zwei ungesättigte oder gesättigte 4- bis 8-gliedrige Ringe anneliert sein können, deren Kohlenstoffkette ebenfalls durch diese Gruppierungen und/oder -N= unterbrochen sein kann, wobei das gesamte Ringsystem ein- oder mehrfach substituiert sein kann durch: C₁-C₂₄-Alkyl, das durch C₁-C₁₈-Alkoxy, C₁-C₁₈-Alkylthio und/oder -NR⁵R⁶ substituiert sein kann, (Het)Aryl, das ein- oder mehrfach durch C₁-C₁₈-Alkyl und/oder die vorstehenden, als Substituenten für Alkyl genannten Reste substituiert sein kann, C₁-C₁₈-Alkoxy, C₁-C₁₈-Alkylthio und/oder -NR⁵R⁶;
m 0, 1 oder 2;
n 0, 2 oder 4 für m = 0;
0, 2 oder 4 für m = 1;
0, 4 oder 6 für m = 2;
p 0, 2 oder 4 für m = 0, wobei n + p = 2 oder 4 ist oder auch 0 sein kann, wenn einer der beiden Reste Y einen Rest der Formel (y1) oder (y2) und der andere Rest Wasserstoff bedeutet, wobei mindestens einer der beiden Reste Reste R¹ oder R² im Rest (y1) einen der als Substituenten für die Reste R genannten (Het)Arylreste (iii) bedeutet, wenn L für eine chemische Bindung steht;
0, 2 oder 4 für m = 1, wobei n + p = 0, 2 oder 4 ist;
0, 4 oder 6 für m = 2, wobei n + p = 0, 4 oder 6 ist

## Claims

1. The use of rylene derivatives of the general formula I 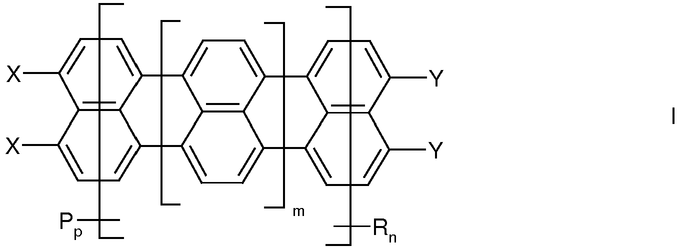 in which the variables are each defined as follows:
X are joined to one another with formation of a six-membered ring to give a radical of the formula (x1), (x2) or (x3) 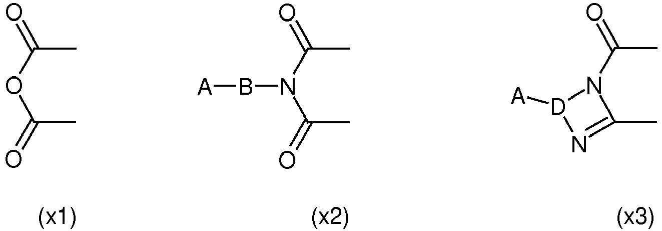
both are a -COOM radical;
Y one of the two radicals is a radical of the formula (y1)
-L-NR¹R² (y1)
or a radical of the formula (y2)
-L-Z-R³ (y2)
and the other radical in each case is hydrogen; are joined together with formation of a six-membered ring to give a radical of the formula (y3) or (y4) 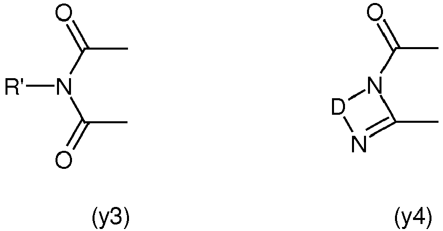
or both are hydrogen;
R are identical or different radicals: aryloxy, arylthio, hetaryloxy or hetarylthio, to each of which may be fused further saturated or unsaturated 5- to 7-membered rings whose carbon skeleton may be interrupted by one or more -0-, -S-, -NR⁴-, -N=CR⁴-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -0-, -S-, -NR⁴-,-N=CR⁴-, -C≡C-, -CR⁴=CR⁴-, -CO-, -SO- and/or -SO₂-moieties and which may be mono- or polysubstituted by: C₁-C₁₂-alkoxy, C₁-C₆-alkylthio, -C≡CR⁴, -CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (het)aryl and/or saturated or unsaturated C₄-C₇-cycloalkyl whose carbon skeleton may be interrupted by one or more -0-, -S-, -NR⁴-, -N=CR⁴-, -CR⁴=CR⁴-, -CO-, -SO- and/or -SO₂- moieties, where the (het)aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -0-, -S-, -NR⁴-,-N=CR⁴-, -CR⁴=CR⁴-, -CO-, -SO- and/or -SO₂-moieties and to which may be fused further saturated or unsaturated 5- to 7-membered rings whose carbon skeleton may be interrupted by one or more -0-, -S-, -NR⁴-, -N=CR⁴-, -CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkylthio,-C≡CR⁴, -CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂ and/or -POR⁷R⁷;
(iii) aryl or hetaryl to which may be fused further saturated or unsaturated 5- to 7-membered rings whose carbon skeleton may be interrupted by one or more -0-, -S-, -NR⁴-,-N=CR⁴-, -CR⁴=CR⁴-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR⁴, -CR⁴=CR⁴₂, hydroxyl, mercapto, halogen, cyano, nitro,-NR⁹R¹⁰, -NR⁵COR⁶, -CONR⁵R⁶, -SO₂NR⁵R⁶, -COOR⁷,-SO₃R⁷, -PR⁷₂, -POR⁷R⁷, (het) aryl, (het) aryloxy and/or (het)arylthio, where the (het)aryl radicals may each be mono- or polysubstituted by C₁-C₁₈-alkyl, C₁-C₁₂-alkoxy, C₁-C₆-alkylthio, hydroxyl, mercapto, halogen, cyano, nitro,-NR⁹R¹⁰, -NR⁵COR⁶, -CONR⁵R⁶, -SO₂NR⁵R⁶, -COOR⁷,-SO₃R⁷, -PR⁷₂, -POR⁷R⁷;
(iv) a -U-aryl radical which may be mono- or polysubstituted by the above radicals specified as substituents for the aryl radicals (iii), where U is a -0-, -S-, -NR⁴-, -CO-, -SO- or-SO₂- moiety;
(v) C₁-C₁₂-alkoxy, C₁-C₆-alkylthio, -C≡CR⁴,-CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂ and/or -POR⁷R⁷;
P is an amino radical -NR¹R²;
B is C₁-C₆-alkylene, phenylene or combinations of these bridging members, where the phenylene radicals may be mono- or polysubstituted by C₁-C₁₂-alkyl, nitro, cyano and/or halogen;
A is -COOM, -SO₃M or -PO₃M₂;
D is phenylene, naphthylene or pyridylene, each of which may be mono- or polysubstituted by C₁-C₁₂-alkyl, C₁-C₁₂-alkoxy, hydroxyl, nitro and/or halogen;
M is hydrogen, monovalent or divalent metal cation, especially alkaline earth metal or alkali metal cation, ammonium salts of cyclic amines, guanidinium salts or [NR⁵]₄⁺;
L is a chemical bond or an arylene or hetarylene radical, bonded to the rylene skeleton directly or via ethenylene or ethynylene, of the formulae
-Ar- -Ar-E-Ar-
in which the (het)arylene radicals Ar may be the same or different, may comprise heteroatoms as ring atoms and/or may have fused saturated or unsaturated 5- to 7-membered rings which may likewise comprise heteroatoms, where the entire ring system may be mono- or polysubstituted by phenyl, C₁-C₁₂-alkyl, C₁-C₁₂-alkoxy, C₁-C₁₂-alkylthio and/or-NR⁵R⁶;
E is a chemical bond or an -O-, -S-, -NR⁴-, -C≡C-, -CR⁴=CR⁴- or C₁-C₆-alkylene moiety;
R¹, R² are each independently one of the alkyl radicals (i), cycloalkyl radicals (ii) or (het)aryl radicals (iii) specified as substituents for the R radicals;
joined together to form a saturated or unsaturated, 5- to 7-membered ring which comprises the nitrogen atom and whose carbon chain may be interrupted by one or more -O-,-S- and/or -NR⁴- moieties, to which may be fused one or two unsaturated or saturated 4- to 8-membered rings whose carbon chain may likewise be interrupted by these moieties and/or -N=, where the entire ring system may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the aforementioned radicals as substituents for alkyl, C₁-C₁₈-alkoxy, C₁-C₁₈-alkylthio and/or-NR⁵R⁶;
Z is -0- or -S-;
R³ is one of the alkyl radicals (i) or (het)aryl radicals (iii) specified as substituents for the R radicals;
R' is hydrogen;
C₁-C₃₀-alkyl whose carbon chain may be interrupted by one or more -0-, -S-, -NR⁴-,-N=CR⁴-, -C≡C-, -CR⁴=CR⁴-, -CO-, -SO- and/or-SO₂- moieties and which may be mono- or polysubstituted by the (ii), (iii), (iv) and/or (v) radicals specified as substituents for the R radicals;
C₃-C₈-cycloalkyl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by the (i), (ii), (iii), (iv) and/or (v) radicals specified as substituents for the R radicals;
aryl or hetaryl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or
-SO₂- moieties, where the entire ring system may be mono- or polysubstituted by the (i), (ii), (iii), (iv), (v) radicals specified as substituents for the R radicals, and/or aryl-and/or hetarylazo, each of which may be substituted by C₁-C₁₀-alkyl, C₁-C₆-alkoxy and/or cyano;
R⁴ is hydrogen or C₁-C₁₈-alkyl, where the R⁴ radicals may be the same or different when they occur more than once;
R⁵, R⁶ are each independently: hydrogen;
C₁-C₁₈-alkyl whose carbon chain may be interrupted by one or more -0-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸; aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl,
where the R⁵ radicals may be the same or different when they occur more than once;
R⁷ is C₁-C₁₈-alkyl whose carbon chain may be interrupted by one or more -0-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸;
aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl,
where the R⁷ radicals may be the same or different when they occur more than once;
R⁸ is C₁-C₁₈-alkyl;
R⁹, R¹⁰ are each C₁-C₃₀-alkyl whose carbon chain may be interrupted by one or more -0-, -S-, -NR⁴-, -N=CR⁴-, -C≡C-, -CR⁴=CR⁴-, -CO-, -SO- and/or -SO₂- moieties and which may be mono- or polysubstituted by: C₁-C₁₂-alkoxy, C₁-C₆-alkylthio, -C≡CR⁴, -CR⁴=CR⁴₂, hydroxyl, -NR⁵R⁶, -NR⁵COR⁶, (het)aryl and/or saturated or unsaturated C₄-C₇-cycloalkyl whose carbon skeleton may be interrupted by one or more -O-, -S-, -NR⁴-, -N=CR⁴- and/or -CR⁴=CR⁴ moieties, where the (het)aryl and cycloalkyl radicals may each be mono- or polysubstituted by C₁-C₁₈-alkyl and/or the above radicals specified as substituents for alkyl;
aryl or hetaryl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR⁴, -CR⁴=CR⁴₂, hydroxyl,-NR⁵R⁶, -NR⁵COR⁶, (het)aryl, (het)aryloxy and/or (het)arylthio, where the (het)aryl radicals may in each case be mono- or polysubstituted by C₁-C₁₈-alkyl, C₁-C₁₂-alkoxy, hydroxyl, -NR⁵R⁶ and/or -NR⁵COR⁶;
joined to the nitrogen atom to form a saturated or unsaturated, 5- to 7-membered ring whose carbon chain may be interrupted by one or more -0-, -S- and/or -NR⁴- moieties, to which may be fused one or two unsaturated or saturated 4- to 8-membered rings whose carbon chain may likewise be interrupted by these moieties and/or-N=, where the entire ring system may be mono- or polysubstituted by C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the above radicals specified as substituents for alkyl, C₁-C₁₈-alkoxy, C₁-C₁₈-alkylthio and/or-NR⁵R⁶;
m is 0, 1 or 2;
n is 0, 2 or 4 when m = 0 or 1;
is 0, 4 or 6 when m = 2;
p is 0, 2 or 4 when m = 0, where n + p = 2 or 4 or may also be 0 when the two X radicals are joined together with formation of a six-membered ring to give a radical of the formula (x1) or (x2) or both are a -COOM radical and one of the two Y radicals is a radical of the formula (y1) or (y2) and the other radical is hydrogen, where at least one of the two R¹ or R² radicals in the (y1) radical is one of the (het)aryl radicals (iii) specified as substituents for the R radicals when L is a chemical bond;
is 0, 2 or 4 when m = 1, where n + p = 0, 2 or 4;
is 0, 4 or 6 when m = 2, where n + p = 0, 4 or 6,
or of mixtures of the rylene derivatives as photosensitizers in solar cells.

2. The use according to claim 1, wherein the variables in formula I are each defined as follows:
X are joined together with formation of a six-membered ring to give a radical of the formula (x1) or both are a -COOM radical;
Y one of the two radicals is a radical of the formula (y1) or (y2) and the other radical is hydrogen, or are joined together with formation of a six-membered ring to give a radical of the formula (y3);
R is phenoxy or thiophenoxy, each of which may be mono- or polysubstituted by identical or different (i), (ii), (iii), (iv) and/or (v) radicals:
(i) C₁-C₃₀-alkyl whose carbon chain may be interrupted by one or more -0-, -S-, -NR⁴-,-C≡C-, -CR⁴=CR⁴- and/or -CO- and/or -SO₂-moieties and which may be mono- or polysubstituted by: C₁-C₁₂-alkoxy, hydroxyl, halogen, cyano, and/or aryl which may be mono-or polysubstituted by C₁-C₁₈-alkyl or C₁-C₆-alkoxy;
(ii) C₃-C₈-cycloalkyl whose carbon skeleton may be interrupted by one or more -0-, -S-,-NR⁴-, -CR⁴=CR⁴- and/or -CO- moieties and which may be mono- or polysubstituted by: C₁-C₁₈-alkyl, C₁-C₁₂-alkoxy and/or C₁-C₆-alkyl thio;
(iii) aryl or hetaryl, to which may be fused further saturated or unsaturated 5- to 7-membered rings whose carbon skeleton may be interrupted by one or more -0-, -S-, -NR⁴-,-N=CR⁴-, -CR⁴=CR⁴-, -CO-, -SO- and/or -SO₂-moieties, where the entire ring system may be mono- or polysubstituted by: C₁-C₁₈-alkyl, C₁-C₁₂-alkoxy, -C=CR⁴₂, -CR⁴=CR⁴₂, hydroxyl, halogen, cyano, -NR⁹R¹⁰, -NR⁵COR⁶, -CONR⁵R⁶,-SO₂NR⁵R⁶, -COOR⁷, -SO₃R⁷, (het)aryl, (het)aryloxy and/or (het)arylthio, where the (het)aryl radicals may each be mono- or polysubstituted by C₁-C₁₈-alkyl, C₁-C₁₈-alkoxy and/or cyano;
(iv) a -U-aryl radical which may be mono- or polysubstituted by the above radicals specified as substituents for the aryl radicals (iii), where U is an -0-, -S-, -NR⁴-, -CO-, -SO- or -SO₂- moiety;
(v) C₁-C₁₂-alkoxy, C₁-C₆-alkylthio, -C=CR⁴,-CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷ and/or -SO₃R⁷;
M is hydrogen, monovalent or divalent metal cation, especially alkaline earth metal or alkali metal cation, ammonium salts of cyclic amines, guanidinium salts or [NR⁵]₄⁺;
L is a chemical bond or phenylene;
R¹, R² are identical or different phenyl radicals, each of which may be mono- or polysubstituted by C₁-C₁-alkyl, C₁-C₁₂-alkoxy, C₁-C₆-alkylthio, -NR⁵R⁶, and/or phenoxy and/or phenylthio, each of which may be mono- or polysubstituted by C₁-C₁₈-alkyl, C₁-C₁₂-alkoxy, C₁-C₆-alkylthio and/or -NR⁵R⁶;
joined to the nitrogen atom to give a piperidyl, pyrrolidinyl, dibenzopyrryl, dibenzo-1,4-oxiranyl, dibenzo-1,4-thiazinyl, dibenzo-1,4-pyrazyl or dibenzopiperidyl ring system, each of which may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the above radicals mentioned as substituents for alkyl, C₁-C₁₈-alkoxy, C₁-C₁₈-alkylthio and/or-NR⁵R⁶;
Z is -0- or -S-;
R³ is C₁-C₃₀-alkyl whose carbon chain may be interrupted by one or more -0-, -S- and/or-NR⁴- moieties and which may be mono- or polysubstituted by: C₁-C₁₂-alkoxy, hydroxyl and/or aryl which may be mono- or polysubstituted by C₁-C₁₈-alkyl or C₁-C₆-alkoxy; phenyl which may be mono- or polysubstituted by: C₁-C₁₈-alkyl, C₁-C₁₂-alkoxy, C₁-C₆-alkylthio, -NR⁵R⁶, and/or phenoxy and/or phenylthio, each of which may be mono- or polysubstituted by C₁-C₁₈-alkyl, C₁-C₁₂-alkoxy, C₁-C₆-alkylthio and/or -NR⁵R⁶;
R' is C₆-C₃₀-alkyl whose carbon chain may be interrupted by one or more -0-, -S- and/or-NR⁴- moieties and which may be mono- or polysubstituted by: C₁-C₆-alkoxy, C₁-C₆-alkylthio, -NR⁹R¹⁰ and/or aryl which may be mono- or polysubstituted by C₁-C₁₈-alkyl or C₁-C₆-alkoxy;
phenyl, naphthyl, pyridyl or pyrimidyl, each of which may be mono- or polysubstituted by: C₁-C₁₈-alkyl, C₁-C₆-alkoxy, halogen, cyano, nitro, -NR⁹R¹⁰, -CONR⁵R⁶, -SO₂NR⁵R⁶ and/or phenoxy, phenylthio, phenylazo and/or naphthylazo, each of which may be substituted by C₁-C₁₀-alkyl, C₁-C₆-alkoxy and/or cyano;
R⁴ is hydrogen or C₁-C₆-alkyl;
R⁵, R⁶ are each independently: hydrogen;
C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl,
where the R⁵ radicals may be the same or different when they occur repeatedly;
R⁷ is C₁-C₁₈-alkyl which may be mono- or polysubstituted by C₁-C₆-alkoxy, hydroxyl, halogen and/or cyano;
aryl or hetaryl, each of which may be mono- or polysubstituted by C₁-C₆-alkyl and/or the above radicals specified as substituents for alkyl;
R⁹, R¹⁰ are each C₁-C₃₀-alkyl whose carbon chain may be interrupted by one or more -0-, -S-, -NR⁴-, -N=CR⁴-, -C=C- and/or -CR⁴=CR⁴- moieties and which may be mono- or polysubstituted by: C₁-C₁₂-alkoxy, C₁-C₆-alkylthio, -C=CR⁴, -CR⁴=CR⁴₂, hydroxyl, -NR⁵R⁶, -NR⁵COR⁶ and/or (het)aryl which may be mono- or polysubstituted by C₁-C₁₈-alkyl and/or the above radicals specified as substituents for alkyl;
aryl or hetaryl, to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkyl thio, -C=CR⁴, -CR⁴=CR⁴₂, hydroxyl,-NR⁵R⁶, -NR⁵COR⁶, (het)aryl, (het)aryloxy and/or (het)arylthio, where the (het)aryl radicals may each be mono- or polysubstituted by C₁-C₁₈-alkyl, C₁-C₁₂-alkoxy, hydroxyl, -NR⁵R⁶ and/or-NR⁵COR⁶;
joined to the nitrogen atom to give a piperidyl, pyrrolidinyl, dibenzopyrryl, dibenzo-1,4-oxiranyl, dibenzo-1,4-thiazinyl, dibenzo-1,4-pyrazyl or dibenzopiperidyl ring system, each of which may be mono- or polysubstituted by C₁-C₂₄-alkyl which may be substituted by C₁-C₁₈-alkoxy, C₁-C₁₈-alkylthio and/or -NR⁵R⁶;
m is 0, 1 or 2;
n is 4 when m = 0 or 1;
is 4 or 6 when m = 2;
p is 0.

3. A dye-sensitized solar cell which comprises at least one rylene derivative of the formula I according to claim 1 or 2 as photosensitizers.

4. A rylene derivative of the general formula Ia 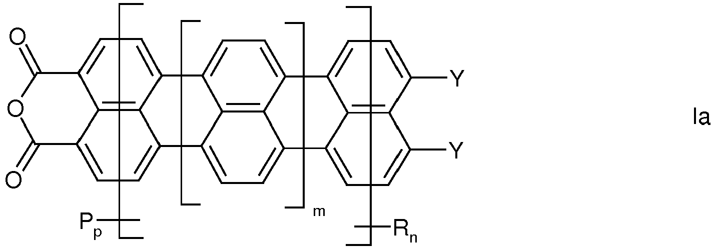 in which the dicarboxylic anhydride radical may also represent two carboxyl radicals -COOM and the variables are each defined as follows:
Y one of the two radicals is a radical of the formula (y1)
-L-NR¹R² (y1)
or a radical of the formula (y2)
-L-Z-R³ (y2)
and the other radical in each case is hydrogen;
R are identical or different radicals: aryloxy, arylthio, hetaryloxy or hetarylthio, to each of which may be fused further saturated or unsaturated 5- to 7-membered rings whose carbon skeleton may be interrupted by one or more -0-, -S-, -NR⁴-, -N=CR⁴-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -O-, -S-, -NR⁴-,-N=CR⁴-, -C=C-, -CR⁴=CR⁴-, -CO-, -SO- and/or -SO₂-moieties and which may be mono- or polysubstituted by: C₁-C₁₂-alkoxy, C₁-C₆-alkylthio, -C=CR⁴, -CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (het)aryl and/or saturated or unsaturated C₄-C₇-cycloalkyl whose carbon skeleton may be interrupted by one or more -0-, -S-, -NR⁴-, -N=CR⁴-, -CR⁴=CR⁴-, -CO-, -SO- and/or -SO₂- moieties, where the (het)aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -0-, -S-, -NR⁴-,-N=CR⁴-, -CR⁴=CR⁴-, -CO-, -SO- and/or -SO₂-moieties and to which may be fused further saturated or unsaturated 5- to 7-membered rings whose carbon skeleton may be interrupted by one or more -0-, -S-, -NR⁴-, -N=CR⁴-, -CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkyl thio,-C=CR⁴, -CR⁴=CR⁴₂, hydroxyl, mercapto, halogen, cyano, nitro, -NR⁹R¹⁰, -NR⁵COR⁶, -CONR⁵R⁶,-SO₂NR⁵R⁶, -COOR⁷, -SO₃R⁷, -PR⁷₂ and/or -POR⁷R⁷;
(iii) aryl or hetaryl to which may be fused further saturated or unsaturated 5- to 7-membered rings whose carbon skeleton may be interrupted by one or more -0-, -S-, -NR⁴-,-N=CR⁴-, -CR⁴=CR⁴-, -CO-, -SO- and/or -SO₂-moieties, where the entire ring system may be mono- or polysubstituted by: C₁-C₁₈-alkyl, C₁-C₁₂-alkoxy, C₁-C₆-alkyl thio, -C=CR⁴, -CR⁴=CR⁴₂, hydroxyl, mercapto, halogen, cyano, nitro,-NR⁷R⁸, -NR⁵COR⁶, -CONR⁵R⁶, -SO₂NR⁵R⁶, -COOR⁷,-SO₃R⁷, -PR⁷₂, -POR⁷R⁷, (het)aryl, (het)aryloxy and/or (het)arylthio, where the (het)aryl radicals may each be mono- or polysubstituted by C₁-C₁₈-alkyl, C₁-C₁₂-alkoxy, C₁-C₆-alkyl thio, hydroxyl, mercapto, halogen, cyano, nitro,-NR⁷R⁸, -NR⁵COR⁶, -CONR⁵R⁶, -SO₂NR⁵R⁶, -COOR⁷,-SO₃R⁷, -PR⁷₂ and/or -POR⁷R⁷ ;
(iv) a -U-aryl radical which may be mono- or polysubstituted by the above radicals specified as substituents for the aryl radicals (iii), where U is a -0-, -S-, -NR³-, -CO-, -SO- or-SO₂- moiety;
(v) C₁-C₁₂-alkoxy, C₁-C₆-alkylthio, -C=CR⁴,-CR⁴=CR⁴₂, hydroxyl, mercapto, halogen, cyano, nitro, -NR⁷R⁸, -NR⁵COR⁶, -CONR⁵R⁶, -SO₂NR⁵R⁶, -COOR⁷, -SO₃R⁷, -PR⁷₂ and/or -POR⁷R⁷;
P is an amino radical -NR¹R²;
M is hydrogen, monovalent or divalent metal cation, especially alkaline earth metal or alkali metal cation, ammonium salts of cyclic amines, guanidinium salts or [NR⁵]₄⁺;
L is a chemical bond or an arylene or hetarylene radical, bonded to the rylene skeleton directly or via ethenylene or ethynylene, of the formulae
-Ar- -Ar-E-Ar-
in which the (het)arylene radicals Ar may be the same or different, may comprise heteroatoms as ring atoms and/or may have fused saturated or unsaturated 5- to 7-membered rings which may likewise comprise heteroatoms, where the entire ring system may be mono- or polysubstituted by phenyl, C₁-C₁₂-alkyl, C₁-C₁₂-alkoxy, C₁-C₁₂-alkylthio and/or-NR⁵R⁶;
E is a chemical bond or an -0-, -S-, -NR⁴-, -C=C-, -CR⁴=CR⁴- or C₁-C₆-alkylene moiety;
R¹, R² are each independently one of the alkyl radicals (i), cycloalkyl radicals (ii) or (het)aryl radicals (iii) specified as substituents for the R radicals;
joined together to form a saturated or unsaturated, 5- to 7-membered ring which comprises the nitrogen atom and whose carbon chain may be interrupted by one or more -O-,-S- and/or -NR⁴- moieties, to which may be fused one or two unsaturated or saturated 4- to 8-membered rings whose carbon chain may likewise be interrupted by these moieties and/or -N=, where the entire ring system may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the aforementioned radicals as substituents for alkyl, C₁-C₁₈-alkoxy, C₁-C₁₈-alkylthio and/or-NR⁵R⁶;
Z is -0- or -S-;
R³ is one of the alkyl radicals (i) or (het)aryl radicals (iii) specified as substituents for the R radicals;
R⁴ is hydrogen or C₁-C₁₈-alkyl, where the R⁴ radicals may be the same or different when they occur more than once;
R⁵, R⁶ are each independently: hydrogen;
C₁-C₁₈-alkyl whose carbon chain may be interrupted by one or more -0-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸; aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl; where the R⁵ radicals may be the same or different when they occur more than once;
R⁷ is C₁-C₁₈-alkyl whose carbon chain may be interrupted by one or more -0-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸;
aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl,
where the R⁷ radicals may be the same or different when they occur more than once;
R⁸ is C₁-C₁₈-alkyl;
R⁹, R¹⁰ are each C₁-C₃₀-alkyl whose carbon chain may be interrupted by one or more -0-, -S-, -NR⁴-, -N=CR⁴-, -C≡C-, -CR⁴=CR⁴-, -CO-, -SO- and/or -SO₂- moieties and which may be mono- or polysubstituted by: C₁-C₁₂-alkoxy, C₁-C₆-alkylthio, -C≡CR⁴, -CR⁴=CR⁴₂, hydroxyl, -NR⁵R⁶, -NR⁵COR⁶, (het)aryl and/or saturated or unsaturated C₄-C₇-cycloalkyl whose carbon skeleton may be interrupted by one or more -O-, -S-, -NR⁴-, -N=CR⁴- and/or -CR⁴=CR⁴ moieties, where the (het)aryl and cycloalkyl radicals may each be mono- or polysubstituted by C₁-C₁₈-alkyl and/or the above radicals specified as substituents for alkyl;
aryl or hetaryl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkyl thio, -C≡CR⁴, -CR⁴=CR⁴₂, hydroxyl,-NR⁵R⁶, -NR⁵COR⁶, (het)aryl, (het)aryloxy and/or (het)arylthio, where the (het)aryl radicals may in each case be mono- or polysubstituted by C₁-C₁₈-alkyl, C₁-C₁₂-alkoxy, hydroxyl, -NR⁵R⁶ and/or -NR⁵COR⁶
joined to the nitrogen atom to form a saturated or unsaturated, 5- to 7-membered ring whose carbon chain may be interrupted by one or more -0-, -S- and/or -NR⁴- moieties, to which may be fused one or two unsaturated or saturated 4- to 8-membered rings whose carbon chain may likewise be interrupted by these moieties and/or-N=, where the entire ring system may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the above radicals specified as substituents for alkyl, C₁-C₁₈-alkoxy, C₁-C₁₈-alkylthio and/or-NR⁵R⁶;
m is 0, 1 or 2;
n is 0, 2 or 4 when m = 0;
is 0, 2 or 4 when m = 1;
is 0, 4 or 6 when m = 2;
p is 0, 2 or 4 when m = 0, where n + p = 0, 2 or 4, where, in the case that n + p = 0 and one of the two Y radicals is a radical of the formula (y1) and the other radical is hydrogen, at least one of the two R¹ or R² radicals is one of the (het)aryl radicals (iii) specified as substituents for the R radicals when L is a chemical bond; is 0, 2 or 4 when m = 1, where n + p = 0, 2 or 4;
is 0, 4 or 6 when m = 2, where n + p = 0, 4 or 6.

5. A rylene derivative of the general formula Ib 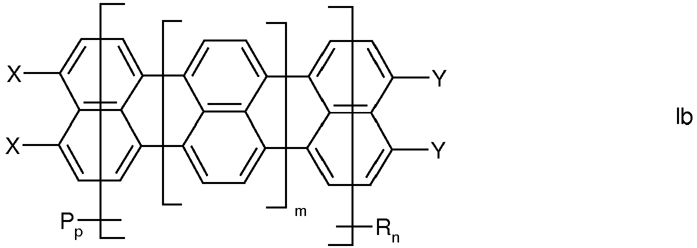 in which the variables are each defined as follows:
X are joined to one another with formation of a six-membered ring to give a radical of the formula (x2) or (x3) 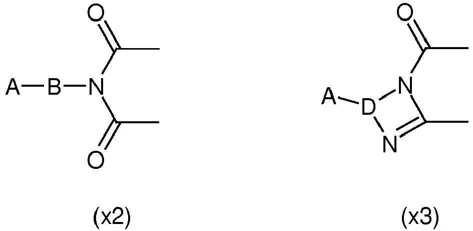
Y one of the two radicals is a radical of the formula (y1)
-L-NR¹R² (y1)
or a radical of the formula (y2)
-L-Z-R³ (y2)
or one radical is R and the other radical in each case is hydrogen;
are joined together with formation of a six-membered ring to give a radical of the formula (y3) or (y4) 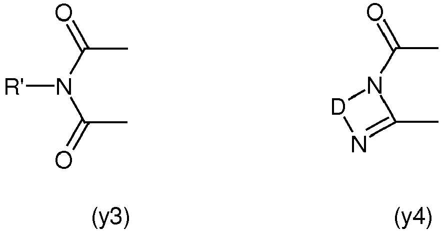 or both are hydrogen;
R are identical or different radicals: aryloxy, arylthio, hetaryloxy or hetarylthio, to each of which may be fused further saturated or unsaturated 5- to 7-membered rings whose carbon skeleton may be interrupted by one or more -0-, -S-, -NR⁴-, -N=CR⁴-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -0-, -S-, -NR⁴-,-N=CR⁴-, -C≡C-, -CR⁴=CR⁴-, -CO-, -SO- and/or -SO₂-moieties and which may be mono- or polysubstituted by: C₁-C₁₂-alkoxy, C₁-C₆-alkylthio, -C≡CR⁴, -CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂, -POR⁷R⁷, (het)aryl and/or saturated or unsaturated C₄-C₇-cycloalkyl whose carbon skeleton may be interrupted by one or more -0-, -S-, -NR⁴-, -N=CR⁴-, -CR⁴=CR⁴-, -CO-, -SO- and/or -SO₂- moieties, where the (het)aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -0-, -S-, -NR⁴-,-N=CR⁴-, -CR⁴=CR⁴-, -CO-, -SO- and/or -SO₂-moieties and to which may be fused further saturated or unsaturated 5- to 7-membered rings whose carbon skeleton may be interrupted by one or more -0-, -S-, -NR⁴-, -N=CR⁴-, -CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkylthio,-C≡CR⁴, -CR⁴=CR⁴₂, hydroxyl, mercapto, halogen, cyano, nitro, -NR⁹R¹⁰, -NR⁵COR⁶, -CONR⁵R⁶,-SO₂NR⁵R⁶, -COOR⁷, -SO₃R⁷, -PR⁷₂ and/or -POR⁷R⁷ ;
(iii) aryl or hetaryl to which may be fused further saturated or unsaturated 5- to 7-membered rings whose carbon skeleton may be interrupted by one or more -0-, -S-, -NR⁴-,-N=CR⁴-, -CR⁴=CR⁴-, -CO-, -SO- and/or -SO₂-moieties, where the entire ring system may be mono- or polysubstituted by: C₁-C₁₈-alkyl, C₁-C₁₂-alkoxy, C₁-C₆-alkyl thio, -C≡CR⁴, -CR⁴=CR⁴₂, hydroxyl, mercapto, halogen, cyano, nitro,-NR⁹R¹⁰, -NR⁵COR⁶, -CONR⁵R⁶, -SO₂NR⁵R⁶, -COOR⁷,-SO₃R⁷, -PR⁷₂, -POR⁷R⁷, (het)aryl, (het)aryloxy and/or (het)arylthio, where the (het)aryl radicals may each be mono- or polysubstituted by C₁-C₁₈-alkyl, C₁-C₁₂-alkoxy, C₁-C₆-alkylthio, hydroxyl, mercapto, halogen, cyano, nitro,-NR⁹R¹⁰, -NR⁵COR⁶, -CONR⁵R⁶, -SO₂NR⁵R⁶, -COOR⁷,-SO₃R⁷, -PR⁷₂ and/or -POR⁷R⁷;
(iv) a -U-aryl radical which may be mono- or polysubstituted by the above radicals specified as substituents for the aryl radicals (iii), where U is an -0-, -S-, -NR⁴-, -CO-, -SO- or-SO₂- moiety;
(v) C₁-C₁₂-alkoxy, C₁-C₆-alkylthio, -C≡CR⁴,-CR⁴=CR⁴₂, hydroxyl, mercapto, halogen, cyano, nitro, -NR⁹R¹⁰, -NR⁵COR⁶, -CONR⁵R⁶, -SO₂NR⁵R⁶, -COOR⁷, -SO₃R⁷, -PR⁷₂ and/or -POR⁷R⁷;
P is an amino radical -NR¹R²;
B is C₁-C₆-alkylene, phenylene or combinations of these bridging members, where the phenylene radicals may be mono- or polysubstituted by C₁-C₁₂-alkyl, nitro, cyano and/or halogen;
A is -COOM, -SO₃M or -PO₃M₂;
D is phenylene, naphthylene or pyridylene, each of which may be mono- or polysubstituted by C₁-C₁₂-alkyl, C₁-C₆-alkoxy, hydroxyl, nitro and/or halogen;
M is hydrogen, monovalent or divalent metal cation, especially alkaline earth metal or alkali metal cation, ammonium salts of cyclic amines, guanidinium salts or [NR⁵]₄⁺;
L is a chemical bond or an arylene or hetarylene radical, bonded to the rylene skeleton directly or via ethenylene or ethynylene, of the formulae
-Ar- -Ar-E-Ar-
in which the (het)arylene radicals Ar may be the same or different, may comprise heteroatoms as ring atoms and/or may have fused saturated or unsaturated 5- to 7-membered rings which may likewise comprise heteroatoms, where the entire ring system may be mono- or polysubstituted by phenyl, C₁-C₁₂-alkyl, C₁-C₁₂-alkoxy, C₁-C₁₂-alkylthio and/or-NR⁵R⁶;
E is a chemical bond or an -0-, -S-, -NR⁴-, -C=C-, -CR⁴=CR⁴- or C₁-C₆-alkylene moiety;
R¹, R² are each independently one of the alkyl radicals (i), cycloalkyl radicals (ii) or (het)aryl radicals (iii) specified as substituents for the R radicals;
joined together to form a saturated or unsaturated, 5- to 7-membered ring which comprises the nitrogen atom and whose carbon chain may be interrupted by one or more -O-,-S- and/or -NR⁴- moieties, to which may be fused one or two unsaturated or saturated 4- to 8-membered rings whose carbon chain may likewise be interrupted by these moieties and/or -N=, where the entire ring system may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the aforementioned radicals as substituents for alkyl, C₁-C₁₈-alkoxy, C₁-C₁₈-alkylthio and/or-NR⁵R⁶;
Z is -0- or -S-;
R³ is one of the alkyl radicals (i) or (het)aryl radicals (iii) specified as substituents for the R radicals;
R' is hydrogen; C₁-C₃₀-alkyl whose carbon chain may be interrupted by one or more -O-, -S-, -NR⁴-,-N=CR⁴-, -C≡C-, -CR⁴=CR⁴-, -CO-, -SO- and/or-SO₂- moieties and which may be mono- or polysubstituted by the (ii), (iii), (iv) and/or (v) radicals specified as substituents for the R radicals;
C₃-C₈-cycloalkyl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by the (i), (ii), (iii), (iv) and/or (v) radicals specified as substituents for the R radicals;
aryl or hetaryl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or
-SO₂- moieties, where the entire ring system may be mono- or polysubstituted by the (i), (ii), (iii), (iv), (v) radicals specified as substituents for the R radicals, and/or aryl-and/or hetarylazo, each of which may be substituted by C₁-C₁₀-alkyl, C₁-C₆-alkoxy and/or cyano;
R⁴ is hydrogen or C₁-C₁₈-alkyl, where the R⁴ radicals may be the same or different when they occur more than once;
R⁵, R⁶ are each independently: hydrogen;
C₁-C₁₈-alkyl whose carbon chain may be interrupted by one or more -O-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸;
aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl;
where the R⁵ radicals may be the same or different when they occur more than once;
R⁷ is C₁-C₁₈-alkyl whose carbon chain may be interrupted by one or more -0-, -S-, -CO-,-SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR⁸;
aryl or hetaryl to each of which may be fused further saturated or unsaturated 5- to 7-membered rings whose carbon skeleton may be interrupted by one or more -0-, -S-, -CO-and/or -SO₂- moieties, where the entire ring system may be mono- or polysubstituted by C₁-C₁₂-alkyl and/or the above radicals specified as substituents for alkyl;
where the R⁷ radicals may be the same or different when they occur more than once;
R⁸ is C₁-C₁₈-alkyl;
R⁹, R¹⁰ are each C₁-C₃₀-alkyl whose carbon chain may be interrupted by one or more -0-, -S-, -NR⁴-, -N=CR⁴-, -C≡C-, -CR⁴=CR⁴-, -CO-, -SO- and/or -SO₂- moieties and which may be mono- or polysubstituted by: C₁-C₁₂-alkoxy, C₁-C₆-alkylthio, -C≡CR⁴, -CR⁴=CR⁴₂, hydroxyl, -NR⁵R⁶, -NR⁵COR⁶, (het)aryl and/or saturated or unsaturated C₄-C₇-cycloalkyl whose carbon skeleton may be interrupted by one or more -O-, -S-, -NR⁴-, -N=CR⁴- and/or -CR⁴=CR⁴ moieties, where the (het)aryl and cycloalkyl radicals may each be mono- or polysubstituted by C₁-C₁₈-alkyl and/or the above radicals specified as substituents for alkyl;
aryl or hetaryl to which may be fused further saturated or unsaturated 5- to 7-membered rings whose carbon skeleton may be interrupted by one or more -0-, -S-, -NR⁴-, -N=CR⁴-,-CR⁴=CR⁴-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR⁴, -CR⁴=CR⁴₂, hydroxyl,-NR⁵R⁶, -NR⁵COR⁶, (het)aryl, (het)aryloxy and/or (het)arylthio, where the (het)aryl radicals may be mono- or polysubstituted by C₁-C₁₈-alkyl, C₁-C₁₂-alkoxy, hydroxyl, -NR⁵R⁶ and/or-NR⁵COR⁶;
joined to the nitrogen atom to form a saturated or unsaturated, 5- to 7-membered ring whose carbon chain may be interrupted by one or more -0-, -S- and/or -NR⁴- moieties, to which may be fused one or two unsaturated or saturated 4- to 8-membered rings whose carbon chain may likewise be interrupted by these moieties and/or -N=, where the entire ring system may be mono- or polysubstituted by: C₁-C₂₄-alkyl which may be substituted by C₁-C₁₈-alkoxy, C₁-C₁₈-alkylthio and/or -NR⁵R⁶, (het)aryl which may be mono- or polysubstituted by C₁-C₁₈-alkyl and/or the above radicals specified as substituents for alkyl, C₁-C₁₈-alkoxy, C₁-C₁₈-alkylthio and/or-NR⁵R⁶;
m is 0, 1 or 2;
n is 0, 2 or 4 when m = 0;
is 0, 2 or 4 when m = 1;
is 0,4 or 6 when m = 2;
p is 0, 2 or 4 when m = 0, where n + p = 2 or 4 or may also be 0 when one of the two Y radicals is a radical of the formula (y1) or (y2) and the other radical is hydrogen, where at least one of the two R¹ or R² radicals in the (y1) radical is one of the (het)aryl radicals (iii) specified as substituents for the R radicals when L is a chemical bond;
is 0, 2 or 4 when m = 1, where n + p = 0, 2 or 4; is 0, 4 or 6 when m = 2, where n + p = 0, 4 or 6.

## Revendications

1. Utilisation de dérivés de rylène de formule générale I 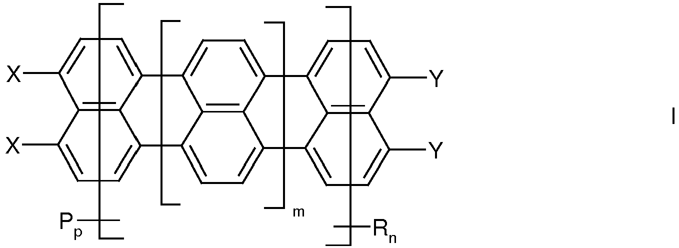 dans laquelle les variables ont la signification suivants :
X reliés l'un à l'autre avec formation d'un cycle à six éléments pour former un radical de formule (x1), (x2) ou (x3) 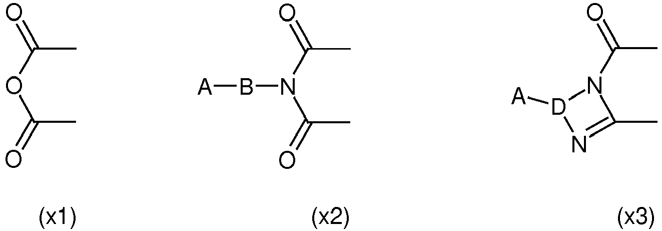 tous les deux un radical -COOM ;
Y un des deux radicaux un radical de formule (y1)
-L-NR¹R² (y1)
ou un radical de formule (y2)
-L-Z-R³ (y2)
et l'autre radical à chaque fois l'hydrogène ; reliés l'un à l'autre avec formation d'un cycle à six éléments pour former un radical de formule (y3) ou (y4) 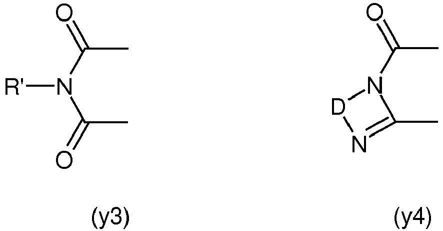 ou tous les deux l'hydrogène ;
R des radicaux identiques ou différents :
aryloxy, arylthio, hétaryloxy ou hétarylthio, à chacun desquels des cycles de 5 à 7 éléments saturés ou insaturés supplémentaires, dont le squelette carboné peut être interrompu par un ou plusieurs groupes -0-, -S-, - NR⁴-, -N=CR⁴-, -CO-, -SO- et/ou -SO₂-, peuvent être annelés, le système cyclique entier pouvant être substitué une ou plusieurs fois par les radicaux (i), (ii), (iii), (iv) et/ou (v) :
(i) alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C=CR⁴, -CR⁴=CR⁴₂, hydroxy, mercapto, halogène, cyano, nitro, -NR⁹R¹⁰, -NR⁵COR⁶, CONR⁵R⁶, -SO₂NR⁵R⁶, -COOR⁷, -SO₃R⁷, -PR⁷₂, -POR⁷R⁷, (het)aryle et/ou cycloalkyle en C₄-C₇ saturé ou insaturé, dont le squelette carboné peut être interrompu par un ou plusieurs groupes -O-, -S-, -NR⁴-, -N=CR⁴-, -CR⁴=CR⁴-,-CO-, -SO- et/ou -SO₂-, les radicaux (het) aryle et cycloalkyle pouvant chacun être substitués une ou plusieurs fois par alkyle en C₁-C₁₈ et/ou les radicaux précédents indiqués en tant que substituants pour alkyle ;
(ii) cycloalkyle en C₃-C₈, dont le squelette carboné peut être interrompu par un ou plusieurs groupes -O-, - S-, -NR⁴-, -N=CR⁴-, -CR⁴=CR⁴-, -CO-, -SO- et/ou -SO₂-, et auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-, -CO-, -SO- et/ou -SO₂-, peuvent être annelés, le système cyclique entier pouvant être substitué une ou plusieurs fois par : alkyle en C₁-C₁₈ , alcoxy en C₁-C₁₂, alkylthio en C₁-C₆, -C=CR⁴, -CR⁴=CR⁴₂, hydroxy, mercapto, halogène, cyano, nitro, -NR⁹R¹⁰,-NR⁵COR⁶, -CONR⁵R⁶, -SO₂NR⁵R⁶; -COOR⁷, -SO₃R⁷, -PR⁷₂ et/ou - POR⁷R⁷ ;
(iii) aryle ou hétaryle, auquel des cycles de 5 à 7 éléments saturés ou insaturés supplémentaires, dont le squelette carboné peut être interrompu par un ou plusieurs groupes -O-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C=CR⁴, -CR⁴=CR⁴₂, hydroxy, mercapto, halogène, cyano, nitro, -NR⁹R¹⁰, -NR⁵COR⁶, -CONR⁵R⁶, -SO₂NR⁵R⁶ ;-COOR⁷, -SO₃R⁷, -PR⁷₂, -POR⁷R⁷, (het) aryle, (het) aryloxy et/ou (het) arylthio, les radicaux (het) aryle pouvant chacun être substitués une ou plusieurs fois par alkyle en C₁-C₁₈, alcoxy en C₁-C₁₂, alkylthio en C₁-C₆, hydroxy, mercapto, halogène, cyano, nitro, -NR⁹R¹⁰, -NR⁵COR⁶, CONR⁵R⁶, -SO₂NR⁵R⁶, -COOR⁷, -SO₃R⁷, -PR⁷₂, -POR⁷R⁷ ;
(iv) un radical -U-aryle, qui peut être substitué une ou plusieurs fois par les radicaux précédents indiqués en tant que substituants pour les radicaux aryle (iii), U signifiant un groupe -0-, -S-, -NR⁴-, -CO-, -SO- ou-SO₂- ;
(v) alcoxy en C₁-C₁₂, alkylthio en C₁-C₆, -C=CR⁴,-CR⁴=CR⁴₂, hydroxy, mercapto, halogène, cyano, nitro,-NR⁹R¹⁰, -NR⁵COR⁶ , -CONR⁵R⁶, -SO₂NR⁵R⁶, -COOR⁷, -SO₃R⁷, -PR⁷₂ et/ou -POR⁷R⁷ ;
P un radical amino -NR¹R² ;
B alkylène en C₁-C₈, phénylène ou des combinaisons de ces éléments de pont, les radicaux phénylène pouvant être substitués une ou plusieurs fois par alkyle en C₁-C₁₂, nitro, cyano et/ou halogène ;
A -COOM, -SO₃M et/ou -PO₃M₂ ;
D phénylène, naphtylène ou pyridylène, qui peuvent chacun être substitués une ou plusieurs fois par alkyle en C₁-C₁₂, alcoxy en C₁-C₁₂, hydroxy, nitro et/ou halogène ;
M hydrogène, cation métallique monovalent ou
bivalent, notamment cation de métal alcalino-terreux ou
alcalin, sels d'ammonium d'amines cycliques, sels de guanidinium ou [NR⁵₄]⁺ ;
L une liaison chimique ou un radical arylène ou hétarylène relié directement ou par un éthénylène ou un éthynylène au squelette rylène, de formules
-Ar- -Ar-E-Ar-
dans lesquelles les radicaux (het) arylène Ar peuvent être identiques ou différents, peuvent contenir des hétéroatomes en tant qu'atomes de cycle et/ou peuvent comprendre des cycles de 5 à 7 éléments saturés ou insaturés annelés, qui peuvent également contenir des hétéroatomes, le système cyclique entier pouvant être substitué une ou plusieurs fois par phényle, alkyle en C₁-C₁₂, alcoxy en C₁-C₁₂, alkylthio en C₁-C₁₂ et/ou -NR⁵R⁶ ;
E une liaison chimique ou un groupe -0-, -S-, -NR⁴-, -C≡C-, -CR⁴=CR⁴- ou alkylène en C₁-C₆ ;
R¹, R² indépendamment l'un de l'autre un des radicaux alkyle (i), radicaux cycloalkyle (ii) ou radicaux (het) aryle (iii) indiqués en tant que substituants pour les radicaux R ;
reliés l'un à l'autre pour former un cycle de 5 à 7 éléments saturé ou insaturé, contenant l'atome d'azote, dont la chaîne carbonée peut être interrompue par un ou plusieurs groupes -0-, -S- et/ou -NR⁴-, auquel un ou deux cycles de 4 à 8 éléments insaturés ou saturés peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈, alkylthio en C₁-C₁₈ et/ou -NR⁵R⁶ ; (het) aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
Z -0- ou -S- ;
R³ un des radicaux alkyle (i) ou radicaux (het) aryle (iii) indiqués en tant que substituants pour les radicaux R ;
R¹ hydrogène ;
alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par les radicaux (ii), (iii), (iv) et/ou (v) indiqués en tant que substituants pour les radicaux R ;
cycloalkyle en C₃-C₈, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par les radicaux (i), (ii) , (iii), (iv) et/ou (v) indiqués en tant que substituants pour les radicaux R ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par les radicaux (i), (il), (iii), (iv), (v) indiqués en tant que substituants pour les radicaux R et/ou aryl- et/ou hétarylazo, qui peuvent chacun être substitués par alkyle en C₁-C₁₀, alcoxy en C₁-C₆ et/ou cyano ;
R⁴ hydrogène ou alkyle en C₁-C₁₈, les radicaux R⁴ pouvant être identiques ou différents lorsqu'ils sont présents plusieurs fois ;
R⁵, R⁶ indépendamment l'un de l'autre :
hydrogène ;
alkyle en C₁-C₁₈ , dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR⁸ ;
aryle ou hétaryle, auquel des cycles de 5 à 7 éléments saturés ou insaturés supplémentaires, dont le squelette carboné peut être interrompu par un ou plusieurs groupes -0-, -S-, -CO- et/ou -SO₂-, peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁵ pouvant être identiques ou différents lorsqu'ils sont présents plusieurs fois ;
R⁷ alkyle en C₁-C₁₈, dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR₈ ;
aryle ou hétaryle, auquel des cycles de 5 à 7 éléments saturés ou insaturés supplémentaires, dont le squelette carboné peut être interrompu par un ou plusieurs groupes -0-, -S-, -CO- et/ou -SO₂-, peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁷ pouvant être identiques ou différents lorsqu'ils sont présents plusieurs fois ;
R⁸ alkyle en C₁-C₁₈ ;
R⁹, R¹⁰alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C=CR⁴, -CR⁴=CR⁴2, hydroxy, -NR⁵R⁶, -NR⁵COR⁶, (het) aryle et/ou cycloalkyle en C₄-C₇ saturé ou insaturé, dont le squelette carboné peut être interrompu par un ou plusieurs groupes -0-, -S-, -NR⁴-, -N=CR⁴- et/ou -CR⁴=CR⁴-, les radicaux (het) aryle et cycloalkyle pouvant chacun être substitués une ou plusieurs fois par alkyle en C₁-C₁₈ et/ou les radicaux précédents indiqués en tant que substituants pour alkyle ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C≡CR⁴, -CR⁴=CR⁴₂, hydroxy, -NR⁵R⁶, -NR⁵COR⁶, (het) aryle, (het) aryloxy et/ou (het) arylthio, les radicaux (het) aryle pouvant chacun être substitués une ou plusieurs fois par alkyle en C₁-C₁₈, alcoxy en C₁-C₁₂, hydroxy, -NR⁵R⁶ et/ou -NR⁵COR⁶ ;
reliés avec l'atome d'azote pour former un cycle de 5 à 7 éléments saturé ou insaturé, dont la chaîne carbonée peut être interrompue par un ou plusieurs groupes -0-, -S- et/ou -NR⁴-, auquel un ou deux cycles de 4 à 8 éléments insaturés ou saturés peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈ , alkylthio en C₁-C₁₈ et/ou -NR⁵R⁶ ; (het) aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₉ et/ou -NR⁵R⁶ ;
m 0, 1 ou 2 ;
n 0, 2 ou 4 pour m = 0 ou 1 ;
0, 4 ou 6 pour m = 2 ;
p 0, 2 ou 4 pour m = 0, avec n + p = 2 ou 4 ou
également 0, lorsque les deux radicaux X sont reliés l'un à l'autre avec formation d'un cycle à six éléments pour former un radical de formule (x1) ou (x2), ou signifient tous les deux un radical -COOM ; et
un des deux radicaux Y signifie un radical de formule (y1) ou (y2) et l'autre radical signifie l'hydrogène, au moins un des deux radicaux R¹ ou R² dans le radical (y1) signifiant un des radicaux (het) aryle (iii) indiqués en tant que substituants pour les radicaux R lorsque L représente une liaison chimique ;
0, 2 ou 4 pour m = 1, avec n + p = 0, 2 ou 4 ;
0, 4 ou 6 pour m = 2, avec n + p = 0, 4 ou 6,
ou de mélanges des dérivés de rylène en tant que photosensibilisateurs dans des cellules solaires.

2. Utilisation selon la revendication 1, **caractérisée en ce que** les variables dans la formule I ont la signification suivants :
X reliés l'un à l'autre avec formation d'un cycle à six éléments pour former un radical de formule (x1) ou tous les deux un radical -COOM ;
Y un des deux radicaux un radical de formule (y1) ou (y2), et l'autre radical l'hydrogène, ou reliés l'un à l'autre avec formation d'un cycle à six éléments pour former un radical de formule (y3) ;
R phénoxy ou thiophénoxy, qui peuvent chacun être substitués une ou plusieurs fois par des radicaux (i), (ii), (iii), (iv) et/ou (v) identiques ou différents :
(i) alkyle en C₁-C₃₀, dont la chaîne carbonée peut être interrompue par un ou plusieurs groupes -0-, -S-, -NR⁴-, -C=C-, -CR⁴=CR⁴- et/ou -CO- et/ou -SO₂-, et qui peut être substitué une ou plusieurs fois par : alcoxy en C₁-C₁₂, hydroxy, halogène, cyano et/ou aryle, qui peut être substitué une ou plusieurs fois par alkyle en C₁-C₁₈ ou alcoxy en C₁-C₈ ;
(ii) cycloalkyle en C₃-C₈, dont le squelette carboné peut être interrompu par un ou plusieurs groupes -O-,-S-, -NR⁴-, -CR⁴=CR⁴- et/ou -CO-, et qui peut être substitué une ou plusieurs fois par alkyle en C₁-C₁₈, alcoxy en C₁-C₁₂ et/ou alkylthio en C₁-C₆ ;
(iii) aryle ou hétaryle, à chacun desquels des cycles de 5 à 7 éléments saturés ou insaturés supplémentaires, dont le squelette carboné peut être interrompu par un ou plusieurs groupes -O-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, -C=CR⁴₂, - CR⁴=CR⁴₂, hydroxy, halogène, cyano, -NR⁹R¹⁰, -NR⁵COR⁶, CONR⁵R⁶, -SO₂NR⁵R⁶, -COOR⁷, -SO₃R⁷, (het) aryle, (het) aryloxy et/ou (het) arylthio, les radicaux (het) aryle pouvant chacun être substitués une ou plusieurs fois par alkyle en C₁-C₁₈, alcoxy en C₁-C₁₈ et/ou cyano ;
(iv) un radical -U-aryle, qui peut être substitué une ou plusieurs fois par les radicaux précédents indiqués en tant que substituants pour les radicaux aryle (iii), U signifiant un groupe -0-, -S-, -NR⁴-, -CO-, -SO- ou-SO₂- ;
(v) alcoxy en C₁-C₁₂, alkylthio en C₁-C₆, -C=CR⁴,-CR⁴=CR⁴₂, hydroxy, mercapto, halogène, cyano, nitro,-NR⁹R¹⁰, -NR⁵COR⁶, -CONR⁵R⁶, -SO₂NR⁵R⁶, -COOR⁷ et/ou -SO₃R⁷ ;
M hydrogène, cation métallique monovalent ou bivalent, notamment cation de métal alcalino-terreux ou alcalin, sels d'ammonium d'amines cycliques, sels de guanidinium ou [NR⁵₄]⁺ ;
L une liaison chimique ou phénylène ;
R¹, R² radicaux phényle identiques ou différents, qui peuvent chacun être substitués une ou plusieurs fois par alkyle en C₁-C₁₆, alcoxy en C₁-C₁₂, alkylthio en C₁-C₆, NR⁵R⁶ et/ou phénoxy et/ou phénylthio, qui peuvent chacun être substitués une ou plusieurs fois par alkyle en C₁-C₁₈, alcoxy en C₁-C₁₂, alkylthio en C₁-C₆ et/ou -NR⁵R⁶ ;
reliés avec l'atome d'azote pour former un système cyclique pipéridyle, pyrrolidinyle, dibenzopyrryle, dibenzo-1,4-oxiranyle, dibenzo-1,4-thiazinyle, dibenzo-1,4-pyrazyle ou dibenzopipéridyle, qui peuvent chacun être substitués une ou plusieurs fois par : alkyle en C₁-C₂₄, qui peut être substitué par alcoxy en C₁-C₁₈, alkylthio en C₁-C₁₈ et/ou -NR⁵R⁶ ; (het) aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
Z -0- ou -S- ;
R³ alkyle en C₁-C₃₀, dont la chaîne carbonée peut être interrompue par un ou plusieurs groupes -0-, -S- et/ou-NR⁴-, et qui peut être substitué une ou plusieurs fois par : alcoxy en C₁-C₁₂, hydroxy et/ou aryle, qui peut être substitué une ou plusieurs fois par alkyle en C₁-C₁₈ ou alcoxy en C₁-C₆ ;
phényle, qui peut être substitué une ou plusieurs fois par : alkyle en C₁-C₁₈, alcoxy en C₁-C₁₂, alkylthio en C₁-C₆, -NR⁵R⁶ et/ou phénoxy et/ou phénylthio, qui peuvent chacun être substitués une ou plusieurs fois par alkyle en C₁-C₁₈ , alcoxy en C₁-C₁₂, alkylthio en C₁-C₆ et/ou -NR⁵R⁶ ;
R¹ alkyle en C₆-C₃₀, dont la chaîne carbonée peut être interrompue par un ou plusieurs groupes -0-, -S- et/ou-NR⁴-, et qui peut être substitué une ou plusieurs fois par : alcoxy en C₁-C₆, alkylthio en C₁-C₆, -NR⁹R¹⁰ et/ou aryle, qui peut être substitué une ou plusieurs fois par alkyle en C₁-C₁₈ ou alcoxy en C₁-C₆ ;
phényle, naphtyle, pyridyle ou pyrimidyle, qui peuvent chacun être substitués une ou plusieurs fois par : alkyle en C₁-C₁₈, alcoxy en C₁-C₆, halogène, cyano, nitro, -NR⁹R¹⁰, -CONR⁵R⁶, -SO₂R⁵R⁶ et/ou phénoxy, phénylthio, phénylazo et/ou naphtylazo, qui peuvent chacun être substitués par alkyle en C₁-C₁₀ alcoxy en C₁-C₆ et/ou cyano ;
R⁴ hydrogène ou alkyle en C₁-C₆;
R⁵, R⁶ indépendamment l'un de l'autre :
hydrogène ;
alkyle en C₁-C₁₈ qui peut être substitué une ou plusieurs fois par alcoxy en C₁-C₆, hydroxy, halogène et/ou cyano ;
aryle ou hétaryle, qui peuvent chacun être substitués une ou plusieurs fois par alkyle en C₁-C₆ et/ou les radicaux précédents indiqués en tant que substituants pour alkyle,
les radicaux R⁵ pouvant être identiques ou différents lorsqu'ils sont présents plusieurs fois ;
R⁷ alkyle en C₁-C₁₈ qui peut être substitué une ou plusieurs fois par alcoxy en C₁-C₆, hydroxy, halogène et/ou cyano ;
aryle ou hétaryle, qui peuvent chacun être substitués une ou plusieurs fois par alkyle en C₁-C₆ et/ou les radicaux précédents indiqués en tant que substituants pour alkyle,
R⁹, R¹⁰alkyle en C₁-C₃₀, dont la chaîne carbonée peut être interrompue par un ou plusieurs groupes -0-, -S-, -NR⁴-, -N=CR⁴-, -C=C- et/ou -CR⁴=CR⁴-, et qui peut être substitué une ou plusieurs fois par : alcoxy en C₁-C₁₂ alkylthio en C₁-C₆, -C=CR⁴, -CR⁴=CR⁴₂, hydroxy, -NR⁵R⁶, -NR⁵COR⁶ et/ou (het) aryle, qui peut être substitué une ou plusieurs fois par alkyle en C₁-C₁₈ et/ou les radicaux précédents indiqués en tant que substituants pour alkyle ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈ alcoxy en C₁-C₁₂, alkylthio en C₁-C₆, -C≡CR⁴, -CR⁴=CR⁴₂, hydroxy, -NR⁵R⁶, -NR⁵COR⁶, (het) aryle, (het) aryloxy et/ou (het) arylthio, les radicaux (het) aryle pouvant chacun être substitués une ou plusieurs fois par alkyle en C₁-C₁₈, alcoxy en C₁-C₁₂, hydroxy, -NR⁵R⁶ et/ou -NR⁵COR⁶ ;
reliés avec l'atome d'azote pour former un système cyclique pipéridyle, pyrrolidinyle, dibenzopyrryle, dibenzo-1,4-oxiranyle, dibenzo-1,4-thiazinyle, dibenzo-1,4-pyrazyle ou dibenzopipéridyle, qui peuvent chacun être substitués une ou plusieurs fois par alkyle en C₁-C₂₄, qui peut être substitué par alcoxy en C₁-C₁₈, alkylthio en C₁-C₁₈ et/ou -NR⁵R⁶ ;
m 0, 1 ou 2 ;
n 4 pour m = 0 ou 1 ;
4 ou 6 pour m = 2 ;
p 0.

3. Cellules solaires à pigment photosensibilisé, qui contiennent au moins un dérivé de rylène de formule I selon la revendication 1 ou 2 en tant que photosensibilisateurs.

4. Dérivés de rylène de formule générale la 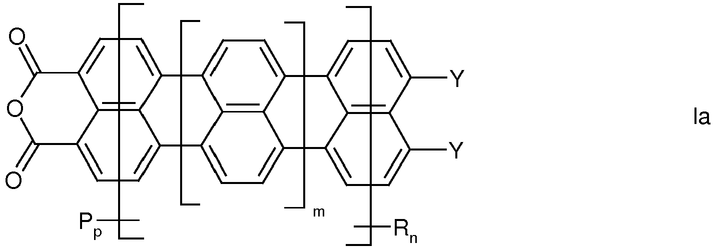 dans laquelle le radical anhydride d'acide dicarboxylique peut également représenter deux radicaux carboxyle -COOM, et les variables ont la signification suivants :
Y un des deux radicaux un radical de formule (y1)
-L-NR¹R² (y1)
ou un radical de formule (y2)
-L-Z-R³ (y2)
et l'autre radical à chaque fois l'hydrogène ;
R des radicaux identiques ou différents :
aryloxy, arylthio, hétaryloxy ou hétarylthio, à chacun desquels des cycles de 5 à 7 éléments saturés ou insaturés supplémentaires, dont le squelette carboné peut être interrompu par un ou plusieurs groupes -0-, -S-,-NR⁴-, -N=CR⁴-, -CO-, -SO- et/ou -SO₂-, peuvent être annelés, le système cyclique entier pouvant être substitué une ou plusieurs fois par les radicaux (i), (ii), (iii), (iv) et/ou (v) :
(i) alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C=CR⁴, -CR⁴=CR⁴₂, hydroxy, mercapto, halogène, cyano, nitro, -NR⁹R¹⁰, -NR⁵COR⁶, CONR⁵R⁶, -SO₂NR⁵R⁶, -COOR⁷, -SO₃R⁷, -PR⁷₂, -POR⁷R⁷, (het) aryle et/ou cycloalkyle en C₄-C₇ saturé ou insaturé, dont le squelette carboné peut être interrompu par un ou plusieurs groupes -0-, -S-, -NR⁴-, -N=CR⁴-, -CR⁴=CR⁴-,-CO-, -SO- et/ou -SO₂-, les radicaux (het) aryle et cycloalkyle pouvant chacun être substitués une ou plusieurs fois par alkyle en C₁-C₁₈ et/ou les radicaux précédents indiqués en tant que substituants pour alkyle ;
(ii) cycloalkyle en C₃-C₈, dont le squelette carboné peut être interrompu par un ou plusieurs groupes -O-,-S-, -NR⁴-, -N=CR⁴-, -CR⁴=CR⁴-, -CO-, -SO- et/ou -SO₂-, et auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-, -CO-, -SO- et/ou -SO₂-, peuvent être annelés, le système cyclique entier pouvant être substitué une ou plusieurs fois par : alkyle en C₁-C₁₈ , alcoxy en C₁-C₁₂, alkylthio en C₁-C₆, -C≡CR⁴, -CR⁴=CR⁴₂, hydroxy, mercapto, halogène, cyano, nitro, -NR⁹R¹⁰, NR⁵COR⁶, -CONR⁵R⁶, -SO₂NR⁵R⁶, -COOR⁷, -SO₃R⁷, -PR⁷₂ et/ou - POR⁷R⁷ ;
(iii) aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C=CR⁴, -CR⁴=CR⁴₂, hydroxy, mercapto, halogène, cyano, nitro, -NR⁷R⁸, -NR⁵COR⁶, -CONR⁵R⁶, -SO₂NR⁵R⁶, -COOR⁷, -SO₃R⁷, -PR⁷₂, -POR⁷R⁷, (het) aryle, (het) aryloxy et/ou (het) arylthio, les radicaux (het) aryle pouvant chacun être substitués une ou plusieurs fois par alkyle en C₁-C₁₈, alcoxy en C₁-C₁₂, alkylthio en C₁-C₆, hydroxy, mercapto, halogène, cyano, nitro, -NR⁷R⁸, -NR⁵COR⁶, CONR⁵R⁶, -SO₂NR⁵R⁶, -COOR⁷, -SO₃R⁷, -PR⁷₂ et/ou -POR⁷R⁷ ;
(iv) un radical -U-aryle, qui peut être substitué une ou plusieurs fois par les radicaux précédents indiqués en tant que substituants pour les radicaux aryle (iii), U signifiant un groupe -0-, -S-, -NR³-, -CO-, -SO- ou-SO₂- ;
(v) alcoxy en C₁-C₁₂, alkylthio en C₁-C₆, -C=CR⁴, - CR⁴=CR⁴₂, hydroxy, mercapto, halogène, cyano, nitro,-NR⁷R⁸, -NR⁵COR⁶, -CONR⁵R⁶, -SO₂NR⁵R⁶, -COOR⁷, -SO₃R⁷, -PR⁷₂ et/ou -POR⁷R⁷ ;
P un radical amino -NR¹R² ;
M hydrogène, cation métallique monovalent ou bivalent, notamment cation de métal alcalino-terreux ou alcalin, sels d'ammonium d'amines cycliques, sels de guanidinium ou [NR⁵₄]⁺ ;
L une liaison chimique ou un radical arylène ou hétarylène relié directement ou par un éthénylène ou un éthynylène au squelette rylène, de formules
-Ar- -Ar-E-Ar-
dans lesquelles les radicaux (het)arylène Ar peuvent être identiques ou différents, peuvent contenir des hétéroatomes en tant qu'atomes de cycle et/ou peuvent comprendre des cycles de 5 à 7 éléments saturés ou insaturés annelés, qui peuvent également contenir des hétéroatomes, le système cyclique entier pouvant être substitué une ou plusieurs fois par phényle, alkyle en C₁-C₁₂, alcoxy en C₁-C₁₂, alkylthio en C₁-C₁₂ et/ou -NR⁵R⁶ ;
E une liaison chimique ou un groupe -O-, -S-, -NR⁴-, -C=C-, -CR⁴=CR⁴- ou alkylène en C₁-C₆ ;
R¹, R² indépendamment l'un de l'autre un des radicaux alkyle (i), radicaux cycloalkyle (ii) ou radicaux (het) aryle (iii) indiqués en tant que substituants pour les radicaux R ;
reliés l'un à l'autre pour former un cycle de 5 à 7 éléments saturé ou insaturé, contenant l'atome d'azote, dont la chaîne carbonée peut être interrompue par un ou plusieurs groupes -0-, -S- et/ou -NR⁴-, auquel un ou deux cycles de 4 à 8 éléments insaturés ou saturés peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈, alkylthio en C₁-C₁₈ et/ou -NR⁵R⁶ ; (het) aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
Z -0- ou -S- ;
R³ un des radicaux alkyle (i) ou radicaux (het) aryle (iii) indiqués en tant que substituants pour les radicaux R ;
R⁴ hydrogène ou alkyle en C₁-C₁₈, les radicaux R⁴ pouvant être identiques ou différents lorsqu'ils sont présents plusieurs fois ;
R⁵, R⁶ indépendamment l'un de l'autre :
hydrogène ;
alkyle en C₁-C₁₈, dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR⁸ ;
aryle ou hétaryle, à chacun desquels des cycles de 5 à 7 éléments saturés ou insaturés supplémentaires, dont le squelette carboné peut être interrompu par un ou plusieurs groupes -0-, -S-, -CO-, et/ou -SO₂- , peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁵ pouvant être identiques ou différents lorsqu'ils sont présents plusieurs fois ; R⁷ alkyle en C₁-C₁₈, dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR⁸ ;
aryle ou hétaryle, à chacun desquels des cycles de 5 à 7 éléments saturés ou insaturés supplémentaires, dont le squelette carboné peut être interrompu par un ou plusieurs groupes -0-, -S-, -CO- et/ou -SO₂-, peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁷ pouvant être identiques ou différents lorsqu'ils sont présents plusieurs fois ;
R⁸ alkyle en C₁-C₁₈ ;
R⁹, R¹⁰alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C=CR⁴, -CR⁴=CR⁴₂, hydroxy, -NR⁵R⁶, -NR⁵COR⁶, (het) aryle et/ou cycloalkyle en C₄-C₇ saturé ou insaturé, dont le squelette carboné peut être interrompu par un ou plusieurs groupes -0-, -S-, -NR⁴-, -N=CR⁴- et/ou -CR⁴=CR⁴-, les radicaux (het) aryle et cycloalkyle pouvant chacun être substitués une ou plusieurs fois par alkyle en C₁-C₁₈ et/ou les radicaux précédents indiqués en tant que substituants pour alkyle ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C≡CR⁴, -CR⁴=CR⁴₂, hydroxy, -NR⁵R⁶, -NR⁵COR⁶, (het)aryle, (het)aryloxy et/ou (het)arylthio, les radicaux (het)aryle pouvant chacun être substitués une ou plusieurs fois par alkyle en C₁-C₁₈, alcoxy en C₁-C₁₂, hydroxy, -NR⁵R⁶ et/ou -NR⁵COR⁶ ;
reliés avec l'atome d'azote pour former un cycle de 5 à 7 éléments saturé ou insaturé, dont la chaîne carbonée peut être interrompue par un ou plusieurs groupes -0-, -S- et/ou -NR⁴-, auquel un ou deux cycles de 4 à 8 éléments insaturé ou saturé peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈, alkylthio en C₁-C₁₈ et/ou -NR⁵R⁶ ; (het)aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
m 0, 1 ou 2 ;
n 0, 2 ou 4 pour m = 0 ;
0, 2 ou 4 pour m = 1 ;
0, 4 ou 6 pour m = 2 ;
p 0, 2 ou 4 pour m = 0, avec n + p = 0, 2 ou 4 lorsque n + p = 0, et un des deux radicaux Y signifie un radical de formule (y1) et l'autre radical signifie l'hydrogène, au moins un des deux radicaux R¹ ou R² signifiant un des radicaux (het)aryle (iii) indiqués en tant que substituants pour les radicaux R lorsque L représente une liaison chimique ;
0, 2 ou 4 pour m = 1, avec n + p = 0, 2 ou 4 ;
0, 4 ou 6 pour m = 2, avec n + p = 0, 4 ou 6.

5. Dérivés de rylène de formule générale Ib 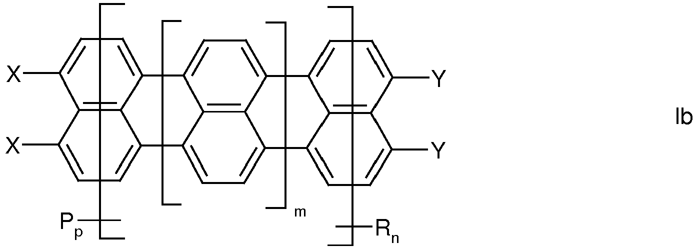 dans laquelle les variables ont la signification suivante :
X reliés l'un à l'autre avec formation d'un cycle à six éléments pour former un radical de formule (x2) ou (x3) 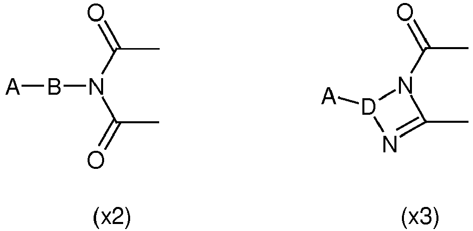
Y un des deux radicaux un radical de formule (y1)
-L-NR¹R² (y1)
ou un radical de formule (y2)
-L-Z-R³ (y2)
ou un radical R et l'autre radical à chaque fois l'hydrogène ;
reliés l'un à l'autre avec formation d'un cycle à six éléments pour former un radical de formule (y3) ou (y4) 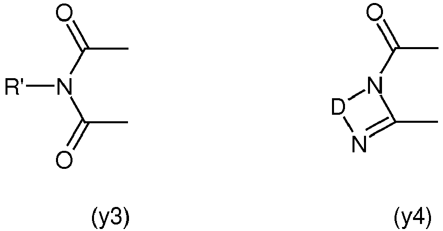 ou tous les deux l'hydrogène ;
R des radicaux identiques ou différents :
aryloxy, arylthio, hétaryloxy ou hétarylthio, à chacun desquels des cycles de 5 à 7 éléments saturés ou insaturés supplémentaires, dont le squelette carboné peut être interrompu par un ou plusieurs groupes -0-, -S-, - NR⁴-, -N=CR⁴-, -CO-, -SO- et/ou -SO₂-, peuvent être annelés, le système cyclique entier pouvant être substitué une ou plusieurs fois par les radicaux (i), (ii), (iii), (iv) et/ou (v) :
(i) alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C≡CR⁴, -CR⁴=CR⁴₂, hydroxy, mercapto, halogène, cyano, nitro, -NR⁹R¹⁰, -NR⁵COR⁶, - CONR⁵R⁶, -SO₂NR⁵R⁶, -COOR⁷, -SO₃R⁷, -PR⁷₂, -POR⁷R⁷, (het)aryle et/ou cycloalkyle en C₄-C₇ saturé ou insaturé, dont le squelette carboné peut être interrompu par un ou plusieurs groupes -0-, -S-, -NR⁴-, -N=CR⁴-, -CR⁴=CR⁴-,-CO-, -SO- et/ou -SO₂-, les radicaux (het)aryle et cycloalkyle pouvant chacun être substitués une ou plusieurs fois par alkyle en C₁-C₁₈ et/ou les radicaux précédents indiqués en tant que substituants pour alkyle ;
(ii) cycloalkyle en C₃-C₈, dont le squelette carboné peut être interrompu par un ou plusieurs groupes -O-,-S-, -NR⁴-, -N=CR⁴-, -CR⁴=CR⁴-, -CO-, -SO- et/ou -SO₂-, et auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-, -CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C≡CR⁴, -CR⁴=CR⁴₂, hydroxy, mercapto, halogène, cyano, nitro, -NR⁹R¹⁰,-NR⁵COR⁶, -CONR⁵R⁶, -SO₂NR⁵R⁶, -COOR⁷, -SO₃R⁷, -PR⁷₂ et/ou - POR⁷R⁷ ;
(iii) aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴⁻, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C≡CR⁴, -CR⁴=CR⁴₂, hydroxy, mercapto, halogène, cyano, nitro, -NR⁹R¹⁰, -NR⁵COR⁶, -CONR⁵R⁶, -SO₂NR⁵R⁶,-COOR⁷, -SO₃R⁷, -PR⁷₂, -POR⁷R⁷, (het)aryle, (het)aryloxy et/ou (het)arylthio, les radicaux (het)aryle pouvant chacun être substitués une ou plusieurs fois par alkyle en C₁-C₁₈, alcoxy en C₁-C₁₂, alkylthio en C₁-C₆, hydroxy, mercapto, halogène, cyano, nitro, -NR⁹R¹⁰, -NR⁵COR⁶,-CONR⁵R⁶, -SO₂NR⁵R⁶, -COOR⁷, -SO₃R⁷, -PR⁷₂ et/ou -POR⁷R⁷ ;
(iv) un radical -U-aryle, qui peut être substitué une ou plusieurs fois par les radicaux précédents indiqués en tant que substituants pour les radicaux aryle (iii), U signifiant un groupe -0-, -S-, -NR⁴-, -CO-, -SO- ou - SO₂- ;
(v) alcoxy en C₁-C₁₂, alkylthio en C₁-C₆, -C≡CR⁴, - CR⁴=CR⁴₂, hydroxy, mercapto, halogène, cyano, nitro,-NR⁹R¹⁰, -NR⁵COR⁶, -CONR⁵R⁶, -SO₂NR⁵R⁶, -COOR⁷, -SO₃R⁷, -PR⁷₂ et/ou -POR⁷R⁷ ;
P un radical amino -NR¹R² ;
B alkylène en C₁-C₈, phénylène ou des combinaisons de ces éléments de pont, les radicaux phénylène pouvant être substitués une ou plusieurs fois par alkyle en C₁-C₁₂, nitro, cyano et/ou halogène ;
A -COOM, -SO₃M et/ou -PO₃M₂ ;
D phénylène, naphtylène ou pyridylène, qui peuvent chacun être substitués une ou plusieurs fois par alkyle en C₁-C₁₂, alcoxy en C₁-C₆, hydroxy, nitro et/ou halogène ;
M hydrogène, cation métallique monovalent ou bivalent, notamment cation de métal alcalino-terreux ou alcalin, sels d'ammonium d'amines cycliques, sels de guanidinium ou [NR⁵₄]⁺ ;
L une liaison chimique ou un radical arylène ou hétarylène relié directement ou par un éthénylène ou un éthynylène au squelette rylène, de formules
-Ar- -Ar-E-Ar-
dans lesquelles les radicaux (het)arylène Ar peuvent être identiques ou différents, peuvent contenir des hétéroatomes en tant qu'atomes de cycle et/ou peuvent comprendre des cycles de 5 à 7 éléments saturés ou insaturés annelés, qui peuvent également contenir des hétéroatomes, le système cyclique entier pouvant être substitué une ou plusieurs fois par phényle, alkyle en C₁-C₁₂, alcoxy en C₁-C₁₂, alkylthio en C₁-C₁₂ et/ou -NR⁵R⁶ ;
E une liaison chimique ou un groupe -0-, -S-, -NR⁴-, -C≡C-, -CR⁴=CR⁴- ou alkylène en C₁-C₆ ;
R¹, R² indépendamment l'un de l'autre un des radicaux alkyle (i), radicaux cycloalkyle (ii) ou radicaux (het)aryle (iii) indiqués en tant que substituants pour les radicaux R ;
reliés l'un à l'autre pour former un cycle de 5 à 7 éléments saturé ou insaturé, contenant l'atome d'azote, dont la chaîne carbonée peut être interrompue par un ou plusieurs groupes -0-, -S- et/ou -NR⁴-, auquel un ou deux cycles de 4 à 8 éléments insaturés ou saturés peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈, alkylthio en C₁-C₁₈ et/ou -NR⁵R⁶ ; (het)aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
Z -0- ou -S- ;
R³ un des radicaux alkyle (i) ou (het)aryle (iii) indiqués en tant que substituants pour les radicaux R ;
R¹ hydrogène ;
alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par les radicaux (ii), (iii), (iv) et/ou (v) indiqués en tant que substituants pour les radicaux R ;
cycloalkyle en C₃-C₈, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par les radicaux (i), (ii), (iii), (iv) et/ou (v) indiqués en tant que substituants pour les radicaux R ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par les radicaux (i), (ii), (iii), (iv), (v) indiqués en tant que substituants pour les radicaux R et/ou aryl- et/ou hétarylazo, qui peuvent chacun être substitués par alkyle en C₁-C₁₀, alcoxy en C₁-C₆ et/ou cyano ;
R⁴ hydrogène ou alkyle en C₁-C₁₈, les radicaux R⁴ pouvant être identiques ou différents lorsqu'ils sont présents plusieurs fois ;
R⁵, R⁶ indépendamment l'un de l'autre :
hydrogène ;
alkyle en C₁-C₁₈, dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR⁸ ;
aryle ou hétaryle, auquel des cycles de 5 à 7 éléments saturés ou insaturés supplémentaires, dont le squelette carboné peut être interrompu par un ou plusieurs groupes -0-, -S-, -CO-, et/ou -SO₂-, peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁵ pouvant être identiques ou différents lorsqu'ils sont présents plusieurs fois ; R⁷ alkyle en C₁-C₁₈, dont la chaîne carbonée peut être interrompue par un ou plusieurs groupes -0-, -S-, -CO-, -SO- et/ou -SO₂-, et qui peut être substitué une ou plusieurs fois par alcoxy en C₁-C₁₂, alkylthio en C₁-C₆, hydroxy, mercapto, halogène, cyano, nitro et/ou -COOR⁸ ;
aryle ou hétaryle, à chacun desquels des cycles de 5 à 7 éléments saturés ou insaturés supplémentaires, dont le squelette carboné peut être interrompu par un ou plusieurs groupes -0-, -S-, -CO- et/ou -SO₂-, peuvent être annelés, le système cyclique entier pouvant être substitué une ou plusieurs fois par alkyle en C₁-C₁₂ et/ou les radicaux précédents indiqués en tant que substituants pour alkyle,
les radicaux R⁷ pouvant être identiques ou différents lorsqu'ils sont présents plusieurs fois ;
R⁸ alkyle en C₁-C₁₈ ;
R⁹, R¹⁰ alkyle en C₁-C₃₀, dont la chaîne carbonée peut être interrompue par un ou plusieurs groupes -0-, -S-, -NR⁴-, -N=CR⁴-, -C≡C-, -CR⁴=CR⁴-, -CO-, -SO- et/ou -SO₂-, et qui peut être substitué une ou plusieurs fois par : alcoxy en C₁-C₁₂, alkylthio en C₁-C₆, -C≡CR⁴, -CR⁴=CR⁴₂, hydroxy, -NR⁵R⁶, -NR⁵COR⁶, (het)aryle et/ou cycloalkyle en C₄-C₇ saturé ou insaturé, dont le squelette carboné peut être interrompu par un ou plusieurs groupes -0-, -S-, -NR⁴-, -N=CR⁴- et/ou -CR⁴=CR⁴-, les radicaux (het)aryle et cycloalkyle pouvant chacun être substitués une ou plusieurs fois par alkyle en C₁-C₁₈ et/ou les radicaux précédents indiqués en tant que substituants pour alkyle ;
aryle ou hétaryle, auquel des cycles de 5 à 7 éléments saturés ou insaturés supplémentaires, dont le squelette carboné peut être interrompu par un ou plusieurs groupes -0-, -S-, -NR⁴-, -N=CR⁴-, -CR⁴=CR⁴-,-CO-, -SO- et/ou -SO₂-, peuvent être annelés, le système cyclique entier pouvant être substitué une ou plusieurs fois par : alkyle en C₁-C₁₈, alcoxy en C₁-C₁₂, alkylthio en C₁-C₆, -C≡CR⁴, -CR⁴=CR⁴₂, hydroxy, -NR⁵R⁶, -NR⁵COR⁶, (het)aryle, (het)aryloxy et/ou (het)arylthio, les radicaux (het)aryle pouvant chacun être substitués une ou plusieurs fois par alkyle en C₁-C₁₈, alcoxy en C₁-C₁₂, hydroxy, -NR⁵R⁶ et/ou -NR⁵COR⁶ ;
reliés avec l'atome d'azote pour former un cycle de 5 à 7 éléments saturé ou insaturé, dont la chaîne carbonée peut être interrompue par un ou plusieurs groupes -0-, -S- et/ou -NR⁴-, auquel un ou deux cycles de 4 à 8 éléments insaturés ou saturés peuvent être annelés, dont la chaîne carbonée peut également être interrompue par ces groupes et/ou -N=, le système cyclique entier pouvant être substitué une ou plusieurs fois par : alkyle en C₁-C₂₄, qui peut être substitué par alcoxy en C₁-C₁₈, alkylthio en C₁-C₁₈ et/ou -NR⁵R⁶ ; (het)aryle, qui peut être substitué une ou plusieurs fois par alkyle en C₁-C₁₈ et/ou les radicaux précédents indiqués en tant que substituants pour alkyle, alcoxy en C₁-C₁₈, alkylthio en C₁-C₁₈ et/ou -NR⁵R⁶ ;
m 0, 1 ou 2 ;
n 0, 2 ou 4 pour m = 0 ;
0, 2 ou 4 pour m = 1 ;
0, 4 ou 6 pour m = 2 ;
p 0, 2 ou 4 pour m = 0, avec n + p = 2 ou 4 ou également 0, lorsqu'un des deux radicaux Y signifie un radical de formule (y1) ou (y2) et l'autre radical signifie l'hydrogène, au moins un des deux radicaux R¹ ou R² dans le radical (y1) signifiant un des radicaux (het)aryle (iii) indiqués en tant que substituants pour les radicaux R lorsque L représente une liaison chimique ;
0, 2 ou 4 pour m = 1, avec n + p = 0, 2 ou 4 ;
0, 4 ou 6 pour m = 2, avec n + p = 0, 4 ou 6.
